# EUROPEAN PATENT SPECIFICATION

(11) **EP 4 511 119 B1**
(45) Date of publication and mention of the grant of the patent: **15.07.2026**
(21) Application number: 23723033.9
(22) Date of filing: 19.04.2023
(51) Int. Cl.: A61P 17/00, A61P 29/00, A61P 31/12, A61P 35/00, A61P 37/00, C07D 413/14, C07D 471/04, C07D 491/048, C07D 519/00, A61K 31/4709, A61K 31/5377

(54) **COMPOUNDS ACTIVE TOWARDS BROMODOMAINS**
VERBINDUNGEN MIT BROMDOMÄNEN-AKTIVITÄT
COMPOSÉS ACTIFS VIS-À-VIS DE BROMODOMAINES

(30) Priority: 19.04.2022 SE 2250473
(43) Date of publication of application: 26.02.2025
(73) Proprietor: Nuevolution A/S, 2100 Copenhagen (DK)
(72) Inventor: SMITH, Garrick, Paul, 2100 Copenhagen (DK); RAST, Slavko, 2100 Copenhagen (DK); STASI, Luigi, Piero, 2100 Copenhagen (DK); PEIRÓ CADAHÍA, Jorge, 2100 Copenhagen (DK); POLJAK, Visnja, 2100 Copenhagen (DK)
(74) Representative: Aamand, Jesper L.
(86) International application number: PCT/US2023/019121
(87) International publication number: WO 2023/205251

(56) References cited:
- EP-A1- 3 563 851
- WO-A1-2016/016316
- GHAZY EHAB ET AL: "Design, synthesis, and biological evaluation of dual targeting inhibitors of histone deacetylase 6/8 and bromodomain BRPF1", EUROPEAN JOURNAL OF MEDICINAL CHEMISTRY, ELSEVIER, AMSTERDAM, NL, vol. 200, 18 May 2020 (2020-05-18), XP086205557, ISSN: 0223-5234, [retrieved on 20200518], DOI: 10.1016/J.EJMECH.2020.112338
- BAI PING ET AL: "Discovery of a Positron Emission Tomography Radiotracer Selectively Targeting the BD1 Bromodomains of BET Proteins", ACS MEDICINAL CHEMISTRY LETTERS, vol. 12, no. 2, 11 February 2021 (2021-02-11), US, pages 282 - 287, XP093049860, ISSN: 1948-5875, DOI: 10.1021/acsmedchemlett.0c00650

## Description

### Field

The present application relates to compounds active towards bromodomains, pharmaceutical compositions comprising the compounds, and methods of treating diseases or disorders using the compounds.

### Background

Bromodomains are protein domains of biological and pharmaceutical interest, for example as components of transcription factor complexes and determinants of epigenetic memory. The human genome codes for 61 bromodomains that are present in 46 human proteins, and which may be categorized into 8 distinct bromodomain families based on primary sequence conservation (Nat Rev Drug Discov. 2014 May; 13(5):337-56). One such family, the BET family, or bromodomain and extraterminal domain family, includes BRD2, BRD3, BRD4 and BRDT all of which are found in humans. Bromodomains are capable of recognizing acetylated lysine residues in histones and other proteins. The BET family has a common domain architecture featuring two amino-terminal bromodomains that exhibit high levels of sequence conservation, and a more divergent carboxy-terminal recruitment domain (Filippakopoulos, P. et al., Nature 2010,468, 1067-1073). The two bromodomains of BET proteins are typically called BD1 and BD2, where BD1 refers to the most N-terminal of the two bromodomains. BRD2 and BRD3 are reported to associate with histones along actively transcribed genes and may be involved in facilitating transcriptional elongation (Leroy et al, Mol. Cell. 2008, 30, 51-60). It has also been reported that BRD4 or BRD3 may fuse with NUT (nuclear protein in testis) forming novel fusion oncogenes in a highly malignant form of epithelial neoplasia called NUT-midline carcinoma. It has been suggested that BRD-NUT fusion proteins contribute to carcinogenesis (Oncogene 2008, 27, 2237-2242). BRDT is uniquely expressed in the testes and ovary.

All BET family members have been reported to have some involvement in aspects of the cell cycle. In addition, some viruses make use of these proteins to tether their genomes to the host cell chromatin, as part of the process of viral replication (You et al. Cell 2004 117, 349-60). BRD4 appears to be involved in the recruitment of the pTEF-P complex to inducible genes resulting in phosphorylation of RNA polymerase and increased transcriptional output (Hargreaves et al, Cell 2009 138, 129-145). WO2009084693, WO2012075383, WO2011054553, WO2011054841, WO2011054844, WO2011054845, WO2011054846, WO2011054848, WO2011143669, WO2011161031, WO2013027168, WO2014095774, WO2014095775, and WO2016016316 relate to bromodomains and modulators thereof.

EP3563851 discloses a nitrogen-containing heterocyclic compound having a specific structure, which has a bromodomain inhibitory activity and is useful as antitumor agent. E. Ghazy et al. describe dual inhibitors of both HDAC6/8 and the bromodomain and PHD finger containing protein 1 (BRPF1) (E. Ghazy et al., European Journal of Medicinal Chemistry, 2020 May 18, vol 200, pp.112338, XP086205557, PMID: 32497960). P. Bai et al. disclose a positron emission tomography radiotracer selectively targeting the BD1 bromodomains of BET proteins (P. Bai et al., ACS Med Chem Lett. 2021 Jan 8;vol. 12, no. 2, pp. 282-287, XP093049860, PMID: 33603976).

Despite the progress in the field of molecules that modulate the function of bromodomains there is a need for further bromodomain inhibitors with improved tolerability and reduced side effects. A particular concerning side effect of some known bromodomain modulators is phosphodiesterase inhibitor 3 (PDE3) inhibitory activity. The PDE3 family in mammals consists of two members, PDE3A and PDE3B. The PDE3A is mainly implicated in cardiovascular function and it is well known that chronic treatment with PDE3 inhibitors increases mortality in patients with heart failure. The most common and severe side effect of PDE3 inhibitors is ventricular arrhythmias which may be life-threatening. PDE3 inhibitors are generally not recommended for long-term use in patients with heart failure because of their strong cardiostimulatory effects. There is a clear need for bromodomain inhibitors with improved tolerability and without potential cardiovascular side effects by inhibition of phosphodiesterase 3.

### Summary

The invention is set out in the appended set of claims. In addition, any reference to methods of treatment in the subsequent paragraphs of this description is to be interpreted as references to the compounds, pharmaceutical compositions and medicaments of the present invention for use in a method for treatment of the human or animal body by therapy.

An aspect disclosed herein relates to compounds of Formula (I) 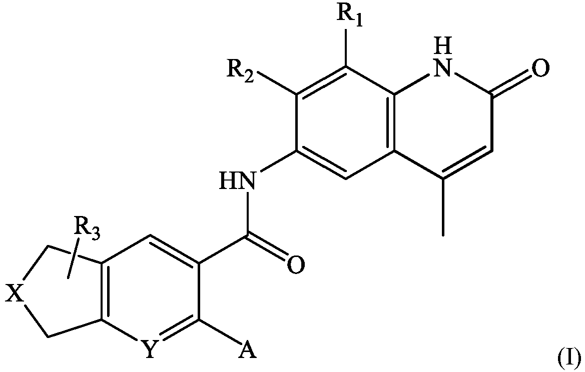 or pharmaceutically acceptable salts, polymorphs, stereoisomers, and tautomers thereof, wherein X is selected from the group consisting of -C(R₄R₅)-, -S-, -O- and -N(R₆)-;Y is -N- or -C(R₇)-, wherein R₇ is selected from the group consisting of hydrogen and C₁₋₄ alkyl; A is selected from the group consisting of unsubstituted or substituted 5 or 6 membered alicyclic ring systems; unsubstituted or substituted 5-10 membered heteroalicyclic ring systems; and -NR₈R₉; R₁ is selected from the group consisting of hydrogen, hydroxy, halogen, unsubstituted or substituted C₁₋₄ alkyl, unsubstituted or substituted C₁₋₄ alkoxy, and unsubstituted or substituted -O-R₁₀, wherein R₁₀ is selected from C₃₋₆ cycloalkyl, 4-6 membered heteroalicyclyl, and C₁₋₄ hydroxyalkyl; R₂ is selected from the group consisting of hydrogen, hydroxy, halogen, unsubstituted or substituted C₁₋₄ alkyl, and unsubstituted or substituted C₁₋₄ alkoxy; provided that R₁ and R₂ cannot be simultaneously selected from hydrogen; or R₁, R₂ and the carbon atom to which they are attached are taken together to form a ring; R₃, may be absent or present 1 or 2 times, and when present is C₁₋₄ alkyl; R₄ and R₅ are independently selected from the group consisting of hydrogen, unsubstituted or substituted C₁₋₆ alkyl, unsubstituted or substituted C₁₋₆ alkenyl, unsubstituted or substituted C₁₋₆ alkynyl, unsubstituted or substituted C₁₋₆ alkoxy, - OH, and -CN; R₆ is selected from the group consisting of hydrogen and unsubstituted or substituted C₁₋₆ alkyl, unsubstituted or substituted C₃₋₆ cycloalkyl, unsubstituted or substituted C₂₋₆ heteroalicyclyl; and R₈, and R₉ are independently selected from the group consisting of hydrogen, unsubstituted or substituted C₁₋₄ alkyl, and unsubstituted or substituted C₃₋₄ cycloalkyl, provided that at least one of R₈ and R₉ is unsubstituted or substituted C₁₋₄ alkyl, and unsubstituted or substituted C₃₋₄ cycloalkyl.

In another aspect the compounds of Formulae (I), (II) and (III), and pharmaceutically acceptable salts, polymorphs, stereoisomers, and tautomers thereof are active bromodomain inhibitors, without inhibiting the activity of phosphodiesterase enzyme 3 (PDE3).

In another aspect the compounds of Formulae (I), (II) and (III), and pharmaceutically acceptable salts, polymorphs, stereoisomers, and tautomers thereof and at least one pharmaceutically acceptable excipitent are comprised in a pharmaceutical composition.

In another aspect, disclosed herein compounds of Formulae (I), (II) and (III), and pharmaceutically acceptable salts, polymorphs, stereoisomers, and tautomers thereof, are used for treating diseases or conditions related to systemic or tissue inflammation, inflammatory responses to infection or products of infectious organisms or hypoxia, autoimmune and allergic processes, cellular activation and proliferation, cancer, metabolism, fibrosis and in the prevention and treatment of viral infections.

In another aspect, disclosed herein are compounds of Formulae (I), (II) and (III), and pharmaceutically acceptable salts, polymorphs, stereoisomers, and tautomers thereof, comprised in a composition for use in treating diseases or conditions related to systemic or tissue inflammation, inflammatory responses to infection or products of infectious organisms or hypoxia, autoimmune and allergic processes, cellular activation and proliferation, cancer, metabolism, fibrosis and in the prevention and treatment of viral infections.

In one aspect the disease or conditions are as rheumatoid arthritis, osteoarthritis, gout, psoriasis, psoriatric arthritis, systemic lupus erythematosus, multiple sclerosis, inflammatory bowel disease, inflammatory bowel syndrome, Crohn's disease, ulcerative colitis, colitis, asthma, chronic obstructive airways disease, pneumonitis, myocarditis, pericarditis, myositis, eczema, dermatitis, atopic dermatitis, allergy, ankylosing spondylitis, lupus erythematosus, Hashimoto's disease, pancreatitis, autoimmune ocular disease, Sjögren's disease, optic neuritis, neuromyelitis optica, Myasthenia Gravis, Guillain Barre syndrome, Graves' disease, alopecia, vitiligo, bullous skin diseases, nephritis, vasculitis, atherosclerosis, Alzheimer's disease, depression, retinitis, uveitis, scleritis, hepatitis, primary biliary cirrhosis, sclerosing cholangitis, hypophysitis, thyroiditis, Addison's disease, type I diabetes and acute rejection of transplanted organs, giant cell arteritis, nephritis including lupus nephritis, vasculitis with organ involvement such as glomerulonephritis, vasculitis including giant cell arteritis, Polyarteritis nodosa, Behcet's disease, Wegener's granulomatosis, Kawasaki disease, Takayasu's Arteritis, vasculitis with organ involvement and acute rejection of transplanted organs; or
treating sepsis, sepsis syndrome, septic shock, endotoxaemia, systemic inflammatory response syndrome (SIRS), multi-organ dysfunction syndrome, toxic shock syndrome, acute lung injury, ARDS (adult respiratory distress syndrome), acute renal failure, fulminant hepatitis, burns, acute pancreatitis, post-surgical syndromes, sarcoidosis, Herxheimer reactions, encephalitis, myelitis, meningitis, malaria and SIRS associated with viral infections such as influenza, herpes zoster, herpes simplex and coronavirus; or
treating ischaemia-reperfusion injury,myocardial infarction, cerebrovascular ischaemia (stroke), acute coronary syndromes, renal reperfusion injury, organ transplantation, coronary artery bypass grafting, cardio-pulmonary bypass procedures, pulmonary, renal, hepatic, gastro-intestinal or peripheral limb embolism; or
treating disorders or conditions such as hypercholesterolemia, atherosclerosis and Alzheimer's disease; or
treating disorders or conditions such as idiopathic pulmonary fibrosis, lung fibrosis, intestinal fibrosis, liver fibrosis, non-alcoholic steatohepatitis, cirrhosis, renal fibrosis, post-operative stricture, myelofibrosis, keloid formation, skin fibrosis, hand fibrosis, systemic sclerosis, scleroderma and cardiac fibrosis; or
treating or preventing viral infections such as herpes virus, human papilloma virus, human immunodeficiency virus (HIV), adenovirus and poxvirus; or
treating colon carcinomas, midline carcinomas, sarcomas, mesenchymal, hepatic, renal and neurological tumours; acute lymphoblastic leukemia, acute myelogenous leukemia, adult T-cell leukemia/lymphoma, bladder cancer, blastoma, bone cancer, breast cancer, brain cancer, burkitts lymphoma, carcinoma, myeloid sarcoma, cervical cancer, chronic lymphocytic leukemia, chronic myelogenous leukemia, colorectal cancer, diffuse large B-cell lymphoma, endometrial cancer, esophageal cancer, follicular lymphoma, gastrointestinal cancer, glioblastoma multiforme, glioma, gallbladder cancer, gastric cancer, head and neck cancer, Hodgkin's lymphoma, non-Hodgkin's lymphoma, intestinal cancer, kidney cancer, laryngeal cancer, leukemia, lung cancer, lymphoma, liver cancer, small cell lung cancer, non-small cell lung cancer, melanoma, mesothelioma, multiple myeloma, ocular cancer, optic nerve tumor, oral cancer, ovarian cancer, pituitary tumor, primary central nervous system lymphoma, prostate cancer, pancreatic cancer, pharyngeal cancer, renal cell carcinoma, rectal cancer, sarcoma, skin cancer, spinal tumor, small intestine cancer, stomach cancer, T-cell lymphoma, testicular cancer, thyroid cancer, throat cancer, urogenital cancer, urothelial carcinoma, uterine cancer, vaginal cancer, or Wilms' tumor; or treating obesity, such as obesity associated with cancer treatment or obesity associated with diabetes and cardiac hypertrophy.

### Detailed description of embodiments

### Definitions

Unless defined otherwise, all technical and scientific terms used herein have the same meaning as is commonly understood by one of ordinary skill in the art. In the event that there is a plurality of definitions for a term herein, those in this section prevail unless stated otherwise.

As used herein, any "R" group(s) such as, without limitation, R₁, R₂, R₃, R₄, R₅, R₆, R₇, R₈, R₉, and R₁₀, represent substituents that can be attached to the indicated atom. A non-limiting list of R groups include but are not limited to hydrogen, hydroxy, alkyl, alkenyl, alkynyl, alkoxy, cycloalkyl, cycloalkenyl, cycloalkynyl, aryl, heteroaryl, and heteroalicyclyl. If two "R" groups are covalently bonded to the same atom or to adjacent atoms, then they may be "taken together" or "combined to" as defined herein to form a cycloalkyl, aryl, heteroaryl or heteroalicyclyl group. For example, without limitation, if Rₐ and R_{b} of an NRₐR_{b} group are indicated to be "taken together" or "combined to", it means that they are covalently bonded to one another at their terminal atoms to form a ring that includes the nitrogen:

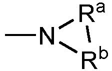

Whenever a group is described as being "unsubstituted or substituted" or "substituted or unsubstituted", if substituted, the substituent(s) (which may be present one or more times, such as 1, 2, 3 or 4 times) are independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, oxo, alkoxy, aryloxy, acyl, ester, O-carboxy, alkylthio, arylthio, cyano, halogen, carbonyl, thiocarbonyl, C-amido, N-amido, S-sulfonamido, N-sulfonamido, nitro, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. Whenever a group is not defined as "unsubstituted or substituted", e.g. C₁₋₄ alkyl, said group is unsubstituted.

When a substituent is deemed to be "substituted," the substitutent itself is substituted. When the referenced substituent is substituted, it is meant that one or more hydrogen atoms on the referenced group may be replaced with a group(s) individually and independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, oxo, alkoxy, aryloxy, acyl, ester, O-carboxy, alkylthio, arylthio, cyano, halogen, carbonyl, thiocarbonyl, C-amido, N-amido, S-sulfonamido, N-sulfonamido, nitro, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. The protecting groups that may form the protective derivatives of the above substituents are known to those of skill in the art and may be found in references Greene and Wuts, Protective Groups in Organic Synthesis, 3rd Ed., John Wiley & Sons, New York, NY, 1999.

As used herein, "Cₘ to Cₙ," "Cₘ-Cₙ" or "Cₘ₋ₙ" in which "m" and "n" are integers refers to the number of carbon atoms in the relevant group. That is, the group can contain from "m" to "n", inclusive, carbon atoms. Thus, for example, a "C₁ to C₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)-, CH₃CH(CH₃)CH₂- and (CH₃)₃C-. If no "m" and "n" are designated with regard to a group, the broadest range described in these definitions is to be assumed.

As used herein, "alkyl" refers to a straight or branched hydrocarbon chain group that is fully saturated (no double or triple bonds). The alkyl group may have 1 to 20 carbon atoms (whenever it appears herein, a numerical range such as "1 to 20" refers to each integer in the given range; *e.g.,* "1 to 20 carbon atoms" means that the alkyl group may consist of 1 carbon atom, 2 carbon atoms, 3 carbon atoms, *etc.,* up to and including 20 carbon atoms, although the present definition also covers the occurrence of the term "alkyl" where no numerical range is designated). The alkyl group may also be a medium size alkyl having 1 to 10 carbon atoms, such as "C₁₋₆". The alkyl group could also be a lower alkyl having 1 to 4 carbon atoms. The alkyl group of the compounds may be designated as "C₁-C₄ alkyl," "C₁₋₄ alkyl" or similar designations. By way of example only, "C₁-C₄ alkyl" or "C₁₋₄ alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl, and the like. When substituted, the substituent group(s) is(are) one or more group(s) individually and independently selected from alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, oxo, alkoxy, aryloxy, acyl, ester, O-carboxy, alkylthio, arylthio, cyano, halogen, carbonyl, thiocarbonyl, C-amido, N-amido, S-sulfonamido, N-sulfonamido, nitro, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof.

As used herein, "alkenyl" refers to an alkyl group that contains in the straight or branched hydrocarbon chain one or more double bonds. If more than one double bond is present, the double bonds may be conjugated or not conjugated. The alkenyl group may have 2 to 20 carbon atoms (whenever it appears herein, a numerical range such as "2 to 20" refers to each integer in the given range; *e.g.,* "2 to 20 carbon atoms" means that the alkenyl group may consist of 2 carbon atom, 3 carbon atoms, 4 carbon atoms, *etc.,* up to and including 20 carbon atoms, although the present definition also covers the occurrence of the term "alkenyl" where no numerical range is designated). When substituted, the substituent group(s) is(are) one or more group(s) individually and independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, oxo, alkoxy, aryloxy, acyl, ester, O-carboxy, alkylthio, arylthio, cyano, halogen, carbonyl, thiocarbonyl, C-amido, N-amido, S-sulfonamido, N-sulfonamido, nitro, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof.

As used herein, "alkynyl" refers to an alkyl group that contains in the straight or branched hydrocarbon chain one or more triple bonds The alkynyl group may have 2 to 20 carbon atoms (whenever it appears herein, a numerical range such as "2 to 20" refers to each integer in the given range; *e.g.,* "2 to 20 carbon atoms" means that the alkynyl group may consist of 2 carbon atom, 3 carbon atoms, 4 carbon atoms, *etc.,* up to and including 20 carbon atoms, although the present definition also covers the occurrence of the term "alkynyl" where no numerical range is designated). An alkynyl group may be unsubstituted or substituted. When substituted, the substituent(s) may be selected from the same groups disclosed above with regard to alkenyl group substitution.

As used herein, "hetero" may be attached to a group and refers to one or more carbon atom(s) and the associated hydrogen atom(s) in the attached group have been independently replaced with the same or different heteroatoms selected from nitrogen, oxygen, phosphorus and sulfur.

As used herein, "heteroalkyl," by itself or in combination with another term, refers to a straight or branched alkyl group consisting of the stated number of carbon atoms, where one or more carbon atom(s), such as 1, 2, 3 or 4 carbon atom(s), and the associated hydrogen atom(s) have been independently replaced with the same or different heteroatoms selected from nitrogen, oxygen and sulfur. The carbon atom(s) being replaced may be in the middle or at the end of the alkyl group. Examples of heteroalkyl include, but are not limited to, -S-alkyl, -O-alkyl, -NH-alkyl, alkyl-O-alkyl, etc.

As used herein, "aryl" refers to a carbocyclic (all carbon) ring or two or more fused rings (rings that share two adjacent carbon atoms) that have a fully delocalized pi-electron system. Aryl groups may range from 6 to 10 carbon atoms (C₆₋₁₀ aryl). In some embodiments the aryl group comprises 6 carbon atoms (C₆ aryl). Examples of aryl groups include, but are not limited to, benzene, naphthalene and azulene. An aryl group may be substituted. When substituted, hydrogen atoms are replaced by substituent group(s) that is(are) one or more group(s) independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, oxo, alkoxy, aryloxy, acyl, ester, O-carboxy, alkylthio, arylthio, cyano, halogen, carbonyl, thiocarbonyl, C-amido, N-amido, S-sulfonamido, N-sulfonamido, nitro, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. When substituted, substituents on an aryl group may form a non-aromatic ring fused to the aryl group, including a cycloalkyl, cycloalkenyl, cycloalkynyl, and heterocyclyl.

As used herein, "heteroaryl" refers to a monocyclic or multicyclic aromatic ring system (a ring system with fully delocalized pi-electron system), in which at least one of the atoms in the ring system is a heteroatom, that is, an element other than carbon, including but not limited to, nitrogen, oxygen and sulfur. Heteroaryl groups may range from 5 to 10 atoms, wherein one or more of the atoms, such as 1, 2, 3 or 4 atom(s), are independently selected from oxygen, sulfur and nitrogen. In some embodiments the heteroaryl group comprises 5 to 7 atoms. Examples of "heteroaryl" include, but are not limited to, furan, thiophene, phthalazine, pyrrole, oxazole, thiazole, imidazole, pyrazole, isoxazole, isothiazole, triazole, thiadiazole, pyridine, pyridazine, pyrimidine, pyrazine, tetrazole, and triazine. A heteroaryl may be substituted. When substituted, hydrogen atoms are replaced by substituent group(s) that is(are) one or more group(s) independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, oxo, alkoxy, aryloxy, acyl, ester, O-carboxy, alkylthio, arylthio, cyano, halogen, carbonyl, thiocarbonyl, C-amido, N-amido, S-sulfonamido, N-sulfonamido, nitro, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. When substituted, substituents on a heteroaryl group may form a non-aromatic ring fused to the aryl group, including a cycloalkyl, cycloalkenyl, cycloalkynyl, and heterocyclyl.

An "aralkyl" or "arylalkyl" is an aryl group connected, as a substituent, via an alkylene group. The alkylene and aryl group of an aralkyl may be substituted. Examples include but are not limited to benzyl, substituted benzyl, 2-phenylethyl, 3-phenylpropyl, and naphthylalkyl. In some cases, the alkylene group is a lower alkylene group. In some cases, the aryl group is a C₆ aryl.

A "heteroaralkyl" or "heteroarylalkyl" is heteroaryl group connected, as a substituent, via an alkylene group. The alkylene and heteroaryl group of heteroaralkyl may be substituted. Examples include but are not limited to 2-thienylmethyl, 3-thienylmethyl, furylmethyl, thienylethyl, pyrrolylalkyl, pyridylalkyl, isoxazolylalkyl, pyrazolylalkyl and imidazolylalkyl, and their substituted as well as benzo-fused analogs. In some cases, the alkylene group is a lower alkylene group, and in some cases the alkylene group is -CH₂- or -CH₂CH₂-.

An "alkylene" is a tethering group, forming bonds to connect molecular fragments via their terminal carbon atoms. The alkylene may have 1 to 20 carbon atoms. The alkylene may also be a medium size alkylene having 1 to 10 carbon atoms, such as "C₁₋₆". The alkylene could also be a lower alkylene having 1 to 4 carbon atoms. The alkylene may be designated as "C₁-C₄ alkylene", "C₁₋₄ alkylene" or similar designations. Non-limiting examples include, methylene (-CH₂-), ethylene (-CH₂CH₂-), propylene (-CH₂CH₂CH₂-), and butylene (-(CH₂)₄-) groups. In the case of methylene, the two connected fragments are connected to the same carbon atom. A lower alkylene group may be substituted, e.g. by a lower alkyl.

As used herein, "heteroalkylene" by itself or in combination with another term refers to an alkylene group consisting of the stated number of carbon atoms in which one or more of the carbon atoms, such as 1, 2, 3 or 4 carbon atom(s), are independently replaced with the same or different heteroatoms selected from oxygen, sulfur and nitrogen. Examples of heteroalkylene include, but not limited to -CH₂-O-, -CH₂-CH₂-O-, -CH₂-CH₂-CH₂-O-, -CH₂-NH-, -CH₂-CH₂-NH-, -CH₂-CH₂-CH₂-NH-, -CH₂-CH₂-NH-CH₂-, -O-CH₂-CH₂-O-CH₂-CH₂-O-, -O-CH₂-CH₂-O-CH₂-CH₂-, and the like.

As used herein, "alkylidene" refers to a divalent group, such as =CR'R'', which is attached to one carbon of another group, forming a double bond. Alkylidene groups include, but are not limited to, methylidene (=CH₂) and ethylidene (=CHCH₃). As used herein, "arylalkylidene" refers to an alkylidene group in which either R' or R'' is an aryl group. An alkylidene group may be substituted.

As used herein, "alkoxy" refers to the group -OR wherein R is an alkyl, e.g. methoxy, ethoxy, n-propoxy, 1-methylethoxy (isopropoxy), cyclopropoxy, n-butoxy, iso-butoxy, sec-butoxy, tert-butoxy, amoxy, tert-amoxy and the like. An alkoxy may be substituted.

As used herein, "alkylthio" refers to the formula -SR wherein R is an alkyl is defined as above, e.g. methylmercapto, ethylmercapto, n-propylmercapto, 1-methylethylmercapto (isopropylmercapto), n-butylmercapto, iso-butylmercapto, sec-butylmercapto, tert-butylmercapto, and the like. An alkylthio may be substituted.

As used herein, "aryloxy" and "arylthio" refers to RO- and RS-, in which R is an aryl as defined above, e.g., phenoxy, naphthalenyloxy, azulenyloxy, anthracenyloxy, naphthalenylthio, phenylthio and the like. Both an aryloxy and arylthio may be substituted.

As used herein, "alkenyloxy" refers to the formula -OR wherein R is an alkenyl as defined above, e.g., vinyloxy, propenyloxy, n-butenyloxy, iso-butenyloxy, sec-pentenyloxy, tert-pentenyloxy, and the like. The alkenyloxy may be substituted.

As used herein, "acyl" refers to a hydrogen, alkyl, alkenyl, alkynyl, or aryl connected, as substituents, via a carbonyl group. Examples include formyl, acetyl, propanoyl, benzoyl, and acryl. An acyl may be substituted.

As used herein, "cycloalkyl" refers to a completely saturated (no double bonds) mono- or multi- cyclic hydrocarbon ring system. When composed of two or more rings, the rings may be joined together in a fused, bridged or spiro-connected fashion. Cycloalkyl groups may range from C₃ to C₁₀, in other embodiments it may range from C₃ to C₆. A cycloalkyl group may be unsubstituted or substituted. Typical cycloalkyl groups include, but are in no way limited to, cyclopropyl, cyclobutyl, cyclopentyl, cyclohexyl, and the like. If substituted, the substituent(s) may be an alkyl or selected from those indicated above with regard to substitution of an alkyl group unless otherwise indicated. When substituted, substituents on a cycloalkyl group may form an aromatic ring fused to the cycloalkyl group, including an aryl and a heteroaryl.

As used herein, "cycloalkenyl" refers to a cycloalkyl group that contains one or more double bonds in the ring although, if there is more than one, they cannot form a fully delocalized pi-electron system in the ring (otherwise the group would be "aryl," as defined herein). When composed of two or more rings, the rings may be connetected together in a fused, bridged or spiro-connected fashion. A cycloalkenyl group may be unsubstituted or substituted. When substituted, the substituent(s) may be an alkyl or selected from the groups disclosed above with regard to alkyl group substitution unless otherwise indicated. When substituted, substituents on a cycloalkenyl group may form an aromatic ring fused to the cycloalkenyl group, including an aryl and a heteroaryl.

As used herein, "cycloalkynyl" refers to a cycloalkyl group that contains one or more triple bonds in the ring. When composed of two or more rings, the rings may be joined together in a fused, bridged or spiro-connected fashion. Cycloalkynyl groups may range from C₃ to C₁₀. A cycloalkynyl group may be unsubstituted or substituted. When substituted, the substituent(s) may be an alkyl or selected from the groups disclosed above with regard to alkyl group substitution unless otherwise indicated. When substituted, substituents on a cycloalkynyl group may form an aromatic ring fused to the cycloalkynyl group, including an aryl and a heteroaryl.

As used herein, "heteroalicyclic" or "heteroalicyclyl" refers to a 3- to 18 membered ring which consists of carbon atoms and from one to five heteroatoms selected from the group consisting of nitrogen, oxygen and sulfur. The heteroalicyclic or heteroalicyclyl groups may range from C₂ to C₁₀, in other embodiments it may range from C₂ to C₉ and in other embodiments it may range from C₂ to C₈. The "heteroalicyclic" or "heteroalicyclyl" may be monocyclic, bicyclic, tricyclic, or tetracyclic ring system, which may be joined together in a fused, bridged or spiro-connected fashion; and the nitrogen, carbon and sulfur atoms in the "heteroalicyclic" or "heteroalicyclyl" may be oxidized; the nitrogen may be quaternized; and the rings may also contain one or more double bonds provided that they do not form a fully delocalized pi-electron system throughout all the rings. Heteroalicyclyl groups may be unsubstituted or substituted. When substituted, the substituent(s) may be one or more groups independently selected from the group consisting of alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, oxo, alkoxy, aryloxy, acyl, ester, O-carboxy, alkylthio, arylthio, cyano, halogen, C-amido, N-amido, S-sulfonamido, N-sulfonamido, isocyanato, thiocyanato, isothiocyanato, nitro, haloalkyl, haloalkoxy, trihalomethanesulfonyl, trihalomethanesulfonamido, and amino, including mono- and di-substituted amino groups, and the protected derivatives thereof. Examples of such "heteroalicyclic" or "heteroalicyclyl" include but are not limited to, azepinyl, azetidinyl, dioxolanyl, imidazolinyl, imidazolinolyl morpholinyl, oxetanyl, oxiranyl, piperidinyl N-Oxide, piperidinyl (e.g. 1-piperidinyl, 2-piperidinyl, 3-piperidinyl and 4-piperidinyl) , pyrrolidinyl, (e.g. 1-pyrrolidinyl, 2-pyrrolidinyl and 3-pyrrolidinyl), piperazinyl, pyranyl, 4-piperidonyl, tetrahydrofuranyl, tetrahydropyranyl, pyrazolidinyl, 2-oxopyrrolidinyl, thiamorpholinyl, thiamorpholinyl sulfoxide, and thiamorpholinyl sulfone. When substituted, substituents on a heteroalicyclyl group may form an aromatic ring fused to the heteroalicyclyl group, including an aryl and a heteroaryl.

A "(cycloalkyl)alkyl" is a cycloalkyl group connected, as a substituent, via an alkylene group. The alkylene and cycloalkyl of a (cycloalkyl)alkyl may be substituted. Examples include but are not limited cyclopropylmethyl, cyclobutylmethyl, cyclopropylethyl, cyclopropylbutyl, cyclobutylethyl, cyclopropylisopropyl, cyclopentylmethyl, cyclopentylethyl, cyclohexylmethyl, cyclohexylethyl, cycloheptylmethyl, and the like. In some cases, the alkylene group is a lower alkylene group (C₁₋₄ alkylene).

A "(cycloalkenyl)alkyl" is a cycloalkenyl group connected, as a substituent, via an alkylene group. The alkylene and cycloalkenyl of a (cycloalkenyl)alkyl may be substituted. In some cases, the alkylene group is a lower alkylene group.

A "(cycloalkynyl)alkyl" is a cycloalkynyl group connected, as a substituent, via an alkylene group. The alkylene and cycloalkynyl of a (cycloalkynyl)alkyl may be substituted. In some cases, the alkylene group is a lower alkylene group.

As used herein, "halo" or "halogen" refers to F (fluoro), Cl (chloro), Br (bromo) or I (iodo).

As used herein, "haloalkyl" refers to an alkyl group in which one or more of the hydrogen atoms are replaced by halogen. Examples of haloalkyl are C₁₋₃ alkyl in which one ore more of the hydrogen atoms are replaced by halogen, e.g. fluoro. Such groups include but are not limited to, chloromethyl, fluoromethyl, difluoromethyl, trifluoromethyl and 1-chloro-2-fluoromethyl, 2-fluoroisobutyl. A haloalkyl may be substituted.

As used herein, "haloalkoxy" refers to a RO-group in which R is a haloalkyl group. Such groups include but are not limited to, chloromethoxy, fluoromethoxy, difluoromethoxy, trifluoromethoxy and 1-chloro-2-fluoromethoxy, 2-fluoroisobutoxy. A haloalkoxy may be substituted.

An "O-carboxy" group refers to a "RC(=O)O-" group in which R can be hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl, as defined herein. An O-carboxy may be substituted.

A "C-carboxy" group refers to a "-C(=O)OR" group in which R can be the same as defined with respect to O-carboxy. A C-carboxy may be substituted.

A "trihalomethanesulfonyl" group refers to an "X₃CSO₂-" group" wherein X is a halogen.

A dashed bond, - - - - -, represents an optional unsaturation between the atoms forming the bond. This bond may be unsaturated (e.g. C=C, C=N, C=O) or saturated (e.g. C-C, C-N, C-O). When a dashed bond is present in a ring system it may form part of an aromatic ring system.

A "nitro" group refers to a "-NO₂" group
A "cyano" group refers to a "-CN" group.

A "cyanato" group refers to an "-OCN" group.

An "isocyanato" group refers to a "-NCO" group.

A "thiocyanato" group refers to a "-SCN" group.

A "carbonyl" group refers to a "-C(=O)-" group.

A "thiocarbonyl" group refers to a "-C(=S)-" group.

An "oxo" group refers to a " =O " group.

An "isothiocyanato" group refers to an " -NCS" group.

A "sulfinyl" group refers to an "-S(=O)-R" group in which R can be the same as defined with respect to O-carboxy. A sulfinyl may be substituted.

A "sulfonyl" group refers to an "SO₂R" group in which R can be the same as defined with respect to O-carboxy. A sulfonyl may be substituted.

An "S-sulfonamido" group refers to a "-SO₂NR_{A}R_{B}" group in which R_{A} and R_{B} independently of each other can be the same as defined with respect to the R group as defined for O-carboxy, or combined to form a ring system selected from the group consisting of substituted or unsubstituted C₃₋₈ cycloalkyl, substituted or unsubstituted C₃₋₈ cycloalkenyl, substituted or unsubstituted C₃₋₈ cycloalkyl, substituted or unsubstituted C₃₋₈ cycloalkenyl, substituted or unsubstituted heteroalicyclyl, substituted or unsubstituted aryl, and substituted or unsubstituted heteroaryl. A S-sulfonamido may be substituted.

An "N-sulfonamido" group refers to a "RSO₂N(R_{A})-" group in which R and R_{A} independently of each other can be the same as defined with respect to the R group as defined for O-carboxy. An N-sulfonamido may be substituted.

A "trihalomethanesulfonamido" group refers to an "X₃CSO₂N(R)-" group with X as halogen and R can be the same as defined with respect to O-carboxy. A trihalomethanesulfonamido may be substituted.

An "amido" group refers to "C-amido" group, i.e. to a "-C(=O)NR_{A}R_{B}" group in which R_{A} and R_{B} independently of each other can be the same as defined with respect to the R group as defined for O-carboxy, or combined to form a ring system selected from the group consisting of substituted or unsubstituted C₃₋₈ cycloalkyl, substituted or unsubstituted C₃₋₈ cycloalkenyl, substituted or unsubstituted C₃₋₈ cycloalkyl, substituted or unsubstituted C₃₋₈ cycloalkenyl, substituted or unsubstituted heteroalicyclyl, substituted or unsubstituted aryl, and substituted or unsubstituted heteroaryl. A C-amido may be substituted; or the "amido"group refers to an "N-amido" group, i.e. to a "RC(=O)NR_{A}-" group in which R and R_{A} independently of each other can be the same as defined with respect to the R group as defined for O-carboxy. An N-amido may be substituted.

An "ester" refers to a "-C(=O)OR" group in which R can be the same as defined with respect to O-carboxy. An ester may be substituted.

A lower alkoxyalkyl refers to an alkoxy group connected via a lower alkylene group. A lower alkoxyalkyl may be substituted.

An "amino" refers to "RNH₂" (primary amines), "R₂NH" (secondary amines), and "R₃N" (tertiary amines), in which each R independently can be selected from hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. An amino group may be substituted.

An aminoalkyl refers to an amino group connected via a alkylene group. A aminoalkyl may be substituted.

The terms "ring" and "ring system" are used interchangeably throughout the disclosure.

Any unsubstituted or monosubstituted amine group on a compound herein can be converted to an amide, any hydroxyl group can be converted to an ester and any carboxyl group can be converted to either an amide or ester using techniques well-known to those skilled in the art (see, for example, Greene and Wuts, Protective Groups in Organic Synthesis, 3rd Ed., John Wiley & Sons, New York, NY, 1999).

As used herein, the abbreviations for any protective groups, amino acids and other compounds, are, unless indicated otherwise, in accord with their common usage, recognized abbreviations, or the IUPAC-IUB Commission on Biochemical Nomenclature (See, Biochem. 11:942-944 (1972)).

As employed herein, the following terms have their accepted meaning in the chemical literature.

| | |
|---|---|
| AcOH | Acetic acid |
| BrettPhos | dicyclohexyl-[3,6-dimethoxy-2-(2,4,6-triisopropylphenyl)phenyl]phosphane |
| CHAPS | 3-[(3-Cholamidopropyl)dimethylammonio]-1-propanesulfonate hydrate |
| Cs₂CO₃ | Cesium carbonate, 99% |
| DCM | Methylene chloride, dichloromethane |
| DIC | (3-Dimethylamino-propyl)-ethyl-carbodiimide |
| DIPEA | N,N-Diisopropylethylamine |
| DIEA | N,N-Diisopropylethylamine |
| DMAP | 4-Dimethylaminopyridine |
| DMF | N,N-dimethylformamide |
| DMSO | Dimethylsulfoxide |
| Dppf | Diphenylphosphinoferrocene |
| EDC | (3-Dimethylamino-propyl)-ethyl-carbodiimide |
| EtOAc | Ethyl acetate |
| EtOH | Ethanol |
| Fe | Iron |
| H₂ | Hydrogen |
| H₂SO₄ | Sulfuric acid |
| HCl | Hydrochloric acid |
| HEPES | 4-(2-Hydroxyethyl)piperazine-1-ethanesulfonic acid, N-(2-Hydroxyethyl)piperazine-N'-(2-ethanesulfonic acid) |
| HOAt | [1,2,3]Triazolo[4,5-b]pyridin-3-ol |
| HOBt | 1-Hydroxy-benzotriazole |
| K₂CO₃ | Potassium carbonate |
| LCMS | Liquid Chromatography - Mass spectrometry |
| LiI | Lithium Iodide |
| LiOH | Lithium hydroxide |
| mAb | Monoclonal antibody |
| MeCN | Acetonitrile |
| MeOH | Methanol |
| MgSO₄ | Magnesium sulfate |
| Na₂CO₃ | Sodium Carbonate |
| Na₂SO₄ | Sodium Sulfate, anhydrous |
| NaBH(OAc)₃ | Sodium triacetoxyborohydride |
| NaH | Sodium hydride |
| NaHCO₃ | sodium hydrogen carbonate |
| NaOH | Sodium hydroxide |
| NaOtBu | Sodium-tert-butylat |
| NH₃-aq | ammonium hydroxide |
| NH₄Cl | Ammonium chloride |
| NMP | 1-methylpyrrolidin-2-one |
| NT | Not Tested |
| Pd(OAc)₂ | Palladium acetate |
| Pd(PPh₃)₄ | Tetrakis(triphenylphosphine)palladium |
| Pd(dppf)Cl₂ | 1'-Bis(diphenylphosphino)ferrocene]dichloropalladium(II) |
| Pd/C | Palladium on activated charcoal |
| PtO₂ | Platinum(IV) oxide |
| Rt | room temperature |
| RuPhos | Dicyclohexyl-[2-(2,6-diisopropoxyphenyl)phenyl]phosphane |
| t-BuOH | Tert-Butanol |
| t-But-phos-pd(o) | Palladium(0) and tri-tert-butylphospine. |
| TEA | Triethylamine |
| TFA | Trifluoroacetic acid |
| THF | Tetrahydrofuran |
| TMS | Trimethylsilyl |
| TPP | Triphenylphosphine |
| TrixiePhos | Di-tert-butyl-[1-(1-naphthyl)-2-naphthyl]phosphane |
| Zn(CN)₂ | zinc dicyanide |

It is understood that, in any compound disclosed herein having one or more chiral centers, if an absolute stereochemistry is not expressly indicated, then each center may independently be of R-configuration or S-configuration or a mixture thereof. The terms "R" and "S" used herein are configurations as defined in IUPAC 1974 Recommendations for Section E, Fundamental Stereochemistry, in Pure Appl. Chem., 1976, 45: 13-30. The disclosure contemplates various stereoisomers and mixtures thereof and these are specifically included within the scope of the disclosure. Stereoisomers include enantiomers and diastereomers, and mixtures of enantiomers or diastereomers. Individual stereoisomers of compounds of the present disclosure may be prepared synthetically from commercially available starting materials which contain asymmetric or chiral centers or by preparation of racemic mixtures followed by methods of resolution well-known to those of ordinary skill in the art. These methods of resolution are exemplified by (1) attachment of a mixture of enantiomers to a chiral auxiliary, separation of the resulting mixture of diastereomers by recrystallization or chromatography and optional liberation of the optically pure product from the auxiliary as described in Furniss, Hannaford, Smith, and Tatchell, "Vogel's Textbook of Practical Organic Chemistry", 5th edition (1989), Longman Scientific & Technical, Essex CM20 2JE, England, or (2) direct separation of the mixture of optical enantiomers on chiral chromatographic columns or (3) fractional recrystallization methods. Further, compounds provided herein may be scalemic mixtures. In addition, it is understood that in any compound having one or more double bond(s) generating geometrical isomers that can be defined as E or Z each double bond may independently be E or Z or a mixture thereof. Likewise, all tautomeric forms are also intended to be included.

Compounds disclosed herein may exist as cis or trans isomers, wherein substituents on a ring may be attached in such a manner that they are on the same side of the ring (cis) relative to each other, or on opposite sides of the ring relative to each other (trans). For example, cyclobutane may be present in the cis or trans configuration, and may be present as a single isomer or a mixture of the cis and trans isomers. Individual cis or trans isomers of compounds disclosed herein may be prepared synthetically from commercially available starting materials using selective organic transformations, or prepared in single isomeric form by purification of mixtures of the cis and trans isomers. Such methods are well-known to those of ordinary skill in the art, and may include separation of isomers by recrystallization or chromatography.

"Tautomers" refer to compounds that are interchangeable forms of a particular compound structure which vary in the displacement of hydrogen atoms and electrons, a typical example is the "enol" - "keto" forms:

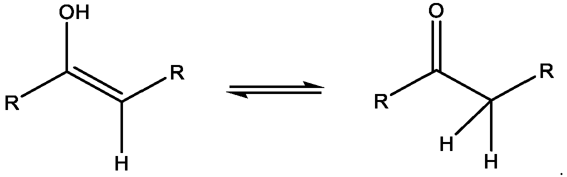

"Enol" - "keto" tautomerism may be exemplified byFormula I and its tautomeric form :

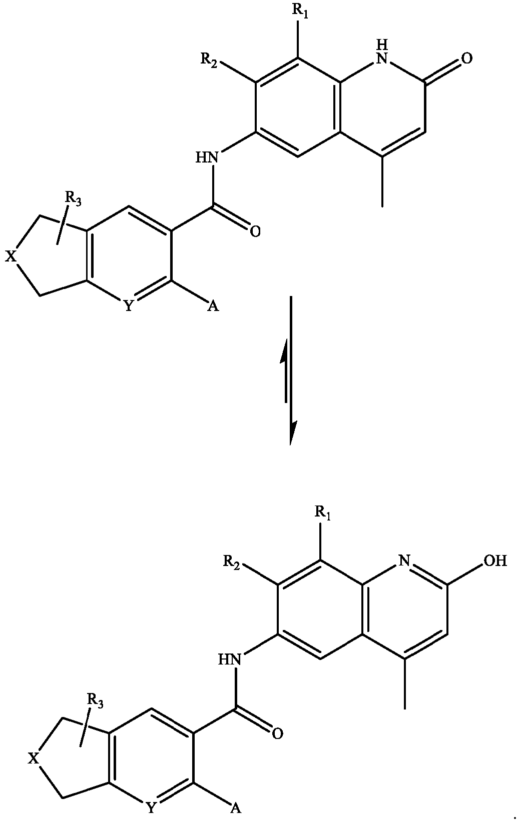

Additional non-limiting examples of tautomers include imine-enamine tautomers (-CH₂-CH=NH and -CH=CH-NH₂), or the tautomeric forms of heteroaryl groups containing a ring atom attached to both a ring -NH- moiety and a ring =N- moiety such as pyrazoles, imidazoles, benzimidazoles, triazoles, and tetrazoles.

It is understood that isotopes may be present in the compounds described herein. Each chemical element as represented in a compound structure may include any isotope of said element. For example, in a compound described herein a hydrogen atom can be any isotope of hydrogen, including but not limited to hydrogen-1 (protium) and hydrogen-2 (deuterium). Thus, reference herein to a compound encompasses all potential isotopic forms unless the context clearly dictates otherwise.

As used herein, "pharmaceutically acceptable salt" refers to a salt of a compound that does not abrogate the biological activity and properties of the compound. Pharmaceutical salts can be obtained by reaction of a compound disclosed herein with an acid or base. Base-formed salts include, without limitation, ammonium salt (NH₄⁺); alkali metal, such as, without limitation, sodium or potassium, salts; alkaline earth, such as, without limitation, calcium or magnesium, salts; salts of organic bases such as, without limitation, dicyclohexylamine, piperidine, piperazine, methylpiperazine, N-methyl-D-glucamine, diethylamine, ethylenediamine, tris(hydroxymethyl)methylamine; and salts with the amino group of amino acids such as, without limitation, arginine and lysine. Useful acid-based salts include, without limitation, hydrochlorides, hydrobromides, acetates, adipates, aspartates, ascorbates, benzoates, butyrates, caparate, caproate, caprylate, camsylates, citrates, decanoates, formates, fumarates, gluconates, glutarate, glycolates, hexanoates, laurates, lactates, maleates, nitrates, oleates, oxalates, octanoates, propanoates, palmitates, phosphates, sebacates, succinates, stearates, sulfates, sulfonates, such as methanesulfonates, ethanesulfonates, p-toluenesulfonates, salicylates, tartrates, tosylates.

Pharmaceutically acceptable solvates and hydrates are complexes of a compound with one or more solvent of water molecules, or 1 to about 100, or 1 to about 10, or one to about 2, 3 or 4, solvent or water molecules.

As used herein, a "prodrug" refers to a compound that may not be pharmaceutically active but that is converted into an active drug upon *in vivo* administration. The prodrug may be designed to alter the metabolic stability or the transport characteristics of a drug, to mask side effects or toxicity, to improve the flavor of a drug or to alter other characteristics or properties of a drug. Prodrugs are often useful because they may be easier to administer than the parent drug. They may, for example, be bioavailable by oral administration whereas the parent drug is not. The prodrug may also have better solubility than the active parent drug in pharmaceutical compositions. An example, without limitation, of a prodrug would be a compound disclosed herein, which is administered as an ester (the "prodrug") to facilitate absorption through a cell membrane where water solubility is detrimental to mobility but which then is metabolically hydrolyzed to a carboxylic acid (the active entity) once inside the cell where water-solubility is beneficial. A further example of a prodrug might be a short peptide (polyaminoacid) bonded to an acid group where the peptide is metabolized *in vivo* to release the active parent compound. By virtue of knowledge of pharmacodynamic processes and drug metabolism *in vivo,* those skilled in the art, once a pharmaceutically active compound is known, can design prodrugs of the compound (see, *e.g.* Nogrady (1985) Medicinal Chemistry A Biochemical Approach, Oxford University Press, New York, pages 388-392).

As used herein, to "modulate" the function of a bromodomain or a bromodomain containing protein means either to increase its cellular function over the base level measured in the particular environment in which it is found, or decrease its cellular function to less than the measured base level in the environment in which it is found and/or render it unable to perform its cellular function at all.

An "agonist" is defined as a compound that increases the basal activity of a receptor (i.e. signal transduction mediated by the receptor).

As used herein, "partial agonist" refers to a compound that has an affinity for a receptor but, unlike an agonist, when bound to the receptor it elicits only a fractional degree of the pharmacological response normally associated with the receptor even if a large number of receptors are occupied by the compound.

An "inverse agonist" is defined as a compound, which reduces, or suppresses the basal activity of a receptor, such that the compound is not technically an antagonist but, rather, is an agonist with negative intrinsic activity.

As used herein, "antagonist" refers to a compound that binds to a receptor to form a complex that does not give rise to any response, as if the receptor was unoccupied. An antagonist attenuates the action of an agonist on a receptor. An antagonist may bind reversibly or irreversibly, effectively eliminating the activity of the receptor permanently or at least until the antagonist is metabolized or dissociates or is otherwise removed by a physical or biological process.

As used herein, a "subject" refers to an animal that is the object of treatment, observation or experiment. "Animal" includes cold- and warm-blooded vertebrates and invertebrates such as birds, fish, shellfish, reptiles and, in particular, mammals. "Mammal" includes, without limitation, mice; rats; rabbits; guinea pigs; dogs; cats; sheep; goats; cows; horses; primates, such as monkeys, chimpanzees, and apes, and, in particular, humans.

As used herein, a "patient" refers to a subject that is being treated by a medical professional such as an M.D. or a D.V.M. to attempt to cure, or at least ameliorate the effects of, a particular disease or disorder or to treat the disease or disorder from occurring in the first place.

A "receptor" is intended to include any molecule present inside or on the surface of a cell that may affect cellular physiology when it is inhibited or stimulated by a ligand. Typically, a receptor comprises an extracellular domain with ligand-binding properties, a transmembrane domain that anchors the receptor in the cell membrane, and a cytoplasmic domain that generates a cellular signal in response to ligand binding ("signal transduction"). A receptor also includes any intracellular molecule that in response to ligation generates a signal. A receptor also includes any molecule having the characteristic structure of a receptor, but with no identifiable ligand. In addition, a receptor includes a truncated, modified, mutated receptor, or any molecule comprising partial or all of the sequences of a receptor. "Ligand" is intended to include any substance that binds to or interacts with a bromodomain or a bromodomain containing protein.

"Selective" or "selectivity" is defined as a compound's ability to bind or inhibit preferentially a particular protein or specific domain of a protein over other proteins or other domains. "Selective" or "selectivity" of a bromodomain binding compound or inhibitor may refer to a compound being able to bind preferentially a bromodomain of the BET family over non-BET family bromodomain containing proteins. It may also refer to a compound being able to bind preferentially to the N-terminal bromodomain of a BET family protein over the C-terminal bromodomain or the compound being able to preferentially bind the C-terminal bromodomain over the N-terminal domain

As used herein, "coadministration" of pharmacologically active compounds refers to the delivery of two or more separate chemical entities, whether in vitro or in vivo. Coadministration means the simultaneous delivery of separate agents; the simultaneous delivery of a mixture of agents; as well as the delivery of one agent followed by delivery of a second agent or additional agents. Agents that are coadministered are typically intended to work in conjunction with each other.

The term "an effective amount" as used herein means an amount of active compound or pharmaceutical agent that elicits the biological or medicinal response in a tissue, system, animal or human that is being sought by a researcher, veterinarian, medical doctor or other clinician, which includes alleviation or palliation of the symptoms of the disease being treated.

### Compounds

An embodiment disclosed herein relates to compounds of Formula (I) 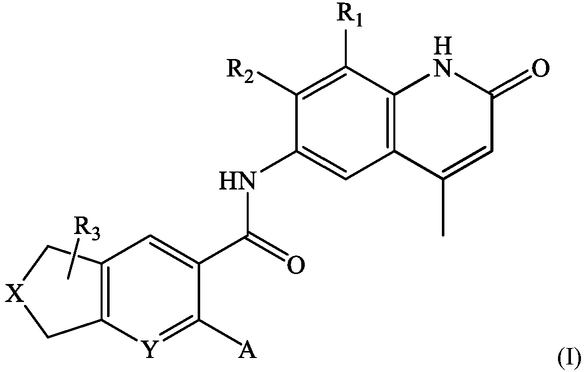 or pharmaceutically acceptable salts, polymorphs, stereoisomers, and tautomers thereof, wherein X is selected from the group consisting of -C(R₄R₅)-, -S-, -O- and -N(R₆)-; Y is -N- or -C(R₇)-, wherein R₇ is selected from the group consisting of hydrogen and C₁₋₄ alkyl; A is selected from the group consisting of unsubstituted or substituted 5 or 6 membered alicyclic ring systems; unsubstituted or substituted 5-10 membered heteroalicyclic ring systems; and -NR₈R₉; R₁ is selected from the group consisting of hydrogen, hydroxy, halogen, unsubstituted or substituted C₁₋₄ alkyl, unsubstituted or substituted C₁₋₄ alkoxy, and unsubstituted or substituted -O-R₁₀, wherein R₁₀ is selected from C₃₋₆ cycloalkyl, 4-6 membered heteroalicyclyl, and C₁₋₄ hydroxyalkyl; R₂ is selected from the group consisting of hydrogen, hydroxy, halogen, unsubstituted or substituted C₁₋₄ alkyl, and unsubstituted or substituted C₁₋₄ alkoxy; provided that R₁ and R₂ cannot be simultaneously selected from hydrogen; or R₁, R₂ and the carbon atom to which they are attached are taken together to form a ring; R₃, may be absent or present 1 or 2 times, and when present is C₁₋₄ alkyl selected from the group selected from C₁₋₄ alkyl; R₄ and R₅ are independently selected from the group consisting of hydrogen, unsubstituted or substituted C₁₋₆ alkyl, unsubstituted or substituted C₁₋₆ alkenyl, unsubstituted or substituted C₁₋₆ alkynyl, unsubstituted or substituted C₁₋₆ alkoxy, -OH, and -CN; R₆ is selected from the group consisting of hydrogen and unsubstituted or substituted C₁₋₆ alkyl, unsubstituted or substituted C₃₋₆ cycloalkyl, unsubstituted or substituted C₂₋₆ heteroalicyclyl; and R₈, and R₉ are independently selected from the group consisting of hydrogen, unsubstituted or substituted C₁₋₄ alkyl, and unsubstituted or substituted C₃₋₄ cycloalkyl, provided that at least one of R₈ and R₉ is selected from the group consisting of unsubstituted or substituted C₁₋₄ alkyl, and unsubstituted or substituted C₃₋₄ cycloalkyl.

In some embodiments the compounds, or pharmaceutically acceptable salts, polymorphs, stereoisomers, and tautomers of Formula (I) relate to X being selected from the group consisting of -C(R₄R₅)-, -O- and -N(R₆)-. In other embodiments X is -C(R₄R₅)-, wherein R₄ and R₅ are independently selected from the the group consisting of hydrogen, hydroxy, C₁₋₄ alkyl, C₁₋₄ alkoxy, e.g. hydrogen or methyl. In some embodiments both R₄ and R₅ are hydrogen, in other embodiments R₄ is hydrogen and R₅ is methyl, and in another embodiment both R₄ and R₅ are methyl. In other embodiments X is -O-. In other embodiments X is -N(R₆)-, wherein R₆ is selected from the group consisting of hydrogen, C₁₋₄ alkyl, C₁₋₄ haloalkyl, and 4 or 5 membered heteroalicyclyl, e.g. hydrogen, methyl, ethyl, n-propyl, isopropyl, and tetrahydrofuranyl, such as hydrogen, methyl, or ethyl.

In some embodiments of Formula (I), Y is -N- or -C(R₇)-, wherein R₇ is hydrogen or C₁₋₄ alkyl, such as hydrogen.

In some embodiments of Formula (I), Y is -N- and X is -CH₂-.

In some embodiments of Formula (I), Y is -N- and X is -O-.

In some embodiments of Formula (I), Y is -N- and X is -N(R₆)-, wherein R₆ is selected from the group consisting of hydrogen, methyl, ethyl, n-propyl, isopropyl, and tetrahydrofuranyl, such as hydrogen, methyl or ethyl.

In some embodiments of Formula (I), A is selected from the group consisting of 5 or 6 membered alicyclic ring systems; 5-10 membered heteroalicyclic ring systems; and - NR₈R₉, wherein each ring systems are unsubstituted or substituted. In some embodiments the ring system, e.g the 5-10 membered heteroalicyclic ring systems is unsubstituted, wherein R₈, and R₉ are independently selected from the group consisting of hydrogen, unsubstituted or substituted C₁₋₄ alkyl, and unsubstituted or substituted C₃₋₄ cycloalkyl, provided that at least one of R₈ and R₉ is selected from the group consisting of substituted C₁₋₄ alkyl, or unsubstituted or substituted C₃₋₄ cycloalkyl.

In some embodiments of Formula (I) the ring system, e.g the 5-10 membered heteroalicyclic ring systems is substituted by a substitutent selected from the group consisting of hydrogen, hydroxy, halogen, unsubstituted or substituted C₁₋₄ alkyl, and unsubstituted or substituted C₁₋₄ alkoxy.

In some embodiments of Formula (I) A is an unsubstituted or substituted a 5-10 membered heteroalicyclic ring system selected from the group consisting of a 5 membered heteroalicyclic ring system comprising at least one nitrogen atom, a 6 membered heteroalicyclic ring system comprising at least one nitrogen atom or an oxygen atom, a 7 membered heteroalicyclic ring system comprising at least one nitrogen atom, an 8 membered heteroalicyclic ring system comprising at least one nitrogen atom, all which may be unsubsituted or substituted, and whenever substituted the substitutent may be present 1, 2 or 3 times and independently selected from the group consisting of methyl, ethyl, propyl, and halogen.

In some embodiments of Formula (I), A is an unsubstituted or substituted morpholine or unsubstituted or substituted morpholine derivative.

In some embodiments of Formula (I), A is an unsubstituted or substituted morpholine or unsubstituted or substituted morpholine derivative, Y is -N- or -CH-, X is - C(R₄R₅)-, wherein R₄ and R₅ independently are hydrogen or C₁₋₄ alkyl, e.g. hydrogen or methyl, or X is -N(R₆)-, wherein R₆ is selected from the group consisting of hydrogen, methyl, ethyl, n-propyl, isopropyl, and tetrahydrofuranyl, such as hydrogen, methyl or ethyl, or X is -O-.

In some embodiments of Formula (I), A is an unsubstituted or substituted morpholine or unsubstituted or substituted morpholine derivative, Y is -N-, X is -CH₂- or X is -N(R₆)-, wherein R₆ is selected from the group consisting of hydrogen, methyl, ethyl, n-propyl, isopropyl, and tetrahydrofuranyl, such as hydrogen, methyl or ethyl, or X is -O-.

In some embodiments of Formula (I), A is an unsubstituted or substituted morpholine or unsubstituted or substituted morpholine derivative, Y is -N-, X is -CH₂-, or X is -N(R₆)-, wherein R₆ is selected from the group consisting of hydrogen, methyl, ethyl, n-propyl, isopropyl, and tetrahydrofuranyl, such as hydrogen, methyl or ethyl.

In some embodiments of Formula (I), Y is -N-, X is -C(R₄R₅)-, wherein R₄ and R₅ independently are hydrogen or C₁₋₄ alkyl, e.g. hydrogen or methyl, or X is -N(R₆)-, wherein R₆ is selected from the group consisting of hydrogen, methyl, ethyl, n-propyl, isopropyl, and tetrahydrofuranyl, such as hydrogen, methyl, or ethyl, or X is -O-, and A is an unsubstituted or substituted morpholine or unsubstituted or substituted morpholine derivative selected from the group consisting of: 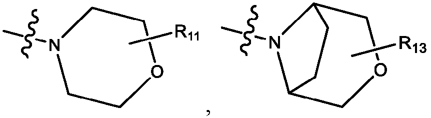 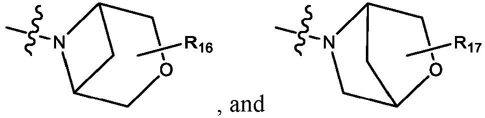 wherein R₁₁, R₁₃, R₁₆, and R₁₇ are absent, or present 1 or 2 times, and when present independently selected from the group consisting of hydroxy, fluoro, methyl, ethyl, and propyl. In other embodiments R₁₁, R₁₃, R₁₆, and R₁₇ are absent. In other embodiments A is unsubstituted morpholine

In some embodiments of Formula (I), A is an unsubstituted or substituted morpholine or unsubstituted or substituted morpholine derivative, Y is -N-, and X is -O-, wherein the unsubstituted or substituted morpholine or unsubstituted or substituted morpholine derivative is selected from the group consisting of: 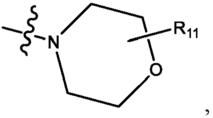 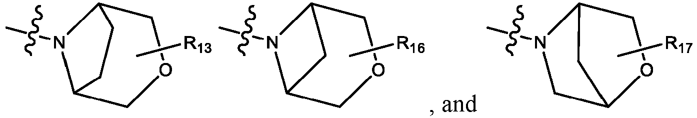 wherein R₁₁, R₁₃, R₁₆, and R₁₇ are absent, or present 1 or 2 times, and when present independently selected from the group consisting of hydroxy, fluoro, methyl, ethyl, and propyl. In other embodiments R₁₁, R₁₃, R₁₆, and R₁₇ are absent. In other embodiments A is unsubstituted morpholine.

In some embodiments of Formula (I), A is an unsubstituted or substituted morpholine or unsubstituted or substituted morpholine derivative, Y is -N-, and X is -O-, wherein the unsubstituted or substituted morpholine or unsubstituted or substituted morpholine derivative is selected from the group consisting of: 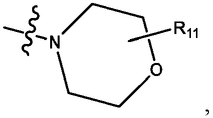 wherein R₁₁ is absent, or present 1 or 2 times, and when present independently selected from the group consisting of hydroxy, fluoro, methyl, ethyl, and propyl. In other embodiments R₁₁, is absent.

In some embodiments of Formula (I), A is an unsubstituted or substituted morpholine or unsubstituted or substituted morpholine derivative, Y is -N-, and X is -O-, wherein the unsubstituted or substituted morpholine or unsubstituted or substituted morpholine derivative is selected from the group consisting of: 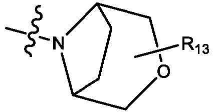 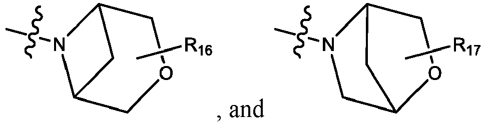 wherein R₁₃, R₁₆, and R₁₇ are absent, or present 1 or 2 times, and when present independently selected from the group consisting of hydroxy, fluoro, methyl, ethyl, and propyl. In other embodiments R₁₃, R₁₆, and R₁₇ are absent.

In some embodiments of Formula (I), Y is -N-, X is -C(R₄R₅)-, wherein R₄ and R₅ independently are selected from the group consisting of hydrogen, hydroxyl, C₁₋₄ alkyl, C₁₋₄ alkoxy, e.g. both are hydrogen or X is -N(R₆)-, wherein R₆ is selected from the group consisting of hydrogen, methyl, ethyl, n-propyl, isopropyl, and tetrahydrofuranyl, such as hydrogen, methyl, or ethyl, or X is -O-, and A is an unsubstituted or substituted a 5-10 membered heteroalicyclic ring system other than an unsubstituted or substituted morpholine or an unsubstituted or substituted morpholine derivative, non-limiting example of such groups are 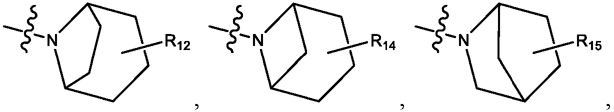 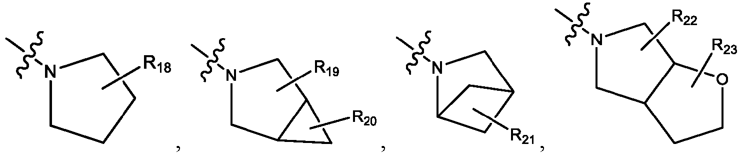 and 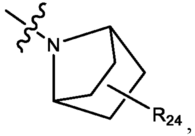
wherein R₁₂, R₁₄, R₁₅, R₁₈, R₁₉, R₂₀, R₂₁, R₂₂, R₂₃ and R₂₄ independently are absent or present 1, 2 or 3 times and when present selected from the group of halogen, hydroxy, C₁₋₄ alkyl, and C₁₋₄ alkoxy. In other embodiments A is selected from the group consisting of 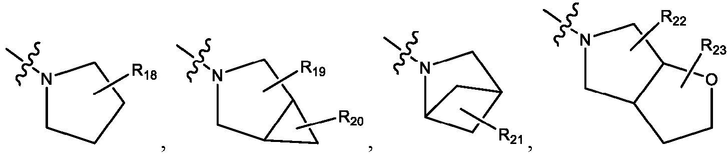 and 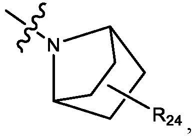
wherein R₁₈, R₁₉, R₂₀, R₂₁, R₂₂, R₂₃ and R₂₄ independently are absent, or present one or two time, and when present independently selected from the group consisting of hydroxy, fluoro, methyl, ethyl, and propyl. In other embodiments R₁₈, R₁₉, R₂₀, R₂₁, R₂₂, R₂₃ and R₂₄ present 1 time and selected from the group consisting of hydroxy, fluoro, and methyl. In other embodiments R₁₈, R₁₉, R₂₀, R₂₁, R₂₂, R₂₃ and R₂₄ present two time and selected from the group consisting of fluoro, and methyl.

In other embodiments of Formul (I), A is selected from the group consisting of 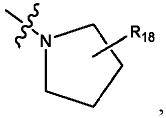 wherein R₁₈, is absent or present 1 or 2 times and selected from the group consisting of fluoro, and methyl. In some embodiments R₁₈ is present 1 time.

In some embodiment A is an unsubstituted or substituted a 5-10 membered heteroalicyclic ring system other than an unsubstituted or substituted morpholine or an unsubstituted or substituted morpholine derivative (including bridged morpholine derivates) which means a heteroalicyclic ring other than (or provided it is not) an unsubstituted or substituted morpholine or an unsubstituted or substituted morpholine derivative. The unsubstituted or substituted morpholine derivative is understood as comprising a morpholine scaffold, examples are:

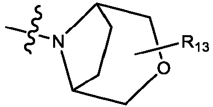

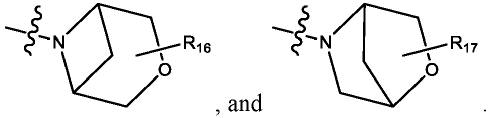

In some embodiment an unsubstituted or substituted a 5-10 membered heteroalicyclic ring system other than an unsubstituted or substituted morpholine or an unsubstituted or substituted morpholine derivative means a heteroalicyclic ring other than the following: 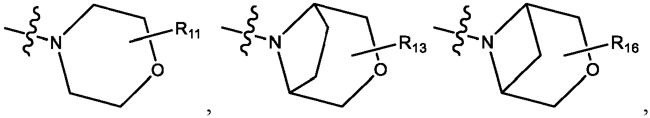 and

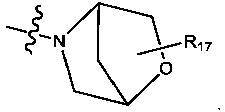

In some embodiments of Formula (I), Y is -N-, X is -CH₂-, or X is -N(R₆)-, wherein R₆ is selected from the group consisting of hydrogen, methyl, ethyl, n-propyl, isopropyl, and tetrahydrofuranyl, such as hydrogen, methyl, or ethyl, or X is -O-, and A is a 5 or 6 membered alicyclic ring systems.

In some embodiments of Formula (I), R₃, may be absent, indicating the carbon atoms adjacent X are unsubstituted, or R₃ is present one or two time, connected to the same or different carbon atom, and R₃ is C₁₋₄ alkyl. In some embodiments R₃ is methyl.

In some embodiments of Formula (I), R₃ is methyl, and A is an unsubstituted or substituted morpholine or unsubstituted or substituted morpholine derivative, Y is -N- or - C(R₇)-, wherein R₇ is hydrogen, X is -CH₂-, or X is -N(R₆)-, wherein R₆ is selected from the group consisting of hydrogen, methyl, ethyl, n-propyl, isopropyl, and tetrahydrofuranyl, such as hydrogen, methyl, or ethyl, or X is -O-.

In some embodiments of Formula (I), R₃ is absent, and A is an unsubstituted or substituted morpholine or unsubstituted or substituted morpholine derivative, Y is -N- or - C(R₇)-, wherein R₇ is hydrogen, X is -CH₂-, or X is -N(R₆)-, wherein R₆ is selected from the group consisting of hydrogen, methyl, ethyl, n-propyl, isopropyl, and tetrahydrofuranyl, such as hydrogen, methyl, or ethyl, or X is -O-.

In some embodiments of Formula (I), A is selected from -NR₈R₉, wherein R₈ and R₉, independently are selected from the group consisting of methyl, ethyl, propyl, cyclopropyl, and cyclopropylmethyl. In some embodiments R₈ and R₉, independently are methyl or cyclopropylmethyl.

In some embodiments of Formula (I), A is selected from -NR₈R₉, wherein R₈ and R₉, independently are selected from the group consisting of methyl, ethyl, propyl, cyclopropyl, and cyclopropylmethyl, provided at least one of R₈ and R₉ is selected from the group consisting of methyl, ethyl, propyl, cyclopropyl, and cyclopropylmethyl, and X is - O-, and Y is -N-.

In some embodiments the compounds, or pharmaceutically acceptable salts, polymorphs, stereoisomers, and tautomers of Formula (I) relate to R₁ being selected from the group consisting of hydrogen, hydroxy, halogen, unsubstituted or substituted C₁₋₄ alkyl, unsubstituted or substituted C₁₋₄ alkoxy, and unsubstituted or substituted -O-R₁₀, wherein R₁₀ is selected from C₃₋₆ cycloalkyl, 4-6 membered heteroalicyclyl, and C₁₋₄ hydroxyalkyl;

In some embodiments of Formula (I), R₂ is selected from the group consisting of hydrogen, hydroxy, halogen, unsubstituted or substituted C₁₋₄ alkyl, and unsubstituted or substituted C₁₋₄ alkoxy; provided that R₁ and R₂ cannot be simultaneously selected from hydrogen;

In some embodiments the compounds, or pharmaceutically acceptable salts, polymorphs, stereoisomers, and tautomers of Formula (I) relate R₁, R₂ and the carbon atom to which they are attached are taken together to form a ring. Particular examples of rings are heteroalicyclic rings, such as 5 and 6 membered rings comprising one or more heteroatoms, such as one ore more oxygen atoms. Some examples are:

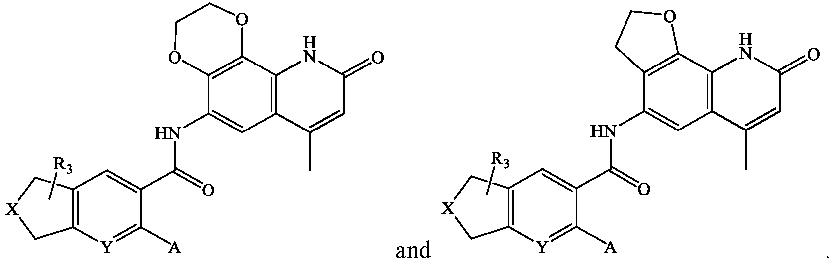

In some embodiments of Formula (I), R₁ is selected from the group consisting of hydrogen, hydroxy, fluoro, methyl, ethyl, propyl, isopropyl, butyl, methoxy, ethoxy, propoxy, butoxy, -O-azetidinyl, -O-CH₂CH₂OH, -O-CH₂CH₂NHCH₃.

In some embodiments of Formula (I), R₁ is selected from the group consisting of methyl, methoxy, and ethoxy. In many embodiments R₁ is methoxy.

In some embodiments of Formula (I), R₂ is selected from the group consisting of hydrogen, fluoro, methyl, ethyl, propyl, isopropyl, butyl, methoxy, ethoxy, propoxy, and butoxy. In many embodiments R₂ is hydrogen.

In some embodiments of Formula (I), R₁ is methoxy and R₂ is hydrogen.

In some embodiments the compounds, or pharmaceutically acceptable salts, polymorphs, stereoisomers, and tautomers according to the following Formula (II): 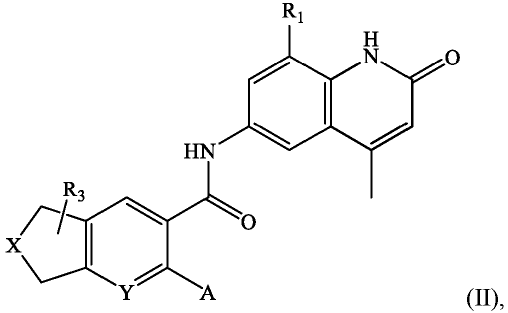 wherein R₁ is C₁₋₄ alkoxy, and X, Y, A and R₃ is a described hereinabove.

In some embodiments of Formula (II), A is selected from the group consisting of: 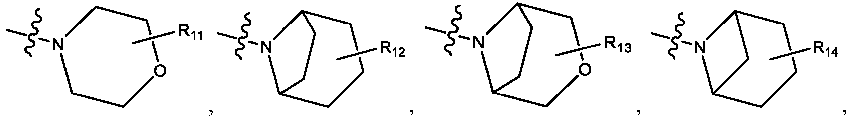 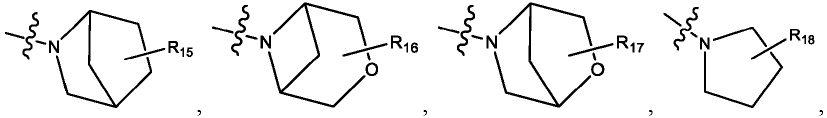 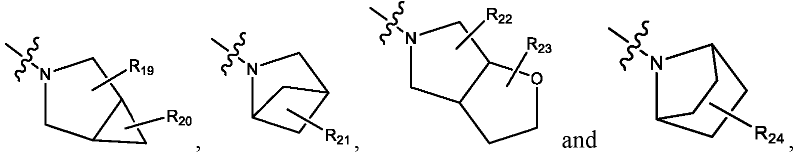 wherein R₁₁, R₁₂, R₁₃, R₁₄, R₁₅, R₁₆, R₁₇, R₁₈, R₁₉, R₂₀, R₂₁, R₂₂, R₂₃ and R₂₄ ndependently are absent or present 1, 2 or 3 times and when present selected from the group of halogen, hydroxy, C₁₋₄ alkyl, and C₁₋₄ alkoxy.

In some embodiments of Formula (II), A is selected from the group consisting of 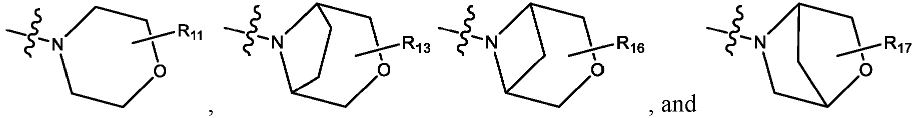 , wherein R₁₁, R₁₃, R₁₆, and R₁₇ are absent, or present 1 or 2 times, and when present independently selected from the group consisting of hydroxy, fluoro, methyl, ethyl, and propyl. In some embodiments of Formula (II), R₁₁, R₁₃, R₁₆, and R₁₇ are absent.

In some embodiments of Formula (II), A is selected from the group consisting of 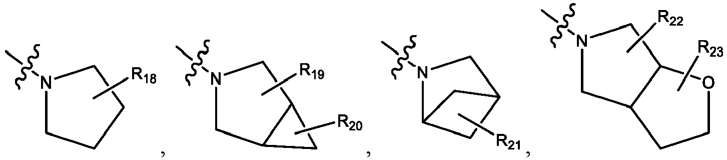 and 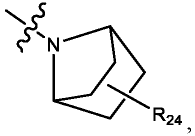 wherein R₁₈, R₁₉, R₂₀, R₂₁, R₂₂, R₂₃ and R₂₄ independently are absent, or present one or two time, and when present independently selected from the group consisting of hydroxy, fluoro, methyl, ethyl, and propyl. In some embodiments of Formula (II), R₁₈, R₁₉, R₂₀, R₂₁, R₂₂, R₂₃ and R₂₄ present 1 time and selected from the group consisting of hydroxy, fluoro, and methyl.

In some embodiments of Formula (II), X is -C(R₄R₅)-, wherein R₄ and R₅ independently are hydrogen or C₁₋₄ alkyl; or X is -N(R₆)-, wherein R₆ is selected from the group consisting of hydrogen, C₁₋₄ alkyl, C₁₋₄ haloalkyl, and 4 or 5 membered heteroaclicyclyl; or X is -O-.

In some embodiment of Formula (II), R₃ is absent or methyl.

In some embodiments of Formula (II), R₁ is methoxy and R₂ is hydrogen, and R₃ is absent or methyl.

In some embodiments the compounds, or pharmaceutically acceptable salts, polymorphs, stereoisomers, and tautomers according to the following Formula (III): 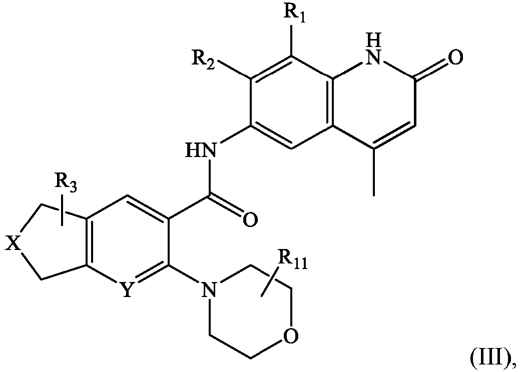 wherein R₁ is C₁₋₄ alkoxy; R₂ is hydrogen; R₃ is absent or present 1 time and when present selected from methyl; R₁₁ is absent; Y is -N- or -CH-; X is -O- or N(R₆), wherein R₆ is selected from the group consisting of hydrogen, methyl, ethyl, n-propyl, isopropyl, and oxetanyl.

In some embodiments of Formula (III), R₁ is methoxy; R₃ is absent; Y is -N- and X is -O-.

In other embodiments of Formula (III), R₁ is methoxy; R₃ is absent; Y is -CH- and X is -O-.

In some embodiments the compounds, or pharmaceutically acceptable salts, polymorphs, stereoisomers, and tautomers according of Formula (I) is selected from N-(8-methoxy-4-methyl-2-oxo-1H-quinolin-6-yl)-2-morpholino-5,7-dihydrofuro[3,4-b]pyridine-3-carboxamide,
N-(8-methoxy-4-methyl-2-oxo-1H-quinolin-6-yl)-2-morpholino-6,7-dihydro-5H-cyclopenta[b]pyridine-3-carboxamide,
N-(8-methoxy-4-methyl-2-oxo-1H-quinolin-6-yl)-5-methyl-2-morpholino-5,7-dihydrofuro[3,4-b]pyridine-3-carboxamide,
N-(8-methoxy-4-methyl-2-oxo-1H-quinolin-6-yl)-2-morpholino-6,7-dihydro-5H-pyrrolo[3,4-b]pyridine-3-carboxamide,
(5R)-N-(8-methoxy-4-methyl-2-oxo-1H-quinolin-6-yl)-5-methyl-2-morpholino-5,7-dihydrofuro[3,4-b]pyridine-3-carboxamide,
N-(8-methoxy-4-methyl-2-oxo-1H-quinolin-6-yl)-2-(3-oxa-8-azabicyclo[3.2.1]octan-8-yl)-5,7-dihydrofuro[3,4-b]pyridine-3-carboxamide,
N-(8-methoxy-4-methyl-2-oxo-1H-quinolin-6-yl)-2-[(1S,4S)-2-oxa-5-azabicyclo[2.2.1]heptan-5-yl]-5,7-dihydrofuro[3,4-b]pyridine-3-carboxamide,
N-(4,8-dimethyl-2-oxo-1H-quinolin-6-yl)-2-morpholino-5,7-dihydrofuro[3,4-b]pyridine-3-carboxamide,
N-(6-methyl-8-oxo-3,9-dihydro-2H-furo[3,2-h]quinolin-4-yl)-2-morpholino-5,7-dihydrofuro[3,4-b]pyridine-3-carboxamide,
N-[8-(2-hydroxyethoxy)-4-methyl-2-oxo-1H-quinolin-6-yl]-2-morpholino-5,7-dihydrofuro[3,4-b]pyridine-3-carboxamide,
N-[8-(azetidin-3-yloxy)-4-methyl-2-oxo-1H-quinolin-6-yl]-2-morpholino-5,7-dihydrofuro[3,4-b]pyridine-3-carboxamide,
N-(7-fluoro-8-methoxy-4-methyl-2-oxo-1H-quinolin-6-yl)-2-morpholino-5,7-dihydrofuro[3,4-b]pyridine-3-carboxamide,
N-(7-methyl-9-oxo-3,10-dihydro-2H-[1,4]dioxino[2,3-h]quinolin-5-yl)-2-morpholino-5,7-dihydrofuro[3,4-b]pyridine-3-carboxamide,
N-(4,7-dimethyl-2-oxo-1H-quinolin-6-yl)-2-morpholino-5,7-dihydrofuro[3,4-b]pyridine-3-carboxamide,
N-(8-methoxy-4-methyl-2-oxo-1H-quinolin-6-yl)-2-[(1R,4R)-2-oxa-5-azabicyclo[2.2.1]heptan-5-yl]-5,7-dihydrofuro[3,4-b]pyridine-3-carboxamide,
N-(8-ethoxy-4-methyl-2-oxo-1H-quinolin-6-yl)-2-morpholino-5,7-dihydrofuro[3,4-b]pyridine-3-carboxamide,
N-[4-methyl-8-[2-(methylamino)ethoxy]-2-oxo-1H-quinolin-6-yl]-2-morpholino-5,7-dihydrofuro[3,4-b]pyridine-3-carboxamide,
2-morpholino-N-(4,7,8-trimethyl-2-oxo-1H-quinolin-6-yl)-5,7-dihydrofuro[3,4-b]pyridine-3-carboxamide,
N-(7-methoxy-4-methyl-2-oxo-1H-quinolin-6-yl)-2-morpholino-5,7-dihydrofuro[3,4-b]pyridine-3-carboxamide,
N-(8-methoxy-4-methyl-2-oxo-1H-quinolin-6-yl)-6-methyl-2-morpholino-5,7-dihydropyrrolo[3,4-b]pyridine-3-carboxamide,
N-(8-methoxy-4-methyl-2-oxo-1H-quinolin-6-yl)-2-morpholino-6-(oxetan-3-yl)-5,7-dihydropyrrolo[3,4-b]pyridine-3-carboxamide,
6-ethyl-N-(8-methoxy-4-methyl-2-oxo-1H-quinolin-6-yl)-2-morpholino-5,7-dihydropyrrolo[3,4-b]pyridine-3-carboxamide,
6-isopropyl-N-(8-methoxy-4-methyl-2-oxo-1H-quinolin-6-yl)-2-morpholino-5,7-dihydropyrrolo[3,4-b]pyridine-3-carboxamide,
2-(dimethylamino)-N-(8-methoxy-4-methyl-2-oxo-1H-quinolin-6-yl)-5,7-dihydrofuro[3,4-b]pyridine-3-carboxamide,
2-[cyclopropylmethyl(methyl)amino]-N-(8-methoxy-4-methyl-2-oxo-1H-quinolin-6-yl)-5,7-dihydrofuro[3,4-b]pyridine-3-carboxamide,
2-(diethylamino)-N-(8-methoxy-4-methyl-2-oxo-1H-quinolin-6-yl)-5,7-dihydrofuro[3,4-b]pyridine-3-carboxamide,
2-[ethyl(isopropyl)amino]-N-(8-methoxy-4-methyl-2-oxo-1H-quinolin-6-yl)-5,7-dihydrofuro[3,4-b]pyridine-3-carboxamide,
2-(3,6-dihydro-2H-pyran-4-yl)-N-(8-methoxy-4-methyl-2-oxo-1H-quinolin-6-yl)-5,7-dihydrofuro[3,4-b]pyridine-3-carboxamide,
2-(cyclopenten-1-yl)-N-(8-methoxy-4-methyl-2-oxo-1H-quinolin-6-yl)-5,7-dihydrofuro[3,4-b]pyridine-3-carboxamide,
2-(3,3-difluoropyrrolidin-1-yl)-N-(8-methoxy-4-methyl-2-oxo-1H-quinolin-6-yl)-5,7-dihydrofuro[3,4-b]pyridine-3-carboxamide,
N-(8-methoxy-4-methyl-2-oxo-1H-quinolin-6-yl)-2-[(2R)-2-methylpyrrolidin-1-yl]-5,7-dihydrofuro[3,4-b]pyridine-3-carboxamide,
2-(2,3,3a,4,6,6a-hexahydrofuro[2,3-c]pyrrol-5-yl)-N-(8-methoxy-4-methyl-2-oxo-1H-quinolin-6-yl)-5,7-dihydrofuro[3,4-b]pyridine-3-carboxamide,
N-(8-methoxy-4-methyl-2-oxo-1H-quinolin-6-yl)-2-(1-methyl-2-azabicyclo[2.1.1]hexan-2-yl)-5,7-dihydrofuro[3,4-b]pyridine-3-carboxamide,
2-(1,3,3a,4,6,6a-hexahydrofuro[3,4-c]pyrrol-5-yl)-N-(8-methoxy-4-methyl-2-oxo-1H-quinolin-6-yl)-5,7-dihydrofuro[3,4-b]pyridine-3-carboxamide,
2-(8-azabicyclo[3.2.1]octan-8-yl)-N-(8-methoxy-4-methyl-2-oxo-1H-quinolin-6-yl)-5,7-dihydrofuro[3,4-b]pyridine-3-carboxamide,
N-(8-methoxy-4-methyl-2-oxo-1H-quinolin-6-yl)-2-[rac-(3aR,6aR)-2,3,3a,4,6,6a-hexahydrofuro[2,3-c]pyrrol-5-yl]-5,7-dihydrofuro[3,4-b]pyridine-3-carboxamide,
N-(8-methoxy-4-methyl-2-oxo-1H-quinolin-6-yl)-2-[(2S)-2-methylpyrrolidin-1-yl]-5,7-dihydrofuro[3,4-b]pyridine-3-carboxamide,
2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide,
2-[(3R)-3-fluoropyrrolidin-1-yl]-N-(8-methoxy-4-methyl-2-oxo-1H-quinolin-6-yl)-5,7-dihydrofuro[3,4-b]pyridine-3-carboxamide,
2-[(3 S)-3-fluoropyrrolidin-1-yl]-N-(8-methoxy-4-methyl-2-oxo-1H-quinolin-6-yl)-5,7-dihydrofuro[3,4-b]pyridine-3-carboxamide,
N-(8-methoxy-4-methyl-2-oxo-1H-quinolin-6-yl)-2-[rac-(3R,4S)-3,4-difluoropyrrolidin-1-yl]-5,7-dihydrofuro[3,4-b]pyridine-3-carboxamide,
N-(8-methoxy-4-methyl-2-oxo-1H-quinolin-6-yl)-2-[rac-(3S,4S)-3,4-difluoropyrrolidin-1-yl]-5,7-dihydrofuro[3,4-b]pyridine-3-carboxamide, and
2-(6,6-difluoro-3-azabicyclo[3.1.0]hexan-3-yl)-N-(8-methoxy-4-methyl-2-oxo-1H-quinolin-6-yl)-5,7-dihydrofuro[3,4-b]pyridine-3-carboxamide.

In another aspect the compounds of Formula (I), and pharmaceutically acceptable salts, polymorphs, stereoisomers, and tautomers thereof are active bromodomain inhibitors, without inhibiting the activity of phosphodiesterase enzyme 3 (PDE3).

In aspect the compounds of Formula (I), and pharmaceutically acceptable salts, polymorphs, stereoisomers, and tautomers thereof and at least one pharmaceutically acceptable excipitent are comprised in a pharmaceutical composition.

In another aspect the compounds of Formula (I), and pharmaceutically acceptable salts, polymorphs, stereoisomers, and tautomers thereof, for use in treating diseases or conditions systemic or tissue inflammation, inflammatory responses to infection or hypoxia, cellular activation and proliferation, metabolism, fibrosis and in the prevention and treatment of viral infections

In another aspect the compounds of Formulae (I), (II), (III) and pharmaceutically acceptable salts, polymorphs, stereoisomers, and tautomers thereof, are comprised in a composition for use in treating diseases or conditions such as rheumatoid arthritis, osteoarthritis, gout, psoriasis, psoriatric arthritis, systemic lupus erythematosus, multiple sclerosis, inflammatory bowel disease, inflammatory bowel syndrome, Crohn's disease, ulcerative colitis, colitis, asthma, chronic obstructive airways disease, pneumonitis, myocarditis, pericarditis, myositis, eczema, dermatitis, atopic dermatitis, allergy, ankylosing spondylitis, lupus erythematosus, Hashimoto's disease, pancreatitis, autoimmune ocular disease, Sjögren's disease, optic neuritis, neuromyelitis optica, Myasthenia Gravis, Guillain Barre syndrome, Graves' disease, alopecia, vitiligo, bullous skin diseases, nephritis, vasculitis, atherosclerosis, Alzheimer's disease, depression, retinitis, uveitis, scleritis, hepatitis, primary biliary cirrhosis, sclerosing cholangitis, hypophysitis, thyroiditis, Addison's disease, type I diabetes and acute rejection of transplanted organs; or
treating diseases or conditions such as acute gout, giant cell arteritis, nephritis including lupus nephritis, vasculitis with organ involvement such as glomerulonephritis, vasculitis including giant cell arteritis, Polyarteritis nodosa, Behcet's disease, Wegener's granulomatosis, Kawasaki disease, Takayasu's Arteritis, vasculitis with organ involvement and acute rejection of transplanted organs; or
treating sepsis, sepsis syndrome, septic shock, endotoxaemia, systemic inflammatory response syndrome (SIRS), multi-organ dysfunction syndrome, toxic shock syndrome, acute lung injury, ARDS (adult respiratory distress syndrome), acute renal failure, fulminant hepatitis, burns, acute pancreatitis, post-surgical syndromes, sarcoidosis, Herxheimer reactions, encephalitis, myelitis, meningitis, malaria and SIRS associated with viral infections such as influenza, herpes zoster, herpes simplex and coronavirus; or
treating ischaemia-reperfusion injury,myocardial infarction, cerebrovascular ischaemia (stroke), acute coronary syndromes, renal reperfusion injury, organ transplantation, coronary artery bypass grafting, cardio-pulmonary bypass procedures, pulmonary, renal, hepatic, gastro-intestinal or peripheral limb embolism; or
treating disorders or conditions such as hypercholesterolemia, atherosclerosis and Alzheimer's disease; or
treating disorders or conditions such as idiopathic pulmonary fibrosis, lung fibrosis, intestinal fibrosis, liver fibrosis, non-alcoholic steatohepatitis, cirrhosis, renal fibrosis, post-operative stricture, myelofibrosis, keloid formation, skin fibrosis, hand fibrosis, systemic sclerosis, scleroderma and cardiac fibrosis; or
treating or preventing viral infections such as herpes virus, human papilloma virus, human immunodeficiency virus (HIV), adenovirus and poxvirus; or
treating colon carcinomas, midline carcinomas, sarcomas, mesenchymal, hepatic, renal and neurological tumours; acute lymphoblastic leukemia, acute myelogenous leukemia, adult T-cell leukemia/lymphoma, bladder cancer, blastoma, bone cancer, breast cancer, brain cancer, burkitts lymphoma, carcinoma, myeloid sarcoma, cervical cancer, chronic lymphocytic leukemia, chronic myelogenous leukemia, colorectal cancer, diffuse large B-cell lymphoma, endometrial cancer, esophageal cancer, follicular lymphoma, gastrointestinal cancer, glioblastoma multiforme, glioma, gallbladder cancer, gastric cancer, head and neck cancer, Hodgkin's lymphoma, non-Hodgkin's lymphoma, intestinal cancer, kidney cancer, laryngeal cancer, leukemia, lung cancer, lymphoma, liver cancer, small cell lung cancer, non-small cell lung cancer, melanoma, mesothelioma, multiple myeloma, ocular cancer, optic nerve tumor, oral cancer, ovarian cancer, pituitary tumor, primary central nervous system lymphoma, prostate cancer, pancreatic cancer, pharyngeal cancer, renal cell carcinoma, rectal cancer, sarcoma, skin cancer, spinal tumor, small intestine cancer, stomach cancer, T-cell lymphoma, testicular cancer, thyroid cancer, throat cancer, urogenital cancer, urothelial carcinoma, uterine cancer, vaginal cancer, or Wilms' tumor; or
treating obesity, such as obesity associated with cancer treatment or obesity associated with diabetes and cardiac hypertrophy.

Additional examples of diseases, disorders or conditions include obesity, such as obesity associated with cancer treatment or obesity associated with diabetes and cardiac hypertrophy.

### Methods of Administration

The compounds or pharmaceutical compositions may be administered to the patient by any suitable means. Non-limiting examples of methods of administration include, among others, (a) administration though oral pathways, which administration includes administration in capsule, tablet, granule, spray, syrup, or other such forms; (b) administration through non-oral pathways such as rectal, vaginal, intraurethral, intraocular, intranasal, or intraauricular, which administration includes administration as an aqueous suspension, an oily preparation or the like or as a drip, spray, suppository, salve, ointment or the like; (c) administration via injection, subcutaneously, intraperitoneally, intravenously, intramuscularly, intradermally, intraorbitally, intracapsularly, intraspinally, intrasternally, or the like, including infusion pump delivery; (d) administration locally such as by injection directly in the renal or cardiac area, e.g., by depot implantation by intratumoral injection, or by intra-lymph node injection; as well as (e) administration topically; as deemed appropriate by those of skill in the art for bringing the compound disclosed herein into contact with living tissue.

Pharmaceutical compositions suitable for administration include compositions where the active ingredients are contained in an amount effective to achieve its intended purpose. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize. More specifically, a therapeutically effective amount means an amount of compound effective to treat, alleviate or ameliorate symptoms of disease or prolong the survival of the subject being treated. Determination of a therapeutically effective amount is well within the capability of those skilled in the art, especially in light of the detailed disclosure provided herein.

As will be readily apparent to one skilled in the art, the useful *in vivo* dosage to be administered and the particular mode of administration will vary depending upon the age, weight and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

In non-human animal studies, applications of potential products are commenced at higher dosage levels, with dosage being decreased until the desired effect is no longer achieved or adverse side effects disappear. The dosage administered to a human or non-human subject may range broadly, depending upon the desired effects and the therapeutic indication. Typically, dosages may be between about 10 microgram/kg and 1000 mg/kg body weight, preferably between about 100 microgram/kg and 10 mg/kg body weight. Alternatively dosages may be based and calculated upon the surface area of the patient, as understood by those of skill in the art.

The exact formulation, route of administration and dosage for the pharmaceutical compositions disclosed herein can be chosen by the individual physician in view of the patient's condition. (See *e.g.,* Fingl et al. 1975, in "The Pharmacological Basis of Therapeutics", with particular reference to Ch. 1, p. 1). Typically, the dose range of the composition administered to the patient can be from about 0.5 to 1000 mg/kg of the patient's body weight. The dosage may be a single one or a series of two or more given in the course of one or more days, as is needed by the patient. In instances where human dosages for compounds have been established for at least some condition, those same dosages may be used, or dosages that are between about 0.1% and 500%, more preferably between about 25% and 250% of the established human dosage. Where no human dosage is established, as will be the case for newly-discovered pharmaceutical compounds, a suitable human dosage can be inferred from ED₅₀ or ID₅₀ values, or other appropriate values derived from *in vitro* or *in vivo* studies, as qualified by toxicity studies and efficacy studies in animals.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Although the exact dosage will be determined on a drug-by-drug basis, in most cases, some generalizations regarding the dosage can be made. The daily dosage regimen for an adult human patient may be, for example, an oral dose of between 0.1 mg and 2000 mg of each active ingredient, preferably between 1 mg and 500 mg, e.g. 5 to 200 mg. An ocular eye drop may range in concentration between 0.005 and 5 percent. In one embodiment, an eye drop may range between 0.01 and 1 percent, or between 0.01 and 0.3 percent in another embodiment. In other embodiments, an intravenous, subcutaneous, or intramuscular dose of each active ingredient of between 0.01 mg and 100 mg, preferably between 0.1 mg and 60 mg, e.g. 1 to 40 mg is used. In cases of administration of a pharmaceutically acceptable salt, dosages may be calculated as the free base. In some embodiments, the composition is administered 1 to 4 times per day. Alternatively the compositions disclosed herein may be administered by continuous intravenous infusion, preferably at a dose of each active ingredient up to 1000 mg per day. As will be understood by those of skill in the art, in certain situations it may be necessary to administer the compounds disclosed herein in amounts that exceed, or even far exceed, the above-stated, preferred dosage range or frequency in order to effectively and aggressively treat particularly aggressive diseases or infections. In some embodiments, the compounds will be administered for a period of continuous therapy, for example for a week or more, or for months or years.

Dosage amount and interval may be adjusted individually to provide plasma or tissue levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays or bioassays can be used to determine plasma concentrations.

Dosage intervals can also be determined using MEC value. Compositions should be administered using a regimen which maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered may be dependent on the subject being treated, on the subject's weight, the severity of the affliction, the manner of administration and the judgment of the prescribing physician.

Compounds disclosed herein can be evaluated for efficacy and toxicity using known methods. For example, the toxicology of a particular compound, or of a subset of the compounds, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular compound may be established using several recognized methods, such as *in vitro* methods, animal models, or human clinical trials. Recognized *in vitro* models exist for nearly every class of condition, including but not limited to cancer, cardiovascular disease, and various immune dysfunction. Similarly, acceptable animal models may be used to establish efficacy of chemicals to treat such conditions. When selecting a model to determine efficacy, the skilled artisan can be guided by the state of the art to choose an appropriate model, dose, and route of administration, and regime. Of course, human clinical trials can also be used to determine the efficacy of a compound in humans.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions comprising a compound disclosed herein formulated in a compatible pharmaceutical carrier may also be prepared, placed in an appropriate container, and labeled for treatment of an indicated condition.

### General remarks

As described above with reference to specific illustrative embodiments, it is not intended to be limited to the specific form set forth herein. Any combination of the above mentioned embodiments should be appreciated as being within the scope of the disclosure. Rather, the disclosure is limited only by the accompanying claims and other embodiments than the specific above are equally possible within the scope of these appended claims.

In the claims, the term "comprises/comprising" does not exclude the presence of other species or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc. do not preclude a plurality.

The phrases "at least one" and "one or more" refer to 1 or a number greater than 1, such as to 1, 2, 3, 4, 5, 6, 7, 8, 9, or 10.

### Experimental

The following examples are mere examples and should by no means be interpreted to limit the scope of the disclosure. Rather, the disclosure is limited only by the accompanying claims.

The compounds described below have, unless specifically stated, been prepared using commercially available starting materials. The following is a non-comprehensive list of starting materials used for the synthesis of compounds prepared herein.

As can be seen in the table below, the compounds were found to possess beneficial activity. All reagents were of commercial grade and were used as received without further purification, unless otherwise stated. Commercially available anhydrous solvents were used for reactions conducted under inert atmosphere. Reagent grade solvents were used in all other cases, unless otherwise specified.

| **IUPAC-NAME** | **SUPPLIER** |
|---|---|
| acetaldehyde | Sigma-Aldrich |
| diazomethyl(trimethyl)silane | Sigma-Aldrich |
| morpholine | Sigma-Aldrich |
| formaldehyde | Sigma-Aldrich |
| N-ethylethanamine | Sigma-Aldrich |
| 2-(benzotriazol-1-yl)acetic acid | Enamine-BB |
| 1-cyclopropyl-N-methyl-methanamine | Enamine-BB |
| N-methylmethanamine | Sigma-Aldrich |
| N-ethylpropan-2-amine | Enamine-BB |
| 8-azabicyclo[3.2.1]octane | Fluorochem |
| acetone | Sigma-Aldrich |
| 3-oxa-8-azabicyclo[3.2.1]octane | Combi-Blocks |
| 6-amino-4,7-dimethyl-1H-quinolin-2-one | Enamine-BB |
| 3,3-difluoropyrrolidine | Enamine-BB |
| oxetan-3-one | Combi-Blocks |
| 3,3a,4,5,6,6a-hexahydro-1H-furo[3,4-c]pyrrole | Combi-Blocks |
| 7-azabicyclo[2.2.1]heptane | Combi-Blocks |
| 6,6-difluoro-3-azabicyclo[3.1.0]hexane | Enamine-BB |
| (2R)-2-methylpyrrolidine | Sigma-Aldrich |
| 2-(3,6-dihydro-2H-pyran-4-yl)-4,4,5,5-tetramethyl-1,3,2-dioxaborolane | Sigma-Aldrich |
| 2-bromoethanol | Sigma-Aldrich |
| 4-amino-6-methyl-3,9-dihydro-2H-furo[3,2-h]quinolin-8-one | Sundia |
| tert-butyl 3-bromoazetidine-1-carboxylate | Combi-Blocks |
| 3-bromo-6,7-dihydro-SH-pyrrolo[3,4-b]pyridine | Astatech |
| 2-chloro-6,7-dihydro-5H-cyclopenta[b]pyridine-3-carboxylic acid | Chembridge |
| 5-bromo-2-(bromomethyl)pyrimidine | Fluorochem |
| 2-(cyclopenten-1-yl)-4,4,5,5-tetramethyl-1,3,2-dioxaborolane | Fluorochem |
| 2-(2-aminophenyl)ethanol | Fluorochem |
| 3,3a,4,5,6,6a-hexahydro-2H-furo[2,3-c]pyrrole | Fluorochem |
| 1-methyl-2-azabicyclo[2.1.1]hexane | Enamine-BB |
| rac-(3aR,6aR)-3,3a,4,5,6,6a-hexahydro-2H-furo[2,3-c]pyrrole | Enamine-BB |
| (1S,4S)-2-oxa-5-azabicyclo[2.2.1]heptane | Enamine-BB |
| (1R,4R)-2-oxa-5-azabicyclo[2.2.1]heptane | Combi-Blocks |
| (2S)-2-methylpyrrolidine | Combi-Blocks |
| tert-butyl N-(2-chloroethyl)-N-methyl-carbamate | Combi-Blocks |
| (3 S)-3-fluoropyrrolidine | Combi-Blocks |
| (3R)-3-fluoropyrrolidine | Combi-Blocks Advanced |
| (3R,4S)-3,4-difluoropyrrolidine | ChemBlocks Advanced |
| (3R,4R)-3,4-difluoropyrrolidine | ChemBlocks |

Detailed description of the preparation of individual compounds according to formula (I) are described herein below.

Compound names were generated using Accelrys Draw 4.2

Compounds were characterized using LC-MS analysis and/or 1H-Nuclear Magnetic Resonance (NMR) and were in all cases consistent with the proposed structures.

Unless otherwise stated, compounds are analysed and purified using following systems:
Analytical:
   Waters Acquity system equipped with a Acquity BEH C18 (1.7µm, 2.1 x 50 mm) with a linear gradient of the binary solvent system water/acetonitrile/formic acid (A: 100/0.1% and B: 0/100/0.1%) with a flow rate of 0.5 mL/min and DAD at ambient temperature, combined with MS detection SQD I.
Preparative:
   Preparative HPLC was performed on a Waters Acquity system using a C18 reverse phase column (Supelco DISCOVERY C18, 25 cm x 21.2 mm), with a linear gradient of the binary solvent system water/acetonitrile/formic acid (A: 100/0/0.1% and B: 0/100/0.1%) with a flow rate of 45 mL/min and UV detection at 254 nm, combined with MS detecting on a Waters Micromass ZQ Quadrupole MS.

Chemical shifts relating to NMR are reported in parts-per-million (ppm) and referenced from non-deuterated solvent residues. Conventional abbreviations for designation of major peaks were used, e.g. s, singlet; d, doublet; t, triplet; q, quartet; m, multiplet; br, broad. Abbreviations for common solvents are: Chloroform-d or CDCl3, deuterochloroform; DMSO-d6, hexadeuterodimethylsulfoxide; CD3COOD, deuteroacetic acid; and CD3OD, deuteromethanol.

### General Synthesis

The compounds described herein, including compounds of general formula (I) and specific examples, may be prepared, for example, through the reaction routes depicted in schemes 1-7. By way of example, the variables R1, R2, R3, R4, X, A etc. used in the following schemes have the meanings as set forth in the claims, summary and detailed description sections unless otherwise noted

Abbreviations used in the present disclosure have the following meanings:

| | |
|---|---|
| AcOH | Acetic acid |
| BrettPhos | dicyclohexyl-[3,6-dimethoxy-2-(2,4,6-triisopropylphenyl)phenyl]phosphane |
| Cbz | benzyloxycarbonyl |
| Cs₂CO₃ | Cesium carbonate, 99% |
| DCM | Methylene chloride, dichloromethane |
| DIC | (3-Dimethylamino-propyl)-ethyl-carbodiimide |
| DIPEA | N,N-Diisopropylethylamine |
| DIEA | N,N-Diisopropylethylamine |
| DMAP | 4-Dimethylaminopyridine |
| DMF | N,N-dimethylformamide |
| DMSO | Dimethylsulfoxide |
| Dppf | Diphenylphosphinoferrocene |
| EDC | (3-Dimethylamino-propyl)-ethyl-carbodiimide |
| EtOAc | Ethyl acetate |
| EtOH | Ethanol |
| Fe | Iron |
| H₂ | Hydrogen |
| H₂SO₄ | Sulfuric acid |
| HCl | Hydrochloric acid |
| HEPES | 4-(2-Hydroxyethyl)piperazine-1-ethanesulfonic acid, N-(2-Hydroxyethyl)piperazine-N'-(2-ethanesulfonic acid) |
| HOAt | [1,2,3]Triazolo[4,5-b]pyridin-3-ol |
| HOBt | 1-Hydroxy-benzotriazole |
| K₂CO₃ | Potassium carbonate |
| LCMS [M+H]⁺ | Liquid Chromatography - Mass spectrometry |
| LiI | Lithium Iodide |
| LiOH | Lithium hydroxide |
| mAb | Monoclonal antibody |
| MeCN | Acetonitrile |
| MeOH | Methanol |
| MgSO₄ | Magnesium sulfate |
| MsCl | Methanesulfonyl chloride |
| Na₂CO₃ | Sodium Carbonate |
| Na₂SO₄ | Sodium Sulfate, anhydrous |
| NaBH(OAc)₃ | Sodium triacetoxyborohydride |
| NaH | Sodium hydride |
| NaHCO₃ | sodium hydrogen carbonate |
| NaOH | Sodium hydroxide |
| NaOtBu | Sodium-tert-butylat |
| NH₃-aq | ammonium hydroxide |
| NH₄Cl | Ammonium chloride |
| NMP | 1-methylpyrrolidin-2-one |
| NT | Not Tested |
| Pd(OAc)₂ | Palladium acetate |
| Pd(PPh₃)₄ | Tetrakis(triphenylphosphine)palladium |
| Pd(dppf)Cl₂ | 1'-Bis(diphenylphosphino)ferrocene]dichloropalladium(II) |
| Pd/C | Palladium on activated charcoal |
| PtO₂ | Platinum(IV) oxide |
| Rt | room temperature |
| RuPhos | Dicyclohexyl-[2-(2,6-diisopropoxyphenyl)phenyl]phosphane |
| t-BuOH | Tert-Butanol |
| t-But-phos-pd(o) | Palladium(0) and tri-tert-butylphosphine. |
| TEA | Triethylamine |
| TFA | Trifluoroacetic acid |
| THF | Tetrahydrofuran |
| TMS | Trimethylsilyl |
| TPP | Triphenylphosphine |
| Zn(CN)₂ | zinc dicyanide |
| Xantphos | 4,5-Bis(diphenylphosphino)-9,9-dimethylxanthene |
| Xphos | 2-Dicyclohexylphosphino-2',4',6'-triisopropylbiphenyl |

### Synthesis of final compounds:

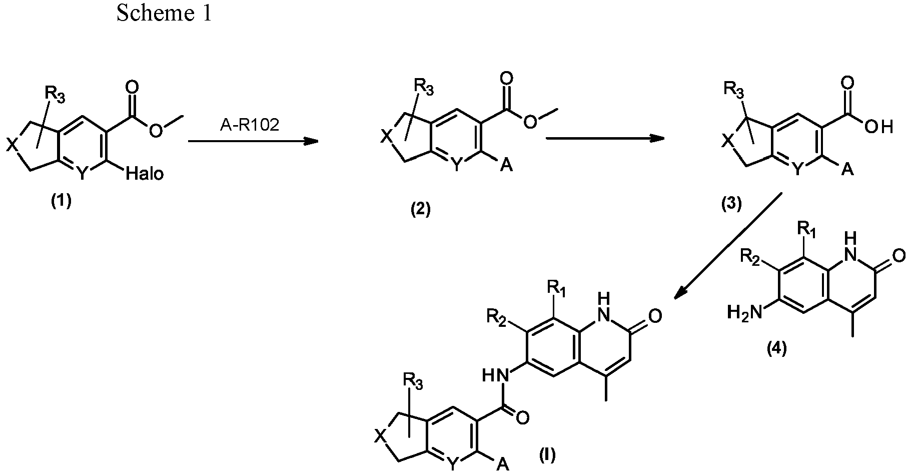

Compounds of formula (I) may be prepared using general synthetic route as shown in Scheme 1, and a specific example disclosed as Example 2 for preparation of 2-(3,6-dihydro-2H-pyran-4-yl)-N-(8-methoxy-4-methyl-2-oxo-1H-quinolin-6-yl)-5,7-dihydrofuro[3,4-b]pyridine-3-carboxamide.

Treatment of compounds of formula (1) wherein halo is (CI, Br, or I) and compounds of formula A-R102 wherein R102 is boronic acid or a derivative thereof (e.g., a pinacol ester), under Suzuki coupling conditions (N. Miyama and A. Suzuki, Chem.Rev. 1995, 95:2457-2483, J. Organomet. Chem. 1999, 576:147-148). For example, the coupling reaction may be conducted in the presence of a palladium catalyst and a base, and optionally in the presence of a ligand, and in a suitable solvent at elevated temperature (about 60 °C to about 150 °C). The reaction may be facilitated by microwave irradiation. Examples of the palladium catalyst include, but are not limited to, tetrakis(triphenylphosphine)palladium(0), tris(dibenzylideneacetone)dipalladium(0), bis(triphenylphosphine)palladium(II) dichloride, and palladium(II)acetate. Examples of suitable bases that may be employed include, but not limited to, carbonates or phosphates of sodium, potassium, and cesium, and cesium fluoride. Examples of suitable ligands include, but are not limited to, 1,3,5,7-tetramethyl-6-phenyl-2,4,8-trioxa-6-phosphaadamante, 2- dicyclohexylphosphino-2',4',6'-triisopropylbiphenyl (X-phos), and 1,1'- bis(diphenylphosphanyl) ferrocene. Non-limiting examples of suitable solvent include methanol, ethanol, dimethoxyethane, N,N -dimethylformamide, dimethylsulfoxide, dioxane, tetrahydrofuran, toluene, and water, or a mixture thereof.

Compounds of formula (2) may also be prepared by displacement of the halogen atom (F, Cl or Br) of the formula (1) with an appropriate amine as defined under the general structure of A. Displacement of the halogen atom may be accomplished in a solvent such as, but not limited to, dimethylsulfoxide, dimethylformamide, dioxane, or tetrahydrofuran and in the presence of a base such as, but not limited to, carbonate of cesium, potassium, or sodium, or sodium hydride, and at a temperature from about 20 °C to about 160 °C. The reaction may be facilitated by microwave irradiation.

Hydrolysis of esters (2) provides acids of formula (3), which are treated with amines of formula (4) in a solvent such as, but not limited to, tetrahydrofuran or N,N-dimethylformamide or dichloromethane at a temperature from about room temperature to about 70 °C, optionally in the presence of a base such as, but not limited to, triethylamine, N,N -diisopropylethylamine, or potassium carbonate, and optionally in the presence of a catalyst such as 4-dimethylaminopyridine and in the presence of a coupling reagent such as 1,1'-carbonyldiimidazole (CDI), bis(2-oxo-3-oxazolidinyl)phosphinic chloride (BOPCl), 1,3-dicyclohexylcarbodiimide (DCC), polymer supported 1,3-dicyclohexylcarbodiimide (PS-DCC), 0-(7-azabenzotriazol-1-yl)-N,N,N',N'-tetramethyluronium hexafluorophosphate (HATU), or O-benzotriazol-1-ylN,N,N ',N '-tetramethyluronium tetrafluoroborate (TBTU), in the presence or absence of a coupling auxiliary such as, but not limited to, 1-hydroxy-7-azabenzotriazole (HOAT) or 1-hydroxybenzotriazole hydrate (HOBT), to provide compounds of formula (I).

A specific example is N-(4,8-dimethyl-2-oxo-1H-quinolin-6-yl)-2-morpholino-5,7-dihydrofuro[3,4-b]pyridine-3-carboxamide (Example 7).

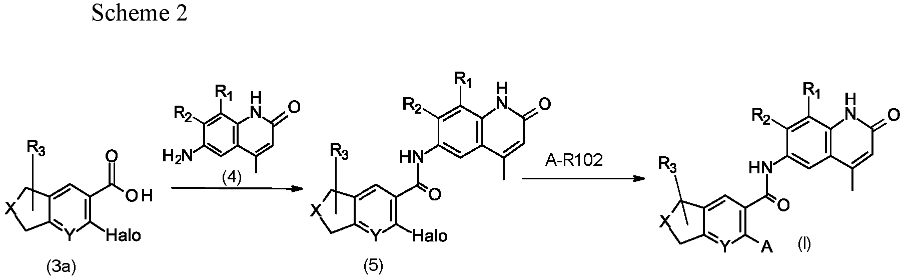

Alternatively, compounds of general formula (I) may be prepared as described in Scheme 2. Reaction of acids (3a) with amines (4) to provide compounds (5), followed by treatment of (5) wherein halo is F, Cl or Br, with compounds A-R102 may be achieved using reaction conditions as described in Scheme 1.

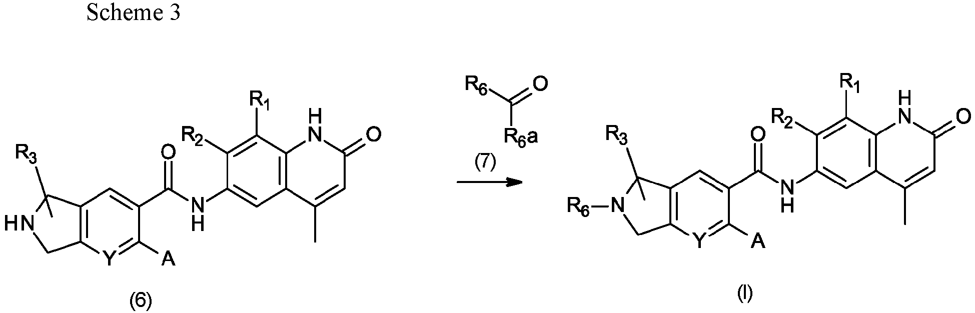

Compounds of general formula (I) wherein X is -N(R₆) may be prepared as described in Scheme 3 from compounds of formula (6) by reductive amination using appropriate aldehydes or ketones (7) in the presence of reducing agents such as, but not limited to, sodium cyanoborohydride, sodium borohydride, or sodium triacetoxyborohydride in solvents such as, but not limited to, tetrahydrofurane, 1,2-Dichloroethane. Acetic acid may be used as catalyst.

A specific example is N-(8-methoxy-4-methyl-2-oxo-1H-quinolin-6-yl)-6-methyl-2-morpholino-5,7-dihydropyrrolo[3,4-b]pyridine-3-carboxamide (Example 32)

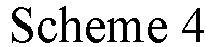

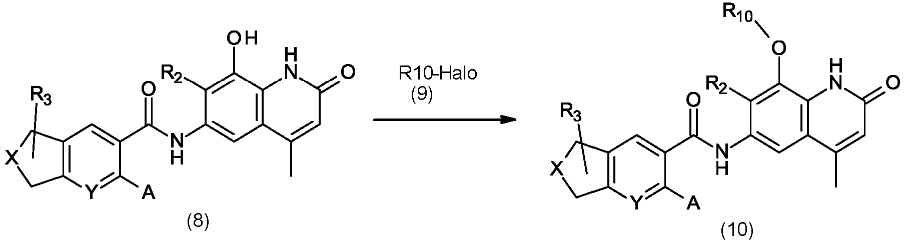

Compounds of general formula (I) wherein R1 is -OR10 may be prepared as described in Scheme 4 from compounds of formula (8) by alkylating -OH group using compounds of formula (9) wherein Halo is Br or Cl, in the presence of base such as, but not limited to, sodium hydride, cesium carbonate, or potassium carbonate, and in a solvent such as, but not limited to, N,N-dimethylformamide or dimethylsulfoxide at a temperature of about room temperature to about 120 °C.

A specific example is N-[8-(2-hydroxyethoxy)-4-methyl-2-oxo-1H-quinolin-6-yl]-2-morpholino-5,7-dihydrofuro[3,4-b]pyridine-3-carboxamide (Example 36)

### Synthesis of intermediates

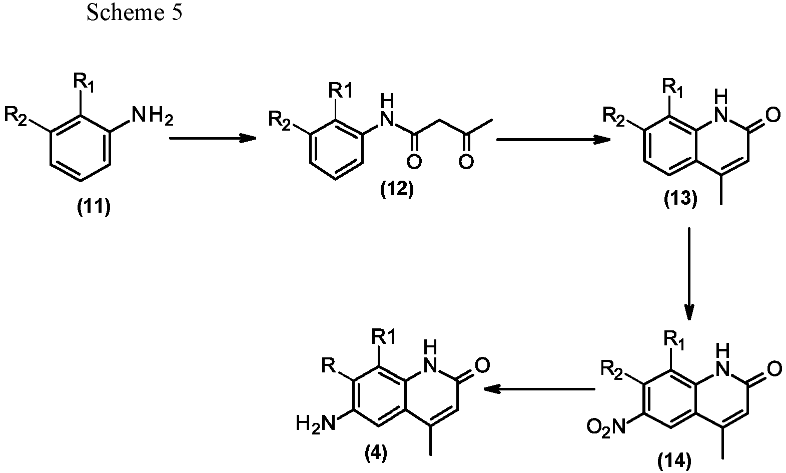

Compounds of formula (4) may be prepared using general synthetic route as shown in Scheme 5. Depending on R1 and R2, compounds of formula (12) may be prepared by treatment of compounds of formula (11) with, for example, methyl acetoacetate or 2,2,6-trimethyl-1,3-dioxin-4-one at elevated temperature (for example, at about 60 °C to about 140 °C), in the presence of a base such as pyridine, and in a solvent such as, but not limited to, toluene. Treatment of (12) with an acid such as, but not limited to, triflic acid or polyphosphoric acid and in the presence or absence of solvent such as, but not limited to, dichloromethane, at about room temperature to about 100°C, provides compounds of formula (13). Preparation of compounds of formula (13) may be achieved by nitration with nitric acid in acetic anhydride at a temperature of about 0°C to 50°. Reduction of the nitro group of compounds of formula (14) provides amines of formula (4) using reagents known to those skilled in the art, such as, but not limited to platinum on carbon, platinum (IV) oxide, Raney nickel, iron metal in acidic media or zinc.

A specific example is 6-amino-8-ethoxy-4-methyl-1H-quinolin-2-one

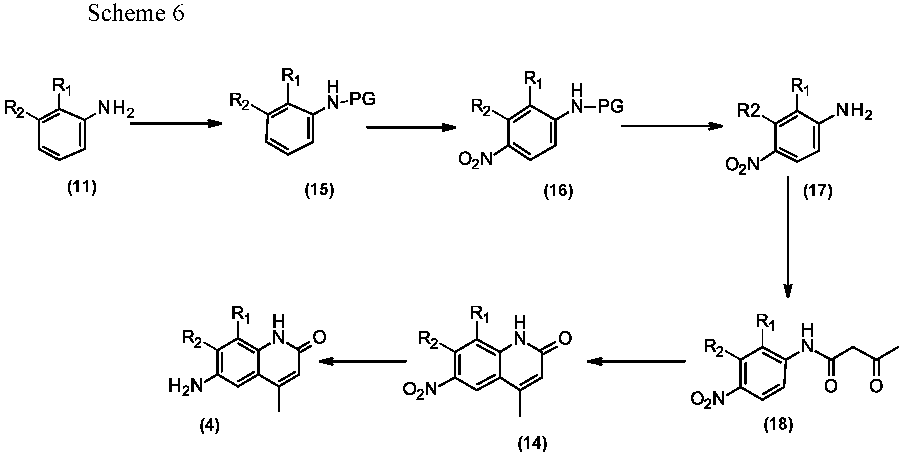

Alternatively, depending on R1 and R2, compounds of formula (4) may be prepared using general synthetic route as shown in Scheme 6 where amino group of compounds (11) is first protected by appropriate protecting group using reagents such as, but not limited to acetic anhydride or Boc anhydride. The protecting group is removed after nitration step described in the Scheme 5 using acid such as, but not limited to hydrochloric acid or trifluoroacetic acid in the presence or absence of solvent such as, but not limited to, dichloromethane.

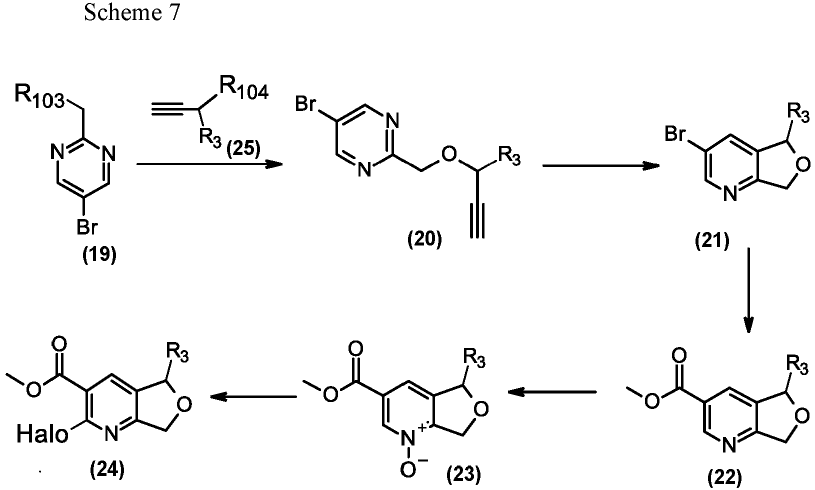

Compounds of formula (1) where R3 is present can be prepared as outlined in the scheme 7. Treatment of compounds of formula (19) wherein R103 is -OH with alkynyl derivatives (25) wherein (R104 is Br) in the presence of a base such as, but not limited to, NaH and in a solvent such as, but not limited to N,N-dimethylformamide or tetrahydrofuran, at a temperature from about -20°C to room temperature give compounds of formula (20). Treatment of (20) with nitrobenzene at a temperature from about 100°C to about 180°C gives compound (21). Insertion of carbonyl group into (21) give compounds of formula (22). It may be achieved by adding CO into the reaction and in general, the conversion may be facilitated by a palladium catalyst such as, but not limited to, tetrakis(triphenylphosphine)palladium(0), tris(dibenzylideneacetone)dipalladium(0), or palladium(II)acetate, an optional ligand such as, but not limited to, 2-dicyclohexyl-phosphino2',4',6'-triisopropylbiphenyl, 2-dicyclohexylphosphino-2',4',6'-triisopropyl-biphenyl (X-phos), or 4,5-Bis(diphenylphosphino)-9,9-dimethylxanthene (Xantphos), and a base such as, but not limited to, triethylamine, N,N -diisopropylethylamine, or potassium carbonate. Non-limiting examples of suitable solvents include methanol, N,N-dimethylformamide, dimethylsulfoxide, dioxane, tetrahydrofuran, or a mixture thereof. CO can be added directly to the reaction in the form of CO gas or can be prepared *in situ* from reagents such as, but not limited to formic acid and mesyl chloride. Compound (23) can be obtained from (22) by treatment with urea/hydrogen peroxide complex and trifluoroacetic anhydride in the solvent such as, but not limited to, dichloromethane, acetonitrile or a mixture thereof, at temperature from about 0 °C to about room temperature.

A specific example is methyl 2-chloro-5,7-dihydrofuro[3,4-b]pyridine-3-carboxylate.

Alternatively, compounds (20) can be obtained under the same conditions from compounds of formula (19) wherein R103 is -Br and compounds (25) wherein R104 is - OH and the compounds (22) can then be obtained from compounds (21) by base-mediated carboxylation using CO2 and a strong base such as, but not limited to, n-BuLi at temperature from about -78 °C to about -50 °C in a solvent such as, but not limited to, tetrahydrofuran. A specific example is methyl (5R)-2-chloro-5-methyl-5,7-dihydrofuro[3,4-b]pyridine-3-carboxylate.

It can be appreciated that the synthetic schemes and specific examples as described in the present disclosure are examples, and are not to be considered as limiting the scope of the disclosure.

Optimum reaction conditions and reaction times for each individual step can vary depending on the particular reactants employed and substituents present in the reactants used. Unless otherwise specified, solvents, temperatures and other reaction conditions can be readily selected by one of ordinary skill in the art. Specific procedures are provided in the experimental section. Reactions can be worked up in the conventional manner, e.g. by eliminating the solvent from the residue and further purified according to methodologies generally known in the art such as, but not limited to, crystallization, distillation, extraction, trituration and chromatography. Unless otherwise described, the starting materials and reagents are either commercially available or can be prepared by one skilled in the art from commercially available materials using methods described in the chemical literature.

Routine experimentations, including appropriate manipulation of the reaction conditions, reagents and sequence of the synthetic route, protection of any chemical functionality that cannot be compatible with the reaction conditions, and deprotection at a suitable point in the reaction sequence of the method are included in the scope of the invention. Suitable protecting groups and the methods for protecting and deprotecting different substituents using such suitable protecting groups are well known to those skilled in the art; examples of which can be found in T. Greene and P. Wuts, Protecting Groups in Organic Synthesis (3 rd ed.), John Wiley & Sons, NY (1999). Synthesis of the compounds of the invention can be accomplished by methods analogous to those described in the synthetic schemes described hereinabove and in specific examples.

Starting materials, if not commercially available, can be prepared by procedures selected from standard organic chemical techniques, techniques that are analogous to the synthesis of known, structurally similar compounds, or techniques that are analogous to the above described schemes or the procedures described in the synthetic examples section.

When an optically active form of a compound is required, it can be obtained by carrying out one of the procedures described herein using an optically active starting material (prepared, for example, by asymmetric induction of a suitable reaction step), or by resolution of a mixture of the stereoisomers of the compound or intermediates using a standard procedure (such as chromatographic separation, recrystallization or enzymatic resolution).

Similarly, when a pure geometric isomer of a compound is required, it can be prepared by carrying out one of the above procedures using a pure geometric isomer as a starting material, or by resolution of a mixture of the geometric isomers of the compound or intermediates using a standard procedure such as chromatographic separation.

### Examples:

### Synthesis of intermediates

### Preparation of 6-amino-8-ethoxy-4-methyl-1H-quinolin-2-one (Intermediate 1)

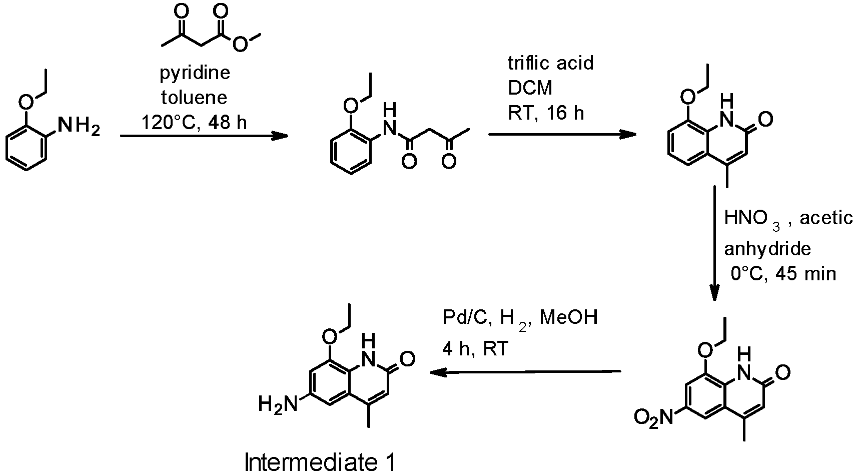

### Preparation of N-(2-ethoxyphenyl)-3-oxo-butanamide:

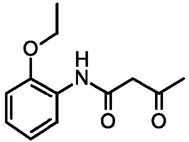

To a stirred solution of 2-ethoxyaniline (5.0 g, 36.49 mmol) in Toluene (500 mL) were added methyl acetoacetate (4.23 g, 36.5 mmol) followed by Pyridine (15 mL, 3 vol) and reaction mixture was stirred at 120°C for 40 h. After completion, the reaction mixture was allowed to warm up to RT and the solvent was evaporated. The residue was taken in diethyl ether and stirred for 15 min, the precipitated solid was filtered to afford N-(2-ethoxyphenyl)-3-oxo-butanamide as brown solid (4.0 g, 50%).

### Preparation 8-ethoxy-4-methyl-1H-quinolin-2-one:

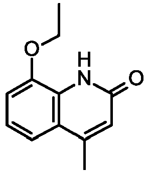

To a stirred solution of N-(2-ethoxyphenyl)-3-oxo-butanamide (3.5 g, 15.83 mmol) in DCM (350 mL) was added triflic acid (3.4 ml, 39.59 mmol) and the reaction mixture was stirred at RT for 16 h. After completion, the reaction mixture was poured into ice water and basified with solid NaHCO₃ and the solid was filtered. The solid was washed with diethyl ether and dried under vacuum to afford 8-ethoxy-4-methyl-1H-quinolin-2-one as brown solid (2.0 g, 62%)

### Preparation of 8-ethoxy-4-methyl-6-nitro-1H-quinolin-2-one:

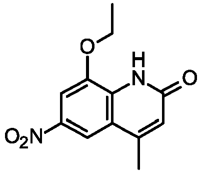

To a solution of 8-ethoxy-4-methyl-1H-quinolin-2-one (1.4 g, 6.9 mmol) in acetic anhydride (42 ml, 30 vol) was added nitric acid (0.57 ml, 13.79 mmol) at 0 °C and the reaction mixture was stirred at same temperature for 45 minutes. After completion, the reaction mixture was quenched in ice water and the solid was collected by filtration. Solid was washed with water and n-pentane (3 x 50 mL) to afford 8-ethoxy-4-methyl-6-nitro-1H-quinolin-2-one (compound-4) (750 mg, 44 %) as a yellow solid.

### Preparation of 6-amino-8-ethoxy-4-methyl-1H-quinolin-2-one (Intermediate 1):

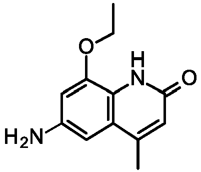

A suspension of 8-ethoxy-4-methyl-6-nitro-1H-quinolin-2-one (750 mg, 3.024 mmol) in methanol (7.5 mL) was degassed with nitrogen for 10 minutes. After that Pd/C (10%) (75 mg) was added and reaction was stirred under hydrogen atmosphere (40 psi) in a par shaker at room temperature for 16 h. After completion, the reaction mixture was filtered through celite bed. Filtrate was evaporated to afford 6-amino-8-ethoxy-4-methyl-1H-quinolin-2-one (500 mg, 75%) as a yellow solid. ¹H NMR (400 MHz, DMSO-*d₆*): δ 9.99 (brs, 1H), 6.52 (d, *J* = 1.6 Hz, 1H), 6.39 (d, *J* = 1.6 Hz, 1H), 6.13 (brs, 1H), 4.99 (s, 2H), 4.04 (dd, *J* = 14, 7.2 Hz, 2H), 2.29 (d, *J* = 0.8 Hz, 3H), 1.40 (t, *J* = 6.8 Hz, 3H); LCMS [M+H]+: 219.24 m/z, HPLC: 95.14%.

### Preparation of 6-amino-4,7,8-trimethyl-1H-quinolin-2-one (Intermediate 2):

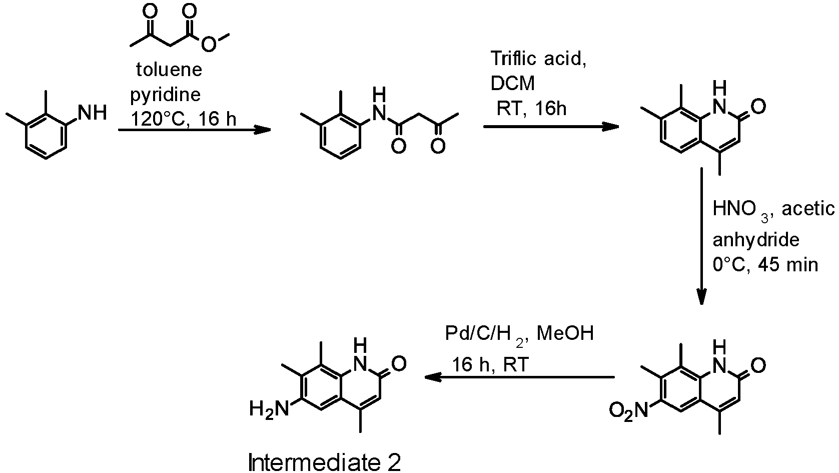

### Preparation of N-(2,3-dimethylphenyl)-3-oxobutanamide:

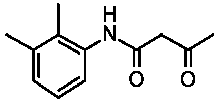

To a stirred solution of 2,3-dimethylaniline (25 g, 206.6 mmol) in toluene (25 mL, 10 vol) were added methyl-3-oxobutanoate (35 g, 309.9 mmol 1.0 eq) and Pyridine (75 ml, 3 vol) and stirred at 120°C for 16 h. After completion, the reaction mixture was allowed to warm up to RT and the solvent evaporated in vacuo. The residue was purified by column chromatography using 100-200 mesh, eluting with 50% of EtOAc in Pet.Ether to afford N-(2,3-dimethylphenyl)-3-oxobutanamide as a brown solid (18 g, 46%).

### Preparation of 4,7,8-trimethyl-1H-quinolin-2-one:

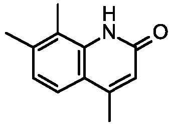

To a stirred solution of N-(2,3-dimethylphenyl)-3-oxobutanamide (5.0g, 24.3 mmol, 1.0 eq) in DCM (50 mL, 10 vol) was added triflic acid (21.5 ml, 243.0 mmol, 10 eq) at 0°C and allowed to stir for 16 h at rt. After completion, the reaction mixture was poured into ice water and basified with solid NaHCO₃ and extracted with EtOAc (2 x 300 mL). The crude was purified by column chromatography using 100-200 mesh, eluting with 5% methanol in dichloromethane to afford 4,7,8-trimethyl-1H-quinolin-2-one as a brown solid (4.0 g, 87%)

### Preparation of 4,7,8-trimethyl-6-nitro-1H-quinolin-2-one:

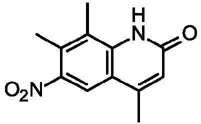

To a solution of 4,7,8-trimethyl-1H-quinolin-2-one (320 mg, 1.71 mmol) in acetic anhydride (9.6 ml, 30 vol)) was added nitric acid (0.14 ml, 3.42 mmol, 2 eq) at 0°C and the reaction mixture was stirred at 0°C for 45 min. After completion, reaction mixture was quenched with ice water, basified with aq. NaHCO₃ and extracted with ethyl acetate (2 x 50 mL). The organic layer was dried over Na₂SO₄ and concentrated under reduced pressure. The crude was purified by column chromatography using 100-200 mesh, eluting with 30% EtOAc in Pet ether to afford 4,7,8-trimethyl-6-nitro-1H-quinolin-2-one (100 mg, 25 %) as a yellow solid.

### Preparation of 6-amino-4,7,8-trimethyl-1H-quinolin-2-one (Intermediate 2):

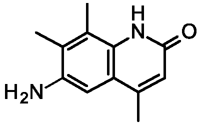

A suspension of 4,7,8-trimethyl-6-nitro-1H-quinolin-2-one (100 mg, 0.43 mmol) in methanol (1 mL, 10 vol) was degassed under nitrogen for 10 minutes. After that Pd/C (10%) (10 mg) was added and reaction was stirred under hydrogen atmosphere (40 psi) at room temperature for 16 h. After completion, the reaction mixture was filtered through a celite bed, filtrate was evaporated to afford 6-amino-4,7,8-trimethyl-1H-quinolin-2-one (80 mg, 97%) as a brown solid. LCMS [M+H]⁺ 203.17 m/z; ¹H NMR (400 MHz, DMSO-d6): δ 10.22 (br s, 1H), 6.83 (s, 1H), 6.26 (s, 1H), 4.74 (brs, 2H), 2.34 (s, 3H), 2.31 (s, 3H), 2.13 (s, 3H).

### Preparation of 6-amino-7-fluoro-8-methoxy-4-methyl-1H-quinolin-2-one (Intermediate 3):

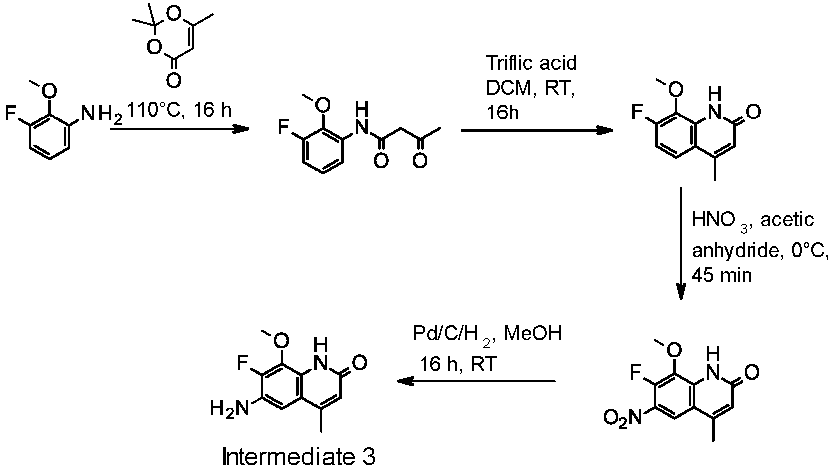

### Preparation of N-(3-fluoro-2-methoxy-phenyl)-3-oxo-butanamide:

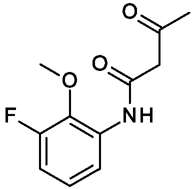

3-Fluoro-2-methoxy-aniline (2.8g, 20mmol, leq) was dissolved in toluene (5ml) and heated to 70°C and 2,2,6-trimethyl-1,3-dioxin-4-one (3.3g, 23mmol, 1.15eq) was added. The reaction mixture and heated at 110°C and stirred for 1.5h. The reaction mixture was cooled to RT. The reaction mixture was dissolved in a minimal amount of DCM and purified on Biotage Isolera: Heptane/EtOAc 95/5 then 95/5->40/60 and again Heptane/EtOAc 90/10 then 95/5->40/60. Fractions with the product were evaporated to afford N-(3-fluoro-2-methoxy-phenyl)-3-oxo-butanamide 2.5g (55%) as a yellow oil LCMS [M+H]⁺ 226 m/z

### Preparation of 7-fluoro-8-methoxy-4-methyl-1H-quinolin-2-one:

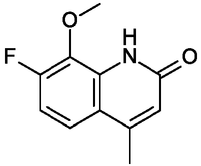

N-(3-fluoro-2-methoxy-phenyl)-3-oxo-butanamide (2.5g, 11mmol, leq) was dissolved in DCM (20ml). Trifluoromethanesulfonic acid (8ml, 90.6mmol, 8.23 eq) was added at RT dropwise during 15 min, then stirred at RT for 2h then another portion of trifluoromethanesulfonic acid (2ml, 22.5mmol, 2.5 eq) was added.

After 3h the reaction mixture was poured onto 70ml of ice and stirred for 10 min. and then filtered. The product was washed with water (3x20ml) and then dried at 1 mbar vacuum at room temperature overnight to afford 7-fluoro-8-methoxy-4-methyl-1H-quinolin-2-one 1.5g (66%) as a white powder. LCMS [M+H]+ 208 m/z. ¹H NMR (300 MHz, DMSO-d6) δ 11.17 (s, 1H), 7.46 (dd, J = 9.0, 5.3 Hz, 1H), 7.12 (dd, J = 11.1, 9.0 Hz, 1H), 6.38 (d, J = 1.3 Hz, 1H), 2.39 (d, J = 1.2 Hz, 3H).

### Preparation of 7-fluoro-8-methoxy-4-methyl-6-nitro-1H-quinolin-2-one:

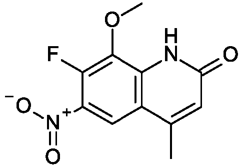

7-fluoro-8-methoxy-4-methyl-1H-quinolin-2-one (1.5g, 7.2mmol, leq) was suspended in Ac₂O and cooled to 0°C. HNO₃ 65% aq (0.6ml, 8.6mmol, 1.19eq) was added dropwise, stirred at 0°C for 1.5h and then poured into 150 ml of ice cold water and stirred until solidified. The product was filtered and washed with water (3x25ml) and then dried at 1 mbar vacuum at room temperature and then triturated with DCM/MeOH/EtOAc (5+0.5+5ml). The product was filtered, washed with EtOAc (2x10ml) and dried at 5 mbar vacuum at 45°C to afford 7-fluoro-8-methoxy-4-methyl-6-nitro-1H-quinolin-2-one, 860 mg as an off-white powder (~70% desired product+ 20% of other regioisomer). ¹H NMR (300 MHz, DMSO-d6) δ 11.79 (s, 1H), 8.22 (d, J = 7.4 Hz, 1H), 6.56 (s, 1H), 3.97 (d, J = 1.4 Hz, 3H), 2.47 (d, J = 1.2 Hz, 3H).

### Preparation of 6-amino-7-fluoro-8-methoxy-4-methyl-1H-quinolin-2-one (Intermediate 3):

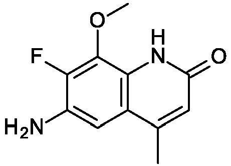

7-fluoro-8-methoxy-4-methyl-6-nitro-1H-quinolin-2-one (860 mg) was dissolved in MeOH (35ml) and 10% Pd/C (55mg) was added. Stirred with a H₂ baloon at RT for 2h. The reaction mixture was diluted with acetone (15ml) and filtered through Celite. The filtrate was evaporated and the residue was triturated with boiling acetone (30ml).

The filtered product was washed with acetone (5ml) and dried at 1 mbar vacuum at room temperature overnight to afford 6-amino-7-fluoro-8-methoxy-4-methyl-1H-quinolin-2-one 440mg (58%) as yellow powder. LCMS [M+H]+ 223 m/z. ¹H NMR (300 MHz, DMSO-d6) δ 10.73 (s, 1H), 6.78 (d, J = 8.8 Hz, 1H), 6.30 (d, J = 1.3 Hz, 1H), 5.12 (s, 2H), 3.86 (d, J = 1.3 Hz, 3H).

### Preparation of 6-amino-7-methoxy-4-methyl-1H-quinolin-2-one (Intermediate 4):

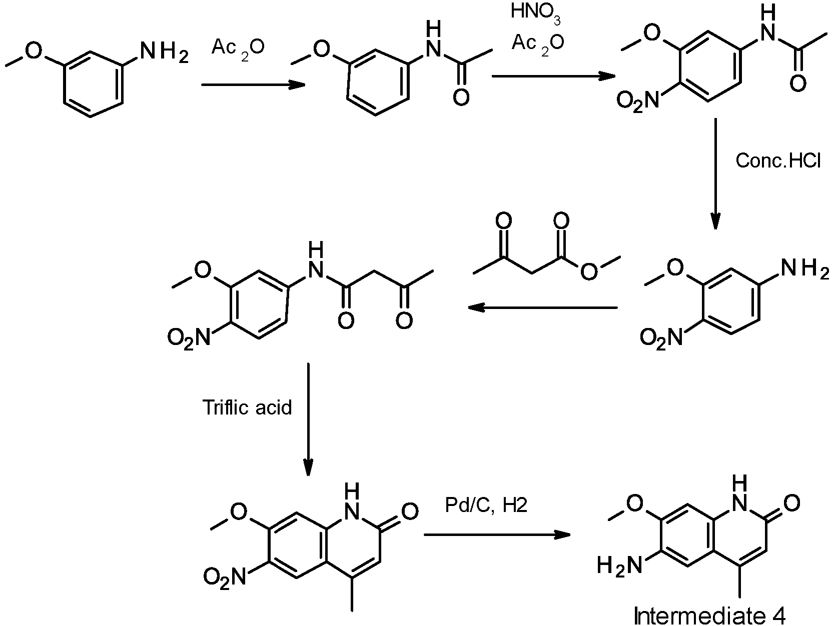

### Preparation of N-(3-methoxyphenyl)acetamide:

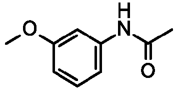

3-Methoxyaniline (10.0 g, 81.3 mmol) was added to acetic anhydride (24.8 g, 243.9 mmol) at 0°C. The reaction mixture was then allowed to warm to room temperature and was stirred for 2 h. After completion, the reaction mixture was basified with NaHCO₃ and extracted with ethyl acetate (2 x 50 mL). The combined organic layers were dried over Na₂SO₄ and concentrated under reduced pressure and the residue was washed with n-pentane to afford N-(3-methoxyphenyl)acetamide as light brown solid (10 g, 76%).

### Preparation of N-(3-methoxy-4-nitro-phenyl)acetamide:

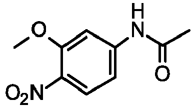

To a solution of N-(3-methoxyphenyl)acetamide (9.5 g, 57.5 mmol, 1.0 eq) in acetic anhydride (150 mL, 15 vol) was added nitric acid (4.8 mL, 115.1 mmol, 2.0 eq) at 0°C and the reaction mixture was stirred at the same temperature for 30 min. After completion, reaction mixture was quenched in ice water, basified with aq. NaHCO₃ and extracted with ethyl acetate (2 x 500 mL). The combined organic layers were dried over Na₂SO₄ and concentrated under reduced pressure. The crude was purified by column chromatography using 100-200 mesh silica gel, eluting with 30% of EtOAc in petrolether to afford N-(3-methoxy-4-nitrophenyl)acetamide (2.4 g, 20 %) as a yellow solid.

### Preparation of 3-methoxy-4-nitroaniline:

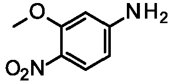

To a stirred solution of 1N HCl (24 mL, 10 vol) was added N-(3-methoxy-4-nitrophenyl)acetamide (2.4 g, 11.42 mmol) at room temperature and reaction mixture was allowed to stir at 100 °C for 2 h. After completion, reaction mixture was poured into ice water, basified with aq. NaHCO₃ and extracted with ethyl acetate (2 x 50 mL). The organic layer was dried over Na₂SO₄ and concentrated under reduced pressure. The residue was washed with n-pentane to afford 3-methoxy-4-nitroaniline as brown solid (1.8 g, 94%).

### Preparation of N-(3-methoxy-4-nitrophenyl)-3-oxobutanamide:

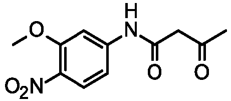

To a stirred solution of 3-methoxy-4-nitroaniline (1.8 g, 10.71 mmol, 1 eq) in toluene (1.8 mL, 10 vol) were added methyl-3-oxobutanoate (1.2 g, 10.71 mmol) followed by Pyridine (15 ml, 3 vol) at rt and reaction mixture was stirred at 100°C for 16h. After completion, the reaction mixture was allowed to warm up to rt and the solvent was evaporated. The crude was purified by silica gel column chromatography using (100-200 mesh) 50% of EtOAc in Pet ether to afford N-(3-methoxy-4-nitrophenyl)-3-oxobutanamide as brown solid (1.7 g, 63%).

### Preparation 7-methoxy-4-methyl-6-nitro-1H-quinolin-2-one:

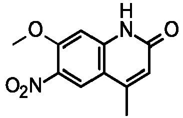

To a stirred solution of N-(3-methoxy-4-nitrophenyl)-3-oxobutanamide (600 mg, 2.38 mmol) in DCM (6 mL) was added triflic acid (2.02 ml, 7.93 mmol) at 0°C and allowed to stir at RT for 16 h. After completion, the reaction mixture was poured into ice water and basified with solid NaHCO₃ and extracted with EtOAc (2 x 50 mL). The crude product was purified by column chromatography using (100-200 mesh silica gel) eluting with 5% of methanol in dichloromethane to afford 7-methoxy-4-methyl-6-nitro-1H-quinolin-2-one as a brown solid (50 mg, 9%).

### Preparation of 6-amino-7-methoxy-4-methyl-1H-quinolin-2-one (Intermediate 4):

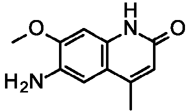

A suspension of 7-methoxy-4-methyl-6-nitro-1H-quinolin-2-one (50 mg, 0.21 mmol) in methanol (0.5 mL, 10 vol) was degassed under argon for 10 min. To this 10% Pd/C (5 mg) was added and reaction was stirred under hydrogen atmosphere (40 psi) at room temperature for 12 h. After completion, the reaction mixture was filtered through a celite bed. The filtrate was evaporated to afford 6-amino-7-methoxy-4-methyl-1H-quinolin-2-one (30 mg, 70%) as a brown solid. ¹H NMR (400 MHz, DMSO-*d₆*): δ 11.14 (brs, 1H), 6.87 (s, 1H), 6.75 (s, 1H), 6.14 (s, 1H), 4.68 (brs, 2H), 3.81 (s, 3H), 2.33 (s, 3H); LCMS [M+H]+: 205.20 m/z

### Preparation of 5-amino-7-methyl-3,10-dihydro-2H-[1,4]dioxino[2,3-h]quinolin-9-one (Intermediate 5):

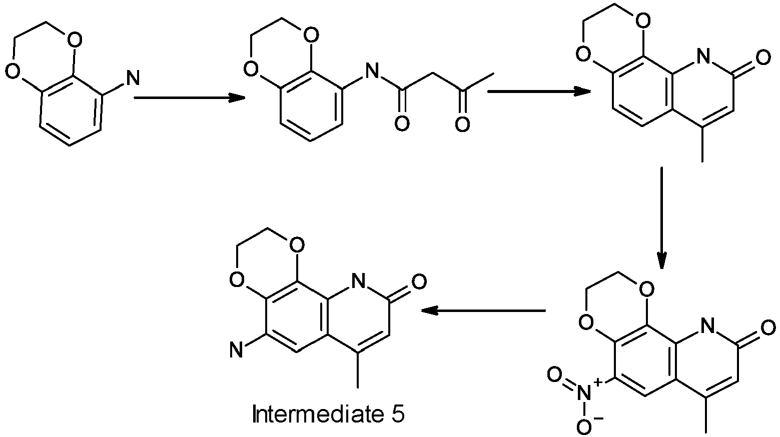

### Preparation of N-(2,3-dihydro-1,4-benzodioxin-5-yl)-3-oxo-butanamide:

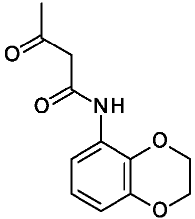

2,3-dihydro-1,4-benzodioxin-5-amine (3g, 20mmol, leq) was dissolved in toluene(10ml) and heated to 110°C and 2,2,6-trimethyl-1,3-dioxin-4-one (3ml, 22.7mmol, 1.1eq) was added. The reaction mixture was stirred for 0.75h and then cooled to RT. Solvent was evaporated and the crude mixture purified on Biotage Isolera: Heptane/EtOAc 93/7 then 93/7->30/70. Fractions with the product were evaporated to afford N-(2,3-dihydro-1,4-benzodioxin-5-yl)-3-oxo-butanamide 2.8g (60%) as a yellow oil. ¹H NMR (300 MHz, Chloroform-d) δ 9.18 (s, 1H), 7.88 (dd, J = 8.2, 1.5 Hz, 1H), 6.83 (t, J = 8.2 Hz, 1H), 6.66 (dd, J = 8.3, 1.5 Hz, 1H), 4.38 (ddd, J = 5.3, 3.1, 1.1 Hz, 2H), 4.34 - 4.24 (m, 2H), 3.62 (s, 2H), 2.35 (s, 3H).

### Preparation of 7-methyl-3,10-dihydro-2H-[1,4]dioxino[2,3-h]quinolin-9-one:

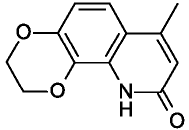

N-(2,3-dihydro-1,4-benzodioxin-5-yl)-3-oxo-butanamide (2.8g, 11.9 mmol, leq) was dissolved in DCM (20ml). Trifluoromethanesulfonic acid (10.5ml, 119mmol, 10 eq) and DCM (5ml) were added at RT dropwise during 20 min. The reaction mixture was stirred at RT for 1h then poured into 100ml of ice and stirred for 20 min. The reaction mixture was filtered and washed with water (2x15ml) and Et2O (10ml). The product was dissolved in EtOAc/DCM/MeOH=1/10.2 (150ml), dried over Na2SO4 and evaporated. The product was dried in vacuo at 1 mbar at RT for 1h to afford 7-methyl-3,10-dihydro-2H-[1,4]dioxino[2,3-h]quinolin-9-one 2.5g (97%) as pale yellow powder. ¹H NMR (300 MHz, Chloroform-d) δ 9.00 (s, 1H), 7.18 (d, J = 8.9 Hz, 1H), 6.80 (d, J = 8.9 Hz, 1H), 6.42 (d, J = 1.2 Hz, 1H), 4.52-4.33 (m, 4H), 2.45 (d, J = 1.2 Hz, 3H).

### Preparation of 7-methyl-5-nitro-3,10-dihydro-2H-[1,4]dioxino[2,3-h]quinolin-9-one:

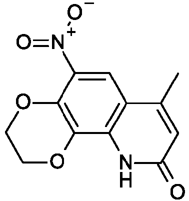

7-methyl-3,10-dihydro-2H-[1,4]dioxino[2,3-h]quinolin-9-one (2.5g, 11.5mmol, leq) was suspended in Ac₂O (40ml) and cooled to 0oC. HNO₃ 65% aq(1.1ml, 17.3mmol, 1.5eq) was added dropwise and stirred at 0°C for 40min then poured into 400 ml of ice and stirred at RT for 10 min. The product was filtered and washed with water (2x30ml). The product was triturated with ultrasound in DCM/EtOAc=1/1 (10ml), filtered and washed with EtoAc/heptane=1/1 (10ml), heptane (10ml), DCM/EtOAc=1/1 (10ml) and heptane (2x10ml). The product was dried in air, triturated with ultrasound in DCM/MeOH=1/1 (20ml) and filtered. The product was purified on Biotage Isolera: ACN(0.1%FA)/H2O(0.1%FA) 1/99 then 1/99->40/60. Relevant fractions were evaporated. The front fraction of the purification was repurified on Biotage Isolera: ACN(0.1%FA)/H20(0.1%FA) 0/100 then 0/100->80/20. The product, 7-methyl-5-nitro-3,10-dihydro-2H-[1,4]dioxino[2,3-h]quinolin-9-one was obtained (150 mg) as a mixture with the other regioisomer in the ratio 4:6. ¹H NMR (300 MHz, DMSO-d6) δ 11.21 (s, 1H), 7.95 (s, 1H), 6.42 (s, 1H), 4.52 - 4.45 (m, 4H), 2.42 (d, J = 1.2 Hz, 3H).

### Preparation of 5-amino-7-methyl-3,10-dihydro-2H-[1,4]dioxino[2,3-h]quinolin-9-one (Intermediate 5):

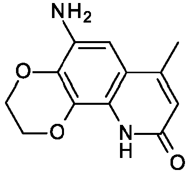

7-methyl-5-nitro-3,10-dihydro-2H-[1,4]dioxino[2,3-h]quinolin-9-one (150 mg) was dissolved in MeOH (10ml) and 10%Pd/C (15mg) was added and the mixture ran through 3x vacuum/H2 cycles. The reaction was stirred with a H₂ balloon at RT for 4h. The reaction mixture was filtered through Celite and evaporated to afford 5-amino-7-methyl-3,10-dihydro-2H-[1,4]dioxino[2,3-h]quinolin-9-one (120mg, 90%) , as a brown oil. LCMS [M+H]⁺ 233 m/z as a mixture of regioisomers.

### Preparation of methyl 2-chloro-5,7-dihydrofuro[3,4-b]pyridine-3-carboxylate (Intermediate 6):

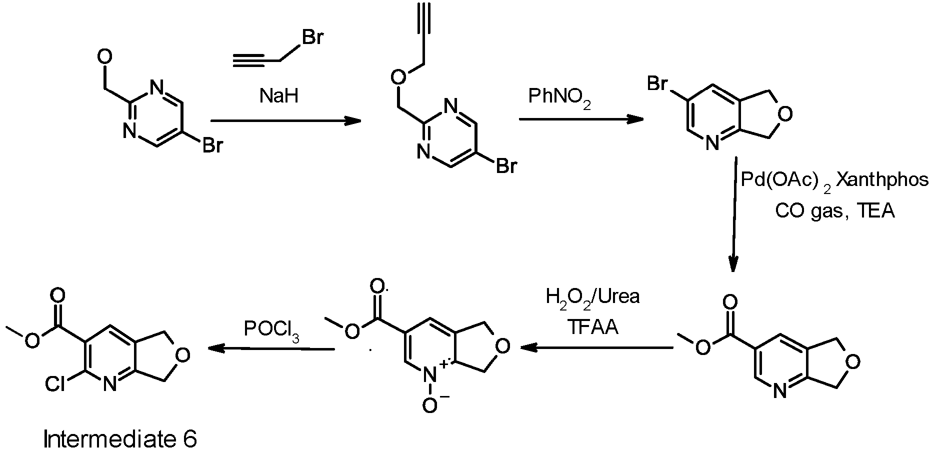

### Preparation of 5-bromo-2-(prop-2-ynoxymethyl)pyrimidine:

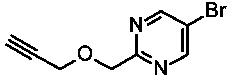

To a stirred solution of (5-bromopyrimidin-2-yl) methanol (20 g, 105.82 mmol) in dry DMF (100 mL) was added NaH (3.8 g, 246 mmol) portion wise at 0°C and the reaction mixture was stirred for 15 minutes at the same temperature. To this was added propargyl bromide (9.6 mL, 1.2 eq) dropwise over 20 minutes and the reaction mixture was stirred at 0°C for 30 minutes. After 30 minutes the reaction mixture was allowed to warm up to room temperature, and to this 0.12 eq of propargyl bromide (0.96 mL, 0.12 eq) was added dropwise. Again the reaction mixture was stirred for 45 minutes at the same temperature. After completion, the reaction mixture was quenched with 15% Na₂SO₃ solution (100 mL), 5% LiCl solution (100 mL), diluted with water (100 mL) and extracted with EtOAc (2 x 500 mL). The combined organic layers were dried over Na₂SO₄, filtered and concentrated under reduced pressure to afford crude product, which was purified by silica gel (100-200 mesh) column chromatography eluted at 5% EtOAc in pet ether to afford 5-bromo-2-(prop-2-ynoxymethyl)pyrimidine (18 g, 75% Yield) as a yellow solid. ¹H NMR (400 MHz, CDCl₃): *δ* 8.80 (s, 2H), 4.82 (s, 2H), 4.39 (d, *J* = 2.4 Hz, 2H), 2.48 (t, *J* = 2.4 Hz, 1H): LCMS [M+H]⁺: 227.01 m/z.

### Preparation of 3-bromo-5,7-dihydrofuro[3,4-b]pyridine:

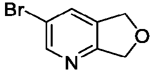

To a stirred solution of 5-bromo-2-(prop-2-ynoxymethyl)pyrimidine (25 g, 110 mmol) in nitrobenzene (75 mL) was heated at 180 °C for 4 h with argon flowing over the reaction mixture and the outlet into a flask containing a cooled hypochlorite solution to quench the HCN, that is formed in the reaction. After completion, the reaction mixture was cooled to RT and directly purified by silica gel (100-200 mesh) column chromatography eluted with 25% EtOAc in pet ether to afford 3-bromo-5,7-dihydrofuro[3,4-b]pyridine (18g, 81% yield) as an off white solid. ¹H NMR (300 MHz, DMSO-*d₆*): δ 8.57 (t, *J* = 0.6 Hz, 1H), 8.03 (d, *J* = 0.6 Hz, 1H), 5.06 (s, 2H), 4.90 (t, *J* = 1.8 Hz, 2H): LCMS [M+H]⁺: 202.00 m/z.

### Preparation of methyl 5,7-dihydrofuro[3, 4-b]pyridine-3-carboxylate:

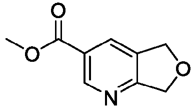

In a 1 L autoclave, 3-bromo-5,7-dihydrofuro[3,4-b]pyridine (30 g, 150 mmol) DMF (90 mL), MeOH (90 mL), 4A° MS sieves followed by Et₃N (61 mL, 450 mmol) were charged at room temperature and the reaction mixture was degassed with argon for 10 minutes. To this were added Pd(OAc)₂ (5.04 g, 7.5 mmol), Xantphos (4.34 g, 7.5 mmol) and filled with CO gas (100 psi).The reaction mixture was heated at 90 °C for 4h. After completion, the reaction mixture was concentrated under reduced pressure to obtain crude compound, which was purified by silica gel (100-200 mesh) column chromatography eluted with 15% EtOAc in pet ether to afford methyl 5,7-dihydrofuro[3,4-b]pyridine-3-carboxylate (21.6 g, 81% yield) as an off white solid. ¹H NMR (400 MHz, CDCl₃): *δ* 9.11 (s, 1H), 8.15 (s, 1H), 5.20 (t, *J* = 0.8 Hz, 2H), 5.11 (t, *J* = 1.6 Hz, 2H), 3.96 (s, 3H); LCMS [M+H]⁺: 180.08 m/z

### Preparation of methyl 1-oxido-5,7-dihydrofuro[3,4-b]pyridin-1-ium-3-carboxylate:

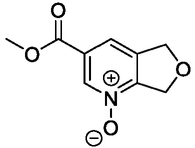

To a stirred solution of methyl 5,7-dihydrofuro[3,4-b]pyridine-3-carboxylate (30 g, 167.59 mmol) in DCM (250 mL) and ACN (250 mL), urea/hydrogen peroxide complex was added (29.4 g, 313.4 mmol) and the reaction mixture was cooled to 0°C. To this TFAA (41 mL, 293.29) was added drop wise over 25 minutes. Then the reaction mixture was allowed to warm to rt and stirred for 30 minutes at the same temperature. Further TFAA (3 mL, 21.78 mmol) was added and stirred for 10 minutes. After completion, the reaction mixture was diluted with DCM (300 mL), quenched with NaHCO₃ solution (800 mL) and 5M NaOH solution (12 mL) to adjust the pH up to 7.5. The layers were separated and the aqueous layer was extracted with DCM (2x500 mL). The combined organic layers were dried over Na₂SO₄ concentrated under reduced pressure to afford crude product. The crude product was purified by silica gel (100-200 mesh) column chromatography eluted with 5% MeOH in DCM to afford methyl 1-oxido-5,7-dihydrofuro[3,4-b]pyridin-1-ium-3-carboxylate (29.8 g, 94% yield) as an off white solid. ¹H NMR (400 MHz, CDCl₃): *δ* 8.69 (s, 1H), 7.72 (s, 1H), 5.20 (dd, *J* = 8.4, 2 Hz, 4H), 3.97 (s, 3H); LCMS [M+H]⁺: 195.97 m/z.

### Preparation of methyl 2-chloro-5,7-dihydrofuro[3,4-b]pyridine-3-carboxylate (Intermediate 6):

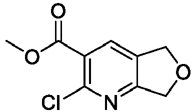

To methyl 1-oxido-5,7-dihydrofuro[3,4-b]pyridin-1-ium-3-carboxylate (20 g, 102.56 mmol) was added POCl₃ (70.4 mL, 753.84 mmol, freshly distilled) at 0°C. The reaction mixture was heated at 100 °C for 1 h under argon. After completion, the reaction mixture was diluted with DCM (100 mL) and quenched with ice cold mixture of DCM (400 mL)/sat.aq.NaHCO₃ solution (800 mL) during 15 minutes with stirring, shaken until gas evolution ceased. Brine solution (200 mL) was added and the layers were separated. The aqueous layer was extracted with DCM (2 x 800 mL). The combined organic layers were dried over Na₂SO₄, filtered and concentrated under reduced pressure to afford crude compound, which was purified by silica gel (100-200 mesh) column chromatography and eluted with 15% EtOAc in pet ether to afford methyl 2-chloro-5,7-dihydrofuro[3,4-b] pyridine-3-carboxylate (13.2 g, 61% yield) as an off white solid. ¹H NMR (400 MHz, CDCl₃): *δ* 8.03 (t, *J* = 0.8, 1H), 5.17 - 5.16 (m, 2H), 5.06 (t, *J* = 2 Hz, 2H), 3.96 (s, 3H); LCMS [M+H]⁺: 214.09 m/z.

### Preparation of methyl (5R)-2-chloro-5-methyl-5,7-dihydrofuro[3,4-b]pyridine-3-carboxylate (Intermediate 7):

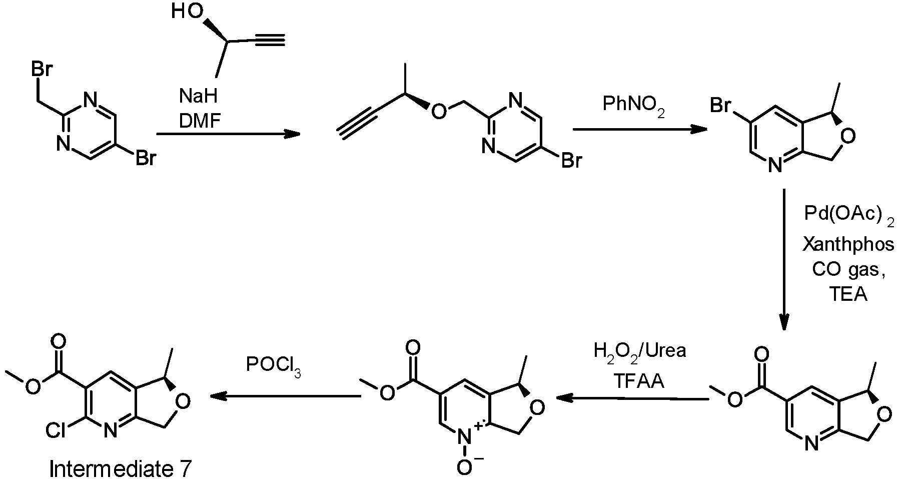

### Preparation of 5-bromo-2-[[(1R)-1-methylprop-2-ynoxy]methyl]pyrimidine

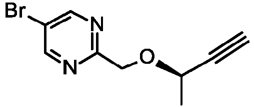

To a stirred solution of 5-bromo-2-(bromomethyl)pyrimidine (470 mg, 1.87 mmol) in dry THF (4 mL), was added NaH (60% in mineral oil, 97 mg, 2.3 mmol) portionwise at -10 °C under argon and the reaction mixture was stirred for 10 minutes at same temperature. To this (2R)-but-3-yn-2-ol (153 mg, 2.2 mmol) was added and the reaction mixture stirred for 1h. After completion, the reaction mixture was quenched with saturated aq. NH₄Cl solution (6 mL) and extracted with EtOAc (3 x 6 mL). The combined organic layers were dried over Na₂SO₄, filtered and concentrated under reduced pressure to afford crude product, which was purified by silica gel (100-200 mesh) column chromatography and eluted with 2-30% EtOAc in heptane to afford 5-bromo-2-[[(1R)-1-methylprop-2-ynoxy]methyl]pyrimidine (254 mg, 56% yield). LCMS [M+H]⁺: 241.06/243 m/z. ¹H NMR (300 MHz, Chloroform-d) δ 8.82 (s, 2H), 4.98 (d, *J* = 14.0 Hz, 1H), 4.78 (d, *J* = 14.0 Hz, 1H), 4.47 (qd, *J* = 6.6, 2.1 Hz, 1H), 2.49 (d, *J* = 2.1 Hz, 1H), 1.60 (s, 3H).

### Preparation of (5R)-3-bromo-5-methyl-5,7-dihydrofuro[3,4-b]pyridine:

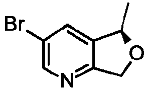

A stirred solution of 5-bromo-2-[[(1R)-1-methylprop-2-ynoxy]methyl]pyrimidine (254 mg, 1.05 mmol) in nitrobenzene (2 mL) was heated at 180 °C for 2 h in argon atmosphere. After completion, the reaction mixture was cooled to RT and directly purified by silica gel flash column chromatography eluting by 1-30% EtOAc in heptane to afford (5R)-3-bromo-5-methyl-5,7-dihydrofuro[3,4-b]pyridine (203 mg, 90% yield). LCMS [M+H]⁺: 214/216.7 m/z. ¹H NMR (300 MHz, Chloroform-d) δ 8.55 (dd, *J* = 2.1, 0.8 Hz, 1H), 7.62 (dd, *J* = 2.1, 1.0 Hz, 1H), 5.41-5.31 (m, 1H), 5.08 - 4.91 (m, 2H), 1.54 (d, *J* = 6.4 Hz, 3H).

### Preparation of methyl (5R)-5-methyl-5,7-dihydrofuro[3,4-b]pyridine-3-carboxylate:

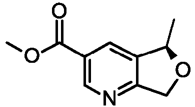

In a two-chamber reaction vial, one chamber was charged with methyl 3-bromo-5,7-dihydrofuro [3,4-b]pyridine (202 g, 0.94 mmol), DMF (1.6 mL), MeOH (0.8 mL) and Et₃N (393 uL, 2.82 mmol) and flushed with argon at RT for 10 minutes. To this were added Pd(OAc)₂ (11 mg, 0.05 mmol) and Xantphos (27 mg, 0.05 mmol). The second chamber was charged with CHOOH (142 uL, 3.76 mmol) and MsCl (289 uL, 3.76 mmol) in toluene (7 mL) and flushed with argon. To start CO formation, Et₃N (800 uL, 5.64 mmol) was quickly added to the second chamber. The reaction mixture was heated at 85°C for 45 min. After completion, the reaction mixture was poured into water and extracted with EtOAc. The combined extracts were washed with water, 5% LiCl and brine, dried over anhydrous Na₂SO₄, filtered and evaporated. The crude compound was purified by flash chromatography using EtOAc/heptane, (10-70% EtOAc) to afford methyl (5R)-5-methyl-5,7-dihydrofuro[3,4-b]pyridine-3-carboxylate (373.5 mg, 73 % yield). LCMS [M+H]⁺: 194.16 m/z. ¹H NMR (300 MHz, Chloroform-d) δ 9.13 (dd, J = 1.9, 0.8 Hz, 1H), 8.10 (dd, J = 1.8, 1.0 Hz, 1H), 5.49 - 5.31 (m, 1H), 5.24 - 4.97 (m, 2H), 3.99 (s, 3H), 1.58 (d, J = 6.4 Hz, 3H).

### Preparation of methyl (5R)-5-methyl-1-oxido-5,7-dihydrofuro[3,4-b]pyridin-1-ium-3-carboxylate:

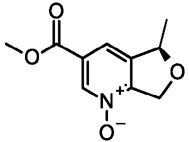

A stirred solution of (5R)-5-methyl-5,7-dihydrofuro[3,4-b]pyridine-3-carboxylate (130 mg, 0.673 mmol) in DCM (3 mL) and ACN (6 mL) was cooled to 0 °C and to this was added urea hydrogen peroxide complex (165 mg, 1.75 mmol) and TFAA (41 mL, 293.29). Then the reaction mixture was stirred for 30 minutes at room temperature. Another portion of urea hydrogen peroxide complex (16 mg) and TFAA (24.4 uL) was added and stirred for 30 minutes. After completion, to the reaction mixture was added TEA (1 mL) at 0°C, stirred for 10 min and evaporated in vacuo to afford methyl (5R)-5-methyl-1-oxido-5,7-dihydrofuro[3,4-b]pyridin-1-ium-3-carboxylate (131 mg, 93% yield). LCMS [M+H]⁺: 210.17 m/z.¹H NMR (300 MHz, Chloroform-d) δ 8.73 (s, 1H), 7.70 (d, J = 1.2 Hz, 1H), 5.54 - 5.38 (m, 1H), 5.38 - 5.06 (m, 2H), 4.00 (s, 3H), 1.58 (d, J = 6.4 Hz, 3H).

### Preparation of methyl (5R)-2-chloro-5-methyl-5,7-dihydrofuro[3,4-b]pyridine-3-carboxylate (Intermediate 7):

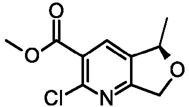

To (5R)-5-methyl-1-oxido-5,7-dihydrofuro[3,4-b]pyridin-1-ium-3-carboxylate (130 mg, 0.62 mmol) was added POCl₃ (400 uL, 4.4 mmol, freshly distilled). The reaction mixture was heated at 100 °C for 3 h under argon. After completion, the excess reagent was removed in vacuo to afford methyl (5R)-2-chloro-5-methyl-5,7-dihydrofuro[3,4-b]pyridine-3-carboxylate (90 % yield). LCMS [M+H]^{+:} 228.13 m/z.

### Preparation of methyl 5-methyl-2-morpholino-5,7-dihydrofuro[3,4-b]pyridine-3-carboxylate (Intermediate 8)

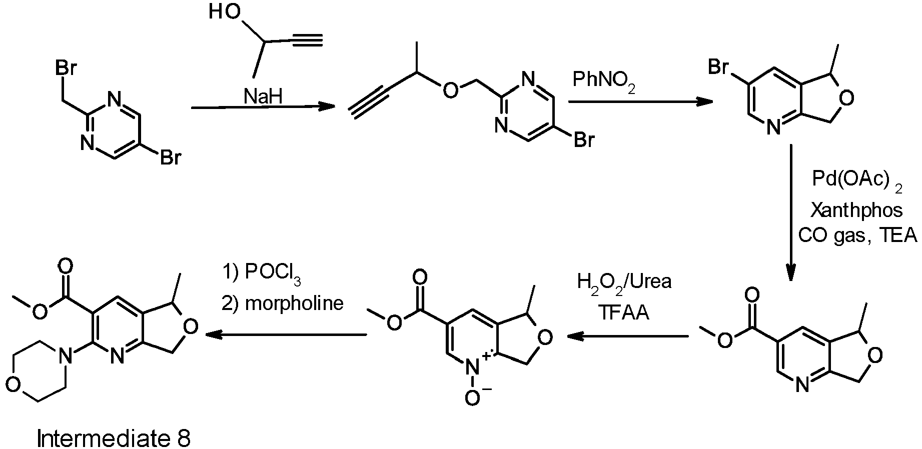

### Preparation of 5-bromo-2-(1-methylprop-2-ynoxymethyl)pyrimidine:

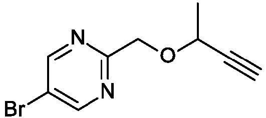

To a solution of but-3-yn-2-ol (140 mg, 2 mmol, 1.17 eq) in dry THF (4 mL) at - 10 °C under argon, NaH 60% wt (84mg, 2.1 mmol, 1.235 eq) was added in one portion and stirred at -10°C for 10 min. Then 5-bromo-2-(bromomethyl)pyrimidine (430mg, 1.7 mmol, 1 eq) was added and stirred in the ice-acetone bath for 1h. Then NH₄Cl sat.aq. (6ml) was added followed by EtOAc (6ml). Layers were separated, and the aqueous layer washed with EtOAc (6ml). Organic extracts were combined washed with brine and evaporated. The crude product was purified on Biotage Isolera: Heptane/EtOAc 98/2 ->70/30. Fractions with the product were evaporated to afford 5-bromo-2-(1-methylprop-2-ynoxymethyl)pyrimidine 251 mg (62 %) as a colourless oil. ¹H NMR (300 MHz, CDCl₃) δ 8.82 (s, 2H), 4.97 (d, J = 14.0 Hz, 1H), 4.78 (d, J = 13.9 Hz, 1H), 4.47 (dd, J = 6.6, 2.1 Hz, 1H), 2.49 (d, J = 2.1 Hz, 1H), 1.58 (d, J = 6.6 Hz, 3H).

### Preparation of 3-bromo-5-methyl-5,7-dihydrofuro[3,4-b]pyridine:

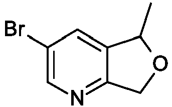

A solution of 5-bromo-2-(1-methylprop-2-ynoxymethyl)pyrimidine ( 251mg, 1.05 mmol, 1 eq) in PhNO₂ (4 mL) was stirred at 180°C for 2h. Then it was directly purified on Biotage Isolera: Heptane/EtOAc 99/1 then Heptane/EtOAc 99/1 ->70/30. Fractions with the product were evaporated to afford 3-bromo-5-methyl-5,7-dihydrofuro[3,4-b]pyridine 180 mg (81 %) as a colourless oil. ¹H NMR (300 MHz, CDCl₃) δ 8.55 (dd, J = 2.1, 0.9 Hz, 1H), 7.62 (dd, J = 2.1, 1.0 Hz, 1H), 5.43 - 5.29 (m, 1H), 5.12 - 4.90 (m, 2H), 1.54 (d, J = 6.4 Hz, 3H).

### Preparation of methyl 5-methyl-5,7-dihydrofuro[3,4-b]pyridine-3-carboxylate:

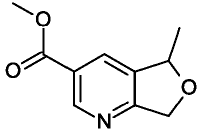

3-Bromo-5-methyl-5,7-dihydrofuro[3,4-b]pyridine (180mg, 0.84mmol, 1eq) was dissolved under argon in dry THF (4ml) and cooled to -78°C. n-BuLi 1.6M/hexane (0.55ml, 0.88mmol, 1.05eq) was added dropwise during 3min and then stirred for 5min. Two lumps of solid CO₂ were added, the bath removed and stirred for 20 min. The reaction mixture was evaporated to afford 250 mg of crude (5-methyl-5,7-dihydrofuro[3,4-b]pyridine-3-carbonyl)oxylithium as a yellow foam. LCMS [M+H]⁺ 180 m/z. This was dissolved in DCM/MeOH/DMF (4+2+1ml) and to the solution was added TEA (0.3ml, 2.19mmol,2.6eq) followed by HATU (350mg, 0.92mmol, 1.1eq). The reaction mixture was stirred at RT for 1.5h and then concentrated. The residue was partitioned between EtOAc (8ml) and 5% LiCl aq. (5ml). Layers were separated and the aqueous layer was washed with EtOAc (5ml). The organic extracts were combined, evaporated in vacuo and purified on a Biotage Isolera: Heptane/EtOAc 99/1 ->60/40. The fractions with the product were evaporated to afford methyl 5-methyl-5,7-dihydrofuro[3,4-b]pyridine-3-carboxylate 75 mg (42% over two steps) as a colourless oil. LCMS [M+H]⁺ 194 m/z.

### Preparation of methyl 5-methyl-1-oxo-5,7-dihydrofuro[3,4-b]pyridine-3-carboxylate:

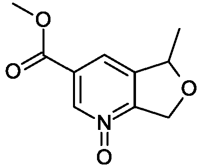

Methyl 5-methyl-5,7-dihydrofuro[3,4-b]pyridine-3-carboxylate (75 mg, 0.386 mmol, 1 eq) was dissolved in ACN/DCM (4+2ml). and cooled to 0°C. Added H₂O₂xUrea complex (94 mg, 1 mmol, 2.6 eq) followed by TFAA (0.14ml, 1mmol, 2.6 eq). Bath removed. Stirred at RT for 30min. Added H₂O₂xUrea complex (94mg, 1mmol, 2.6 eq) followed by TFAA (0.14 ml, 1 mmol, 2.6 eq). Stirred at RT for 30min. Cooled to 0°C. Added TEA (0.6 ml, 4.39 mmol, 11.4 eq). Stirred for 10 min. RM evaporated. Purified on Biotage Isolera: EtOAc/MeOH 100/0 then EtOAc/MeOH 100/0 ->85/15. The fractions with product were evaporated to afford methyl 5-methyl-1-oxo-5,7-dihydrofuro[3,4-b]pyridine-3-carboxylate 81 mg (100%) as a white substance. LCMS [M+H]⁺ 210 m/z. ¹H NMR (300 MHz, DMSO-d6) δ 8.50 (t, J = 1.0 Hz, 1H), 7.79 (t, J = 1.1 Hz, 1H), 5.42 (dtt, J = 6.4, 1.7, 0.9 Hz, 1H), 5.06 (dd, J = 11.8, 2.3 Hz, 2H), 3.90 (s, 3H), 1.48 (d, J = 6.4 Hz, 3H).

### Preparation of methyl 5-methyl-2-morpholino-5,7-dihydrofuro[3,4-b]pyridine-3-carboxylate (Intermediate 8):

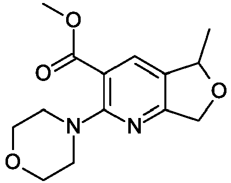

Methyl 5-methyl-1-oxo-5,7-dihydrofuro[3,4-b]pyridine-3-carboxylate (81 mg, 0.387mmol, leq) was mixed with POCl₃ (0.2 ml, 2.14 mmol, 5.5 eq) in a 7 ml vial. The mixture was shaken at 80°C for 30min and then at 100°C for 1h. The reaction mixture was concentrated and to the mixture was added morpholine (1ml, 11mmol, 35eq) with cooling followed by DMF (1 ml). The reaction mixture was shaken at 80°C for 30min and then at 100°C for 1h. The reaction mixture was cooled to RT, diluted with EtOAc (7ml) and washed with 5% LiCl aq.(7ml). The layers were separated and the aqueous layer washed with EtOAc (7ml). The organic extracts were combined, washed with brine and evaporated. The crude was dissolved in a minimal amount of DCM and purified on Biotage Isolera: Heptane/EtOAc 99/1 ->50/50. Fractions with the product were evaporated to afford methyl 5-methyl-2-morpholino-5,7-dihydrofuro[3,4-b]pyridine-3-carboxylate 50 mg (46% over two steps) as a brown oil. LCMS [M+H]⁺ 279 m/z. ¹H NMR (300 MHz, Chloroform-d) δ 7.74 (d, J = 0.9 Hz, 1H), 5.22 (m,1H), 4.95 - 4.76 (m, 2H), 3.82 (s, 3H), 3.79 - 3.70 (m, 4H), 3.38 - 3.28 (m, 4H), 1.41 (d, J = 6.3 Hz, 3H).

### Examples:

### Preparation of N-(8-methoxy-4-methyl-2-oxo-1H-quinolin-6-yl)-2-morpholino-6,7-dihydro-5H-pyrrolo[3,4-b]pyridine-3-carboxamide (Example 1)

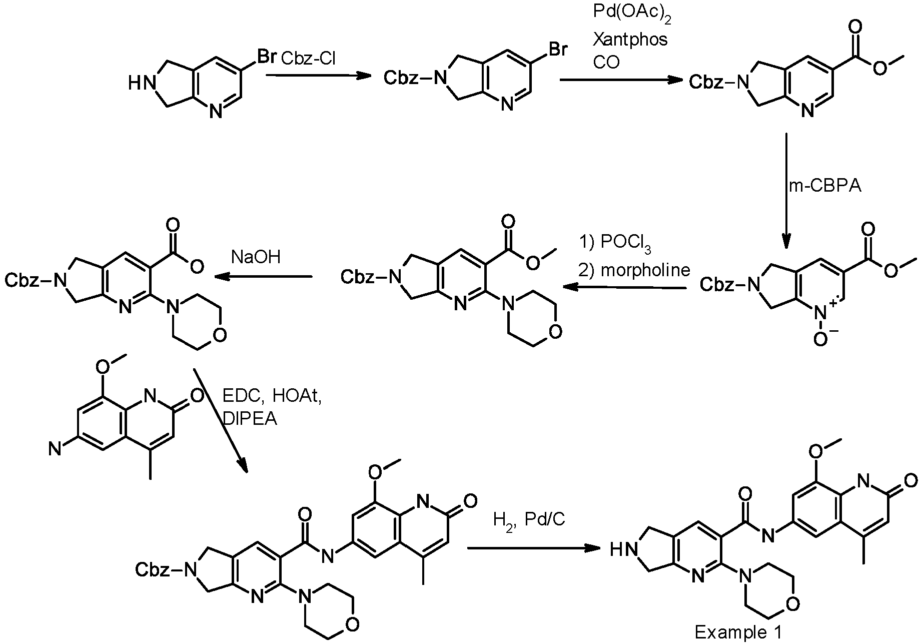

### Preparation of Benzyl 3-bromo-5,7-dihydropyrrolo[3,4-b]pyridine-6-carboxylate:

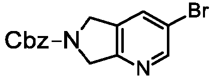

To a suspension of 3-bromo-6,7-dihydro-5H-pyrrolo[3,4-b]pyridine hydrochloride (300 mg, 1.87 mmol) in dry DCM (25 mL) was added DIPEA (812 µL, 4.67 mmol) and the mixture was stirred at room temperature for 15 min followed by drop-wise addition of benzyl chloroformate (295 µL, 2.06 mmol). The reaction mixture was then stirred at room temperature for additional 60 minutes. The reaction was then diluted with EtOAc (125 mL), washed with 0.1 M aq. NaOH (2 x 50 mL) and sat. aq. NaHCO₃ (100 mL), dried over MgSO₄, filtered and concentrated in vacuo. The residue was purified by column chromatography on silica gel (Isolera One, Biotage) using a mixture of Heptane/EtOAc (from 100/0 to 0/100) as the eluent to afford the title product benzyl 3-bromo-5,7-dihydropyrrolo[3,4-b]pyridine-6-carboxylate as a white solid (400 mg, 65%). ¹H NMR (300 MHz, Chloroform-d) δ 8.48 (s, 1H), 7.77 - 7.51 (m, 1H), 7.37 - 7.22 (m, 6H), 5.16 (d, J = 2.3 Hz, 2H), 4.72 - 4.63 (m, 4H).

### Preparation of O6-benzyl O3-methyl 5,7-dihydropyrrolo[3,4-b]pyridine-3,6-dicarboxylate

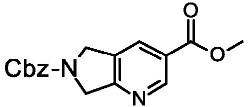

In a two chamber reaction vial, one chamber was charged with benzyl 3-bromo-5,7-dihydropyrrolo[3,4-b]pyridine-6-carboxylate (1.0 g, 3.0 mmol), DMF (8 mL), MeOH (4 mL) and Et₃N (1.25 mL, 9.0 mmol) and flushed with argon at RT for 10 minutes. To this were added Pd(OAc)₂ (100 mg, 0.45 mmol) and Xantphos (260 mg, 0.45 mmol). The second chamber was charged with HCOOH (300 µL, 7.9 mmol) and MsCl (612 µL, 7.9 mmol) in toluene (5 mL) and flushed with argon. To start CO formation, Et₃N (1.6 mL, 11.9 mmol) was quickly added to the second chamber. The reaction mixture was heated at 100°C for 2h. After completion, the reaction mixture was diluted with EtOAc (150 mL) and washed with sat. aq. NaHCO₃ (2 x 75 mL), aq. 5% LiCl (2 x 25 mL) and brine (75 mL), dried over MgSO₄, filtered and concentrated in vacuo. The residue was purified by column chromatography on silica gel (Isolera One, Biotage) using a mixture of DCM/MeOH (from 100/0 to 95/5) as the eluent to afford O6-benzyl O3-methyl 5,7-dihydropyrrolo[3,4-b]pyridine-3,6-dicarboxylate as a white solid (410 mg, 43%). ¹H NMR (300 MHz, Chloroform-d) δ 9.14 (d, J = 1.9 Hz, 1H), 8.20 (dd, J = 18.0, 2.1 Hz, 1H), 7.47 - 7.34 (m, 6H), 5.26 (d, J = 1.9 Hz, 2H), 4.89-4.82 (m, 4H), 3.98 (s, 3H).

### Preparation of O6-benzyl O3-methyl 1-oxido-5,7-dihydropyrrolo[3,4-b]pyridin-1-ium-3,6-dicarboxylate:

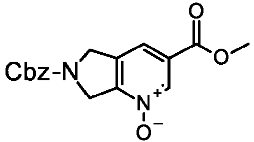

A solution of O6-benzyl O3-methyl 5,7-dihydropyrrolo[3,4-b]pyridine-3,6-dicarboxylate (410 mg, 1.3 mmol) in dry DCM (25 mL) was treated with m-CPBA (321 mg, 77% pure, 1.6 mmol) at 0 oC. The reaction mixture was then allowed to warm to room temperature and stirred for 16h. The reaction was diluted with EtOAc (100 mL), washed with sat. aq. NaHCO₃ (2 x 75 mL) and brine (100 mL). The organic phase was dried over MgSO₄, filtered and concentrated in vacuo. The residue was purified by column chromatography on silica gel (Isolera One, Biotage) using a mixture of DCM/MeOH (from 100/0 to 90/10) as the eluent to afford the title product O6-benzyl O3-methyl 1-oxido-5,7-dihydropyrrolo[3,4-b]pyridin-1-ium-3,6-dicarboxylate as a white solid (360 mg, 85%). LCMS [M+H]⁺: 329.2 m/z

### Preparation of O6-benzyl O3-methyl 2-morpholino-5,7-dihydropyrrolo[3,4-b]pyridine-3,6-dicarboxylate

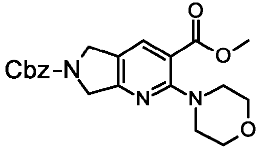

A solution of O6-benzyl O3-methyl 1-oxido-5,7-dihydropyrrolo[3,4-b]pyridin-1-ium-3,6-dicarboxylate (360 mg, 1.1 mmol) in POCl₃ (3.0 mL) was stirred at 80 oC for 1.5h. The crude reaction mixture was concentrated in vacuo and co-evaporated twice with toluene to remove all volatiles. The residue was then redissolved in dry DMF (4.0 mL) and morpholine was added. The mixture was stirred at 120 oC for 1h followed but concentration in vacuo. The residue was purified by column chromatography on silica gel (Isolera One, Biotage) using a mixture of DCM/MeOH (from 100/0 to 90/10) as the eluent to afford the title product O6-benzyl O3-methyl 2-morpholino-5,7-dihydropyrrolo[3,4-b]pyridine-3,6-dicarboxylate as a solid with around 70% UV purity by UPLC-MS (400 mg, 70% pure, 63% yield over two steps).

### Preparation of Benzyl 3-[(8-methoxy-4-methyl-2-oxo-1H-quinolin-6-yl)carbamoyl]-2-morpholino-5,7-dihydropyrrolo[3,4-b]pyridine-6-carboxylate:

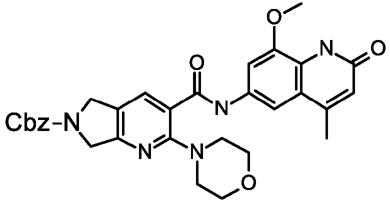

A solution of O6-benzyl O3-methyl 2-morpholino-5,7-dihydropyrrolo[3,4-b]pyridine-3,6-dicarboxylate (400 mg, 70% pure, 1.0 mmol) in MeOH (10 mL) was treated with 0.7 M aq. NaOH (4.5 mL) and the mixture was stirred at room temperature for 16 h followed by quenching with sat. q. NaHCO₃ until pH ~9. The aqueous solution was concentrated in vacuo and the residue used in next step without further purification.

The above residue was suspended in dry DMF (4.0 mL) and treated with EDC·HCl (183 mg, 0.93 mmol) and HOAt (126 mg, 0.96 mmol) followed by DIPEA (472 µL, 2.7 mmol) and 6-amino-8-methoxy-4-methyl-1H-quinolin-2-one (191 mg, 0.93 mmol). The reaction mixture was stirred at 65°C for 4h and then poured into ice-cold water under strong stirring. The formed precipitate was filtered and washed with cold water. The precipitate was purified by column chromatography on silica gel (Isolera One, Biotage) using a mixture of DCM/MeOH (from 100/0 to 80/20) as the eluent to afford the title product benzyl 3-[(8-methoxy-4-methyl-2-oxo-1H-quinolin-6-yl)carbamoyl]-2-morpholino-5,7-dihydropyrrolo[3,4-b]pyridine-6-carboxylate as a white solid (150 mg, 46% yield over two steps).

### Preparation of N-(8-methoxy-4-methyl-2-oxo-1H-quinolin-6-yl)-2-morpholino-6,7-dihydro-5H-pyrrolo[3,4-b]pyridine-3-carboxamide (Example 1):

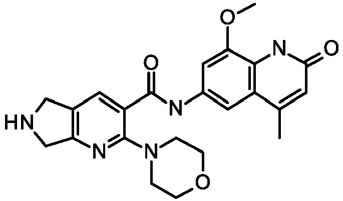

A solution of benzyl 3-[(8-methoxy-4-methyl-2-oxo-1H-quinolin-6-yl)carbamoyl]-2-morpholino-5,7-dihydropyrrolo[3,4-b]pyridine-6-carboxylate (50 mg, 0.087 mmol) in MeOH (5.0 mL) was treated with 10% w/w Pd/C (10 mg) and the reaction mixture was stirred under a H₂ atmosphere for 30 min. The reaction mixture was then filtered over a path of Celite^{®} and concentrated in vacuo. The residue was purified by column chromatography on silica gel (Isolera One, Biotage) using a mixture of DCM/MeOH (from 100/0 to 90/10) as the eluent to afford the title product N-(8-methoxy-4-methyl-2-oxo-1H-quinolin-6-yl)-2-morpholino-6,7-dihydro-5H-pyrrolo[3,4-b]pyridine-3-carboxamide as a pale yellow/white solid (19 mg, 50%).

1H NMR (300 MHz, DMSO-d6) δ 10.62 (s, 2H), 7.79 (s, 1H), 7.76 (d, J = 2.0 Hz, 1H), 7.59 (d, J = 1.9 Hz, 1H), 6.46 (d, J = 1.3 Hz, 1H), 4.12-4.05 (m, 2H), 4.00 (d, J = 1.8 Hz, 2H), 3.90 (s, 3H), 3.71-3.61 (m, 4H), 3.25-3.19 (t, J = 4.6 Hz, 4H), 2.39 (d, J = 1.2 Hz, 3H).

### Preparation of 2-chloro-N-(8-methoxy-4-methyl-2-oxo-1H-quinolin-6-yl)-5,7-dihydrofuro[3,4-b]pyridine-3-carboxamide:

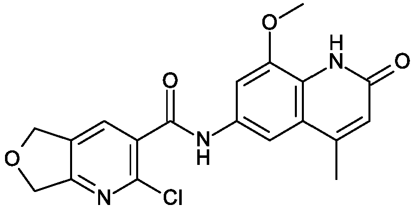

Methyl 2-chloro-5,7-dihydrofuro[3,4-b]pyridine-3-carboxylate (600 mg, 2.8 mmol, 1 eq) was dissolved in THF (15ml), 5M NaOH aq. (1.4 ml, 6 mmol, 2.5 eq) and water (3ml) were added. The mixture was shaken at 45°C overnight and then cooled to 0°C and acidified to pH-7 with 5M HCl aq. The layers were separated and the aqueous layer was extracted with EtOAc (2x15ml). The organic extracts were combined, dried over Na2SO4 and evaporated to afford 600 mg of crude 2-chloro-5,7-dihydrofuro[3,4-b]pyridine-3-carboxylic acid as an orange powder. The crude product was dissolved in DMF (6ml) and 6-amino-8-methoxy-4-methyl-1H-quinolin-2-one (613mg, 3mmol, 1.07eq), DIPEA (1.57ml, 9mmol, 3.21eq), EDC·HCl (573 mg,3 mmol, 1.07 eq) and HOAt (408 mg, 3 mmol, 1.07 eq) were added. The reaction mixture was shaken at 60°C for 2h. After cooling to RT, to the mixture was added water (10ml) and 5%LiCl aq. (10ml) and stirred at RT for 10min. The mixture was filtered, washed with water (2x10ml), dried in air, triturated with ACN (10ml) and filtered. The filtrate was washed with ACN (2x5ml) and dried in vacuo at 1 mbar at RT for 3h to afford 780 mg of 2-chloro-N-(8-methoxy-4-methyl-2-oxo-1H-quinolin-6-yl)-5,7-dihydrofuro[3,4-b]pyridine-3-carboxamide as a yellow powder. LCMS [M+H]⁺ 386 m/z. ¹H NMR (300 MHz, DMSO-d6) δ 10.73 (s, 1H), 10.69 (s, 1H), 8.06 (d, J = 1.0 Hz, 1H), 7.71 (d, J = 1.9 Hz, 1H), 7.52 (d, J = 2.0 Hz, 1H), 6.46 (d, J = 1.3 Hz, 1H), 5.12 (d, J = 2.1 Hz, 2H), 5.00 (d, J = 2.0 Hz, 2H), 3.89 (s, 3H), 2.39 (d, J = 1.2 Hz, 3H).

### Preparation of 2-(3,6-dihydro-2H-pyran-4-yl)-N-(8-methoxy-4-methyl-2-oxo-1H-quinolin-6-yl)-5,7-dihydrofuro[3,4-b]pyridine-3-carboxamide (Example 2)

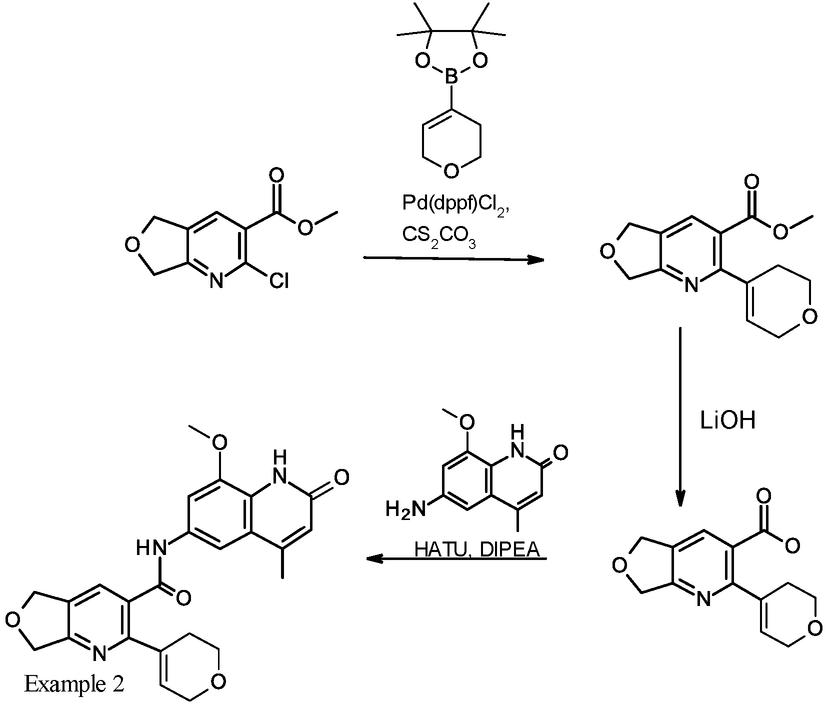

### Preparation of methyl 2-(3,6-dihydro-2H-pyran-4-yl)-5,7-dihydrofuro[3,4-b]pyridine-3-carboxylate:

To a solution of methyl 2-chloro-5,7-dihydrofuro[3,4-b]pyridine-3-carboxylate (66 mg, 0.31 mmol, 1 eq) in dioxane (1.8 mL) and water (0.6 mL), 2-(3,6-dihydro-2H-pyran-4-yl)-4,4,5,5-tetramethyl-1,3,2-dioxaborolane (78 mg, 0.37 mmol, 1.2 eq), Pd(dppf)Cl₂ (6 mg, 0.02 eq) and Cs₂CO₃ (202 mg, 2eq) were added under argon and the reaction mixture stirred at 130°C for 45 min. After completion, solvent was evaporated in vacuo and the product extracted from DCM/water. The combined organic extracts were washed with brine, dried over anhydrous Na2SO4, filtered and evaporated. The crude product was purified by flash chromatography using DCM/MeOH, (0-5% MeOH) to afford methyl 2-(3,6-dihydro-2H-pyran-4-yl)-5,7-dihydrofuro[3,4-b]pyridine-3-carboxylate (54 mg, 67% yield). LCMS [M+H]⁺ 262.20 m/z. ¹H NMR (300 MHz, Chloroform-d) δ 7.92 (s, 1H), 5.85 (s, 1H), 5.21 (s, 2H), 5.11 (s, 2H), 4.32 (q, J = 2.7 Hz, 2H), 3.97 (t, J = 5.3 Hz, 2H), 3.90 (s, 3H), 2.56 (tq, J = 5.2, 2.5 Hz, 2H)

### Preparation of 2-(3,6-dihydro-2H-pyran-4-yl)-5,7-dihydrofuro[3,4-b]pyridine-3-carboxylic acid:

To a solution of methyl 2-(3,6-dihydro-2H-pyran-4-yl)-5,7-dihydrofuro[3,4-b]pyridine-3-carboxylate (112 mg, 0.43 mmol, 1 eq) in methanol (0.6 mL) and THF (0.6mL), 2M LiOH was added (430 µL) and the reaction mixture stirred at 35°C for 3 hours. After completion, 2N HCl was added to neutralize (430 µL). Solvent was evaporated in vacuo with acetonitrile and toluene to remove excess HCl to afford crude 2-(3,6-dihydro-2H-pyran-4-yl)-5,7-dihydrofuro[3,4-b]pyridine-3-carboxylic acid as white solid. LCMS [M+H]⁺ 248.18 m/z.

### Preparation of 2-(3,6-dihydro-2H-pyran-4-yl)-N-(8-methoxy-4-methyl-2-oxo-1H-quinolin-6-yl)-5,7-dihydrofuro[3,4-b]pyridine-3-carboxamide (Example 2):

To a solution of 2-(3,6-dihydro-2H-pyran-4-yl)-5,7-dihydrofuro[3,4-b]pyridine-3-carboxylic acid (0.43 mmol, 1.0 eq) and 6-amino-8-methoxy-4-methyl-1H-quinolin-2-one (96 mg, 0.47 mmol, 1.1 eq) in DMF (3 mL), DIPEA (150 uL, 0.85 mmol, 2 eq) and HATU (79 mg, 0.47 mmol, 1.2 eq) were added and the reaction mixture stirred at room temperature overnight. After completion, the reaction mixture was extracted from EtOAc and 5% LiCl. The combined organic extracts were washed with water and brine. After evaporation of the solvent, the crude product was purified by silicagel flash chromatography using DCM/MeOH, (0-10% MeOH) to afford 82 mg (44% yield) of 2-(3,6-dihydro-2H-pyran-4-yl)-N-(8-methoxy-4-methyl-2-oxo-1H-quinolin-6-yl)-5,7-dihydrofuro[3,4-b]pyridine-3-carboxamide. LCMS [M+H]⁺ 434.19 m/z. ¹H NMR (300 MHz, DMSO-d6) δ 10.65 (s, 1H), 10.48 (s, 1H), 7.88 (s, 1H), 7.62 (d, J = 1.9 Hz, 1H), 7.46 (d, J = 1.9 Hz, 1H), 6.46 (d, J = 1.3 Hz, 1H), 6.14-6.10 (m, 1H), 5.13 (s, 2H), 5.02 - 4.93 (m, 2H), 4.11-4.06 (m, 2H), 3.88 (s, 3H), 3.76 (t, J = 5.4 Hz, 2H), 2.62-2.53 (m, 2H), 2.38 (d, J = 1.2 Hz, 3H)

### Preparation of 2-(cyclopenten-1-yl)-N-(8-methoxy-4-methyl-2-oxo-1H-quinolin-6-yl)-5,7-dihydrofuro[3,4-b]pyridine-3-carboxamide (Example 3)

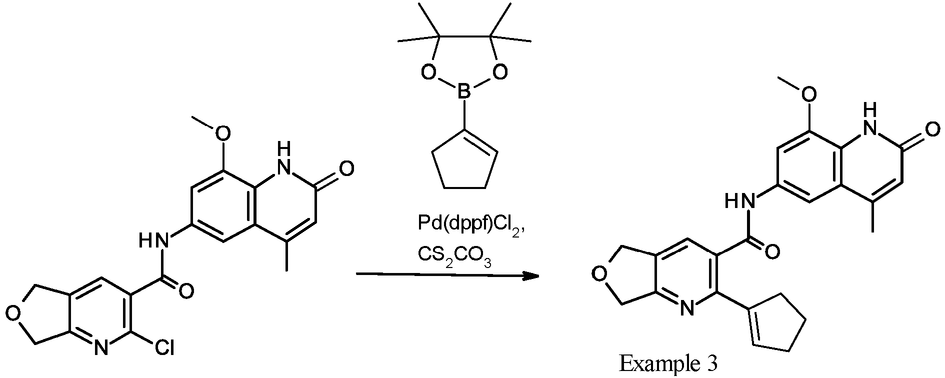

### Preparation of 2-(cyclopenten-1-yl)-N-(8-methoxy-4-methyl-2-oxo-1H-quinolin-6-yl)-5,7-dihydrofuro[3,4-b]pyridine-3-carboxamide (Example 3):

To a solution of 2-chloro-N-(8-methoxy-4-methyl-2-oxo-1H-quinolin-6-yl)-5,7-dihydrofuro[3,4-b]pyridine-3-carboxamide (77 mg, 0.2 mmol, 1 eq) in dioxane (1.6 mL) and water (0.5 mL), 2-(cyclopenten-1-yl)-4,4,5,5-tetramethyl-1,3,2-dioxaborolane (117 mg, 0.6 mmol, 3 eq), Pd(dppf)Cl₂ (3.4 mg, 0.02 eq) and Cs₂CO₃ (130 mg, 2eq) were added under argon and the reaction mixture stirred at 130°C for 45 min. After completion, solvent was evaporated in vacuo and the product extracted from EtOAc/water. The combined organic extracts were washed with brine, dried over anhydrous Na₂SO₄, filtered and evaporated. The crude product was purified by flash chromatography using DCM/MeOH, (0-10% MeOH) to afford methyl 2-(cyclopenten-1-yl)-N-(8-methoxy-4-methyl-2-oxo-1H-quinolin-6-yl)-5,7-dihydrofuro[3,4-b]pyridine-3-carboxamide (29 mg, 35% yield). LCMS [M+H]⁺ 418.21 m/z. ¹H NMR (300 MHz, Chloroform-d) δ 7.92 (s, 1H), 5.85 (s, 1H), 5.21 (s, 2H), 5.11 (s, 2H), 4.32 (q, J = 2.7 Hz, 2H), 3.97 (t, J = 5.3 Hz, 2H), 3.90 (s, 3H), 2.56 (tq, J = 5.2, 2.5 Hz, 2H)

### Preparation of N-(8-methoxy-4-methyl-2-oxo-1H-quinolin-6-yl)-2-(1-methyl-2-azabicyclo[2.1.1]hexan-2-yl)-5,7-dihydrofuro[3,4-b]pyridine-3-carboxamide (Example 4):

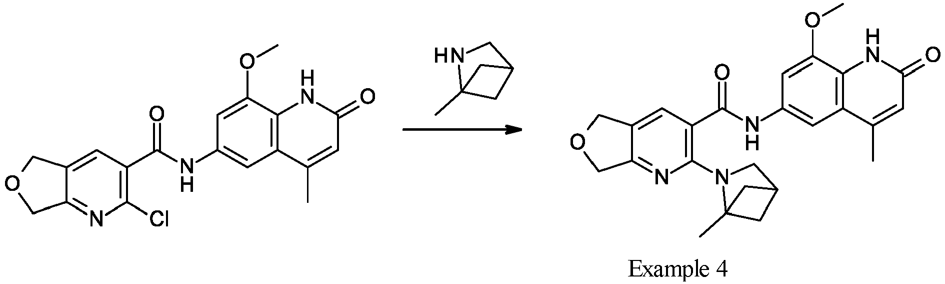

2-Chloro-N-(8-methoxy-4-methyl-2-oxo-1H-quinolin-6-yl)-5,7-dihydrofuro[3,4-b]pyridine-3-carboxamide (50mg, 0.13mmol, leq) and 4-methyl-3-azabicyclo[2.1.1]hexane (30mg, 0.31mmol, 2.38eq) were dissolved in DMSO (2.4ml) and DIPEA (0.1ml, 0.57mmol, 4.38eq) was added. The reaction mixture was heated in a microwave reactor at 135°C for 1h. 4-methyl-3-azabicyclo[2.1.1]hexane (30mg, 0.31mmol, 2.38eq) was added and then heated at 135°C for 2h then 145°C for 45 minutes. Purified on Biotage Isolera: ACN (0.1%FA)/H2O(0.1%FA) 0/100 then 0/100->80/20. Fractions with the product were evaporated, triturated with Et₂O (0.7ml), solvent decanted and dried in vacuo at 1 mbar at room temperature for 3h to afford the title product N-(8-methoxy-4-methyl-2-oxo-1H-quinolin-6-yl)-2-(1-methyl-2-azabicyclo[2.1.1]hexan-2-yl)-5,7-dihydrofuro[3,4-b]pyridine-3-carboxamide, 9mg (yield 15%). LCMS [M+H]⁺ 447 m/z. ¹H NMR (300 MHz, DMSO-d6) δ 11.23 (s, 1H), 10.65 (s, 1H), 8.00 (s, 1H), 7.70 (d, J = 1.9 Hz, 1H), 7.57 (d, J = 2.0 Hz, 1H), 6.46 (s, 1H), 5.06 (s, 2H), 4.89 (d, J = 2.0 Hz, 2H), 3.90 (s, 3H), 3.38 (s, 2H), 2.73 (d, J = 2.7 Hz, 1H), 2.39 (d, J = 1.2 Hz, 3H), 1.78 - 1.68 (m, 4H), 1.63 (s, 3H).

### Preparation of 2-(6,6-difluoro-3-azabicyclo[3.1.0]hexan-3-yl)-N-(8-methoxy-4-methyl-2-oxo-1H-quinolin-6-yl)-5,7-dihydrofuro[3,4-b]pyridine-3-carboxamide (Example 5):

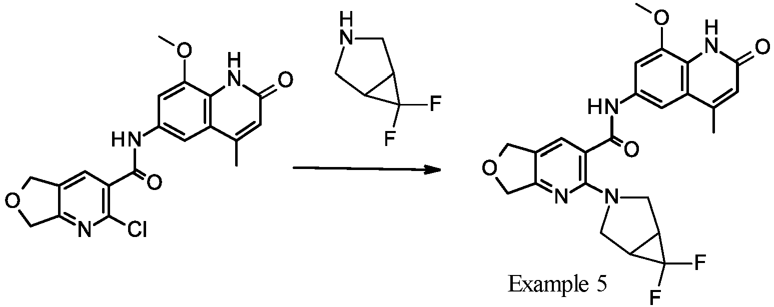

A 100 µl Chromacol 01-CVG vial was charged with 6,6-difluoro-3-azabicyclo[3.1.0]hexane (2.4 µmol), 2-chloro-N-(8-methoxy-4-methyl-2-oxo-1H-quinolin-6-yl)-5,7-dihydrofuro[3,4-b]pyridine-3-carboxamide (2.4 µmol ), dry DMSO (48 µl) and DIPEA (9.6 µmol, 1.7 µl). The mixture was heated at 140 °C overnight, cooled and spinned down. The reaction mixture was transferred to a Twin.Tec plate and evaporated using SpeedVac (45 °C, 1-2 h). The crude product was purified by HPLC-CLND to give the pure title compound 2-(6,6-difluoro-3-azabicyclo[3.1.0]hexan-3-yl)-N-(8-methoxy-4-methyl-2-oxo-1H-quinolin-6-yl)-5,7-dihydrofuro[3,4-b]pyridine-3-carboxamide, 161 µg. LCMS [M+H]⁺ 469.17 m/z

### Preparation of 2-(dimethylamino)-N-(8-methoxy-4-methyl-2-oxo-1H-quinolin-6-yl)-5,7-dihydrofuro[3,4-b]pyridine-3-carboxamide (Example 6):

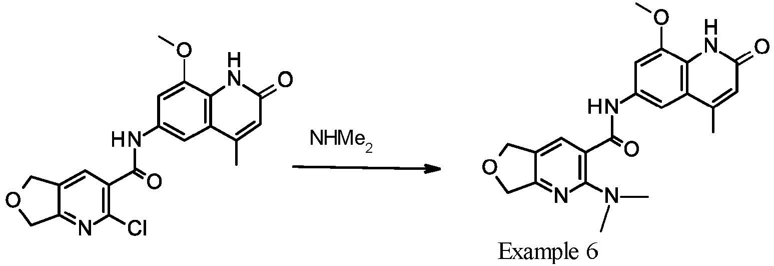

To a solution of 2-chloro-N-(8-methoxy-4-methyl-2-oxo-1H-quinolin-6-yl)-5,7-dihydrofuro[3,4-b]pyridine-3-carboxamide (200 mg, 1 eq) in dry DMF was added NHMe₂ (2M in THF, 1.0 mL) and the reaction mixture was stirred at 120°C for 1h. The crude reaction mixture was filtered through a path of Celite^{®} and directly purified by reverse-phase preparative HPLC to afford the title product 2-(dimethylamino)-N-(8-methoxy-4-methyl-2-oxo-1H-quinolin-6-yl)-5,7-dihydrofuro[3,4-b]pyridine-3-carboxamide as a pale pink solid (55 mg).¹H NMR (300 MHz, DMSO-d6) δ 10.63 (s, 1H), 10.45 (s, 1H), 7.73 (d, J = 2.0 Hz, 1H), 7.70 (s, 1H), 7.56 (d, J = 2.0 Hz, 1H), 6.44 (d, J = 1.3 Hz, 1H), 5.00 (t, J = 2.1 Hz, 2H), 4.84 (t, J = 2.1 Hz, 2H), 3.88 (s, 3H), 2.97 (s, 6H), 2.37 (d, J = 1.1 Hz, 3H).

### Preparation of N-(4,8-dimethyl-2-oxo-1H-quinolin-6-yl)-2-morpholino-5,7-dihydrofuro[3,4-b]pyridine-3-carboxamide (Example 7):

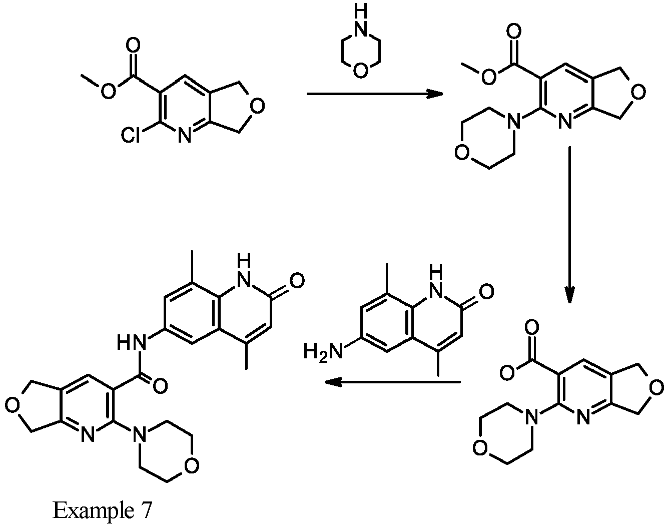

### Preparation of methyl 2-morpholino-5,7-dihydrofuro[3,4-b]pyridine-3-carboxylate:

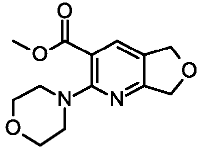

To a solution of methyl 2-chloro-5,7-dihydrofuro[3,4-b]pyridine-3-carboxylate (213.6 mg, 1 mmol, 1 eq) in dry DMSO (5 mL), morpholine (175 µL, 2 mmol, 2 eq), and DIPEA (522 µL, 3 mmol, 3 eq) were added and stirred at 125°C for 3 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude compound was purified by flash chromatography using EtOAc/heptane, (20-70% EtOAc) to afford methyl 2-morpholino-5,7-dihydrofuro[3,4-b]pyridine-3-carboxylate (177.7 mg, 67 % yield) as white solid.

### Preparation of 2-morpholino-5,7-dihydrofuro[3,4-b]pyridine-3-carboxylic acid:

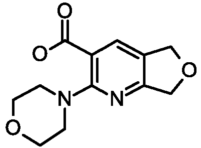

To a solution of methyl 2-morpholino-5,7-dihydrofuro[3,4-b]pyridine-3-carboxylate (40 mg, 0.15 mmol, 1 eq) in methanol (2 mL) and THF (2 mL), was added 2M LiOH (350 µL, 0.35 mmol, 2 eq) and the reaction mixture stirred at 30°C for 3 h. After completion, 2N HCl was added to neutralize (350 µL, 0.35 mmol, 2 eq). Solvent was evaporated in vacuo with acetonitrile and toluene to remove acid to afford crude 2-morpholino-5,7-dihydrofuro[3,4-b]pyridine-3-carboxylic acid as a yellow solid. LCMS [M+H]⁺ 251.17 m/z.

### Preparation of N-(4,8-dimethyl-2-oxo-1H-quinolin-6-yl)-2-morpholino-5,7-dihydrofuro[3,4-b]pyridine-3-carboxamide (Example 7):

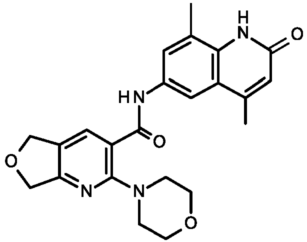

To a solution of 2-morpholino-5,7-dihydrofuro[3,4-b]pyridine-3-carboxylic acid (0.15 mmol, leq) and 6-amino-4,8-dimethyl-1H-quinolin-2-one (31 mg, 0.15 mmol, 1 eq) in DMF (5 mL), DIPEA (80 uL, 0.45 mmol, 3 eq) and HATU (63.3 mg, 0.16 mmol, 1.1 eq) were added and the reaction mixture stirred at 60°C overnight.

After completion, the reaction mixture was poured into water and extracted with EtOAc (2 x 30 mL). The combined extracts were washed with water, 5% LiCl and brine. The precipitated material was filtered off, washed with water and dried to afford 31.3 mg of the title compound N-(4,8-dimethyl-2-oxo-1H-quinolin-6-yl)-2-morpholino-5,7-dihydrofuro[3,4-b]pyridine-3-carboxamide as white solid. (49% yield over two steps), LCMS [M+H]⁺ 421.22 m/z.¹H NMR (300 MHz, DMSO-*d*₆) δ 10.73 (s, 1H), 10.52 (s, 1H), 8.02 (d, *J* = 2.2 Hz, 1H), 7.83 (s, 1H), 7.72 - 7.65 (m, 1H), 6.46 (d, *J* = 1.3 Hz, 1H), 5.04 (d, *J* = 2.2 Hz, 2H), 4.89 (t, *J* = 2.1 Hz, 2H), 3.70 - 3.57 (m, 4H), 3.28-3.22 (m, 4H), 2.44 (s, 3H), 2.40 (d, *J* = 1.2 Hz, 3H).

### Preparation of N-(6-methyl-8-oxo-3,9-dihydro-2H-furo[3,2-h]quinolin-4-yl)-2-morpholino-5,7-dihydrofuro[3,4-b]pyridine-3-carboxamide (Example 8):

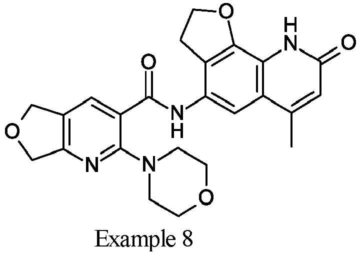

N-(6-methyl-8-oxo-3,9-dihydro-2H-furo[3,2-h]quinolin-4-yl)-2-morpholino-5,7-dihydrofuro[3,4-b]pyridine-3-carboxamide was made following the procedure described in Example 7 (N-(4,8-dimethyl-2-oxo-1H-quinolin-6-yl)-2-morpholino-5,7-dihydrofuro[3,4-b]pyridine-3-carboxamide) from 2-morpholino-5,7-dihydrofuro[3,4-b]pyridine-3-carboxylic acid and 4-amino-6-methyl-3,9-dihydro-2H-furo[3,2-h]quinolin-8-one. Obtained: 22.5 mg (33% yield over two steps) of N-(6-methyl-8-oxo-3,9-dihydro-2H-furo[3,2-h]quinolin-4-yl)-2-morpholino-5,7-dihydrofuro[3,4-b]pyridine-3-carboxamide LCMS [M+H]⁺ 449.19 m/z. ¹HNMR (300 MHz, DMSO-d6) δ 11.06 (s, 1H), 10.21 (s, 1H), 7.87 (s, 1H), 7.62 (s, 1H), 6.37 (s, 1H), 5.05 (s, 2H), 4.89 (s, 2H), 4.78-4.68 (m, 2H), 3.68 (t, J = 4.5 Hz, 4H), 3.00 - 3.50 (m, 4H), 2.37 (s, 3H).

### Preparation of N-(7-fluoro-8-methoxy-4-methyl-2-oxo-1H-quinolin-6-yl)-2-morpholino-5,7-dihydrofuro[3,4-b]pyridine-3-carboxamide (Example 9):

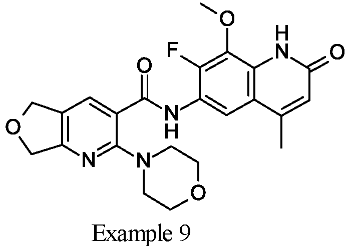

N-(7-fluoro-8-methoxy-4-methyl-2-oxo-1H-quinolin-6-yl)-2-morpholino-5,7-dihydrofuro[3,4-b]pyridine-3-carboxamide was made following the procedure described in Example 7 (N-(4,8-dimethyl-2-oxo-1H-quinolin-6-yl)-2-morpholino-5,7-dihydrofuro[3,4-b]pyridine-3-carboxamide) from 2-morpholino-5,7-dihydrofuro[3,4-b]pyridine-3-carboxylic acid and 6-amino-7-fluoro-8-methoxy-4-methyl-1H-quinolin-2-one. Obtained: 9.4 mg (14% yield) of N-(7-fluoro-8-methoxy-4-methyl-2-oxo-1H-quinolin-6-yl)-2-morpholino-5,7-dihydrofuro[3,4-b]pyridine-3-carboxamide, LCMS [M+H]⁺ 455.16 m/z. ¹H NMR (300 MHz, DMSO-d6) δ 11.25 (s, 1H), 10.87 (s, 1H), 8.07-7.98 (m, 2H), 6.45 (s, 1H), 5.07 (s, 2H), 4.92 (d, J = 2.1 Hz, 2H), 3.93 (s, 3H), 3.78-3.67 (m, 4H), 3.30-3.20 (m, 4H), 2.40 (s, 3H).

### Preparation of N-(7-methyl-9-oxo-3,10-dihydro-2H-[1,4]dioxino[2,3-h]quinolin-5-yl)-2-morpholino-5,7-dihydrofuro[3,4-b]pyridine-3-carboxamide (Example 10):

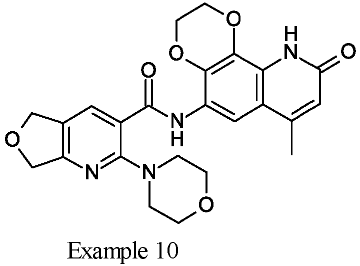

N-(7-methyl-9-oxo-3,10-dihydro-2H-[1,4]dioxino[2,3-h]quinolin-5-yl)-2-morpholino-5,7-dihydrofuro[3,4-b]pyridine-3-carboxamide was made following the procedure described in Example 7 (N-(4,8-dimethyl-2-oxo-1H-quinolin-6-yl)-2-morpholino-5,7-dihydrofuro[3,4-b]pyridine-3-carboxamide) from 2-morpholino-5,7-dihydrofuro[3,4-b]pyridine-3-carboxylic acid and 5-amino-7-methyl-3,10-dihydro-2H-[1,4]dioxino[2,3-h]quinolin-9-one. Precipitate was additionally washed with DMF and 25% water in acetonitrile to afford desired product. Obtained: 21.7 (25% yield) mg of N-(7-methyl-9-oxo-3,10-dihydro-2H-[1,4]dioxino[2,3-h]quinolin-5-yl)-2-morpholino-5,7-dihydrofuro[3,4-b]pyridine-3-carboxamide, LCMS [M+H]⁺ 465.1 m/z. ¹H NMR (300 MHz, DMSO-d6) δ 11.01 (s, 1H), 10.76 (s, 1H), 8.33 (s, 1H), 8.21 (s, 1H), 6.32 (s, 1H), 5.09 (s, 2H), 4.94 (t, J = 2.0 Hz, 2H), 4.54 - 4.38 (m, 4H), 3.78 (dd, J = 6.5, 3.0 Hz, 4H), 3.18 (dd, J = 6.0, 3.3 Hz, 4H), 2.38 (s, 3H).

### Preparation of N-(4,7-dimethyl-2-oxo-1H-quinolin-6-yl)-2-morpholino-5,7-dihydrofuro[3,4-b]pyridine-3-carboxamide (Example 11):

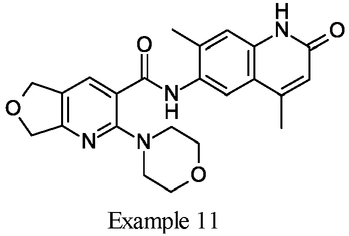

N-(4,7-dimethyl-2-oxo-1H-quinolin-6-yl)-2-morpholino-5,7-dihydrofuro[3,4-b]pyridine-3-carboxamide was made following the procedure described in Example 7 (N-(4,8-dimethyl-2-oxo-1H-quinolin-6-yl)-2-morpholino-5,7-dihydrofuro[3,4-b]pyridine-3-carboxamide) from 2-morpholino-5,7-dihydrofuro[3,4-b]pyridine-3-carboxylic acid and 6-amino-4,7-dimethyl-1H-quinolin-2-one. Precipitate was additionally washed with DMF and 25% water in acetonitrile to afford product.

Obtained: 26.9 mg (38% yield) of N-(4,7-dimethyl-2-oxo-1H-quinolin-6-yl)-2-morpholino-5,7-dihydrofuro[3,4-b]pyridine-3-carboxamide, LCMS [M+H]+ 421.17 m/z.¹H NMR (300 MHz, ) δ 11.57 (s, 1H), 10.08 (s, 1H), 7.91 (s, 1H), 7.85 (s, 1H) 7.18 (s, 1H), 6.37 (s, 1H), 5.06 (s, 2H), 4.89 (t, J = 2.1 Hz, 2H), 3.71 (dd, J = 5.3, 3.8 Hz, 4H), 3.00 - 3.50 (m, 4H), 2.39 (s, 3H), 2.35 (s, 3H).

### Preparation of N-(8-ethoxy-4-methyl-2-oxo-1H-quinolin-6-yl)-2-morpholino-5,7-dihydrofuro[3,4-b]pyridine-3-carboxamide (Example 12):

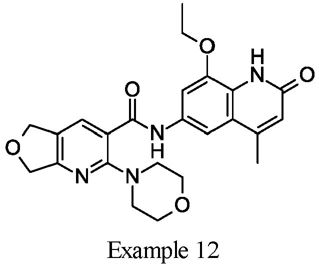

N-(8-ethoxy-4-methyl-2-oxo-1H-quinolin-6-yl)-2-morpholino-5,7-dihydrofuro[3,4-b]pyridine-3-carboxamide was made following the procedure described in Example 7 (N-(4,8-dimethyl-2-oxo-1H-quinolin-6-yl)-2-morpholino-5,7-dihydrofuro[3,4-b]pyridine-3-carboxamide) from 2-morpholino-5,7-dihydrofuro[3,4-b]pyridine-3-carboxylic acid and 6-amino-8-ethoxy-4-methyl-1H-quinolin-2-one.Precipitate was additionally washed with DMF and 25% water in acetonitrile to afford product. Obtained: 31.2 mg (41% yield) of N-(8-ethoxy-4-methyl-2-oxo-1H-quinolin-6-yl)-2-morpholino-5,7-dihydrofuro[3,4-b]pyridine-3-carboxamide, LCMS [M+H]⁺ 451.7 m/z.¹H NMR (300 MHz, DMSO-d6) δ 10.63-10.50 (m, 2H), 7.84 (s, 1H), 7.76 (d, J = 1.9 Hz, 1H), 7.55 (d, J = 1.9 Hz, 1H), 6.46 (s, 1H), 5.04 (s, 2H), 4.89 (s, 2H), 4.14 (q, J = 6.9 Hz, 2H), 3.70 - 3.60 (m, 4H), 3.30-3.18 (m, 4H), 2.39 (s, 3H), 1.45 (t, J = 6.9 Hz, 3H).

### Preparation of 2-morpholino-N-(4,7,8-trimethyl-2-oxo-1H-quinolin-6-yl)-5,7-dihydrofuro[3,4-b]pyridine-3-carboxamide (Example 13):

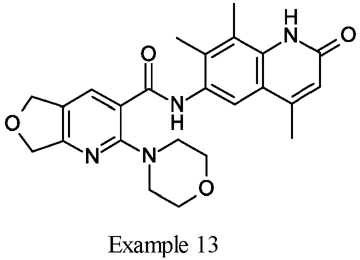

2-Morpholino-N-(4,7,8-trimethyl-2-oxo-1H-quinolin-6-yl)-5,7-dihydrofuro[3,4-b]pyridine-3-carboxamide was made following the procedure described in Example 7 (N-(4,8-dimethyl-2-oxo-1H-quinolin-6-yl)-2-morpholino-5,7-dihydrofuro[3,4-b]pyridine-3-carboxamide) from 2-morpholino-5,7-dihydrofuro[3,4-b]pyridine-3-carboxylic acid and 6-amino-4,7,8-trimethyl-1H-quinolin-2-one. Precipitate was additionally washed with water and EtOAc to afford product. Obtained: 22.0 mg (33% yield) of 2-morpholino-N-(4,7,8-trimethyl-2-oxo-1H-quinolin-6-yl)-5,7-dihydrofuro[3,4-b]pyridine-3-carboxamide, LCMS [M+H]⁺ 435.2 m/z. ¹H NMR (300 MHz, DMSO-d6) δ 10.63 (s, 1H), 10.16 (s, 1H), 7.91 (s, 1H), 7.64 (s, 1H), 6.41 (s, 1H), 5.06 (s, 2H), 4.95 - 4.85 (m, 2H), 3.75-3.68 (m, 4H), 3.00-3.5 (m, 4H), 2.42 - 2.36 (m, 3H), 2.28 (s, 3H).

### Preparation of N-(7-methoxy-4-methyl-2-oxo-1H-quinolin-6-yl)-2-morpholino-5,7-dihydrofuro[3,4-b]pyridine-3-carboxamide (Example 14):

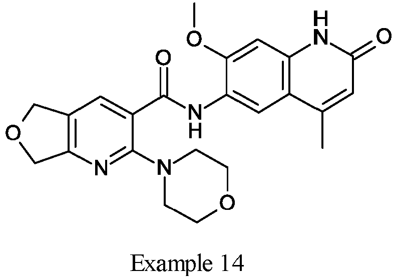

N-(7-methoxy-4-methyl-2-oxo-1H-quinolin-6-yl)-2-morpholino-5,7-dihydrofuro[3,4-b]pyridine-3-carboxamide was made following the procedure described in Example 7 (N-(4,8-dimethyl-2-oxo-1H-quinolin-6-yl)-2-morpholino-5,7-dihydrofuro[3,4-b]pyridine-3-carboxamide) from 2-morpholino-5,7-dihydrofuro[3,4-b]pyridine-3-carboxylic acid and 6-amino-7-methoxy-4-methyl-1H-quinolin-2-one. Precipitate was additionally washed with 5% acetonitrile in water to afford product. Obtained: 34.5 mg (46% yield) of N-(7-methoxy-4-methyl-2-oxo-1H-quinolin-6-yl)-2-morpholino-5,7-dihydrofuro[3,4-b]pyridine-3-carboxamide, LCMS [M+H]⁺ 437.62 m/z. ¹H NMR (300 MHz, DMSO-d6) δ 10.84 (s, 1H), 8.75 (s, 1H), 8.55 (s 1H), 8.18 (s, 1H), 7.00 (s, 1H), 6.30 (s, 1H), 5.09 (s, 2H), 4.94 (s, 2H), 3.98 (s, 3H), 3.79-3.72 (m, 4H), 3.44-3.37 (m,4H), 2.39 (s, 3H).

### Preparation of N-(8-methoxy-4-methyl-2-oxo-1H-quinolin-6-yl)-2-morpholino-5,7-dihydrofuro[3,4-b]pyridine-3-carboxamide (Example 15):

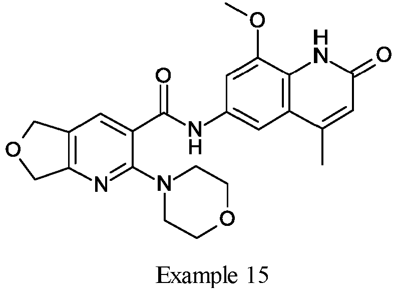

To a solution of 2-morpholino-5,7-dihydrofuro[3,4-b]pyridine-3-carboxylic acid (2.6 g, 10.4 mmol) in DMF (30 mL) were added sequentially 6-amino-8-methoxy-4-methylquinolin-2(1H)-one (2.12 g, 10.4 mmol), EDC·HCl (3.97 g, 20.8 mmol), HOAt (2.82 mg, 20.8 mmol) and DIPEA (7.5 mL, 41.6 mmol) at 0 °C. Then reaction mixture was stirred at room temperature for 16 h. After completion, the reaction mixture was quenched with water (100 mL) and the precipitated solid was filtered, washed with cold water and dried under vacuum to afford N-(8-methoxy-4-methyl-2-oxo-1H-quinolin-6-yl)-2-morpholino-5,7-dihydrofuro[3,4-b]pyridine-3-carboxamide (2.26 g, 50 %) as an off white solid. ¹H NMR (400 MHz, DMSO-d6): δ 10.64 (br s, 1H), 10.56 (br s, 1H), 7.83 (s, 1H), 7.74 (d, J = 1.2 Hz, 1H), 7.56 (d, J = 1.6 Hz, 1H), 6.45 (s, 1H), 5.04 (s, 2H), 4.88 (s, 2H), 3.89 (s, 3H), 3.65 (t, J = 3.6 Hz, 4H), 3.26 (t, J = 3.6 Hz 4H), 2.38 (s, 3H), LCMS [M+H]⁺: 437.17 m/z.

### Preparation of 2-(3,3-difluoropyrrolidin-1-yl)-N-(8-methoxy-4-methyl-2-oxo-1H-quinolin-6-yl)-5,7-dihydrofuro[3,4-b]pyridine-3-carboxamide (Example 16)

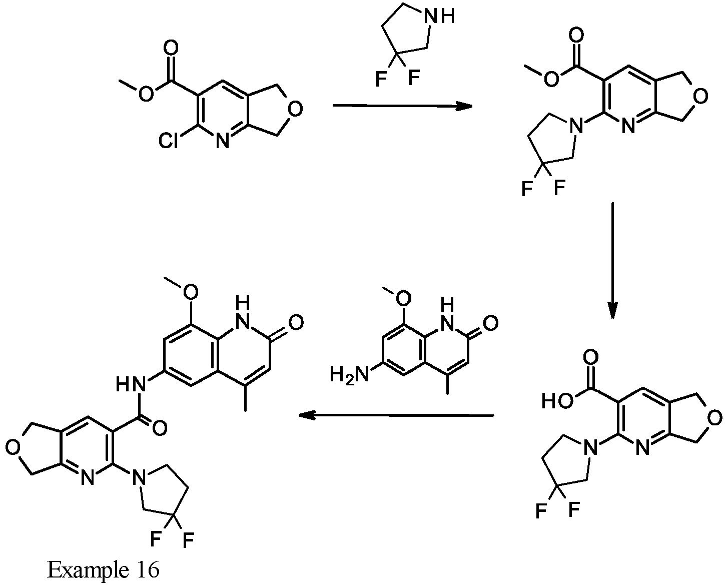

### Preparation of methyl 2-(3,3-difluoropyrrolidin-1-yl)-5,7-dihydrofuro[3,4-b]pyridine-3-carboxylate:

To a solution of methyl 2-chloro-5,7-dihydrofuro[3,4-b]pyridine-3-carboxylate (6) (42.7 mg, 0.2 mmol, 1 eq) in dry DMSO (1 mL), 3,3-difluoropyrrolidine (57.4 mg, 0.4 mmol, 2 eq) and DIPEA (140 µL, 0.8 mmol, 4 eq) were added and the reaction mixture stirred at 125°C for 1 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (10-50% EtOAc) to afford methyl 2-(3,3-difluoropyrrolidin-1-yl)-5,7-dihydrofuro[3,4-b]pyridine-3-carboxylate (128 mg, 56.6 % yield) as a yellow solid. LCMS [M+H]⁺ 285.19 m/z. ¹H NMR (300 MHz, DMSO-d6) δ 7.92 (s, 1H), 4.99 (s, 2H), 4.84 (s, 2H), 3.83 (s, 3H), 3.72 (t, J = 13.2 Hz, 2H), 3.57 (t, J = 7.3 Hz, 2H), 2.47 - 2.36 (m, 2H).

### Preparation of 2-(3,3-difluoropyrrolidin-1-yl)-5,7-dihydrofuro[3,4-b]pyridine-3-carboxylic acid:

To a solution of methyl 2-(3,3-difluoropyrrolidin-1-yl)-5,7-dihydrofuro[3,4-b]pyridine-3-carboxylate (30 mg, 0.125 mmol, 1 eq) in methanol (1 mL) and THF (1 mL), 2M LiOH was added (125 µL, 0.250 mmol, 2 eq) and the reaction mixture stirred at 35°C for 3 h. After completion, 2N HCl was added to neutralize (125 µL, 0.250 mmol, 2 eq). Solvent was evaporated in vacuo with acetonitrile and toluene to remove excess HCl to afford 2-(3,3-difluoropyrrolidin-1-yl)-5,7-dihydrofuro[3,4-b]pyridine-3-carboxylic acid as a yellow solid. LCMS [M+H]⁺ 285.19 m/z.

### Preparation of 2-(3,3-difluoropyrrolidin-1-yl)-N-(8-methoxy-4-methyl-2-oxo-1H-quinolin-6-yl)-5,7-dihydrofuro[3,4-b]pyridine-3-carboxamide (Example 16):

To a solution of 2-(3,3-difluoropyrrolidin-1-yl)-5,7-dihydrofuro[3,4-b]pyridine-3-carboxylic acid (0.125 mmol, 1 eq) and 6-amino-8-methoxy-4-methyl-1H-quinolin-2-one (25.3 mg, 0.125 mmol, 1 eq) in DMF (4 mL), DIPEA (60 uL, 0.35 mmol, 3 eq) and HATU (47 mg, 0.125 mmol, 1 eq) were added and the reaction mixture stirred at 35°C for 3 h and then at RT overnight.

After completion, the reaction mixture was poured into water and extracted with EtOAc (2 x 30 mL). The combined extracts were washed with water, 5% LiCl and brine. After evaporation of the solvent, the crude product was additionally washed with 25% water in acetonitrile, then dried to afford 34 mg of 2-(3,3-difluoropyrrolidin-1-yl)-N-(8-methoxy-4-methyl-2-oxo-1H-quinolin-6-yl)-5,7-dihydrofuro[3,4-b]pyridine-3-carboxamide as off-white solid (17% yield over two steps). LCMS [M+H]⁺ 455.4 m/z.¹H NMR (300 MHz, DMSO-d6) δ 10.66 (s, 1H), 10.59 (s, 1H), 7.77 (s, 1H), 7.73 (d, J = 1.9 Hz, 1H), 7.56 (d, J = 1.9 Hz, 1H), 6.46 (s, 1H), 5.02 (s, 2H), 4.87 (s, 2H), 3.89 (s, 3H), 3.80 (t, J = 13.2 Hz, 2H), 3.65 (t, J = 7.2 Hz, 2H), 2.38 (s, 3H).

### Preparation of N-(8-methoxy-4-methyl-2-oxo-1H-quinolin-6-yl)-2-(3-oxa-8-azabicyclo[3.2.1]octan-8-yl)-5,7-dihydrofuro[3,4-b]pyridine-3-carboxamide (Example 17):

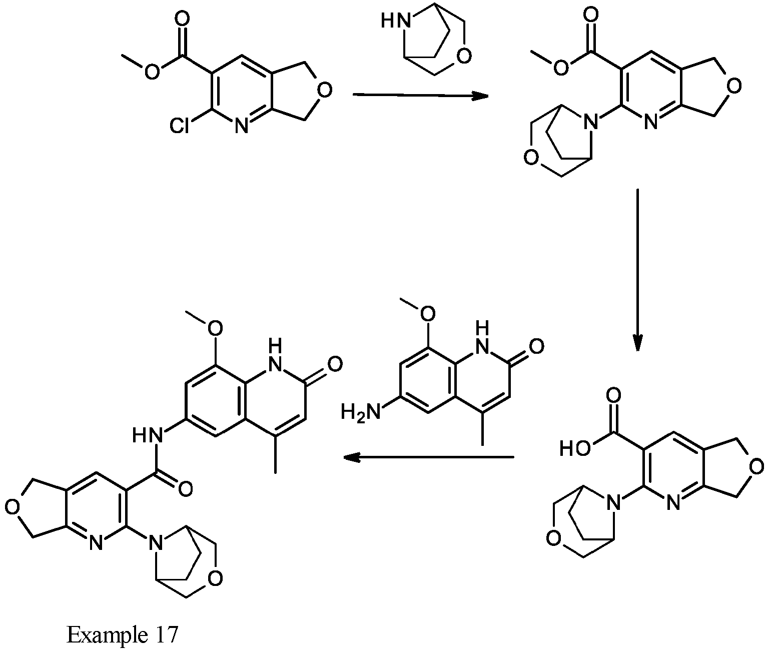

### Preparation of methyl 2-(3-oxa-8-azabicyclo[3.2.1]octan-8-yl)-5,7-dihydrofuro[3,4-b]pyridine-3-carboxylate:

To a solution of methyl 2-chloro-5,7-dihydrofuro[3,4-b]pyridine-3-carboxylate (6) (42.7 mg, 0.2 mmol, 1 eq) in dry DMSO (1 mL), 3-oxa-8-azabicyclo[3.2.1]octane (60 mg, 0.4 mmol, 2 eq) and DIPEA (140 µL, 0.8 mmol, 4 eq) were added and the reaction mixture stirred at 125°C for 1 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (10-50% EtOAc) to afford 48 mg of methyl 2-(3-oxa-8-azabicyclo[3.2.1]octan-8-yl)-5,7-dihydrofuro[3,4-b]pyridine-3-carboxylate (33% yield) as a white solid. LCMS [M+H]⁺ 291.16 m/z.

### Preparation of 2-(3-oxa-8-azabicyclo[3.2.1]octan-8-yl)-5,7-dihydrofuro[3,4-b]pyridine-3-carboxylic acid:

To a solution of methyl 2-(3-oxa-8-azabicyclo[3.2.1]octan-8-yl)-5,7-dihydrofuro[3,4-b]pyridine-3-carboxylate (41 mg, 0.14 mmol, 1 eq) in methanol (1 mL) and THF (1 mL), 2M LiOH was added (400 µL, 0.80 mmol) and the reaction mixture stirred at 35°C for 3 h. After completion, 2N HCl was added (400 µL, 0.80 mmol). Solvent was evaporated in vacuo with acetonitrile and toluene to remove excess HCl to afford 2-(3-oxa-8-azabicyclo[3.2.1]octan-8-yl)-5,7-dihydrofuro[3,4-b]pyridine-3-carboxylic acid as white solid. LCMS [M+H]⁺ 277.14 m/z.

### Preparation of N-(8-methoxy-4-methyl-2-oxo-1H-quinolin-6-yl)-2-(3-oxa-8-azabicyclo[3.2.1]octan-8-yl)-5,7-dihydrofuro[3,4-b]pyridine-3-carboxamide (Example 17):

To a solution of 2-(3-oxa-8-azabicyclo[3.2.1]octan-8-yl)-5,7-dihydrofuro[3,4-b]pyridine-3-carboxylic acid (0.14 mmol, 1 eq) and 6-amino-8-methoxy-4-methyl-1H-quinolin-2-one (31.7 mg, 0.155 mmol, 1.1 eq) in DMF (4 mL), DIPEA (74 uL, 0.42 mmol, 3 eq) and HATU (59 mg, 0.155 mmol, 1.1 eq) were added and the reaction mixture stirred at RT overnight. After completion, the reaction mixture was poured into water and extracted with EtOAc (2 x 30 mL). The combined extracts were washed with water, 5% LiCl and brine. After evaporation of the solvent, the crude product was purified by flash chromatography on a silica gel column in DCM/MeOH, 0-10% MeOH to give 39 mg of N-(8-methoxy-4-methyl-2-oxo-1H-quinolin-6-yl)-2-(3-oxa-8-azabicyclo[3.2.1]octan-8-yl)-5,7-dihydrofuro[3,4-b]pyridine-3-carboxamide as off-white solid (Example 17) (51% yield over two steps). LCMS [M+H]⁺ 463.18 m/z. ¹H NMR (300 MHz, DMSO-d6) δ 10.65 (s, 1H), 10.48 (s, 1H), 7.77-7.71 (m, 2H), 7.56 (d, J = 1.9 Hz, 1H), 6.45 (s, 1H), 5.02 (s, 2H), 4.86 (s, 2H), 4.23 - 4.14 (m, 2H), 3.88 (s, 3H), 3.68 (d, J = 10.5 Hz, 2H), 3.54 - 3.44 (m, 2H), 2.38 (d, J = 1.2 Hz, 3H).

### Preparation of N-(8-methoxy-4-methyl-2-oxo-1H-quinolin-6-yl)-2-[(2R)-2-methylpyrrolidin-1-yl]-5,7-dihydrofuro[3,4-b]pyridine-3-carboxamide (Example 18):

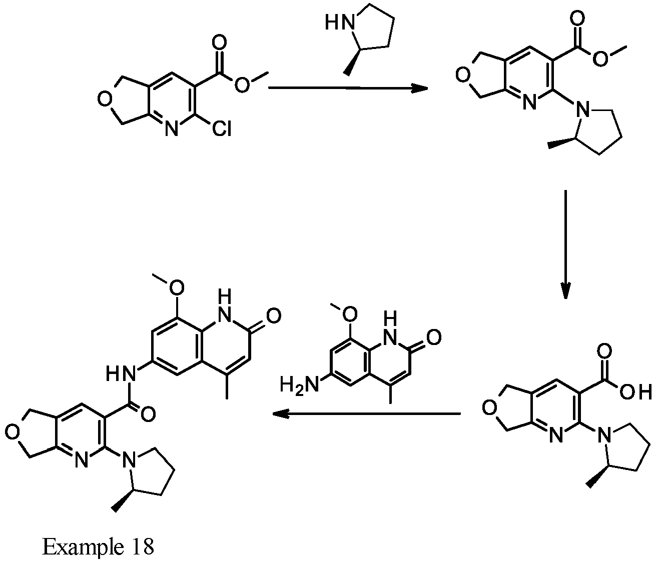

### Preparation of methyl 2-[(2R)-2-methylpyrrolidin-1-yl]-5,7-dihydrofuro[3,4-b]pyridine-3-carboxylate:

To a solution of methyl 2-chloro-5,7-dihydrofuro[3,4-b]pyridine-3-carboxylate (6) (42.7 mg, 0.2 mmol, 1 eq) in dry DMSO (1 mL) were added (2R)-2-methylpyrrolidine (60 mg, 0.4 mmol, 2 eq) and DIPEA (140 µL, 0.8 mmol, 4 eq) and the reaction mixture stirred at 125°C for 1 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (10-50% EtOAc) to afford methyl 2-[(2R)-2-methylpyrrolidin-1-yl]-5,7-dihydrofuro[3,4-b]pyridine-3-carboxylate (43 mg, 83 % yield) as a white solid. LCMS [M+H]⁺ 263.19 m/z. ¹H NMR (300 MHz, DMSO-d6) δ 7.81 (s, 1H), 4.96 (s, 2H), 4.88-4.72 (m, 2H), 4.32-4.17 (m, 1H), 3.80 (s, 3H), 3.58 - 3.42 (m, 1H), 2.86-2.75 (m, 1H), 2.18 - 2.05 (m, 1H), 1.95-1.82 (m, 1H), 1.80 - 1.43 (m, 2H), 1.20 (d, J = 6.0 Hz, 3H).

### Preparation of 2-[(2R)-2-methylpyrrolidin-1-yl]-5,7-dihydrofuro[3,4-b]pyridine-3-carboxylic acid:

To a solution of methyl 2-[(2R)-2-methylpyrrolidin-1-yl]-5,7-dihydrofuro[3,4-b]pyridine-3-carboxylate (42 mg, 0.16 mmol, 1 eq) in methanol (1 mL) and THF (1 mL), 2M LiOH was added (160 µL, 0.32 mmol) and the reaction mixture stirred at 35°C for 3 h. After completion, 2N HCl was added to neutralize (160 µL, 0.32 mmol). Solvent was evaporated in vacuo with acetonitrile and toluene to remove excess HCl to afford 2-[(2R)-2-methylpyrrolidin-1-yl]-5,7-dihydrofuro[3,4-b]pyridine-3-carboxylic acid as white solid. LCMS [M+H]⁺ 249.16 m/z.

### Preparation of N-(8-methoxy-4-methyl-2-oxo-1H-quinolin-6-yl)-2-[(2R)-2-methylpyrrolidin-1-yl]-5,7-dihydrofuro[3,4-b]pyridine-3-carboxamide (Example 18):

To a solution of 2-[(2R)-2-methylpyrrolidin-1-yl]-5,7-dihydrofuro[3,4-b]pyridine-3-carboxylic acid (0.18 mmol, 1.1 eq) and 6-amino-8-methoxy-4-methyl-1H-quinolin-2-one (36 mg, 0.18 mmol, 1.1 eq) in DMF (4 mL), DIPEA (84 uL, 0.48 mmol, 3 eq) and HATU (67 mg, 0.18 mmol, 1.1 eq) were added and the reaction mixture stirred at RT overnight. After completion, the reaction mixture was poured into water and extracted with EtOAc (2 x 30 mL). The combined extracts were washed with water, 5% LiCl and brine. After evaporation of the solvent, the crude product was purified by preparative chromatography to give 16 mg of N-(8-methoxy-4-methyl-2-oxo-1H-quinolin-6-yl)-2-[(2R)-2-methylpyrrolidin-1-yl]-5,7-dihydrofuro[3,4-b]pyridine-3-carboxamide (Example 18) as off-white solid (23% yield over two steps). LCMS [M+H]⁺ 435.23 m/z. ¹H NMR (300 MHz, DMSO-d6) δ 10.62 (s, 1H), 10.46 (s, 1H), 7.74 (d, J = 2.0 Hz, 1H), 7.66 (s, 1H), 7.56 (d, J = 2.0 Hz, 1H), 6.45 (d, J = 1.4 Hz, 1H), 4.99 (s, 2H), 4.90 - 4.75 (m, 2H), 4.35-4.21 (m, 1H), 3.88 (s, 3H), 3.20-3.11 (m, 1H), 2.38 (d, J = 1.2 Hz, 3H), 2.15 - 1.97 (m, 1H), 1.95-1.83 (m, 1H), 1.80 - 1.62 (m, 1H), 1.60-1.47 (m, 1H), 1.20 (d, J = 6.0 Hz, 3H).

### Preparation of 2-(2,3,3a,4,6,6a-hexahydrofuro[2,3-c]pyrrol-5-yl)-N-(8-methoxy-4-methyl-2-oxo-1H-quinolin-6-yl)-5,7-dihydrofuro[3,4-b]pyridine-3-carboxamide (Example 19):

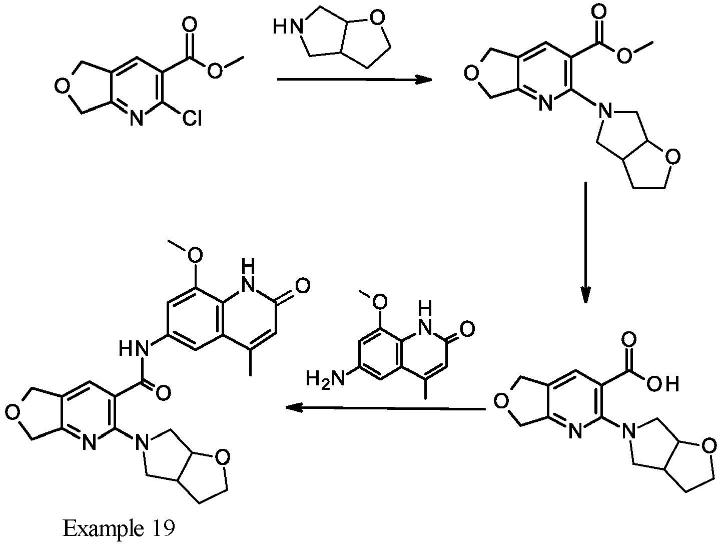

### Preparation of methyl 2-(2,3,3a,4,6,6a-hexahydrofuro[2,3-c]pyrrol-5-yl)-5,7-dihydrofuro[3,4-b]pyridine-3-carboxylate:

To a solution of methyl 2-chloro-5,7-dihydrofuro[3,4-b]pyridine-3-carboxylate (6) (42.7 mg, 0.2 mmol, 1 eq) in dry DMSO (1 mL), 3,3a,4,5,6,6a-hexahydro-2H-furo[2,3-c]pyrrole (60 mg, 0.4 mmol, 2 eq) and DIPEA (140 µL, 0.8 mmol, 4 eq) were added and the reaction mixture stirred at 125°C for 1.5 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (2-50% EtOAc) to afford methyl 2-(2,3,3a,4,6,6a-hexahydrofuro[2,3-c]pyrrol-5-yl)-5,7-dihydrofuro[3,4-b]pyridine-3-carboxylate (39.8 mg, 68% yield) as white solid. LCMS [M+H]⁺ 291.17 m/z. ¹H NMR (300 MHz, DMSO-d6) δ 7.81 (s, 1H), 4.96 (s, 2H), 4.88 - 4.73 (m, 2H), 4.48 - 4.40 (m, 1H), 3.85 (dd, J = 8.3, 7.3 Hz, 1H), 3.81 (s, 3H), 3.78-3.67 (m, 1H), 3.63-3.54 (m, 1H), 3.50-3.38 (m, 1H), 3.38-3.25 (m, 2H), 2.99-2.86 (m, 1H), 2.12 - 1.97 (m, 1H), 1.88-1.77 (m, 1H).

### Preparation of 2-(2,3,3a,4,6,6a-hexahydrofuro[2,3-c]pyrrol-5-yl)-5,7-dihydrofuro[3,4-b]pyridine-3-carboxylic acid:

To a solution of methyl 2-(2,3,3a,4,6,6a-hexahydrofuro[2,3-c]pyrrol-5-yl)-5,7-dihydrofuro[3,4-b]pyridine-3-carboxylate (39 mg, 0.13 mmol, 1 eq) in methanol (1 mL) and THF (1 mL), 2M LiOH was added (134 µL, 0.26 mmol, 2eq) and the reaction mixture stirred at 35°C for 2 days. After completion, 2N HCl was added to neutralize (134 µL, 0.26 mmol). Solvent was evaporated in vacuo with acetonitrile and toluene to remove excess HCl to afford 2-(2,3,3a,4,6,6a-hexahydrofuro[2,3-c]pyrrol-5-yl)-5,7-dihydrofuro[3,4-b]pyridine-3-carboxylic acid as a white solid. LCMS [M+H]⁺ 277.14 m/z.

### Preparation of 2-(2,3,3a,4,6,6a-hexahydrofuro[2,3-c]pyrrol-5-yl)-N-(8-methoxy-4-methyl-2-oxo-1H-quinolin-6-yl)-5,7-dihydrofuro[3,4-b]pyridine-3-carboxamide (Example 19):

To a solution of 2-(2,3,3a,4,6,6a-hexahydrofuro[2,3-c]pyrrol-5-yl)-5,7-dihydrofuro[3,4-b]pyridine-3-carboxylic acid (0.13 mmol, 1.0 eq) and 6-amino-8-methoxy-4-methyl-1H-quinolin-2-one (30 mg, 0.15 mmol, 1.1 eq) in DMF (3 mL), DIPEA (70 uL, 0.4 mmol, 3 eq) and HATU (56 mg, 0.15 mmol, 1.1 eq) were added and the reaction mixture stirred at RT overnight. After completion, the reaction mixture was poured into water and extracted with EtOAc (2 x 30 mL). The combined extracts were washed with water, 5% LiCl and brine. After evaporation of the solvent, the crude product was purified by preparative chromatography to give 26 mg of 2-(2,3,3a,4,6,6a-hexahydrofuro[2,3-c]pyrrol-5-yl)-N-(8-methoxy-4-methyl-2-oxo-1H-quinolin-6-yl)-5,7-dihydrofuro[3,4-b]pyridine-3-carboxamide (Example 19) as off-white solid (42% yield over two steps). LCMS [M+H]⁺ 463.18 m/z. ¹H NMR (300 MHz, DMSO-d6) δ 10.65 (s, 1H), 10.49 (s, 1H), 7.73 (d, J = 1.9 Hz, 1H), 7.67 (s, 1H), 7.55 (d, J = 1.9 Hz, 1H), 6.45 (s, 1H), 4.99 (s, 2H), 4.89-4.77 (m, 2H), 4.47-4.40 (m, 1H), 3.88 (s, 3H), 3.86 - 3.77 (m, 1H), 3.73-3.65 (m, 1H), 3.62 - 3.45 (m, 3H), 3.45 (s, 1H), 2.96-2.84 (m, 1H), 2.38 (s, 3H), 2.09-1.95 (m, 1H), 1.80-1.69 (m, 1H).

### Preparation of 2-(1,3,3a,4,6,6a-hexahydrofuro[3,4-c]pyrrol-5-yl)-N-(8-methoxy-4-methyl-2-oxo-1H-quinolin-6-yl)-5,7-dihydrofuro[3,4-b]pyridine-3-carboxamide (Example 20):

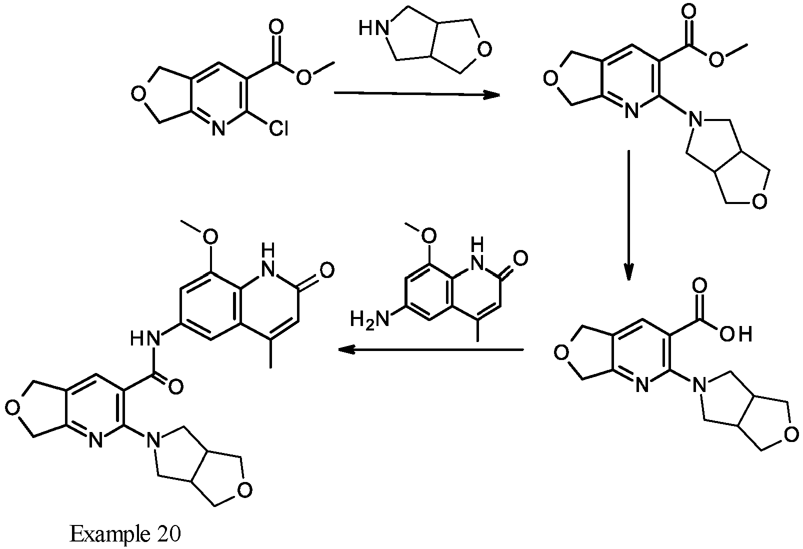

### Preparation of methyl 2-(1,3,3a,4,6,6a-hexahydrofuro[3,4-c]pyrrol-5-yl)-5,7-dihydrofuro[3,4-b]pyridine-3-carboxylate:

To a solution of methyl 2-chloro-5,7-dihydrofuro[3,4-b]pyridine-3-carboxylate (6) (42 mg, 0.2 mmol, 1 eq) in dry DMSO (1 mL), 3,3a,4,5,6,6a-hexahydro-1H-furo[3,4-c]pyrrole (45 mg, 0.4 mmol, 2 eq) and DIPEA (140 µL, 0.8 mmol, 4 eq) were added and the reaction mixture stirred at 125°C for 1 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (10-70% EtOAc) to afford methyl 2-(1,3,3a,4,6,6a-hexahydrofuro[3,4-c]pyrrol-5-yl)-5,7-dihydrofuro[3,4-b]pyridine-3-carboxylate (45.5 mg, 78 % yield) as white solid. LCMS [M+H]⁺ 291.17 m/z. ¹H NMR (300 MHz, DMSO-d6) δ 7.80 (s, 1H), 4.96 (s, 2H), 4.80 (s, 2H), 3.81 (s, 3H), 3.80-3.75 (m, 2H), 3.60 - 3.44 (m, 4H), 3.28 - 3.20 (m, 2H), 2.99-2.87 (m, 2H).

### Preparation of 2-(1,3,3a,4,6,6a-hexahydrofuro[3,4-c]pyrrol-5-yl)-5,7-dihydrofuro[3,4-b]pyridine-3-carboxylic acid:

To a solution of methyl 2-(1,3,3a,4,6,6a-hexahydrofuro[3,4-c]pyrrol-5-yl)-5,7-dihydrofuro[3,4-b]pyridine-3-carboxylate (45 mg, 0.16 mmol, 1 eq) in methanol (1 mL) and THF (1 mL), 2M LiOH was added (200 µL, 3eq) and the reaction mixture stirred at 35°C overnight. 2N HCl was added (200 µL). Solvent was evaporated in vacuo with acetonitrile and toluene to remove excess HCl to afford crude 2-(1,3,3a,4,6,6a-hexahydrofuro[3,4-c]pyrrol-5-yl)-5,7-dihydrofuro[3,4-b]pyridine-3-carboxylic acid as a white solid (42 mg) LCMS [M+H]⁺ 277.14 m/z.

### Preparation of 2-(1,3,3a,4,6,6a-hexahydrofuro[3,4-c]pyrrol-5-yl)-N-(8-methoxy-4-methyl-2-oxo-1H-quinolin-6-yl)-5,7-dihydrofuro[3,4-b]pyridine-3-carboxamide (Example 20):

To a solution of 2-(1,3,3a,4,6,6a-hexahydrofuro[3,4-c]pyrrol-5-yl)-5,7-dihydrofuro[3,4-b]pyridine-3-carboxylic acid (0.16 mmol, 1.0 eq) and 6-amino-8-methoxy-4-methyl-1H-quinolin-2-one (36 mg, 0.17 mmol, 1.1 eq) in DMF (4 mL), DIPEA (84 uL, 0.48 mmol, 3 eq) and HATU (65 mg, 0.17 mmol, 1.1 eq) were added and the reaction mixture stirred at RT overnight. After completion, the reaction mixture was poured into water and extracted with EtOAc (2 x 30 mL). The combined extracts were washed with water, 5% LiCl and brine. After evaporation of the solvent, the crude product was additionally washed with 25% water in acetonitrile, then dried to afford 23.4 mg of 2-(1,3,3a,4,6,6a-hexahydrofuro[3,4-c]pyrrol-5-yl)-N-(8-methoxy-4-methyl-2-oxo-1H-quinolin-6-yl)-5,7-dihydrofuro[3,4-b]pyridine-3-carboxamide (Example 20) as off-white solid (32.6% yield over two steps). LCMS [M+H]⁺ 463.24 m/z. ¹H NMR (300 MHz, DMSO-d6) δ 10.64 (s, 1H), 10.48 (s, 1H), 7.74 (d, J = 1.9 Hz, 1H), 7.68 (s, 1H), 7.55 (d, J = 1.9 Hz, 1H), 6.45 (s, 1H), 5.00 (s, 2H), 4.90 - 4.78 (s, 2H), 3.88 (s, 3H), 3.79 (dd, J = 8.7, 6.6 Hz, 2H), 3.58 (dd, J = 10.6, 7.4 Hz, 2H), 3.51 - 3.43 (m, 2H), 3.41 - 3.36 (m, 2H), 2.96 - 2.90 (m, 2H), 2.38 (s, 3H).

### Preparation of 2-(8-azabicyclo[3.2.1]octan-8-yl)-N-(8-methoxy-4-methyl-2-oxo-1H-quinolin-6-yl)-5,7-dihydrofuro[3,4-b]pyridine-3-carboxamide (Example 21):

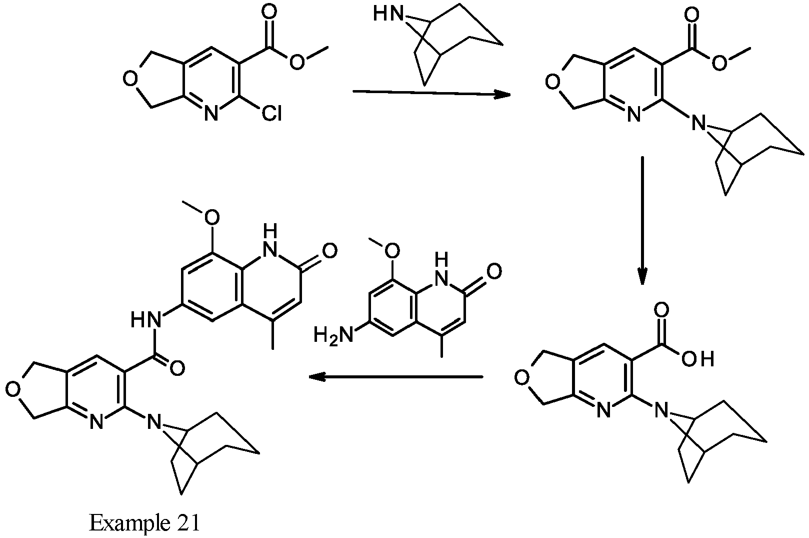

### Preparation of methyl 2-(8-azabicyclo[3.2.1]octan-8-yl)-5,7-dihydrofuro[3,4-b]pyridine-3-carboxylate:

To a solution of methyl 2-chloro-5,7-dihydrofuro[3,4-b]pyridine-3-carboxylate (6) (42 mg, 0.2 mmol, 1 eq) in dry DMSO (1 mL), 8-azabicyclo[3.2.1]octane hydrochloride (60 mg, 0.4 mmol, 2 eq) and DIPEA (140 µL, 0.8 mmol, 4 eq) were added and the reaction mixture stirred at 125°C for 1 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (10-50% EtOAc) to afford methyl 2-(8-azabicyclo[3.2.1]octan-8-yl)-5,7-dihydrofuro[3,4-b]pyridine-3-carboxylate (45.5 mg, 47.5% yield) as a white solid. LCMS [M+H]⁺ 289.18 m/z. 1H NMR (300 MHz, DMSO-d6) δ 7.80 (s, 1H), 4.95 (s, 2H), 4.79 (t, J = 2.0 Hz, 2H), 4.23 (s, 2H), 3.80 (s, 3H), 3.08 (s, 1H), 1.85-1.62 (m, 6H), 1.51-1.35 (m, 3H).

### Preparation of 2-(8-azabicyclo[3.2.1]octan-8-yl)-5,7-dihydrofuro[3,4-b]pyridine-3-carboxylic acid:

To a solution of methyl 2-(8-azabicyclo[3.2.1]octan-8-yl)-5,7-dihydrofuro[3,4-b]pyridine-3-carboxylate (45 mg, 0.16 mmol, 1 eq) in methanol (1 mL) and THF (1 mL), 2M LiOH was added (200 µL, 3eq) and the reaction mixture was stirred at 37°C overnight. 2N HCl was added (200 µL) to the mixture. Solvent was evaporated in vacuo with acetonitrile and toluene to remove excess HCl to afford crude 2-(8-azabicyclo[3.2.1]octan-8-yl)-5,7-dihydrofuro[3,4-b]pyridine-3-carboxylic acid as white solid. LCMS [M+H]+ 275.19 m/z.

### Preparation of 2-(8-azabicyclo[3.2.1]octan-8-yl)-N-(8-methoxy-4-methyl-2-oxo-1H-quinolin-6-yl)-5,7-dihydrofuro[3,4-b]pyridine-3-carboxamide (Example 21):

To a solution of 2-(8-azabicyclo[3.2.1]octan-8-yl)-5,7-dihydrofuro[3,4-b]pyridine-3-carboxylic acid (26 mg,, 0.095 mmol, 1.0 eq) and 6-amino-8-methoxy-4-methyl-1H-quinolin-2-one (21 mg, 0.105 mmol, 1.1 eq) in DMF (4 mL), were added DIPEA (50 uL, 0.28 mmol, 3 eq) and HATU (40 mg, 0.105 mmol, 1.1 eq) and the reaction mixture stirred at RT overnight. After completion, the reaction mixture was poured into water and extracted with EtOAc (2 x 30 mL). The combined extracts were washed with water, 5% LiCl and brine. After evaporation of the solvent, the crude product was additionally washed with 25% water in acetonitrile and 2% MeOH in DCM, then dried to afford 18.1 mg of 2-(8-azabicyclo[3.2.1]octan-8-yl)-N-(8-methoxy-4-methyl-2-oxo-1H-quinolin-6-yl)-5,7-dihydrofuro[3,4-b]pyridine-3-carboxamide (Example 21) as off-white solid (41.4% yield over two steps). LCMS [M+H]⁺ 461.26 m/z. ¹H NMR (300 MHz, DMSO-d6) δ 10.63 (s, 1H), 10.45 (s, 1H), 7.73 (d, J = 1.9 Hz, 1H), 7.67 (s, 1H), 7.54 (d, J = 1.9 Hz, 1H), 6.45 (s, 1H), 5.00 (t, J = 1.9 Hz, 2H), 4.84 (t, J = 2.1 Hz, 2H), 4.33 (s, 2H), 3.87 (s, 3H), 2.37 (s, 3H), 1.89 - 1.58 (m, 7H), 1.51 - 1.28 (m, 3H).

### Preparation of N-(8-methoxy-4-methyl-2-oxo-1H-quinolin-6-yl)-2-[rac-(3aR,6aR)-2,3,3a,4,6,6a-hexahydrofuro[2,3-c]pyrrol-5-yl]-5,7-dihydrofuro[3,4-b]pyridine-3-carboxamide (Example 22):

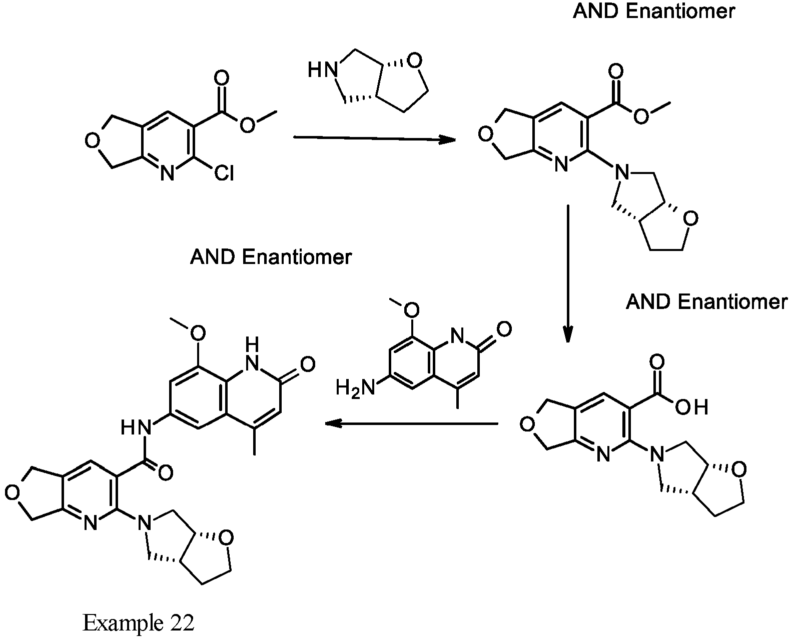

### Preparation of methyl 2-[rac-(3aR,6aR)-2,3,3a,4,6,6a-hexahydrofuro[2,3-c]pyrrol-5-yl]-5,7-dihydrofuro[3,4-b]pyridine-3-carboxylate:

To a solution of methyl 2-chloro-5,7-dihydrofuro[3,4-b]pyridine-3-carboxylate (6) (42 mg, 0.2 mmol, 1 eq) in dry DMSO (1 mL), rac-(3aR,6aR)-hexahydro-2H-furo[2,3-c]pyrrole hydrochloride (45 mg, 0.4 mmol, 2 eq) and DIPEA (140 µL, 0.8 mmol, 4 eq) were added and the reaction mixture stirred at 125°C for 1 h. After completion, the reaction mixture was poured into water and extracted with EtOAc (2 x 30 mL). The combined extracts were washed with water, 5% LiCl and brine, dried over anhydrous Na2SO4, filtered and evaporated. The crude product was purified by flash chromatography using EtOAc/heptane, (20-70% EtOAc) to afford methyl 2-[rac-(3aR,6aR)-2,3,3a,4,6,6a-hexahydrofuro[2,3-c]pyrrol-5-yl]-5,7-dihydrofuro[3,4-b]pyridine-3-carboxylate (43.3 mg, 74% yield) as white solid. LCMS [M+H]⁺ 291.17 m/z. ¹H NMR (300 MHz, DMSO-d6) δ 7.81 (s, 1H), 4.96 (s, 2H), 4.88 - 4.70 (m, 2H), 4.44 (t, J = 5.0 Hz, 1H), 3.81 (s, 3H), 3.77-3.68 (m, 1H), 3.58 (dd, J = 12.5, 4.7 Hz, 1H), 3.50 - 3.38 (m, 2H), 3.38-3.24 (m, 2H), 2.99 - 2.85 (m, 1H), 2.15 - 1.96 (m, 1H), 1.88-1.77 (m, 1H).

### Preparation of 2-[rac-(3aR,6aR)-2,3,3a,4,6,6a-hexahydrofuro[2,3-c]pyrrol-5-yl]-5,7-dihydrofuro[3,4-b]pyridine-3-carboxylic acid:

To a solution of methyl 2-[rac-(3aR,6aR)-2,3,3a,4,6,6a-hexahydrofuro[2,3-c]pyrrol-5-yl]-5,7-dihydrofuro[3,4-b]pyridine-3-carboxylate (41 mg, 0.145 mmol, 1 eq) in methanol (1 mL) and THF (1 mL), 2M LiOH was added (250 µL, 4eq) and the reaction mixture stirred at 35°C overnight. After completion, 2N HCl was added to neutralize (250 µL). The solvent was evaporated in vacuo with acetonitrile and toluene to remove excess HCl to afford crude 2-[rac-(3aR,6aR)-2,3,3a,4,6,6a-hexahydrofuro[2,3-c]pyrrol-5-yl]-5,7-dihydrofuro[3,4-b]pyridine-3-carboxylic acid as white solid. LCMS [M+H]⁺ 277.14 m/z.

### Preparation of N-(8-methoxy-4-methyl-2-oxo-1H-quinolin-6-yl)-2-[rac-(3aR,6aR)-2,3,3a,4,6,6a-hexahydrofuro[2,3-c]pyrrol-5-yl]-5,7-dihydrofuro[3,4-b]pyridine-3-carboxamide (Example 22):

To a solution of 2-[rac-(3aR,6aR)-2,3,3a,4,6,6a-hexahydrofuro[2,3-c]pyrrol-5-yl]-5,7-dihydrofuro[3,4-b]pyridine-3-carboxylic acid (0.145 mmol, 1.0 eq) and 6-amino-8-methoxy-4-methyl-1H-quinolin-2-one (33 mg, 0.16 mmol, 1.1 eq) in DMF (3 mL), DIPEA (76 uL, 0.43 mmol, 3 eq) and HATU (61 mg, 0.16 mmol, 1.1 eq) were added and the reaction mixture stirred at RT overnight. After completion, the reaction mixture was poured into water and extracted with EtOAc (2 x 30 mL). The combined extracts were washed with water, 5% LiCl and brine. After evaporation of the solvent, the crude product was purified by preparative chromatography to afford 28 mg of N-(8-methoxy-4-methyl-2-oxo-1H-quinolin-6-yl)-2-[rac-(3aR,6aR)-2,3,3a,4,6,6a-hexahydrofuro[2,3-c]pyrrol-5-yl]-5,7-dihydrofuro[3,4-b]pyridine-3-carboxamide as off-white solid (Example 22) (41.8% yield over two steps). LCMS [M+H]⁺ 463.24 m/z. ¹H NMR (300 MHz, DMSO-d6) δ 10.63 (s, 1H), 10.49 (s, 1H), 7.73 (d, J = 1.9 Hz, 1H), 7.67 (s, 1H), 7.55 (d, J = 1.9 Hz, 1H), 6.45 (s, 1H), 5.00 (t, J = 1.9 Hz, 2H), 4.90-4.76 (m, 2H), 4.47-4.40 (m, 1H), 3.88 (s, 3H), 3.85 - 3.78 (m, 1H), 3.69 (td, J = 8.2, 4.7 Hz, 1H), 3.62 - 3.45 (m, 3H), 3.40 (dd, J = 6.2, 5.0 Hz, 1H), 2.97 - 2.85 (m, 1H), 2.38 (d, J = 1.2 Hz, 3H), 2.08-1.95 (m, 1H), 1.80-1.70 (m, 1H).

### Preparation of N-(8-methoxy-4-methyl-2-oxo-1H-quinolin-6-yl)-2-[(1S,4S)-2-oxa-5-azabicyclo[2.2.1]heptan-5-yl]-5,7-dihydrofuro[3,4-b]pyridine-3-carboxamide (Example 23):

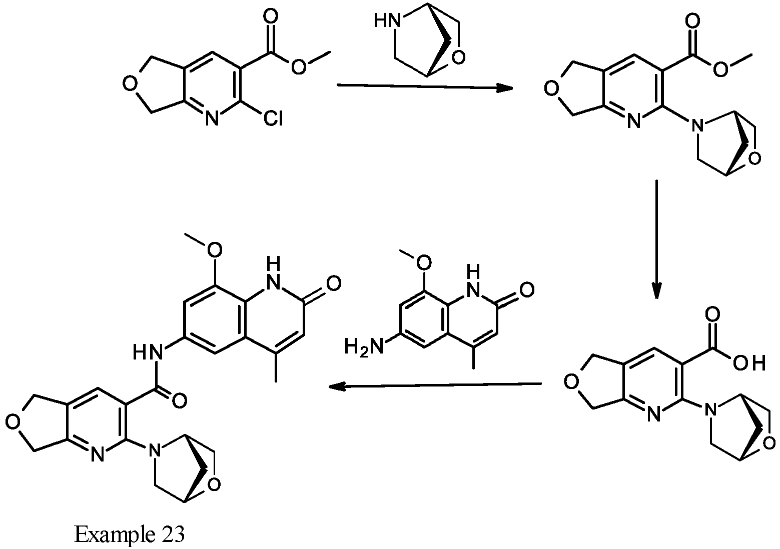

### Preparation of methyl 2-[(1S,4S)-2-oxa-5-azabicyclo[2.2.1]heptan-5-yl]-5,7-dihydrofuro[3,4-b]pyridine-3-carboxylate:

To a solution of methyl 2-chloro-5,7-dihydrofuro[3,4-b]pyridine-3-carboxylate (6) (42 mg, 0.2 mmol, 1 eq) in dry DMSO (1 mL), (1S,4S)-2-oxa-5-azabicyclo[2.2.1]heptane (45 mg, 0.4 mmol, 2 eq) and DIPEA (140 µL, 0.8 mmol, 4 eq) were added and the reaction mixture stirred at 125°C for 1 h. After completion, the reaction mixture was poured into water and extracted with EtOAc (2 x 30 mL). The combined extracts were washed with water, 5% LiCl and brine, dried over anhydrous Na2SO4, filtered and evaporated. The crude product was purified by flash chromatography using EtOAc/heptane, (20-70% EtOAc) to afford methyl 2-[(1S,4S)-2-oxa-5-azabicyclo[2.2.1]heptan-5-yl]-5,7-dihydrofuro[3,4-b]pyridine-3-carboxylate (33 mg, 59.8% yield) as white solid. LCMS [M+H]⁺ 277.17 m/z. ¹H NMR (300 MHz, DMSO-d6) δ 7.85 (s, 1H), 4.97 (s, 2H), 4.89 - 4.73 (m, 3H), 4.58 (s, 1H), 3.80 (s, 3H), 3.78 - 3.70 (m, 2H), 3.54 (dd, J = 10.2, 1.6 Hz, 1H), 2.68 (dd, J = 10.3, 1.4 Hz, 1H), 1.90 - 1.77 (m, 2H), 1.24 (d, J = 3.4 Hz, 1H).

### Preparation of 2-[(1S,4S)-2-oxa-5-azabicyclo[2.2.1]heptan-5-yl]-5,7-dihydrofuro[3,4-b]pyridine-3-carboxylic acid:

To a solution of methyl 2-[(1S,4S)-2-oxa-5-azabicyclo[2.2.1]heptan-5-yl]-5,7-dihydrofuro[3,4-b]pyridine-3-carboxylate (33 mg, 0.12 mmol, 1 eq) in methanol (1 mL) and THF (1 mL), 2M LiOH was added (200 µL) and the reaction mixture stirred at 35°C for two days. 2N HCl was added to neutralize (200 µL). Solvent was evaporated in vacuo with acetonitrile and toluene to remove excess HCl to afford crude 2-[(1S,4S)-2-oxa-5-azabicyclo[2.2.1]heptan-5-yl]-5,7-dihydrofuro[3,4-b]pyridine-3-carboxylic acid as white solid. LCMS [M+H]⁺ 263.13 m/z.

### Preparation of N-(8-methoxy-4-methyl-2-oxo-1H-quinolin-6-yl)-2-[(1S,4S)-2-oxa-5-azabicyclo[2.2.1]heptan-5-yl]-5,7-dihydrofuro[3,4-b]pyridine-3-carboxamide (Example 23):

To a solution of 2-[(1S,4S)-2-oxa-5-azabicyclo[2.2.1]heptan-5-yl]-5,7-dihydrofuro[3,4-b]pyridine-3-carboxylic acid (0.12 mmol, 1.0 eq) and 6-amino-8-methoxy-4-methyl-1H-quinolin-2-one (33 mg, 0.16 mmol, 1.1 eq) in DMF (3 mL), DIPEA (76 uL, 0.43 mmol, 3 eq) and HATU (61 mg, 0.16 mmol, 1.1 eq) were added and the reaction mixture stirred at RT overnight. After completion, the reaction mixture was poured into water and extracted with EtOAc (2 x 30 mL). The combined extracts were washed with water, 5% LiCl and brine. After evaporation of the solvent, the crude product was purified by preparative chromatography to afford 11.6 mg of N-(8-methoxy-4-methyl-2-oxo-1H-quinolin-6-yl)-2-[(1S,4S)-2-oxa-5-azabicyclo[2.2.1]heptan-5-yl]-5,7-dihydrofuro[3,4-b]pyridine-3-carboxamide (Example 23) as off-white solid (21.6% over two steps). LCMS [M+H]⁺ 449.22 m/z. ¹H NMR (300 MHz, DMSO-d6) δ 10.63 (s, 1H), 10.49 (s, 1H), 7.72 (d, J = 1.9 Hz, 1H), 7.68 (s, 1H), 7.54 (d, J = 1.9 Hz, 1H), 6.45 (s, 1H), 5.00 (s, 2H), 4.92 - 4.76 (m, 3H), 4.57 (s, 1H), 3.88 (s, 3H), 3.76 (s, 2H), 3.54 (dd, J = 10.0, 1.6 Hz, 1H), 2.91-3.07 (m, 1H), 2.37 (d, J = 1.2 Hz, 3H), 1.90 - 1.75 (m, 2H).

### Preparation of N-(8-methoxy-4-methyl-2-oxo-1H-quinolin-6-yl)-2-[(1R,4R)-2-oxa-5-azabicyclo[2.2.1]heptan-5-yl]-5,7-dihydrofuro[3,4-b]pyridine-3-carboxamide (Example 24):

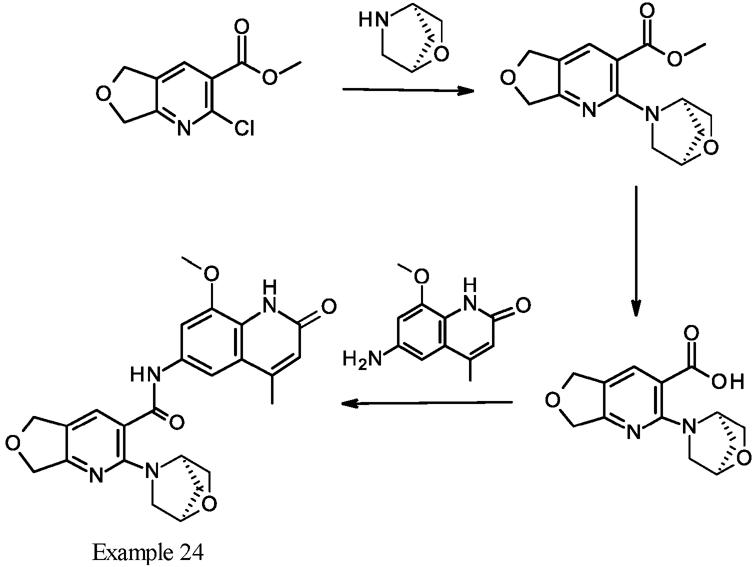

### Preparation of methyl 2-[(1R,4R)-2-oxa-5-azabicyclo[2.2.1]heptan-5-yl]-5,7-dihydrofuro[3,4-b]pyridine-3-carboxylate:

To a solution of methyl 2-chloro-5,7-dihydrofuro[3,4-b]pyridine-3-carboxylate (6) (64 mg, 0.3 mmol, 1 eq) in dry DMSO (2 mL), (1R,4R)-2-oxa-5-azabicyclo[2.2.1]heptane hydrochloride (81 mg, 0.6 mmol, 2 eq) and DIPEA (209 µL, 0.8 mmol, 4 eq) were added and the reaction mixture stirred at 125°C for 1 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (30-70% EtOAc) to afford methyl 2-[(1R,4R)-2-oxa-5-azabicyclo[2.2.1]heptan-5-yl]-5,7-dihydrofuro[3,4-b]pyridine-3-carboxylate (47.5 mg, 60% YIELD)) as a white solid. LCMS [M+H]⁺ 277.14 m/z. ¹H NMR (300 MHz, DMSO-d6) δ 7.85 (s, 1H), 4.97 (s, 2H), 4.87 - 4.73 (m, 3H), 4.58 (s, 1H), 3.80 (s, 3H), 3.77 - 3.70 (m, 2H), 3.54 (dd, J = 10.2, 1.6 Hz, 1H), 2.68 (d, J = 10.2 Hz, 1H), 1.91 - 1.78 (m, 2H).

### Preparation of 2-[(1R,4R)-2-oxa-5-azabicyclo[2.2. 1]heptan-5-yl]-5,7-dihydrofuro[3,4-b]pyridine-3-carboxylic acid:

To a solution of methyl 2-[(1R,4R)-2-oxa-5-azabicyclo[2.2.1]heptan-5-yl]-5,7-dihydrofuro[3,4-b]pyridine-3-carboxylate (47 mg, 0.17 mmol, 1 eq) in methanol (1 mL) and THF (1 mL), 2M LiOH was added (350 µL) and the reaction mixture stirred at 35°C for two days. 2N HCl was added (350 µL). The solvent was evaporated in vacuo with acetonitrile and toluene to remove excess HCl to afford crude 2-[(1R,4R)-2-oxa-5-azabicyclo[2.2.1]heptan-5-yl]-5,7-dihydrofuro[3,4-b]pyridine-3-carboxylic acid as A white solid. LCMS [M+H]⁺ 263.12 m/z.

### Preparation of N-(8-methoxy-4-methyl-2-oxo-1H-quinolin-6-yl)-2-[(1R,4R)-2-oxa-5-azabicyclo[2.2.1]heptan-5-yl]-5,7-dihydrofuro[3,4-b]pyridine-3-carboxamide (Example 24):

To a solution of 2-[(1R,4R)-2-oxa-5-azabicyclo[2.2.1]heptan-5-yl]-5,7-dihydrofuro[3,4-b]pyridine-3-carboxylic acid (0.17 mmol, 1.0 eq) and 6-amino-8-methoxy-4-methyl-1H-quinolin-2-one (39 mg, 0.19 mmol, 1.1 eq) in DMF (3 mL), DIPEA (90 uL, 0.5 mmol, 3 eq) and HATU (71 mg, 0.19 mmol, 1.1 eq) were added and the reaction mixture stirred at RT overnight. After completion, the reaction mixture was poured into water and extracted with EtOAc (2 x 30 mL). The combined extracts were washed with water, 5% LiCl and brine. After evaporation of the solvent, the crude product was purified by preparative chromatography to afford of N-(8-methoxy-4-methyl-2-oxo-1H-quinolin-6-yl)-2-[(1R,4R)-2-oxa-5-azabicyclo[2.2.1]heptan-5-yl]-5,7-dihydrofuro[3,4-b]pyridine-3-carboxamide (Example 24) as off-white solid (22.8 mg, 29.6% yield over two steps). LCMS [M+H]⁺ 449.16 m/z. ¹H NMR (300 MHz, DMSO-d6) δ 10.64 (s, 1H), 10.50 (s, 1H), 7.72 (d, J = 1.9 Hz, 1H), 7.69 (s, 1H), 7.54 (d, J = 1.9 Hz, 1H), 6.45 (s, 1H), 5.01 (s, 2H), 4.94 - 4.75 (m, 3H), 4.56 (d, J = 2.2 Hz, 1H), 3.88 (s, 3H), 3.76 (s, 2H), 3.54 (dd, J = 9.9, 1.6 Hz, 1H), 2.99 (d, J = 9.9 Hz, 1H), 2.37 (d, J = 1.2 Hz, 3H), 1.87 - 1.77(m, 2H).

### Preparation of N-(8-methoxy-4-methyl-2-oxo-1H-quinolin-6-yl)-2-[(2S)-2-methylpyrrolidin-1-yl]-5,7-dihydrofuro[3,4-b]pyridine-3-carboxamide (Example 25):

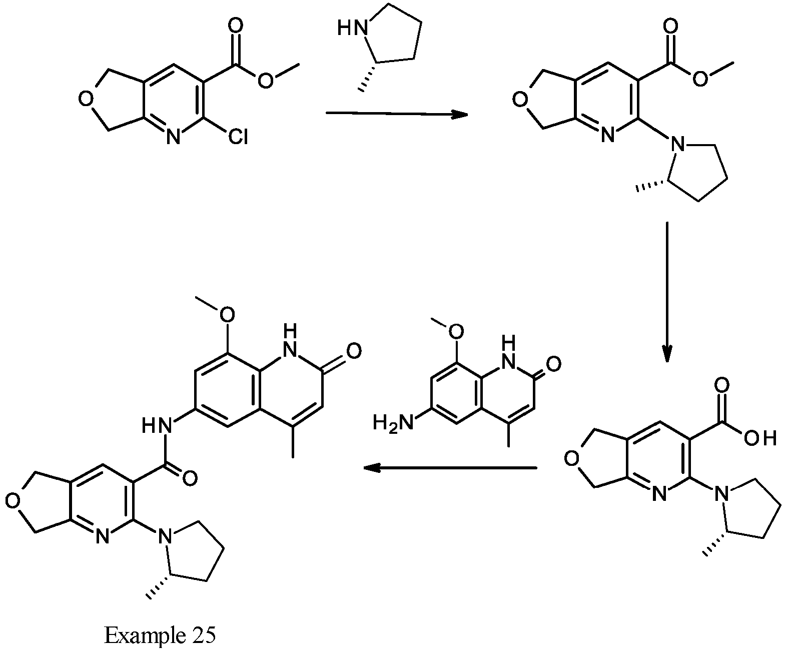

### Preparation of methyl 2-[(2S)-2-methylpyrrolidin-1-yl]-5,7-dihydrofuro[3,4-b]pyridine-3-carboxylate:

To a solution of methyl 2-chloro-5,7-dihydrofuro[3,4-b]pyridine-3-carboxylate (6) (64 mg, 0.3 mmol, 1 eq) in dry DMSO (2 mL), were added (2S)-2-methylpyrrolidine (51 mg, 0.6 mmol, 2 eq) and DIPEA (209 µL, 0.8 mmol, 4 eq) and the reaction mixture was stirred at 125°C for 1 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (10-90% EtOAc) to afford methyl 2-[(2S)-2-methylpyrrolidin-1-yl]-5,7-dihydrofuro[3,4-b]pyridine-3-carboxylate (57 mg, 72% yield) as white solid. LCMS [M+H]⁺ 263.12 m/z. ¹H NMR (300 MHz, DMSO-d6) δ 7.81 (d, J = 0.9 Hz, 1H), 4.96 (s, 2H), 4.80 (qt, J = 13.6, 1.9 Hz, 2H), 4.1-4.19 (m, 1H), 3.80 (s, 3H), 3.50 - 3.42 (m, 1H), 2.85-2.75 (m, 1H), 2.20 - 2.06 (m, 1H), 1.99 - 1.80 (m, 1H), 1.73-1.47 (m, 2H), 1.20 (d, J = 6.0 Hz, 3H).

### Preparation of 2-[(2S)-2-methylpyrrolidin-1-yl]-5,7-dihydrofuro[3,4-b]pyridine-3-carboxylic acid:

To a solution of methyl 2-[(2S)-2-methylpyrrolidin-1-yl]-5,7-dihydrofuro[3,4-b]pyridine-3-carboxylate (56 mg, 0.21 mmol, 1 eq) in methanol (1 mL) and THF (1 mL), 2M LiOH was added (350 µL) and the reaction mixture stirred at 35°C for two days. After completion, 2N HCl was added to neutralize (350 µL). Solvent was evaporated in vacuo with acetonitrile and toluene to remove excess HCl to afford crude 2-[(2S)-2-methylpyrrolidin-1-yl]-5,7-dihydrofuro[3,4-b]pyridine-3-carboxylic acid as white solid. LCMS [M+H]⁺ 249.15 m/z.

### Preparation of N-(8-methoxy-4-methyl-2-oxo-1H-quinolin-6-yl)-2-[(2S)-2-methylpyrrolidin-1-yl]-5,7-dihydrofuro[3,4-b]pyridine-3-carboxamide (Example 25):

To a solution of 2-[(2S)-2-methylpyrrolidin-1-yl]-5,7-dihydrofuro[3,4-b]pyridine-3-carboxylic acid (0.21 mmol, 1.0 eq) and 6-amino-8-methoxy-4-methyl-1H-quinolin-2-one (48 mg, 0.23 mmol, 1.1 eq) in DMF (3 mL), were added DIPEA (149 uL, 0.85 mmol, 4 eq) and HATU (97 mg, 0.25 mmol, 1.2 eq) and the reaction mixture stirred at 35°C overnight. After completion, the reaction mixture was poured into water and extracted with EtOAc (2 x 30 mL). The combined extracts were washed with water, 5% LiCl and brine. After evaporation of the solvent, the crude product was purified by preparative chromatography to afford of N-(8-methoxy-4-methyl-2-oxo-1H-quinolin-6-yl)-2-[(2S)-2-methylpyrrolidin-1-yl]-5,7-dihydrofuro[3,4-b]pyridine-3-carboxamide as off-white solid (16.7 mg, 18% yield over two steps). LCMS [M+H]⁺ 435.17 m/z. ¹H NMR (300 MHz, DMSO-d6) δ 10.63 (s, 1H), 10.46 (s, 1H), 7.74 (d, J = 1.9 Hz, 1H), 7.66 (s, 1H), 7.56 (d, J = 1.9 Hz, 1H), 6.45 (s, 1H), 5.04 - 4.97 (m, 2H), 4.92 - 4.75 (m, 2H), 4.35-4.22 (m, 1H), 3.88 (s, 3H), 3.56-3.45 (m, 1H), 3.20-3.11 (m, 2H), 2.38 (d, J = 1.2 Hz, 3H), 2.12-2.00 (m, 1H), 1.95 - 1.83 (m, 1H), 1.77 - 1.62 (m, 1H), 1.61-1.48 (m, 1H), 1.20 (d, J = 6.0 Hz, 3H).

### Preparation of 2-[cyclopropylmethyl(methyl)amino]-N-(8-methoxy-4-methyl-2-oxo-1H-quinolin-6-yl)-5,7-dihydrofuro[3,4-b]pyridine-3-carboxamide (Example 26)

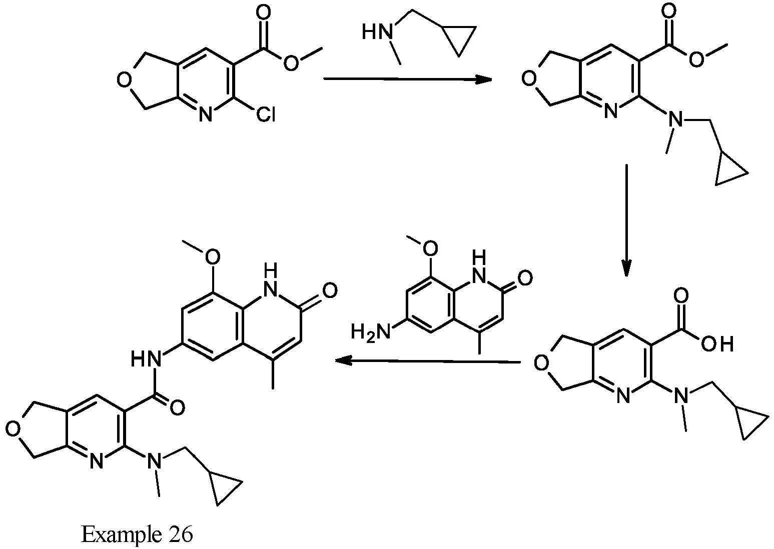

### Preparation of 2-[cyclopropylmethyl(methyl)amino]-N-(8-methoxy-4-methyl-2-oxo-1H-quinolin-6-yl)-5,7-dihydrofuro[3,4-b]pyridine-3-carboxamide (Example 26):

Methyl 2-chloro-5,7-dihydrofuro[3,4-b]pyridine-3-carboxylate (60 mg, 0.28 mmol, 1eq), 1-cyclopropyl-N-methyl-methanamine HCl salt (76 mg 0.63 mmol, 2.25 ml) and DIPEA (0.2 ml, 1.15 mmol,4.1 eq) were dissolved in DMSO (0.5ml) and shaken at 100°C for 3h, then at 60°C overnight and 100°C for 1h and cooled to RT. Aqueous 5%LiCl was added (3.5 ml) and extracted with DCM (3 x 1.5ml). The organic extracts were combined and purified directly on Biotage Isolera: Heptane/EtOAc 98/2 then 98/2->35/65. After completion, the reaction mixture was allowed to warm up to RT and the solvent evaporated in vacuo. Fractions with the product were evaporated to afford methyl 2-[cyclopropylmethyl(methyl)amino]-5,7-dihydrofuro[3,4-b]pyridine-3-carboxylate 40 mg as yellow oil, LCMS [M+H]+ 263 m/z. The oil was dissolved in MeOH(0.5 ml) and 2M LiOH aq. was added (0.2 ml, 0.4 mmol, 2.66 eq). The reaction mixture was shaken at 60°C for 4h, cooled to RT and 5M HCl (0.085 ml, 0.425 mmol, 2.83 eq) was added. The reaction mixture was evaporated, acetonitrile (4 ml) was added and evaporated. Crude 2-[cyclopropylmethyl(methyl)amino]-5,7-dihydrofuro[3,4-b]pyridine-3-carboxylic acid 60 mg was obtained as a pale yellow oil. LC-MS [M+H]⁺ 249 m/z. The crude was dissolved in DMF (0.4 ml) and DIPEA (0.1 ml, 0.57 mmol), 6-amino-8-methoxy-4-methyl-1H-quinolin-2-one (33 mg, 0.162 mmol) and HATU (76 mg,0.2 mmol) were added. The reaction mixture was shaken at 60°C for 1.5h, then cooled to RT. 5%LiCl aq. (3 ml) was added and the product was extracted with EtOAc (2x1.5 ml). The combined extracts were purified directly on Biotage Isolera (SiO2 NH): Heptane/EtOAc 100/0 then 100/0->18/82 then 18/82. Relevant fractions with product were evaporated, triturated with Et₂O (solvent decanted) and dried at 1 mbar/RT for 2h to afford 2-[cyclopropylmethyl(methyl)amino]-N-(8-methoxy-4-methyl-2-oxo-1H-quinolin-6-yl)-5,7-dihydrofuro[3,4-b]pyridine-3-carboxamide, 17 mg as a pale yellow powder. LCMS [M+H]⁺ 435 m/z, UV(254nm) >95%. ¹H NMR (300 MHz, DMSO-d6) δ 10.63 (s, 1H), 10.54 (s, 1H), 7.87 - 7.62 (m, 2H), 7.54 (d, J = 1.9 Hz, 1H), 6.45 (d, J = 1.4 Hz, 1H), 5.00 (s, 2H), 4.84 (t, J = 2.0 Hz, 2H), 3.88 (s, 3H), 3.29 (d, J = 6.6 Hz, 2H), 2.98 (s, 3H), 2.37 (d, J = 1.2 Hz, 3H), 1.09 - 0.94 (m, 1H), 0.50 - 0.33 (m, 2H), 0.25 - 0.10 (m, 2H).

### Preparation of 2-(diethylamino)-N-(8-methoxy-4-methyl-2-oxo-1H-quinolin-6-yl)-5,7-dihydrofuro[3,4-b]pyridine-3-carboxamide (Example 27):

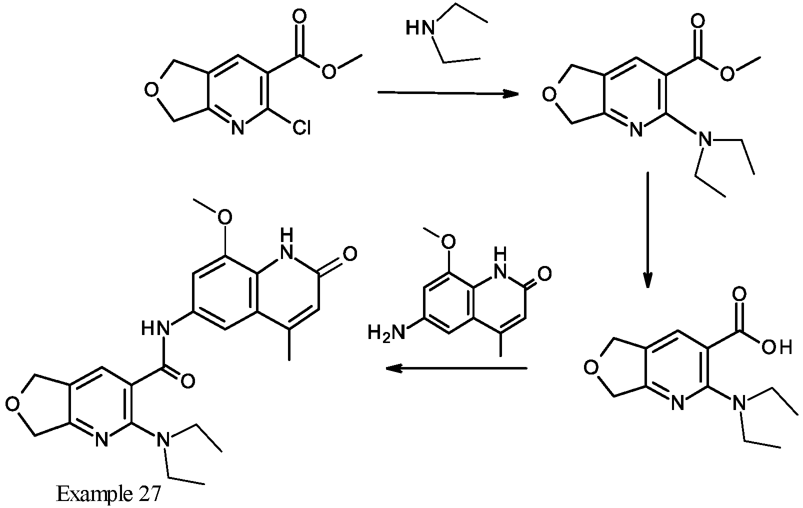

### Preparation of methyl 2-(diethylamino)-5,7-dihydrofuro[3,4-b]pyridine-3-carboxylate:

To a solution of methyl 2-chloro-5,7-dihydrofuro[3,4-b]pyridine-3-carboxylate (128 mg, 0.6 mmol, 1 eq) in dry DMSO (5 mL), N-ethylethanamine (125 mg, 1.2 mmol, 2 eq) and DIPEA (418 µL, 2.4 mmol, 4 eq) were added and the reaction mixture stirred at 125°C for 10 h. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (10-90% EtOAc) to afford methyl 2-(diethylamino)-5,7-dihydrofuro[3,4-b]pyridine-3-carboxylate (65 mg, 43 %) as a white solid. LCMS [M+H]⁺ 251.3 m/z. ¹H NMR (300 MHz, DMSO-d6) δ 7.75 (s, 1H), 4.95 (s, 2H), 4.79 (t, J = 2.0 Hz, 2H), 3.80 (s, 3H), 1.08 (t, J = 7.0 Hz, 6H).

### Preparation of 2-(diethylamino)-5,7-dihydrofuro[3,4-b]pyridine-3-carboxylic acid:

To a solution of methyl 2-(diethylamino)-5,7-dihydrofuro[3,4-b]pyridine-3-carboxylate (65 mg, 0.26 mmol, 1 eq) in methanol (1 mL) and THF (1 mL), 2M LiOH was added (350 µL) and the reaction mixture stirred at 35°C for two days. 2N HCl was added (350 µL). Solvent was evaporated in vacuo with acetonitrile and toluene to remove excess HCl to afford crude 2-(diethylamino)-5,7-dihydrofuro[3,4-b]pyridine-3-carboxylic acid as white solid. LCMS [M+H]⁺ 237.51 m/z.

### Preparation of 2-(diethylamino)-N-(8-methoxy-4-methyl-2-oxo-1H-quinolin-6-yl)-5,7-dihydrofuro[3,4-b]pyridine-3-carboxamide (Example 27):

To a solution of 2-(diethylamino)-5,7-dihydrofuro[3,4-b]pyridine-3-carboxylic acid (0.26 mmol, 1.0 eq) and 6-amino-8-methoxy-4-methyl-1H-quinolin-2-one (58 mg, 0.29 mmol, 1.1 eq) in DMF (3 mL), were added DIPEA (181 uL, 1.09mmol, 4 eq), HOAt (71 mg, 0.29 mmol, 1.1 eq) and EDC·HCl (100 mg, 0.52 mmol, 2eq) and the reaction mixture stirred at 35°C overnight. After completion, the reaction mixture was poured into water and extracted with EtOAc (2 x 30 mL). The combined extracts were washed with water, 5% LiCl and brine. After evaporation in vacuo, the crude product was additionally washed with 25% water in acetonitrile and DMF, then dried to afford 42 mg of 2-(diethylamino)-N-(8-methoxy-4-methyl-2-oxo-1H-quinolin-6-yl)-5,7-dihydrofuro[3,4-b]pyridine-3-carboxamide as off-white solid (38% yield). LCMS [M+H]⁺ 426.63 m/z. ¹H NMR (300 MHz, DMSO-d6) δ 10.72 (s, 1H), 10.66 (s, 1H), 7.73 (s, 1H), 7.71 (d, J = 1.9 Hz, 1H), 7.54 (d, J = 1.9 Hz, 1H), 6.45 (d, J = 1.3 Hz, 1H), 5.01 (t, J = 2.0 Hz, 2H), 4.85 (t, J = 1.9 Hz, 2H), 3.88 (s, 3H), 3.39 (q, J = 7.0 Hz, 4H), 2.38 (d, J = 1.2 Hz, 3H), 1.15 - 1.03 (m, 6H).

### Preparation of 2-[ethyl(isopropyl)amino]-N-(8-methoxy-4-methyl-2-oxo-1H-quinolin-6-yl)-5,7-dihydrofuro[3,4-b]pyridine-3-carboxamide (Example 28)

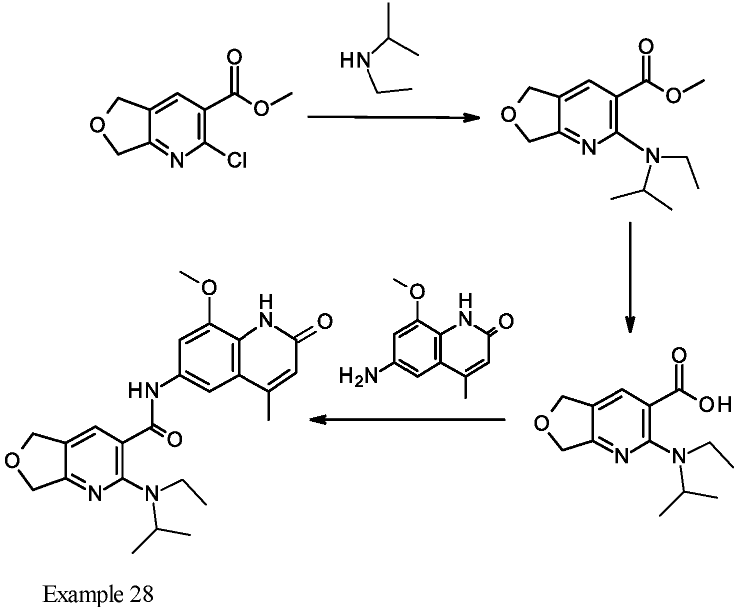

### Preparation of methyl 2-[ethyl(isopropyl)amino]-5,7-dihydrofuro[3,4-b]pyridine-3-carboxylate:

To a solution of methyl 2-chloro-5,7-dihydrofuro[3,4-b]pyridine-3-carboxylate (6) (128 mg, 0.6 mmol, 1 eq) in dry DMSO (5 mL), were added N-ethylpropan-2-amine (125 mg, 1.2 mmol, 2 eq) and DIPEA (418 µL, 2.4 mmol, 4 eq) and the reaction mixture stirred at 125°C for 7 h, then additional 8 h at 80°C. After completion, the reaction mixture was poured into water and extracted with EtOAc (2 x 30 mL). The combined extracts were washed with water, 5% LiCl and brine, dried over anhydrous Na₂SO₄, filtered and evaporated. The crude product was purified by flash chromatography using EtOAc/heptane, (10-90% EtOAc) to afford methyl 2-[ethyl(isopropyl)amino]-5,7-dihydrofuro[3,4-b]pyridine-3-carboxylate (19 mg, 12% yield) as white solid. LCMS [M+H]⁺ 265.52 m/z. ¹H NMR (300 MHz, DMSO-d6) δ 7.77 (s, 1H), 5.01 - 4.92 (m, 2H), 4.80 (t, J = 2.0 Hz, 2H), 3.79 (s, 3H), 1.12 (d, J = 6.6 Hz, 6H), 0.99 (t, J = 6.9 Hz, 3H).

### Preparation of 2-[ethyl(isopropyl)amino]-5,7-dihydrofuro[3,4-b]pyridine-3-carboxylic acid:

To a solution of methyl 2-[ethyl(isopropyl)amino]-5,7-dihydrofuro[3,4-b]pyridine-3-carboxylate (18 mg, 0.07 mmol, 1 eq) in methanol (0.3 mL) and THF (0.3 mL), 2M LiOH was added (100 µL) and the reaction mixture stirred at 35°C for overnight. 2N HCl (100 µL) was then added. Solvent was evaporated in vacuo with acetonitrile and toluene to afford crude 2-[ethyl(isopropyl)amino]-5,7-dihydrofuro[3,4-b]pyridine-3-carboxylic acid as a white solid. LCMS [M+H]⁺ 251.50 m/z.

### Preparation of 2-[ethyl(isopropyl)amino]-N-(8-methoxy-4-methyl-2-oxo-1H-quinolin-6-yl)-5,7-dihydrofuro[3,4-b]pyridine-3-carboxamide (Example 28):

To a solution of 2-[ethyl(isopropyl)amino]-5,7-dihydrofuro[3,4-b]pyridine-3-carboxylic acid (0.07 mmol, 1.0 eq) and 6-amino-8-methoxy-4-methyl-1H-quinolin-2-one (15 mg, 0.075 mmol, 1.1 eq) in DMF (1 mL), were added DIPEA (48 uL, 0.28 mmol, 4 eq), HOAt (19 mg, 0.075 mmol, 1.1 eq) and EDC·HCl (26 mg, 0.14mmol, 2eq) and the reaction mixture stirred at 35°C overnight. After completion, the reaction mixture was poured into water and extracted with EtOAc (2 x 30 mL). The combined extracts were washed with water, 5% LiCl and brine. After evaporation of the solvent, the crude product was purified by preparative chromatography to afford 2-[ethyl(isopropyl)amino]-N-(8-methoxy-4-methyl-2-oxo-1H-quinolin-6-yl)-5,7-dihydrofuro[3,4-b]pyridine-3-carboxamide as an off-white solid (15 mg, 51% yield). LCMS [M+H]⁺ 437.64 m/z. ¹H NMR (300 MHz, DMSO-d6) δ 11.11 (s, 1H), 10.68 (s, 1H), 7.88 (s, 1H), 7.68 (d, J = 1.9 Hz, 1H), 7.53 (d, J = 1.9 Hz, 1H), 6.46 (s, 1H), 5.04 (s, 2H), 4.89 (s, 2H), 3.97 (q, J = 6.6 Hz, 1H), 3.89 (s, 3H), 2.38 (d, J = 1.2 Hz, 3H), 1.11 (d, J = 6.5 Hz, 6H), 1.02 (t, J = 7.0 Hz, 3H).

### Preparation of N-(8-methoxy-4-methyl-2-oxo-1H-quinolin-6-yl)-2-morpholino-6,7-dihydro-5H-cyclopenta[b]pyridine-3-carboxamide (Example 29):

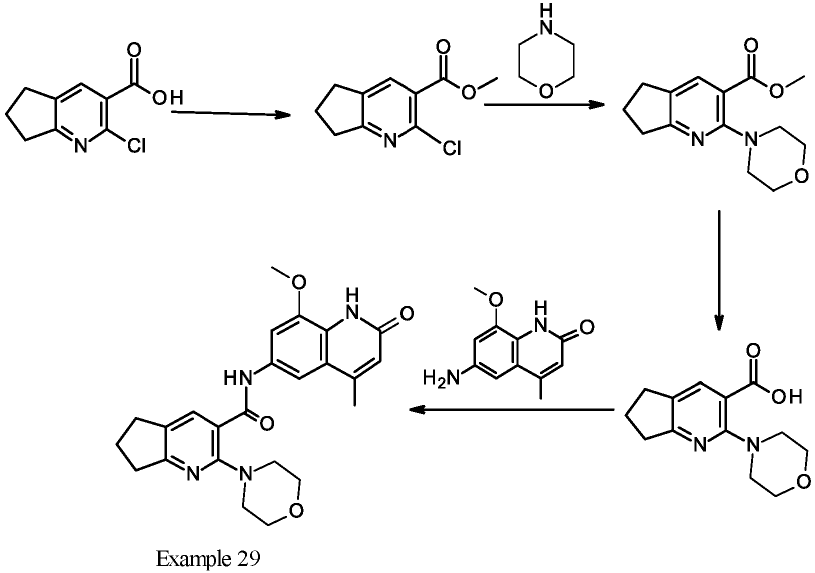

### Preparation of methyl 2-chloro-6,7-dihydro-5H-cyclopenta[b]pyridine-3-carboxylate:

To a solution of 2-chloro-6,7-dihydro-5H-cyclopenta[b]pyridine-3-carboxylic acid (150 mg, 0.7 mmol, 1 eq) in MeOH (2mL) and DCM (4 mL) cooled on an ice bath, diazomethyl(trimethyl)silane 2M in hexane (400 uL) was added and the reaction mixture stirred at RT for 1 h. Additional 300 uL diazomethyl(trimethyl)silane was added (total 700 uL, 1.4 mmol, 2 eq) and stirred for 30 min. After completion, solvent was evaporated in vacuo to give methyl 2-chloro-6,7-dihydro-5H-cyclopenta[b]pyridine-3-carboxylate (145 mg, 98% yield). LCMS [M+H]⁺ 212.12 m/z. ¹H NMR (300 MHz, DMSO-d6) δ 7.93 (t, J = 1.1 Hz, 1H), 3.74 (s, 3H), 2.81 (dd, J = 8.2, 7.1 Hz, 4H), 2.04 - 1.92 (m, 2H).

### Preparation of methyl 2-morpholino-6,7-dihydro-5H-cyclopenta[b]pyridine-3-carboxylate:

To a solution of methyl 2-chloro-6,7-dihydro-5H-cyclopenta[b]pyridine-3-carboxylate (143 mg, 0.68 mmol, 1 eq) in dry DMSO (6 mL) morpholine (118 uL, 1.35 mmol, 2eq) and DIPEA (354 uL, 2.03 mmol, 3 eq) were added and the reaction mixture stirred at 120°C overnight. Reaction mixture was then poured into 5% LiCl and extracted with EtOAc (2 x 30 mL). The combined extracts were washed with water, 5% LiCl and brine. After evaporation of the solvent, the crude product was purified by silica flash chromatography in heptane/EtOAc (20-50% EtOAc) to afford 111 mg of methyl 2-morpholino-6,7-dihydro-5H-cyclopenta[b]pyridine-3-carboxylate (52% yield). LCMS [M+H]+ 263.2 m/z. ¹H NMR (300 MHz, DMSO-d6) δ 7.80 (s, 1H), 3.79 (s, 3H), 3.71 - 3.60 (m, 4H), 3.24 - 3.16 (m, 4H), 2.85 - 2.75 (m, 4H), 2.10 - 1.96 (m, 2H).

### Preparation of 2-morpholino-6,7-dihydro-5H-cyclopenta[b]pyridine-3-carboxylic acid:

To a solution of methyl 2-morpholino-6,7-dihydro-5H-cyclopenta[b]pyridine-3-carboxylate (109 mg, 0.42 mmol, 1 eq) in methanol (3 mL) and THF (4 mL), 2M LiOH was added (1.5 mL) and the reaction mixture stirred at RT overnight. Organic solvent was evaporated in vacuo and the mixture neutralized with 10% KHSO₄. It was then extracted with EtOAc, washed with brine, dried and evaporated give 77 mg of crude 2-morpholino-6,7-dihydro-5H-cyclopenta[b]pyridine-3-carboxylic acid (75% yield). LCMS [M+H]+ 249.21 m/z. ¹H NMR (300 MHz, DMSO-d6) δ 13.55 (s, 1H), 7.88 (s, 1H), 3.74 - 3.61 (m, 4H), 3.24 - 3.15 (m, 4H), 2.90-2.79 (m, 4H), 2.13 - 2.00 (m, 2H).

### Preparation of N-(8-methoxy-4-methyl-2-oxo-1H-quinolin-6-yl)-2-morpholino-6,7-dihydro-5H-cyclopenta[b]pyridine-3-carboxamide (Example 29):

To a solution of 2-morpholino-6,7-dihydro-5H-cyclopenta[b]pyridine-3-carboxylic acid (77 mg, 0.31 mmol, 1.0 eq) and 6-amino-8-methoxy-4-methyl-1H-quinolin-2-one (75 mg, 0.37 mmol, 1.2 eq) in DMF (3 mL), were added DIPEA (267 uL, 1.53 mmol, 5 eq) and HOAt (50 mg, 0.37 mmol, 1.2 eq) and EDC·HCl (70 mg, 0.37 mmol, 1.2 eq) and the reaction mixture was stirred at RT overnight. The solvent was evaporated in vacuo and the crude product purified by silicagel flash chromatography in DCM/MeOH, (0-10% MeOH) to give 80 mg of N-(8-methoxy-4-methyl-2-oxo-1H-quinolin-6-yl)-2-morpholino-6,7-dihydro-5H-cyclopenta[b]pyridine-3-carboxamide (59% yield). LCMS [M+H]⁺ 435.20 m/z. ¹H NMR (300 MHz, DMSO-d6) δ 10.67 (s, 1H), 10.64 (s, 1H), 7.76 (2H), 7.59 (d, J = 1.9 Hz, 1H), 6.45 (s, 1H), 3.89 (s, 3H), 3.67 (t, J = 4.6 Hz, 4H), 3.20 (t, J = 4.6 Hz, 4H), 2.93 - 2.82 (m, 4H), 2.39 (d, J = 1.2 Hz, 3H), 2.09 (q, J = 7.7 Hz, 2H).

### Preparation of (5R)-N-(8-methoxy-4-methyl-2-oxo-1H-quinolin-6-yl)-5-methyl-2-morpholino-5,7-dihydrofuro[3,4-b]pyridine-3-carboxamide (Example 30):

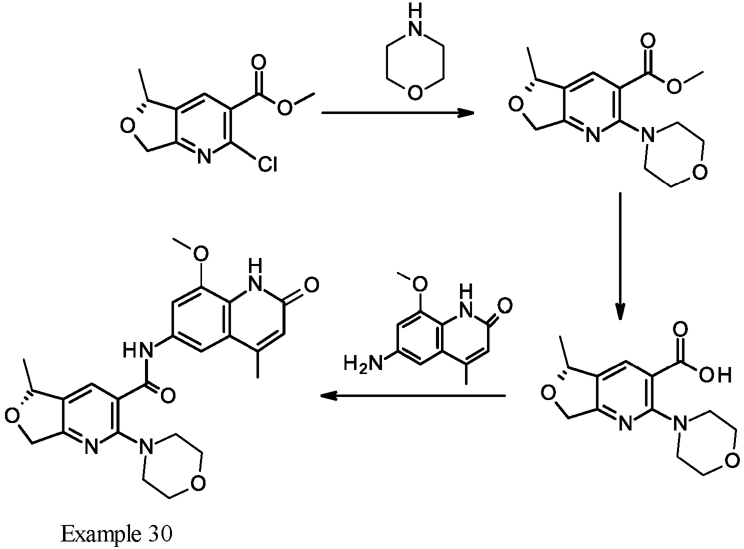

### Preparation of methyl (5R)-5-methyl-2-morpholino-5,7-dihydrofuro[3,4-b]pyridine-3-carboxylate:

To a solution of methyl (5R)-2-chloro-5-methyl-5,7-dihydrofuro[3,4-b]pyridine-3-carboxylate (172 mg, 1 eq) in dry DMF (2 mL), morpholine (2mL), was added and the reaction stirred at 100°C for 1 h. After completion, the reaction mixture was poured into water and extracted with EtOAc. The combined extracts were washed with water, 5% LiCl and brine, dried over anhydrous Na₂SO₄, filtered and evaporated. The crude compound was purified by flash chromatography using EtOAc/heptane, (1-50% EtOAc) to afford methyl (5R)-5-methyl-2-morpholino-5,7-dihydrofuro[3,4-b]pyridine-3-carboxylate (54.6 mg, 31 % yield). LCMS [M+H]+: 279.21 m/z. ¹H NMR (300 MHz, CDCl₃) δ 7.86 (d, J = 0.9 Hz, 1H), 5.34 - 5.24 (m, 1H), 5.05 - 4.87 (m, 2H), 3.92 (s, 3H), 3.87-3.81 (m, 4H), 3.47 - 3.38 (m, 4H), 1.50 (d, 3H).

### Preparation of (5R)-5-methyl-2-morpholino-5,7-dihydrofuro[3,4-b]pyridine-3-carboxylic acid:

To a solution of methyl (5R)-5-methyl-2-morpholino-5,7-dihydrofuro[3,4-b]pyridine-3-carboxylate (52 mg, 0.19 mmol, 1 eq) in methanol (0.5 mL) and THF (0.5 mL), 2M LiOH was added (200 uL) and the reaction mixture stirred at RT overnight. 2N HCl (200 µL) was added. Solvent was evaporated in vacuo with acetonitrile and toluene to remove excess HCl to afford crude (5R)-5-methyl-2-morpholino-5,7-dihydrofuro[3,4-b]pyridine-3-carboxylic acid. (yield 90%) LCMS [M+H]⁺ 279.2 m/z.

### Preparation of (5R)-N-(8-methoxy-4-methyl-2-oxo-1H-quinolin-6-yl)-5-methyl-2-morpholino-5,7-dihydrofuro[3,4-b]pyridine-3-carboxamide (Example 30):

To a solution of (5R)-5-methyl-2-morpholino-5,7-dihydrofuro[3,4-b]pyridine-3-carboxylic acid (0.19 mmol, 1.0 eq) and 6-amino-8-methoxy-4-methyl-1H-quinolin-2-one (42 mg, 0.20 mmol, 1.1 eq) in DMF (1 mL), were added DIPEA (65 uL, 0.37 mmol, 2 eq) and HATU (78 mg, 0.20 mmol, 1.1 eq) and the reaction mixture stirred at 45°C for 1.5 h. The solvent was evaporated in vacuo and the crude product purified by reverse phase column chromatography to give 32 mg of (5R)-N-(8-methoxy-4-methyl-2-oxo-1H-quinolin-6-yl)-5-methyl-2-morpholino-5,7-dihydrofuro[3,4-b]pyridine-3-carboxamide, (38% yield) LCMS [M+H]+ M/z+1 451.28. ¹H NMR (300 MHz, DMSO-d6) δ 10.69 - 10.60 (s, 1H), 10.56 (s, 1H), 7.81 (d, J = 0.9 Hz, 1H), 7.75 (d, J = 1.9 Hz, 1H), 7.57 (d, J = 1.9 Hz, 1H), 6.46 (s, 1H), 5.33-5.23 (m, 1H), 4.95 - 4.80 (m, 2H), 3.89 (s, 3H), 3.69-3.61 (m, 4H), 3.29 - 3.24 (m, 4H), 2.39 (d, J = 1.2 Hz, 3H), 1.43 (d, J = 6.3 Hz, 3H).

### Preparation of N-(8-methoxy-4-methyl-2-oxo-1H-quinolin-6-yl)-5-methyl-2-morpholino-5,7-dihydrofuro[3,4-b]pyridine-3-carboxamide (Example 31)

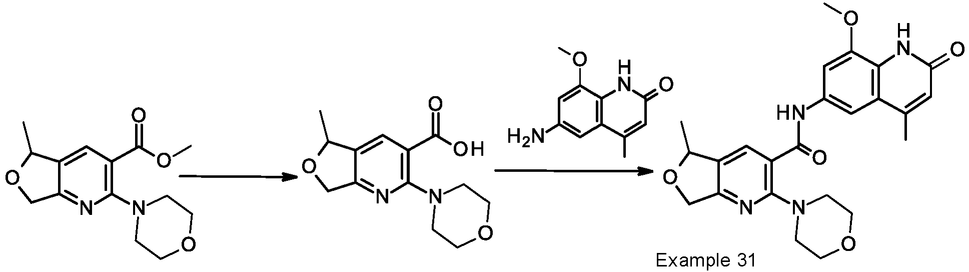

To a solution of ethyl 5-methyl-2-morpholino-5,7-dihydrofuro[3,4-b]pyridine-3-carboxylate (50 mg, 0.18mmol, 1eq) in MeOH (0.5ml), was added LiOH 2M aq. (0.18ml, 0.36mmol, 2eq) and shaken at 45°C for 30 min. LiOH 2M aq. (0.09ml, 0.18mmol, 1eq) was added and shaken at 45°C. After 2h, the reaction mixture was evaporated and 5M HCl aq (0.108ml, 0.540, 3eq) was added, followed by ACN (1ml) and then evaporated. The residue was evaporated from toluene (2x1ml). Crude 5-methyl-2-morpholino-5,7-dihydrofuro[3,4-b]pyridine-3-carboxylic acid was dissolved in DMF (0.7ml) and 6-amino-8-methoxy-4-methyl-1H-quinolin-2-one (40mg, 0.2mmol, 1.1eq) was added followed by DIPEA (0.062 ml, 0.36 mmol, 2 eq). Then HATU (76 mg, 0.2 mmol, 1.1 eq) was added, subjected to ultrasound and shaken at 45°C. After 1.5h, HATU (15 mg, 0.039 mmol, 0.2 eq) was then added and shaken at 45°C for 15 min. The reaction mixture was directly purified on Biotage Isolera: ACN(0.1%FA)/H2O(0.1%FA) 2/98 then 2/98->70/30. Fractions with the product were evaporated, triturated with ACN (0.5ml), filtered and washed with ACN (0.7ml) to afford N-(8-methoxy-4-methyl-2-oxo-1H-quinolin-6-yl)-5-methyl-2-morpholino-5,7-dihydrofuro[3,4-b]pyridine-3-carboxamide (Example 31), 26 mg (32% over two steps) as a brown oil. LCMS [M+H]⁺ 451 m/z. ¹H NMR (300 MHz, DMSO-d6) δ 10.63 (s, 1H), 10.56 (s, 1H), 7.81 (d, J = 0.9 Hz, 1H), 7.75 (d, J = 2.0 Hz, 1H), 7.57 (d, J = 1.9 Hz, 1H), 6.46 (s, 1H), 5.28 (q, J = 6.0 Hz, 1H), 5.00 - 4.71 (m, 2H), 3.89 (s, 3H), 3.69-3.62 (m, 4H), 3.31-3.22 (m, 4H), 2.39 (d, J = 1.2 Hz, 3H), 1.43 (d, J = 6.3 Hz, 3H).

### General method 1

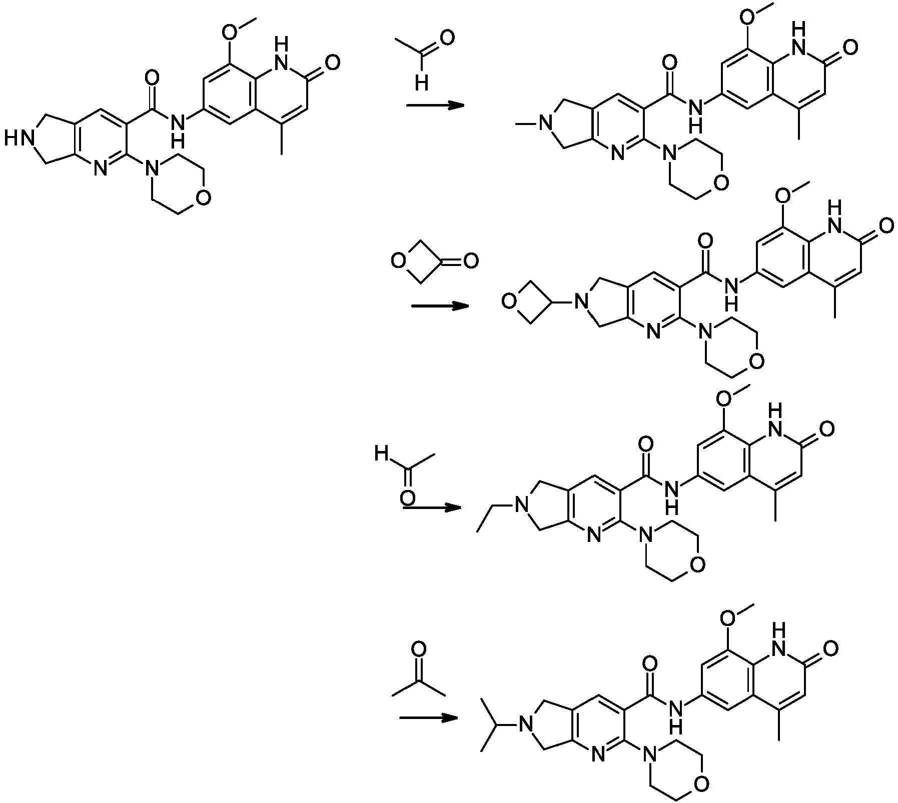

Protocol: A 100 mM solution of N-(8-methoxy-4-methyl-2-oxo-1H-quinolin-6-yl)-2-morpholino-6,7-dihydro-5H-pyrrolo[3,4-b]pyridine-3-carboxamide in dry THF (24 µl, 1 eq) was mixed with of a 300 mM solution of the aldehyde in dry THF (24 µl, 3 eq). The solution was then added to 6.0 µmol SiliaBond cyanoborohydride in a filterplate. The plate was washed with 12 µl of 300 mM acetic acid in dry THF and shaken at RT overnight. THF (100 µl) was then added to the well and the mixture was drained, collected and washed with 100 µl acetonitrile. Solvents were removed by SpeedVac and the compound analyzed and purified by HPLC-CLND.

### Analytical system:

Agilent Infinity I/II -TOF6230B /CLND Antek 8060 equipped with Acquity BEH C18 (1.7µm, 2.1 x 50 mm) with a linear gradient of the binary solvent system water/methanol/formic acid (A: 100/0/0.1% and B: 0/100/0.1%) with a flow rate of 0.75 mL/min and DAD.

### Purification system:

Shimadzu Nexera X2 system using a C18 reverse phase column (Merck Chromolith Speedrod RP-18E, 10 x 100 mm) with a linear gradient of the binary solvent system water/methanol/formic acid (A: 100/0/0.1% and B: 0/100/0.1%) with a flow rate of 7.0 mL/min and UV detection at 254 nm, combined with MS detecting on a Shimadzu LCMS [M+H]+-2020.

After purification, solvents were removed by SpeedVac (45 °C, 2-3 hrs).

### Preparation of N-(8-methoxy-4-methyl-2-oxo-1H-quinolin-6-yl)-6-methyl-2-morpholino-5,7-dihydropyrrolo[3,4-b]pyridine-3-carboxamide (Example 32)

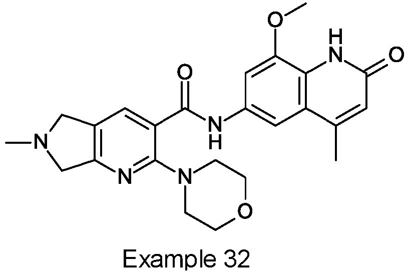

The compound was prepared according to General method 1 reacting (N-(8-methoxy-4-methyl-2-oxo-1H-quinolin-6-yl)-2-morpholino-6,7-dihydro-5H-pyrrolo[3,4-b]pyridine-3-carboxamide) (Example 1) and formaldehyde. Isolated amount after purification 14.8 µg. LCMS [M+H]⁺ 450.22 m/z.

### Preparation of N-(8-methoxy-4-methyl-2-oxo-1H-quinolin-6-yl)-2-morpholino-6-(oxetan-3-yl)-5,7-dihydropyrrolo[3,4-b]pyridine-3-carboxamide (Example 33):

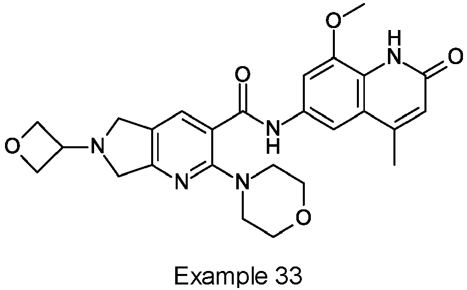

The compound was prepared according to General method 1 reacting (N-(8-methoxy-4-methyl-2-oxo-1H-quinolin-6-yl)-2-morpholino-6,7-dihydro-5H-pyrrolo[3,4-b]pyridine-3-carboxamide) (Example 1) and oxetan-3-one. Isolated amount after purification 150 µg. LCMS [M+H]+ 492.23 m/z.

### Preparation of 6-ethyl-N-(8-methoxy-4-methyl-2-oxo-1H-quinolin-6-yl)-2-morpholino-5,7-dihydropyrrolo[3,4-b]pyridine-3-carboxamide (Example 34):

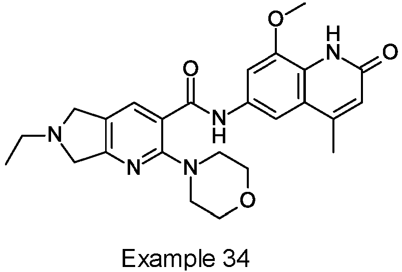

The compound was prepared according to General method 1 reacting (N-(8-methoxy-4-methyl-2-oxo-1H-quinolin-6-yl)-2-morpholino-6,7-dihydro-5H-pyrrolo[3,4-b]pyridine-3-carboxamide) (Example 1) and acetaldehyde. Isolated amount after purification 105 µg. LCMS [M+H]+ 464.24 m/z.

### Preparation of 6-isopropyl-N-(8-methoxy-4-methyl-2-oxo-1H-quinolin-6-yl)-2-morpholino-5,7-dihydropyrrolo[3,4-b]pyridine-3-carboxamide (Example 35):

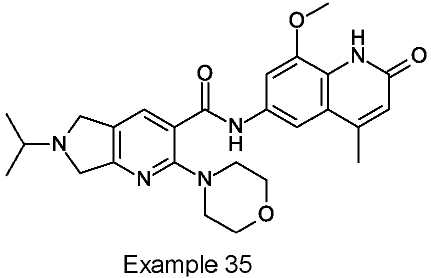

The compound was prepared according to General method 1 reacting (N-(8-methoxy-4-methyl-2-oxo-1H-quinolin-6-yl)-2-morpholino-6,7-dihydro-5H-pyrrolo[3,4-b]pyridine-3-carboxamide) (Example 1) and acetone. Isolated amount after purification 71 µg. LCMS [M+H]⁺ 478.25 m/z.

### General method 2

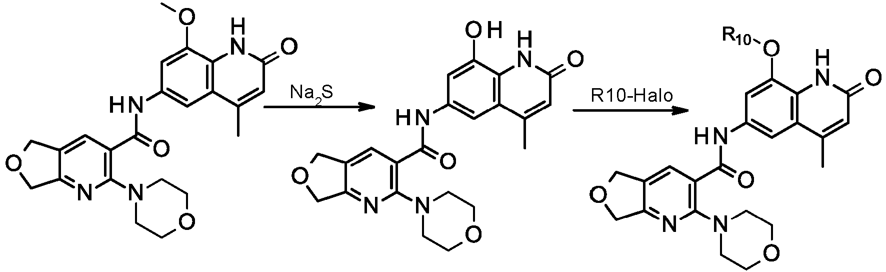

### Preparation of N-(8-hydroxy-4-methyl-2-oxo-1H-quinolin-6-yl)-2-morpholino-5,7-dihydrofuro[3,4-b]pyridine-3-carboxamide:

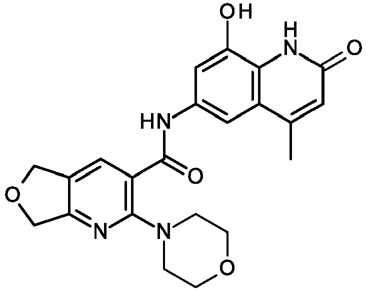

To a suspension of N-(8-methoxy-4-methyl-2-oxo-1H-quinolin-6-yl)-2-morpholino-5,7-dihydrofuro[3,4-b]pyridine-3-carboxamide (800 mg, 1.83 mmol, 1.0 equiv.) in dry NMP (3.0 mL) was added Na₂S (715 mg, 9.2 mmol, 5.0 equiv.) and the mixture was stirred at 140°C for 48 h. The crude reaction mixture was then poured over ice-cold water (100 mL) and the aqueous solution was acidified until pH ~4. The formed precipitate was filtered and washed with H₂O to afford the title compound as a light brown solid (500 mg, 66%) which was used in next steps without further purification.

### General protocol for alkylation

To a solution of N-(8-hydroxy-4-methyl-2-oxo-1H-quinolin-6-yl)-2-morpholino-5,7-dihydrofuro[3,4-b]pyridine-3-carboxamide (1 equiv.) and alkylating agent (2.0 equiv.) in DMF was treated with K₂CO₃ (3.0 equiv.) and the reaction mixture was stirred at 100°C for 2-16h. The crude reaction mixture was then filtered through a cotton path and directly purified by reversed phased preparative HPLC.

### General protocol for deprotection

The isolated intermediates were dissolved in a mixture of DMSO and 4M HCl in dioxane (1: 1) and stirred at 60°C for 2h. The reaction mixture was then concentrated *in vacuo* and the residue purified by reversed-phase preparative-HPLC to afford the final products.

### Preparation of N-[8-(2-hydroxyethoxy)-4-methyl-2-oxo-1H-quinolin-6-yl]-2-morpholino-5,7-dihydrofuro[3,4-b]pyridine-3-carboxamide (Example 36):

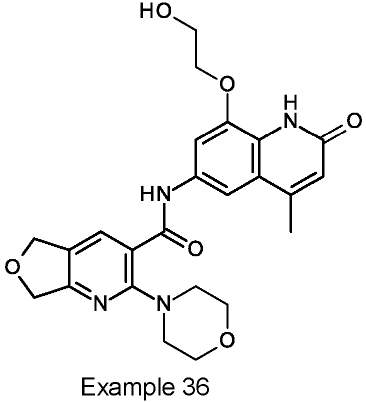

Following the general protocol for alkylation using 0.23 mmol of N-(8-hydroxy-4-methyl-2-oxo-1H-quinolin-6-yl)-2-morpholino-5,7-dihydrofuro[3,4-b]pyridine-3-carboxamide, 0.46 mmol of 2-bromoethanol and 0.39 mmol of K₂CO₃, the title compound N-[8-(2-hydroxyethoxy)-4-methyl-2-oxo-1H-quinolin-6-yl]-2-morpholino-5,7-dihydrofuro[3,4-b]pyridine-3-carboxamide was afforded as a solid (30 mg, 27%). ¹H NMR (300 MHz, DMSO-d6) δ 10.97 (s, 1H), 10.56 (s, 1H), 7.84 (s, 1H), 7.76 (d, J = 1.9 Hz, 1H), 7.53 (d, J = 1.9 Hz, 1H), 6.48 (t, J = 1.6 Hz, 1H), 5.35 (t, J = 6.9 Hz, 1H), 5.04 (s, 2H), 4.88 (s, 2H), 4.03 (t, J = 4.3 Hz, 2H), 3.83 (d, J = 7.7 Hz, 2H), 3.67-3.61 (m, 4H), 3.28-3.23 (m, 4H), 2.40 (d, J = 1.2 Hz, 3H).

### Preparation of N-[8-(azetidin-3-yloxy)-4-methyl-2-oxo-1H-quinolin-6-yl]-2-morpholino-5,7-dihydrofuro[3,4-b]pyridine-3-carboxamide (Example 37):

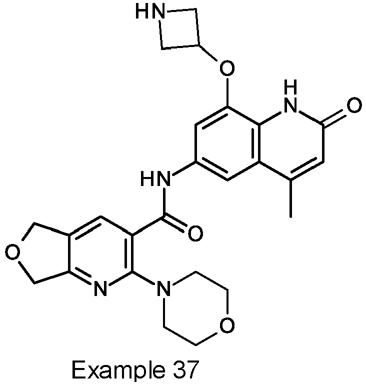

Following the general protocol for alkylation using 0.34 mmol of N-(8-hydroxy-4-methyl-2-oxo-1H-quinolin-6-yl)-2-morpholino-5,7-dihydrofuro[3,4-b]pyridine-3-carboxamide, 0.68 mmol of tert-butyl 3-bromoazetidine-1-carboxylate and 1.02 mmol of K₂CO₃, the intermediate compound tert-butyl 3-[[4-methyl-6-[(2-morpholino-5,7-dihydrofuro[3,4-b]pyridine-3-carbonyl)amino]-2-oxo-1H-quinolin-8-yl]oxy]azetidine-1-carboxylate was afforded as a solid (75 mg, 37%). Following the general procedure for deprotection using 0.1 mmol of tert-butyl 3-[[4-methyl-6-[(2-morpholino-5,7-dihydrofuro[3,4-b]pyridine-3-carbonyl)amino]-2-oxo-1H-quinolin-8-yl]oxy]azetidine-1-carboxylate, the title product N-[8-(azetidin-3-yloxy)-4-methyl-2-oxo-1H-quinolin-6-yl]-2-morpholino-5,7-dihydrofuro[3,4-b]pyridine-3-carboxamide was afforded as a solid (42 mg, 86%). ¹H NMR (300 MHz, DMSO-d6) δ 11.11 (s, 1H), 10.55 (s, 1H), 9.24 (d, J = 29.9 Hz, 2H), 7.87 (d, J = 1.8 Hz, 1H), 7.84 (s, 1H), 7.30 (d, J = 1.9 Hz, 1H), 6.50 (s, 1H), 5.23-5.10 (m, 1H), 5.04 (s, 2H), 4.89 (s, 2H), 4.46 - 4.19 (m, 4H), 3.71-3.58 (m, 4H), 3.30-3.23 (m, 4H), 2.40 (d, J = 1.2 Hz, 3H).

### Preparation of N-[4-methyl-8-[2-(methylamino)ethoxy]-2-oxo-1H-quinolin-6-yl]-2-morpholino-5,7-dihydrofuro[3,4-b]pyridine-3-carboxamide (Example 38):

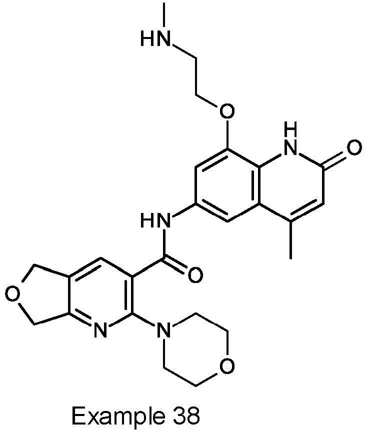

Following the general protocol for alkylation using 0.34 mmol of N-(8-hydroxy-4-methyl-2-oxo-1H-quinolin-6-yl)-2-morpholino-5,7-dihydrofuro[3,4-b]pyridine-3-carboxamide, 0.68 mmol of tert-butyl N-(2-bromoethyl)carbamate and 1.02 mmol of K₂CO₃, the intermediate compound tert-butyl N-methyl-N-[2-[[4-methyl-6-[(2-morpholino-5,7-dihydrofuro[3,4-b]pyridine-3-carbonyl)amino]-2-oxo-1H-quinolin-8-yl]oxy]ethyl]-carbamate was afforded as a solid (19 mg, 9%). Following the general procedure for deprotection using 0.04 mmol of tert-butyl N-methyl-N-[2-[[4-methyl-6-[(2-morpholino-5,7-dihydrofuro[3,4-b]pyridine-3-carbonyl)amino]-2-oxo-1H-quinolin-8-yl]oxy]ethyl]-carbamate, the title product N-[4-methyl-8-[2-(methylamino)ethoxy]-2-oxo-1H-quinolin-6-yl]-2-morpholino-5,7-dihydrofuro[3,4-b]pyridine-3-carboxamide was afforded as a solid (11 mg, 70%). ¹H NMR (300 MHz, Methanol-d4) δ 8.11 (s, 1H), 7.87 (d, J = 1.9 Hz, 1H), 7.81 (d, J = 1.9 Hz, 1H), 6.69 (s, 1H), 5.18 - 5.14 (m, 2H), 5.07-5.02 (m, 2H), 4.57 - 4.51 (m, 2H), 3.86 - 3.79 (m, 4H), 3.68-3.62 (m, 2H), 3.50 - 3.43 (m, 4H), 2.91 (s, 3H), 2.57 (s, 3H).

### Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide (Example 39)

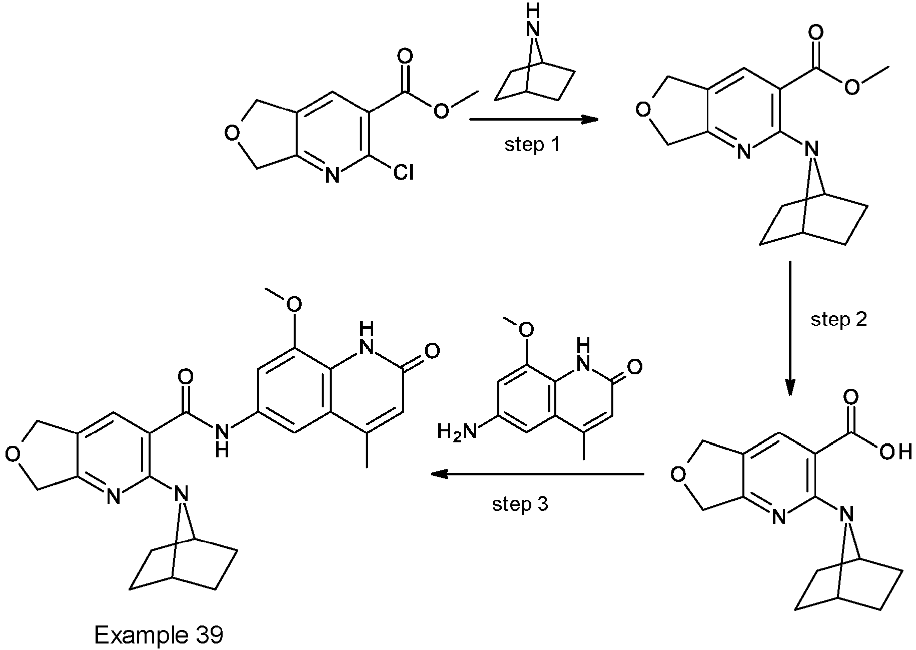

### Step 1: Preparation of 2-(7-azabicyclo[2.2.1]heptan-7-yl)-5,7-dihydrofuro[3,4-b]pyridine-3-carboxylate

A solution of methyl 2-chloro-5,7-dihydrofuro[3,4-b]pyridine-3-carboxylate (0.7 mmol, 1.0 equiv.), 7-azabicyclo[2.2.1]heptane hydrochloride (1.5 mmol, 2.0 equiv.) and DIPEA (1.5 mmol, 2.0 equiv.) in dry DMF (3 mL) was stirred at 120°C for 6h. The reaction mixture was then diluted with EtOAC (75 mL), washed with sat. aq. NaHCO₃ (2 x 25 mL) and 5% aq. LiCl (25 mL). The organic phase was dried over MgSO4, filtered and concentrated in vacuo. The residue was purified by column chromatography on silica gel (Isolera One, Biotage) using a mixture of Heptane/EtOAc (from 100/0 to 60/40) to afford methyl 2-(7-azabicyclo[2.2.1]heptan-7-yl)-5,7-dihydrofuro[3,4-b]pyridine-3-carboxylate as white solid (20 mg, 10%). 1H NMR (300 MHz, Methanol-d4) δ 7.89 (d, J = 1.7 Hz, 1H), 5.07-5.02 (m, 2H), 4.30-4.23 (m, 2H), 3.90 (s, 3H), 1.87-1.83 (m, 4H), 1.54-1.40(m, 4H).

### Step 2: Preparation of 2-(7-azabicyclo[2.2.1]heptan-7-yl)-5,7-dihydrofuro[3,4-b]pyridine-3-carboxylic acid

Methyl 2-(7-azabicyclo[2.2.1]heptan-7-yl)-5,7-dihydrofuro[3,4-b]pyridine-3-carboxylate (20 mg, 1 eq) was dissolved in 20 mL of mixture of MeOH/0.4M aq. NaOH (1/1) and the reaction mixture was stirred at 60°C for 16h. The crude reaction was neutralized with 2M aq. HCl and concentrated *in vacuo* to afford the 2-(7-azabicyclo[2.2.1]heptan-7-yl)-5,7-dihydrofuro[3,4-b]pyridine-3-carboxylic acid as crude product that was used directly in the next step without further purification.

### Step 3: Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide (Example 39):

The crude 2-(7-azabicyclo[2.2.1]heptan-7-yl)-5,7-dihydrofuro[3,4-b]pyridine-3-carboxylic acid (0.07 mmol, 1.0 eq) was suspended on DMF (2.0 mL) and treated with 6-amino-8-methoxy-4-methyl-1H-quinolin-2-one (1.1 equiv.), EDC·HCl (1.1 equiv.), HOAt (1.1 equiv) and DIPEA (2.5 equiv.). The reaction mixture was stirred at 60°C for 16 h. The crude reaction was filtered through a cotton pad and directly purified by reversed-phase preparative-HPLC to afford 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide as a solid (30 mg, 88% over two steps). ¹H NMR (300 MHz, DMSO-d6) δ 10.65 (s, 1H), 10.43 (s, 1H), 7.78 (d, J = 1.9 Hz, 1H), 7.71 (s, 1H), 7.57 (d, J = 1.9 Hz, 1H), 6.46 (s, 1H), 5.01 (s, 2H), 4.85 (s, 2H), 4.28 (s, 2H), 3.88 (s, 3H), 2.38 (d, J = 1.2 Hz, 3H), 1.73-1.62 (m, 4H), 1.44-1.36 (d, J = 6.8 Hz, 4H).

### Preparation of 2-[(3R)-3-fluoropyrrolidin-1-yl]-N-(8-methoxy-4-methyl-2-oxo-1H-quinolin-6-yl)-5,7-dihydrofuro[3,4-b]pyridine-3-carboxamide (Example 40):

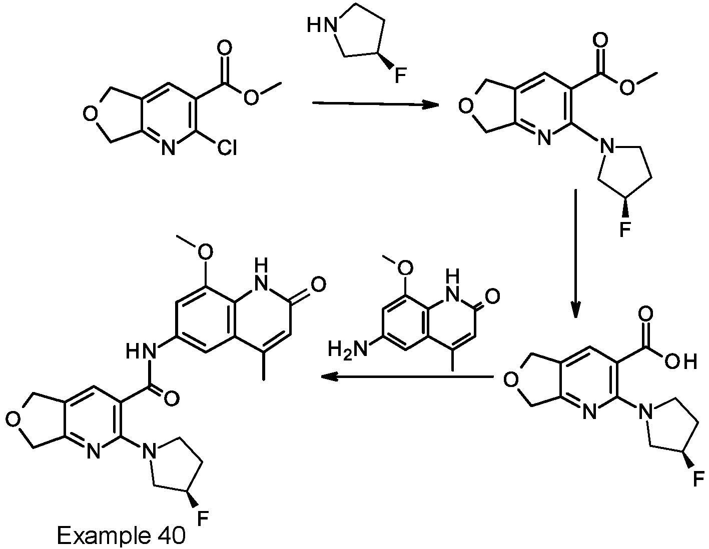

### Preparation of methyl 2-[(3R)-3-fluoropyrrolidin-1-yl]-5,7-dihydrofuro[3,4-b]pyridine-3-carboxylate

Following the procedure described for Step 1, Example 39 Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide, using 0.7 mmol of 2-chloro-5,7-dihydrofuro[3,4-b]pyridine-3-carboxylate and 1.5 mmol of (3R)-3-fluoropyrrolidine hydrochloride, the intermediate product methyl 2-[(3R)-3-fluoropyrrolidin-1-yl]-5,7-dihydrofuro[3,4-b]pyridine-3-carboxylate was afforded as a solid (140 mg, 75%).

### Preparation of 2-[(3R)-3-fluoropyrrolidin-1-yl]-N-(8-methoxy-4-methyl-2-oxo-1H-quinolin-6-yl)-5,7-dihydrofuro[3,4-b]pyridine-3-carboxamide (Example 40):

Following the procedure described for Step 2 and 3, Example 39 Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide using 0.52 mmol of the intermediate compound methyl 2-[(3R)-3-fluoropyrrolidin-1-yl]-5,7-dihydrofuro[3,4-b]pyridine-3-carboxylate followed by the amide coupling with 6-amino-8-methoxy-4-methyl-1H-quinolin-2-one (1.1 equiv.), the final product 2-[(3R)-3-fluoropyrrolidin-1-yl]-N-(8-methoxy-4-methyl-2-oxo-1H-quinolin-6-yl)-5,7-dihydrofuro[3,4-b]pyridine-3-carboxamide was afforded as a solid (35 mg, 20% over two steps) after purification by column chromatography on silica gel (Isolera One, Biotage) using a mixture of DCM/MeOH (from 100/0 to 80/20) followed by recrystallization in MeOH instead of preparative-HPLC purification. ¹H NMR (300 MHz, DMSO-d6) δ 10.65 (s, 1H), 10.54 (s, 1H), 7.74 (d, J = 1.9 Hz, 1H), 7.70 (s, 1H), 7.56 (d, J = 1.9 Hz, 1H), 6.45 (s, 1H), 5.37 (d, J = 53.8 Hz, 1H), 5.01 (s, 2H), 4.93 - 4.77 (m, 2H), 3.88 (s, 3H), 3.86 - 3.45 (m, 4H), 2.38 (d, J = 1.2 Hz, 3H), 2.31-2.09 (m, 2H).

### Preparation of 2-[(3S)-3-fluoropyrrolidin-1-yl]-N-(8-methoxy-4-methyl-2-oxo-1H-quinolin-6-yl)-5,7-dihydrofuro[3,4-b]pyridine-3-carboxamide (Example 41):

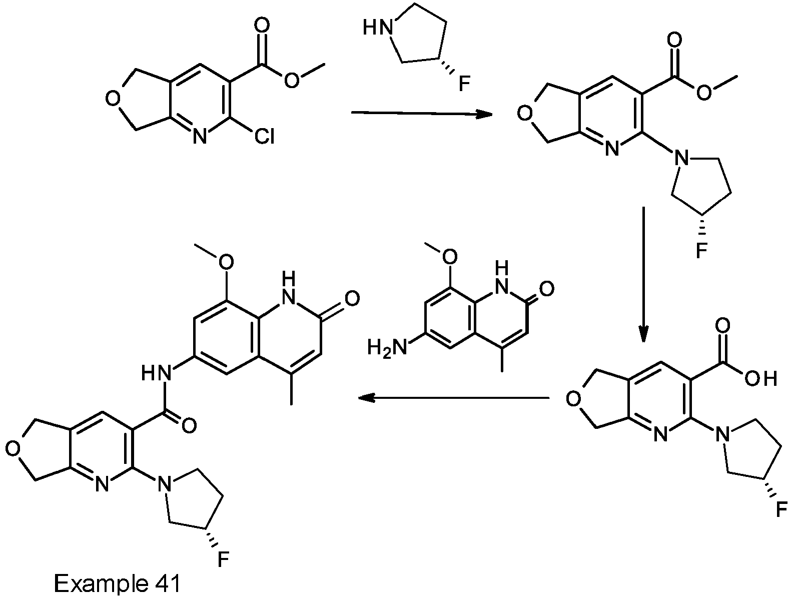

### Preparation of methyl 2-[(3R)-3-fluoropyrrolidin-1-yl]-5,7-dihydrofuro[3,4-b]pyridine-3-carboxylate:

Following procedure described for Step 1, Example 39 Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide using 0.7 mmol of 2-chloro-5,7-dihydrofuro[3,4-b]pyridine-3-carboxylate and 1.5 mmol of (3 S)-3-fluoropyrrolidine hydrochloride, the intermediate product methyl 2-[(3R)-3-fluoropyrrolidin-1-yl]-5,7-dihydrofuro[3,4-b]pyridine-3-carboxylate was afforded as a solid (140 mg, 75%).

### Preparation of 2-[(3S)-3-fluoropyrrolidin-1-yl]-N-(8-methoxy-4-methyl-2-oxo-1H-quinolin-6-yl)-5,7-dihydrofuro[3,4-b]pyridine-3-carboxamide (Example 41):

Following similar procedure as described for Step 2 and 3, Example 39 Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide, using 0.52 mmol of the intermediate compound methyl 2-[(3S)-3-fluoropyrrolidin-1-yl]-5,7-dihydrofuro[3,4-b]pyridine-3-carboxylate, followed by the amide coupling with 6-amino-8-methoxy-4-methyl-1H-quinolin-2-one (1.1 equiv.), the final product 2-[(3S)-3-fluoropyrrolidin-1-yl]-N-(8-methoxy-4-methyl-2-oxo-1H-quinolin-6-yl)-5,7-dihydrofuro[3,4-b]pyridine-3-carboxamide was afforded as a solid (15 mg, 20% over two steps) after purification by column chromatography on silica gel (Isolera One, Biotage) using a mixture of DCM/MeOH (from 100/0 to 80/20) followed by recrystallization in MeOH instead of preparative-HPLC purification. ¹H NMR (300 MHz, DMSO-d6) δ 10.65 (s, 1H), 10.54 (s, 1H), 7.78-7.65 (m, 2H), 7.70 (s, 1H), 7.56 (d, J = 1.9 Hz, 1H), 6.45 (s, 1H), 5.36 (d, J = 53.8 Hz, 1H), 5.01 (s, 2H), 4.93 - 4.76 (m, 2H), 3.88 (s, 3H), 3.85 - 3.58 (m, 4H), 2.37 (s, 3H), 2.31-2.08 (m, 2H).

### Preparation of N-(8-methoxy-4-methyl-2-oxo-1H-quinolin-6-yl)-2-[rac-(3R,4S)-3,4-difluoropyrrolidin-1-yl]-5,7-dihydrofuro[3,4-b]pyridine-3-carboxamide (Example 42):

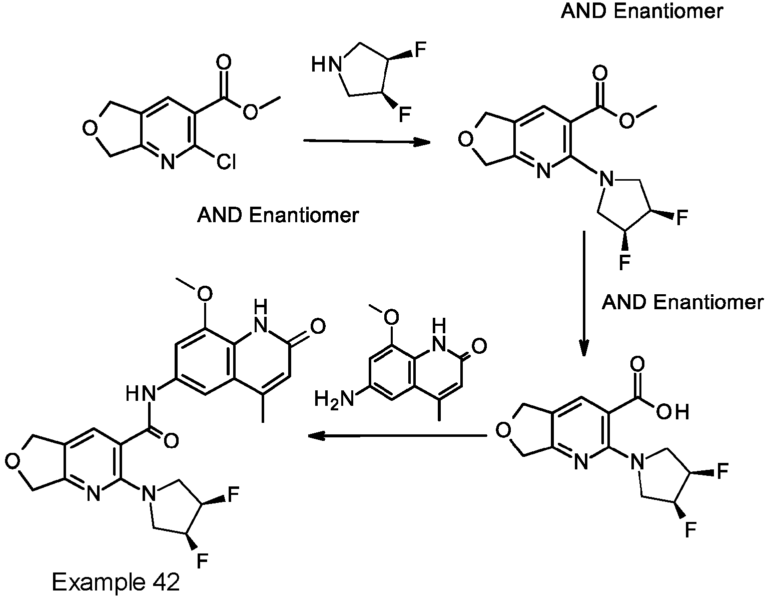

### Preparation of methyl 2-[rac-(3R,4S)-3,4-difluoropyrrolidin-1-yl]-5,7-dihydrofuro[3,4-b]pyridine-3-carboxylate:

Following the procedure described for Step 1, Example 39 Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide, using 0.7 mmol of 2-chloro-5,7-dihydrofuro[3,4-b]pyridine-3-carboxylate and 1.5 mmol of (3R,4S)-3,4-difluoropyrrolidine hydrochloride, the intermediate product methyl 2-[rac-(3R,4S)-3,4-difluoropyrrolidin-1-yl]-5,7-dihydrofuro[3,4-b]pyridine-3-carboxylate was afforded as a solid (140 mg, 70%) using DCM/MeOH (from 100/0 to 85/15) eluent system for purification. ¹H NMR (300 MHz, Chloroform-d) δ 7.84 (s, 1H), 5.33-5.22 (m, 1H), 5.15 - 5.04 (m, 3H), 4.93 (t, J = 1.9 Hz, 2H), 3.92 (s, 3H), 3.90 - 3.60 (m, 4H).

### Preparation of N-(8-methoxy-4-methyl-2-oxo-1H-quinolin-6-yl)-2-[rac-(3R,4S)-3,4-difluoropyrrolidin-1-yl]-5,7-dihydrofuro[3,4-b]pyridine-3-carboxamide (Example 42):

Following the procedure described for Step 2 and 3, Example 39 Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide,using 0.49 mmol of the intermediate compound methyl 2-[rac-(3R,4S)-3,4-difluoropyrrolidin-1-yl]-5,7-dihydrofuro[3,4-b]pyridine-3-carboxylate followed by the amide coupling with 6-amino-8-methoxy-4-methyl-1H-quinolin-2-one (1.1 equiv.), the final product N-(8-methoxy-4-methyl-2-oxo-1H-quinolin-6-yl)-2-[rac-(3R,4S)-3,4-difluoropyrrolidin-1-yl]-5,7-dihydrofuro[3,4-b]pyridine-3-carboxamide was afforded as a solid (98 mg, 74% over two steps).

### Preparation of N-(8-methoxy-4-methyl-2-oxo-1H-quinolin-6-yl)-2-[rac-(3S,4S)-3,4-difluoropyrrolidin-1-yl]-5,7-dihydrofuro[3,4-b]pyridine-3-carboxamide (Example 43):

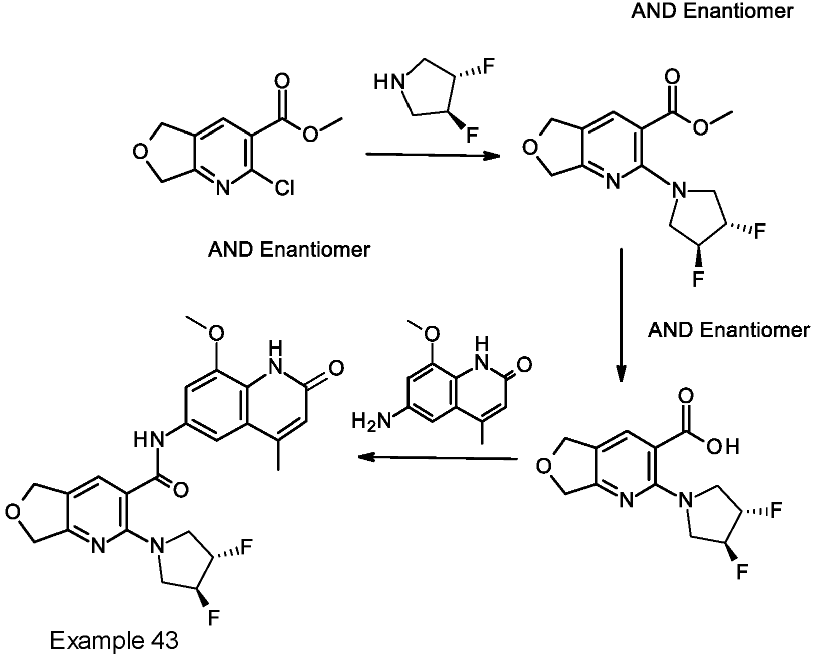

### Preparation of methyl 2-[rac-(3S,4S)-3,4-difluoropyrrolidin-1-yl]-5,7-dihydrofuro[3,4-b]pyridine-3-carboxylate:

Following the procedure described for Step 1, Example 39 Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide using 0.7 mmol of 2-chloro-5,7-dihydrofuro[3,4-b]pyridine-3-carboxylate and 1.5 mmol of (3S,4S)-3,4-difluoropyrrolidine hydrochloride, the intermediate product methyl 2-[rac-(3S,4S)-3,4-difluoropyrrolidin-1-yl]-5,7-dihydrofuro[3,4-b]pyridine-3-carboxylate was afforded as a solid (160 mg, 80%) using DCM/MeOH (from 100/0 to 85/15) eluent system for purification. ¹H NMR (300 MHz, Chloroform-d) δ 7.86 (s, 1H), 5.33-5.29 (m, 1H), 5.20 - 5.11 (m, 1H), 5.10 (s, 2H) 5.03 - 4.88 (m, 2H), 4.23 - 4.10 (m, 1H), 4.09 - 3.99 (m, 1H), 3.92 (s, 3H), 3.65 - 3.48 (m, 2H).

### N-(8-methoxy-4-methyl-2-oxo-1H-quinolin-6-yl)-2-[rac-(3S,4S)-3,4-difluoropyrrolidin-1-yl]-5,7-dihydrofuro[3,4-b]pyridine-3-carboxamide (Example 43):

Following the procedure described for Step 2 and 3, Example 39 Preparation of 2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide, using 0.56 mmol of the intermediate compound methyl 2-[rac-(3S,4S)-3,4-difluoropyrrolidin-1-yl]-5,7-dihydrofuro[3,4-b]pyridine-3-carboxylate followed by the amide coupling with 6-amino-8-methoxy-4-methyl-1H-quinolin-2-one (1.1 equiv.), the final product N-(8-methoxy-4-methyl-2-oxo-1H-quinolin-6-yl)-2-[rac-(3S,4S)-3,4-difluoropyrrolidin-1-yl]-5,7-dihydrofuro[3,4-b]pyridine-3-carboxamide was afforded as a solid (118 mg, 78%).

Compounds were assayed for binding affinity using bromoscan assays and a phosphodiesterase three assay.

Bromoscan assays were run by DiscoveRx Corporation. The relevant assay names are BRD4(1) for the first bromodomain of BRD4 and BRD4(2) for the second bromodomain of BRD4. Further Bromoscan assays covering other bromodomains may be employed to establish the selectivity profile of a compound of interest. To run the Bromoscan assay, a T7 phage strain displaying the relevant bromodomain was grown in parallel in 24-well blocks in an *E. coli* host derived from the BL21 strain. *E. coli* were grown to log-phase and infected with T7 phage from a frozen stock (multiplicity of infection = 0.4) and incubated with shaking at 32°C until lysis (90-150 minutes). The lysates were centrifuged (5,000 x g) and filtered (0.2µm) to remove cell debris. Streptavidin-coated magnetic beads were treated with biotinylated small molecule or acetylated peptide ligands for 30 minutes at room temperature to generate affinity resins for bromodomain assays. The liganded beads were blocked with excess biotin and washed with blocking buffer (SeaBlock (Pierce), 1 % BSA (bovine serum albumin), 0.05 % Tween 20, 1 mM DTT (dithiothreitol)) to remove unbound ligand and to reduce non-specific phage binding. Binding reactions were assembled by combining bromodomains, liganded affinity beads, and test compounds in 1x binding buffer (17% SeaBlock, 0.33x PBS, 0.04% Tween 20, 0.02% BSA, 0.004% Sodium azide, 7.4 mM DTT). Test compounds were prepared as 1000X stocks in 100% DMSO and subsequently diluted 1:10 in monoethylene glycol (MEG) to create stocks at 100X the screening concentration (resulting stock solution is 10% DMSO/90% MEG). The compounds were then diluted directly into the assays such that the final concentration of DMSO and MEG were 0.1% and 0.9%, respectively. All reactions were performed in polystyrene 96-well plates in a final volume of 0.135 ml. The assay plates were incubated at room temperature with shaking for 1 hour and the affinity beads were washed with wash buffer (1x PBS, 0.05% Tween 20). The beads were then re-suspended in elution buffer (1x PBS, 0.05% Tween 20, 2 µM nonbiotinylated affinity ligand) and incubated at room temperature with shaking for 30 minutes. The bromodomain concentration in the eluates was measured by qPCR (quantitative real-time polymerase chain reaction). Binding constants (Kds) were calculated with a standard dose-response curve using the Hill equation: Response=Blackground+ ((Signal-Background)/(1+Kd^{Hill Slope}/Dose^{Hill Slope})). The Hill Slope was set to -1. Curves were fitted using a non-linear least square fit with the Levenberg-Marquardt algorithm.

### Radiometric PDE3A assay

Partially purified human recombinant phosphodiesterase (PDE3A) was obtained by cloning cDNA and expressing in *S. frugiperda* insect cells using a baculovirus expression system. Cells were harvested from culture by centrifuging at 400 x g for 5 minutes. Cell pellet was resuspended in 20ml of RIPA Buffer (150mM NaCl, 10mM Tris, 0.1% NP-40, pH8.3), 4ml/200ml of culture, plus protease inhibitors (100µl/10ml) and incubated on ice for 10-20 minutes then spun at 3500 x g for 10 minutes at 4°C. Supernatant was kept and the pellet thrown away. Tritium-labeled cyclic AMP was hydrolysed to 5'-AMP by PDE3A. The 5'-AMP was then further hydrolysed to adenosine by nucleotidase in snake venom. An anion-exchange resin binds all charged nucleotides and leaves [³H]adenosine as the only labeled compound to be counted by liquid scintillation. The radiometric assay method is a modification of the two-step method of Thompson and Appleman [Biochemistry 10; 311-316; 1971], adapted for a 96 well plate format. 50µl of diluted PDE3A was incubated with 50µl of [³H]-cAMP (specific activity 25 Ci/mmol) and 11µl of 50% DMSO (or compound dilution) for 20 minutes at 30°C. The enzyme was diluted in 20mM Tris HCl pH7.4 (x2.2 final concentration) and [3H]-cAMP were diluted in 10mM MgCl2, 40mM Tris HCl pH 7.4 (x2.2 final concentration). Reactions were carried out in Greiner 96 deep well 1ml master-block. The plates were centrifuged for 9 seconds before the 20 minutes incubation. The reaction was terminated by denaturing the PDE enzyme (at 70°C for 2 minutes, plates were left to cool on ice for 10 minutes) after which 25µl snake venom nucleotidase (225µg/ml final concentration) was added and incubated for 10 minutes (at 30°C), plates were centrifuged for 9 seconds before incubation. After incubation 200µl Dowex resin (1 x 8, 200-400 mesh) was added and the plate was shaken for 20 minutes then centrifuged for 3 minutes at 1000 x g. 50µl of supernatant was removed and added to 200µl of MicroScint-20 in white plates (Greiner 96-well Optiplate) and shaken for 30 minutes before reading on Perkin Elmer TopCount Scintillation Counter. GraphPad Prism^{™} was used to calculate the IC50 values using a sigmoidal dose response (variable slope). The analysis measures 4 parameters including the best fit values for the bottom and top fit of the curve, the hill slope value and the IC50 value.

A suitable range for PDE3A inhibitory activity is a PDE3A IC50>6 µM and a most preferred range is a PDE3A IC50>10 µM.

**Table of results:**

| Example | BRD4(1) Kd µM | BRD4(2) Kd µM | Selectivity BRD4(2)/ BRD4(1) | PDE3A IC50 µM |
|---|---|---|---|---|
| Comparative* Example 128 WO2016016316 | 0.015 | 2.5 | 167 | 0.441 |
| Comparative* Example 99 WO2016016316 | 0.012 | 5.6 | 467 | 3.74 |
| Comparative* Example 107 WO2016016316 | 0.0036 | 0.028 | 8 | 5.4 |

| Example | BRD4(1) Kd µM^{a} [Geomean] | BRD4(2) Kd µM^{a} [Geomean] | Selectivity BRD4(2)/ BRD4(1) | PDE3A IC50 µM |
|---|---|---|---|---|
| 1 | 0.008 | 0.6 | 75 | >10 |
| 2 | 0.064 | 2.6 | 41 | >10 |
| 3 | 0.013 | 0.8 | 62 | |
| 4 | 0.029 | >5 | >172 | |
| 5 | 0.017 | 0.2 | 12 | |
| 6 | 0.120 | 1.3 | 11 | >10 |

| Example | BRD4(1) Kd µM | BRD4(2) Kd µM | Selectivity BRD4(2)/ BRD4(1) | PDE3A IC50 µM |
|---|---|---|---|---|
| 7 | 0.040 | 7.1 | 178 | >10 |
| 8 | 0.056 | >2.5 | 45 | |
| 9 | 0.440 | 7.4 | 17 | |
| 10 | 0.043 | >10 | >232 | |
| 11 | 0.090 | >10 | >111 | 7 |
| 12 | 0.087 | 8.1 | 93 | >10 |
| 13 | 0.280 | >10 | >35 | >10 |
| 14 | 0.026 | 0.4 | 15 | |
| 15 | 0.014 | 2.2 | 157 | >10 |
| 16 | 0.021 | 0.9 | 43 | |
| 17 | 0.025 | 1.1 | 44 | >10 |
| 18 | 0.012 | 0.2 | 17 | |
| 19 | 0.180 | 5.6 | 31 | |
| 20 | 0.070 | 3.4 | 49 | |
| 21 | 0.006 | 1.4 | 233 | |
| 22 | 0.170 | 3.8 | 22 | |
| 23 | 0.190 | 3.2 | 17 | |
| 24 | 0.028 | 0.7 | 25 | >10 |
| 25 | 0.150 | 4.5 | 30 | |
| 26 | 0.051 | 3.1 | 61 | >10 |
| 27 | 0.025 | 0.6 | 24 | >10 |
| 28 | 0.033 | 1 | 30 | |
| 29 | 0.018 | 1.9 | 106 | >10 |
| 30 | 0.014 | 1.2 | 86 | |
| 31 | 0.022 | 1.0 | 45 | |
| 32 | 0.017 | 0.3 | 18 | |
| 33 | 0.013 | 0.3 | 23 | |
| 34 | 0.025 | 0.8 | 32 | |
| 35 | 0.020 | 0.6 | 30 | |
| 36 | 0.027 | 0.4 | 15 | >10 |
| 37 | 0.035 | >10 | >286 | >10 |
| 38 | 0.020 | >5 | >250 | >10 |
| 39 | 0.007 | 0.3 | 43 | >10 |
| 40 | 0.011 | 0.15 | 14 | |
| 41 | 0.092 | >10 | >109 | >10 |
| 42 | 0.026 | 0.4 | 16 | - |
| 43 | 0.018 | 0.4 | 22 | >10 |

| | | | | |
|---|---|---|---|---|
| * comparative example is described as example 128 in WO2016/016316 * comparative example is described as example 99 in WO2016/016316 * comparative example is described as example 107 in WO2016/016316 ^{a}Where multiple measurements have been made the value reported is the geometric mean | | | | |

As can be seen in the table of results the compounds of the present disclosure display in general selectivity for binding to the first bromodomain of BRD4 - BRD4(1) versus PDE3A inhibition. Comparing the compounds disclosed herein under Formula (I), the pharmaceutical profile has been much improved over compounds considered as relevant prior art and disclosed in in WO2016/016316. For example, when comparing the compounds disclosed herein with Example 128 of WO2016/016316, which according the present inventors is considered the best compound disclosed in prior art, it is recognized that the compounds disclosed herein under Formula (I) has substantially improved selectivity against PDE3A inhibition, a beneficial cardiovascular risk profile.

In some aspects compounds and pharmaceutically acceptable salts disclosed herein have a binding constant to the first bromodomain of BRD4 less than 500 nM, e.g. below 100 nM. In some aspect the compounds disclosed herein are considered selective, i.e. the selectivity for the first bromodomain of BRD4 compared to the second bromodomain of BRD4 as calculated by BRD4(2) Kd BRD4(1) Kd is at least 10 fold, e.g. at least 30 fold. In further aspects compounds disclosed herein have a binding constant to the first bromodomain of BRD4 which is less than 500 nM, e.g. less than 100 nM and the selectivity toward the first bromodomain of BRD4 is at least 10 fold, e.g. at least 30 fold.

In some aspect the compounds and pharmaceutically acceptable salts disclosed herein have a PDE3A IC50 of at least 6 µM, such as at least 10 µM (>10 µM).

In some aspect the compounds and pharmaceutically acceptable salts disclosed herein have a PDE3A IC50 of at least 6 µM, such as at least 10 µM (>10 µM), and a binding constant to the first bromodomain of BRD4 less than 500 nM, e.g. below 100 nM, and the selectivity toward the first bromodomain compared to the second bromodomain of BRD4 is at least 10 fold, e.g. at least 30 fold.

### In-vivo PD Studies

Compounds described herein can be evaluated for in-vivo efficacy after dosing (e.g., orally) in animal (e.g., murine) models for various diseases e.g., cancer or atopic dermatitis (AD) (e.g., Li et al, 2006, PNAS, 103(31), 11736-11741). For example, the MC903 murine model of atopic dermatitis can be used wherein mice (e.g., BALB/c mice) are treated with the vitamin D3 analog MC903 (calcipotriol), administered topically for a certain number of days (e.g., 10-12 days) to induce an allergic skin inflammation resembling AD. Compounds described herein are formulated appropriately and are dosed to see whether amelioration of ear inflammation can be observed. For each compound dosed, measurements are taken of ear thickness, terminal plasma exposure and pro-inflammatory markers (such as Th1/Th2 cytokine levels, chemokine CCL2 levels) in mouse ear homogenates.

## Claims

1. A compound according to formula (I) 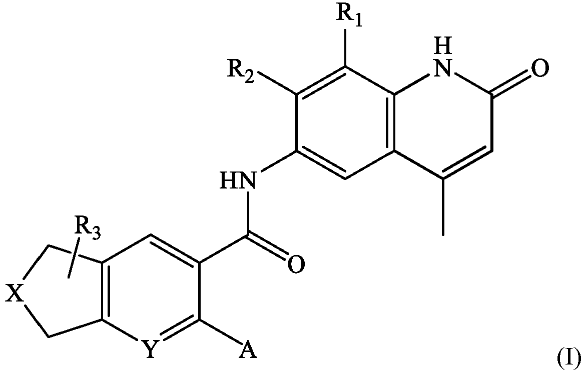
or pharmaceutically acceptable salts, polymorphs, stereoisomers, and tautomers thereof, wherein
X is selected from the group consisting of -C(R₄R₅)-, -S-, -O- and -N(R₆)-;
Y is -N- or -C(R₇)-, wherein R₇ is selected from the group consisting of hydrogen and C₁₋₄ alkyl;
A is selected from the group consisting of unsubstituted or substituted 5 or 6 membered alicyclic ring systems; unsubstituted or substituted 5-10 membered heteroalicyclic ring systems; and -NR₈R₉;
R₁ is selected from the group consisting of hydrogen, hydroxy, halogen, unsubstituted or substituted C₁₋₄ alkyl, unsubstituted or substituted C₁₋₄ alkoxy, and unsubstituted or substituted -O-R₁₀, wherein R₁₀ is selected from C₃₋₆ cycloalkyl, 4-6 membered heteroalicyclyl, and C₁₋₄ hydroxyalkyl;
R₂ is selected from the group consisting of hydrogen, hydroxy, halogen, unsubstituted or substituted C₁₋₄ alkyl, and unsubstituted or substituted C₁₋₄ alkoxy;
provided that R₁ and R₂ cannot be simultaneously selected from hydrogen; or
R₁, R₂ and the carbon atom to which they are attached are taken together to form a ring;
R₃, may be absent or present 1 or 2 times, and when present is C₁₋₄ alkyl;
R₄ and R₅ are independently selected from the group consisting of hydrogen, unsubstituted or substituted C₁₋₆ alkyl, unsubstituted or substituted C₁₋₆ alkenyl, unsubstituted or substituted C₁₋₆ alkynyl, unsubstituted or substituted C₁₋₆ alkoxy, -OH, and -CN;
R₆ is selected from the group consisting of hydrogen and unsubstituted or substituted C₁₋₆ alkyl, unsubstituted or substituted C₃₋₆ cycloalkyl, unsubstituted or substituted C₂₋₆ heteroalicyclyl; and
R₈, and R₉ are independently selected from the group consisting of hydrogen, unsubstituted or substituted C₁₋₄ alkyl, and unsubstituted or substituted C₃₋₄ cycloalkyl, provided that at least one of R₈ and R₉ is selected from the group consisting of unsubstituted or substituted C₁₋₄ alkyl, and unsubstituted or substituted C₃₋₄ cycloalkyl,
and wherein whenever a group is described as being "unsubstituted or substituted", if substituted, the substituent(s), which may be present one or more times, are independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, oxo, alkoxy, aryloxy, acyl, ester, O-carboxy, alkylthio, arylthio, cyano, halogen, carbonyl, thiocarbonyl, C-amido, N-amido, S-sulfonamido, N-sulfonamido, nitro, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof.

2. The compound, pharmaceutically acceptable salt, polymorph, stereoisomer, and tautomer according to claim 1, wherein
a) X is -C(R₄R₅)-, wherein R₄ and R₅ independently are hydrogen or C₁₋₄ alkyl, optionally wherein R₄ and R₅ both are hydrogen;
b) X is -N(R₆)-, wherein R₆ is selected from the group consisting of hydrogen, C₁₋₄ alkyl, C₁₋₄ haloalkyl, and a 4 or 5 membered heteroalicyclyl, optionally wherein R₆ is selected from the group consisting of hydrogen, methyl, ethyl, n-propyl, isopropyl, and tetrahydrofuranyl, further optionally wherein R₆ is hydrogen, methyl or ethyl;
or
c) X is -O-.

3. The compound, pharmaceutically acceptable salt, polymorph, stereoisomer, and tautomer according to claims 1 or 2, wherein Y is -N-.

4. The compound, pharmaceutically acceptable salt, polymorph, stereoisomer, and tautomer according to any one of claims 1-3, wherein
a) R₁ is selected from the group consisting of hydrogen, hydroxy, fluoro, methyl, ethyl, propyl, isopropyl, butyl, isobutyl, tert-butyl, methoxy, ethoxy, propoxy, butoxy, -O-azetidinyl, -O-CH₂CH₂OH, -O-CH₂CH₂NHCH₃,
optionally wherein R₁ is methyl, methoxy, or ethoxy;
b) R₁ is methoxy,
optionally wherein R₂ is selected from the group consisting of hydrogen, fluoro, methyl, ethyl, propyl, isopropyl, butyl, methoxy, ethoxy, propoxy, and butoxy,
further optionally wherein R₂ is hydrogen;
c) R₁, R₂ and the carbon atom to which they are attached are taken together to form a 5 or 6 membered ring system comprising one or more oxygen atom(s);
or
d) R₁, R₂ and the carbon atom to which they are attached are taken together to form a 2,3-dihydro-1,4-dioxine or a 2,3-dihydrofuran.

5. The compound, pharmaceutically acceptable salt, polymorph, stereoisomer, and tautomer according to any one of claims 1-4, wherein R₃ is absent or methyl.

6. The compound, pharmaceutically acceptable salt, polymorph, stereoisomer, and tautomer according to any one of claims 1-5, wherein A is selected from the group consisting of an unsubstituted or substituted morpholine, an unsubstituted or substituted morpholine derivative, and an unsubstituted or substituted a 5-10 membered heteroalicyclic ring system other than an unsubstituted or substituted morpholine or an unsubstituted or substituted morpholine derivative.

7. The compound, pharmaceutically acceptable salt, polymorph, stereoisomer, and tautomer according to any one of claims 1-6, wherein
a) A is selected from the group consisting of: 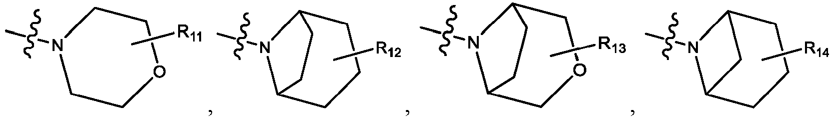 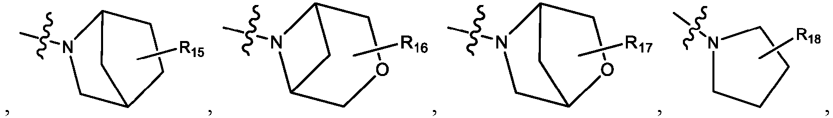 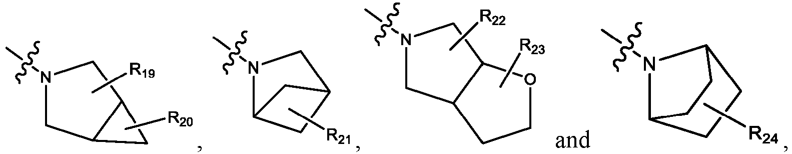 wherein R₁₁, R₁₂, R₁₃, R₁₄, R₁₅, R₁₆, R₁₇, R₁₈, R₁₉, R₂₀, R₂₁, R₂₂, R₂₃ and R₂₄ independently are absent or present 1, 2 or 3 times and when present selected from the group of halogen, hydroxy, C₁₋₄ alkyl, and C₁₋₄ alkoxy;
or
b) wherein the 5-10 membered heteroalicyclic ring system other than an unsubstituted or substituted morpholine or an unsubstituted or substituted morpholine derivative is selected from the group consisting of: 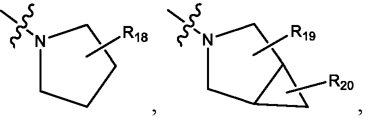 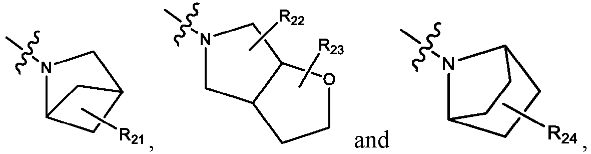
wherein R₁₈, R₁₉, R₂₀, R₂₁, R₂₂, R₂₃ and R₂₄ independently are absent or present 1 or 2 times and when present are independently selected from the group consisting of hydroxy, fluoro, methyl, ethyl, and propyl, or
wherein R₁₈, R₁₉, R₂₀, R₂₁, R₂₂, R₂₃ and R₂₄ are present 1 time and independently selected from the group consisting of fluoro, and methyl.

8. The compound, pharmaceutically acceptable salt, polymorph, stereoisomer, and tautomer according to claim 6, wherein the unsubstituted or substituted morpholine or unsubstituted or substituted morpholine derivative is selected from the group consisting of: 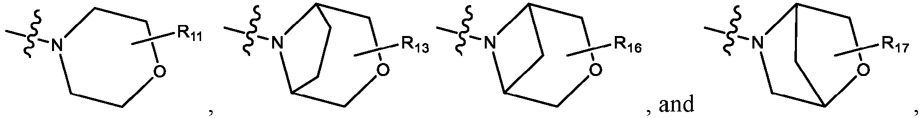
wherein R₁₁, R₁₃, R₁₆, and R₁₇ are absent, or present 1 or 2 times, and when present independently selected from the group consisting of hydroxy, fluoro, methyl, ethyl, and propyl, or
wherein R₁₁, R₁₃, R₁₆, and R₁₇ are absent.

9. The compound, pharmaceutically acceptable salt, polymorph, stereoisomer, and tautomer according to any one of claims 1-6, wherein the 5-10 membered heteroalicyclic ring system other than an unsubstituted or substituted morpholine or an unsubstituted or substituted morpholine derivative is selected from the group consisting of: 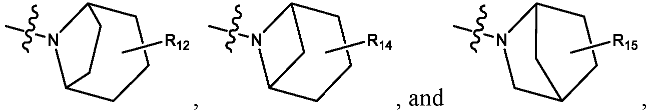
wherein R₁₂, R₁₄, and R₁₅ are absent, or present 1 or 2 times, and when present selected from the group consisting of hydroxy, fluoro, methyl, ethyl, and propyl,
or
wherein R₁₂, R₁₄, and R₁₅ are present 1 time and selected from the group consisting of hydroxy, fluoro, and methyl.

10. The compound, pharmaceutically acceptable salt, polymorph, stereoisomer, and tautomer according to claim 1 having the following Formula (III) 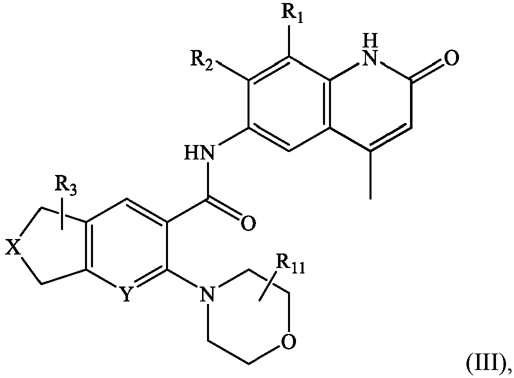 wherein R₁ is C₁₋₄ alkoxy; R₂ is hydrogen; R₃ is absent or present 1 time and when present selected from methyl; R₁₁ is absent; Y is -N- or -CH-; X is -O- or -N(R₆)-, wherein R₆ is selected from the group selected consisting of hydrogen, methyl, ethyl, n-propyl, isopropyl, and oxetanyl,
optionally
wherein the R₁ is methoxy; R₃ is absent; Y is -N- and X is -O-,
or
wherein the R₁ is methoxy; R₃ is absent; Y is -CH- and X is -O-.

11. The compound, pharmaceutically acceptable salt, polymorph, stereoisomer, and tautomer according to any one of claims 1-10, wherein A is selected from -NR₈R₉, wherein R₈ and R₉, independently are selected from the group consisting of hydrogen, methyl, ethyl, propyl, cyclopropyl, and cyclopropylmethyl, wherein at least one of R₈ and R₉ is selected from the group consisting of methyl, ethyl, propyl, cyclopropyl, and cyclopropylmethyl,
optionally wherein R₈ and R₉, independently are methyl, or cyclopropylmethyl.

12. A compound according to claim 1, wherein the compound is selected from the group consisting of
N-(8-methoxy-4-methyl-2-oxo-1H-quinolin-6-yl)-2-morpholino-5,7-dihydrofuro[3,4-b]pyridine-3-carboxamide,
N-(8-methoxy-4-methyl-2-oxo-1H-quinolin-6-yl)-2-morpholino-6,7-dihydro-5H-cyclopenta[b]pyridine-3-carboxamide,
N-(8-methoxy-4-methyl-2-oxo-1H-quinolin-6-yl)-5-methyl-2-morpholino-5,7-dihydrofuro[3,4-b]pyridine-3-carboxamide,
N-(8-methoxy-4-methyl-2-oxo-1H-quinolin-6-yl)-2-morpholino-6,7-dihydro-5H-pyrrolo[3,4-b]pyridine-3-carboxamide,
(5R)-N-(8-methoxy-4-methyl-2-oxo-1H-quinolin-6-yl)-5-methyl-2-morpholino-5,7-dihydrofuro[3,4-b]pyridine-3-carboxamide,
N-(8-methoxy-4-methyl-2-oxo-1H-quinolin-6-yl)-2-(3-oxa-8-azabicyclo[3.2.1]octan-8-yl)-5,7-dihydrofuro[3,4-b]pyridine-3-carboxamide,
N-(8-methoxy-4-methyl-2-oxo-1H-quinolin-6-yl)-2-[(1S,4S)-2-oxa-5-azabicyclo[2.2.1]heptan-5-yl]-5,7-dihydrofuro[3,4-b]pyridine-3-carboxamide,
N-(4,8-dimethyl-2-oxo-1H-quinolin-6-yl)-2-morpholino-5,7-dihydrofuro[3,4-b]pyridine-3-carboxamide,
N-(6-methyl-8-oxo-3,9-dihydro-2H-furo[3,2-h]quinolin-4-yl)-2-morpholino-5,7-dihydrofuro[3,4-b]pyridine-3-carboxamide,
N-[8-(2-hydroxyethoxy)-4-methyl-2-oxo-1H-quinolin-6-yl]-2-morpholino-5,7-dihydrofuro[3,4-b]pyridine-3-carboxamide,
N-[8-(azetidin-3-yloxy)-4-methyl-2-oxo-1H-quinolin-6-yl]-2-morpholino-5,7-dihydrofuro[3,4-b]pyridine-3-carboxamide,
N-(7-fluoro-8-methoxy-4-methyl-2-oxo-1H-quinolin-6-yl)-2-morpholino-5,7-dihydrofuro[3,4-b]pyridine-3-carboxamide,
N-(7-methyl-9-oxo-3,10-dihydro-2H-[1,4]dioxino[2,3-h]quinolin-5-yl)-2-morpholino-5,7-dihydrofuro[3,4-b]pyridine-3-carboxamide,
N-(4,7-dimethyl-2-oxo-1H-quinolin-6-yl)-2-morpholino-5,7-dihydrofuro[3,4-b]pyridine-3-carboxamide,
N-(8-methoxy-4-methyl-2-oxo-1H-quinolin-6-yl)-2-[(1R,4R)-2-oxa-5-azabicyclo[2.2.1]heptan-5-yl]-5,7-dihydrofuro[3,4-b]pyridine-3-carboxamide,
N-(8-ethoxy-4-methyl-2-oxo-1H-quinolin-6-yl)-2-morpholino-5,7-dihydrofuro[3,4-b]pyridine-3-carboxamide,
N-[4-methyl-8-[2-(methylamino)ethoxy]-2-oxo-1H-quinolin-6-yl]-2-morpholino-5,7-dihydrofuro[3,4-b]pyridine-3-carboxamide,
2-morpholino-N-(4,7,8-trimethyl-2-oxo-1H-quinolin-6-yl)-5,7-dihydrofuro[3,4-b]pyridine-3-carboxamide,
N-(7-methoxy-4-methyl-2-oxo-1H-quinolin-6-yl)-2-morpholino-5,7-dihydrofuro[3,4-b]pyridine-3-carboxamide,
N-(8-methoxy-4-methyl-2-oxo-1H-quinolin-6-yl)-6-methyl-2-morpholino-5,7-dihydropyrrolo[3,4-b]pyridine-3-carboxamide,
N-(8-methoxy-4-methyl-2-oxo-1H-quinolin-6-yl)-2-morpholino-6-(oxetan-3-yl)-5,7-dihydropyrrolo[3,4-b]pyridine-3-carboxamide,
6-ethyl-N-(8-methoxy-4-methyl-2-oxo-1H-quinolin-6-yl)-2-morpholino-5,7-dihydropyrrolo[3,4-b]pyridine-3-carboxamide,
6-isopropyl-N-(8-methoxy-4-methyl-2-oxo-1H-quinolin-6-yl)-2-morpholino-5,7-dihydropyrrolo[3,4-b]pyridine-3-carboxamide,
2-(dimethylamino)-N-(8-methoxy-4-methyl-2-oxo-1H-quinolin-6-yl)-5,7-dihydrofuro[3,4-b]pyridine-3-carboxamide,
2-[cyclopropylmethyl(methyl)amino]-N-(8-methoxy-4-methyl-2-oxo-1H-quinolin-6-yl)-5,7-dihydrofuro[3,4-b]pyridine-3-carboxamide,
2-(diethylamino)-N-(8-methoxy-4-methyl-2-oxo-1H-quinolin-6-yl)-5,7-dihydrofuro[3,4-b]pyridine-3-carboxamide,
2-[ethyl(isopropyl)amino]-N-(8-methoxy-4-methyl-2-oxo-1H-quinolin-6-yl)-5,7-dihydrofuro[3,4-b]pyridine-3-carboxamide,
2-(3,6-dihydro-2H-pyran-4-yl)-N-(8-methoxy-4-methyl-2-oxo-1H-quinolin-6-yl)-5,7-dihydrofuro[3,4-b]pyridine-3-carboxamide,
2-(cyclopenten-1-yl)-N-(8-methoxy-4-methyl-2-oxo-1H-quinolin-6-yl)-5,7-dihydrofuro[3,4-b]pyridine-3-carboxamide,
2-(3,3-difluoropyrrolidin-1-yl)-N-(8-methoxy-4-methyl-2-oxo-1H-quinolin-6-yl)-5,7-dihydrofuro[3,4-b]pyridine-3-carboxamide,
N-(8-methoxy-4-methyl-2-oxo-1H-quinolin-6-yl)-2-[(2R)-2-methylpyrrolidin-1-yl]-5,7-dihydrofuro[3,4-b]pyridine-3-carboxamide,
2-(2,3,3a,4,6,6a-hexahydrofuro[2,3-c]pyrrol-5-yl)-N-(8-methoxy-4-methyl-2-oxo-1H-quinolin-6-yl)-5,7-dihydrofuro[3,4-b]pyridine-3-carboxamide,
N-(8-methoxy-4-methyl-2-oxo-1H-quinolin-6-yl)-2-(1-methyl-2-azabicyclo[2.1.1]hexan-2-yl)-5,7-dihydrofuro[3,4-b]pyridine-3-carboxamide,
2-(1,3,3a,4,6,6a-hexahydrofuro[3,4-c]pyrrol-5-yl)-N-(8-methoxy-4-methyl-2-oxo-1H-quinolin-6-yl)-5,7-dihydrofuro[3,4-b]pyridine-3-carboxamide,
2-(8-azabicyclo[3.2.1]octan-8-yl)-N-(8-methoxy-4-methyl-2-oxo-1H-quinolin-6-yl)-5,7-dihydrofuro[3,4-b]pyridine-3-carboxamide,
N-(8-methoxy-4-methyl-2-oxo-1H-quinolin-6-yl)-2-[rac-(3aR,6aR)-2,3,3a,4,6,6a-hexahydrofuro[2,3-c]pyrrol-5-yl]-5,7-dihydrofuro[3,4-b]pyridine-3-carboxamide,
N-(8-methoxy-4-methyl-2-oxo-1H-quinolin-6-yl)-2-[(25)-2-methylpyrrolidin-1-yl]-5,7-dihydrofuro[3,4-b]pyridine-3-carboxamide,
2-(7-azabicyclo[2.2.1]heptan-7-yl)-N-(8-methoxy-4-methyl-2-oxo-1H-quinolin-6-yl)-5,7-dihydrofuro[3,4-b]pyridine-3-carboxamide,
2-[(3R)-3-fluoropyrrolidin-1-yl]-N-(8-methoxy-4-methyl-2-oxo-1H-quinolin-6-yl)-5,7-dihydrofuro[3,4-b]pyridine-3-carboxamide,
2-[(3S)-3-fluoropyrrolidin-1-yl]-N-(8-methoxy-4-methyl-2-oxo-1H-quinolin-6-yl)-5,7-dihydrofuro[3,4-b]pyridine-3-carboxamide,
N-(8-methoxy-4-methyl-2-oxo-1H-quinolin-6-yl)-2-[rac-(3R,4S)-3,4-difluoropyrrolidin-1-yl]-5,7-dihydrofuro[3,4-b]pyridine-3-carboxamide,
N-(8-methoxy-4-methyl-2-oxo-1H-quinolin-6-yl)-2-[rac-(3S,4S)-3,4-difluoropyrrolidin-1-yl]-5,7-dihydrofuro[3,4-b]pyridine-3-carboxamide, and
2-(6,6-difluoro-3-azabicyclo[3.1.0]hexan-3-yl)-N-(8-methoxy-4-methyl-2-oxo-1H-quinolin-6-yl)-5,7-dihydrofuro[3,4-b]pyridine-3-carboxamide.

13. A pharmaceutical composition comprising a compound according to any one of the preceding claims and at least one pharmaceutical acceptable excipient.

14. A compound according to any one of the claims 1-12 or a composition according to claim 13 for use in treating diseases or conditions selected from the group consisting of systemic or tissue inflammation, inflammatory responses to infection or products of infectious organisms or hypoxia, autoimmune and allergic processes, cellular activation and proliferation, cancer, metabolism, fibrosis and in the prevention and treatment of viral infections.

15. A compound according to any one of the claims 1-12 or a composition according to claim 13 for use in treating rheumatoid arthritis, osteoarthritis, gout, psoriasis, psoriatric arthritis, systemic lupus erythematosus, multiple sclerosis, inflammatory bowel disease, inflammatory bowel syndrome, Crohn's disease, ulcerative colitis, colitis, asthma, chronic obstructive airways disease, pneumonitis, myocarditis, pericarditis, myositis, eczema, dermatitis, atopic dermatitis, allergy, ankylosing spondylitis, lupus erythematosus, Hashimoto's disease, pancreatitis, autoimmune ocular disease, Sjögren's disease, optic neuritis, neuromyelitis optica, Myasthenia Gravis, Guillain Barre syndrome, Graves' disease, alopecia, vitiligo, bullous skin diseases, nephritis, vasculitis, atherosclerosis, Alzheimer's disease, depression, retinitis, uveitis, scleritis, hepatitis, primary biliary cirrhosis, sclerosing cholangitis, hypophysitis, thyroiditis, Addison's disease, type I diabetes and acute rejection of transplanted organs; or
for use in treating acute gout, giant cell arteritis, nephritis including lupus nephritis, vasculitis with organ involvement such as glomerulonephritis, vasculitis including giant cell arteritis, Polyarteritis nodosa, Behcet's disease, Wegener's granulomatosis, Kawasaki disease, Takayasu's Arteritis, vasculitis with organ involvement and acute rejection of transplanted organs; or
for use in treating sepsis, sepsis syndrome, septic shock, endotoxaemia, systemic inflammatory response syndrome (SIRS), multi-organ dysfunction syndrome, toxic shock syndrome, acute lung injury, ARDS (adult respiratory distress syndrome), acute renal failure, fulminant hepatitis, burns, acute pancreatitis, post-surgical syndromes, sarcoidosis, Herxheimer reactions, encephalitis, myelitis, meningitis, malaria and SIRS associated with viral infections such as influenza, herpes zoster, herpes simplex and coronavirus; or
for use in treating ischaemia-reperfusion injury, myocardial infarction, cerebrovascular ischaemia (stroke), acute coronary syndromes, renal reperfusion injury, organ transplantation, coronary artery bypass grafting, cardio-pulmonary bypass procedures, pulmonary, renal, hepatic, gastro-intestinal or peripheral limb embolism; or
for use in treating hypercholesterolemia, atherosclerosis and Alzheimer's disease; or
for use in treating idiopathic pulmonary fibrosis, lung fibrosis, intestinal fibrosis, liver fibrosis, non-alcoholic steatohepatitis, cirrhosis, renal fibrosis, post-operative stricture, myelofibrosis, keloid formation, skin fibrosis, hand fibrosis, systemic sclerosis, scleroderma and cardiac fibrosis; or
for use in treating or preventing viral infections such as herpes virus, human papilloma virus, human immunodeficiency virus (HIV), adenovirus and poxvirus; or
for use in treating colon carcinomas, midline carcinomas, sarcomas, mesenchymal, hepatic, renal and neurological tumours; acute lymphoblastic leukemia, acute myelogenous leukemia, adult T-cell leukemia/lymphoma, bladder cancer, blastoma, bone cancer, breast cancer, brain cancer, burkitts lymphoma, carcinoma, myeloid sarcoma, cervical cancer, chronic lymphocytic leukemia, chronic myelogenous leukemia, colorectal cancer, diffuse large B-cell lymphoma, endometrial cancer, esophageal cancer, follicular lymphoma, gastrointestinal cancer, glioblastoma multiforme, glioma, gallbladder cancer, gastric cancer, head and neck cancer, Hodgkin's lymphoma, non-Hodgkin's lymphoma, intestinal cancer, kidney cancer, laryngeal cancer, leukemia, lung cancer, lymphoma, liver cancer, small cell lung cancer, non-small cell lung cancer, melanoma, mesothelioma, multiple myeloma, ocular cancer, optic nerve tumor, oral cancer, ovarian cancer, pituitary tumor, primary central nervous system lymphoma, prostate cancer, pancreatic cancer, pharyngeal cancer, renal cell carcinoma, rectal cancer, sarcoma, skin cancer, spinal tumor, small intestine cancer, stomach cancer, T-cell lymphoma, testicular cancer, thyroid cancer, throat cancer, urogenital cancer, urothelial carcinoma, uterine cancer, vaginal cancer, or Wilms' tumor; or
for use in treating obesity, such as obesity associated with cancer treatment or obesity associated with diabetes and cardiac hypertrophy.

## Patentansprüche

1. Verbindung nach Formel (I) 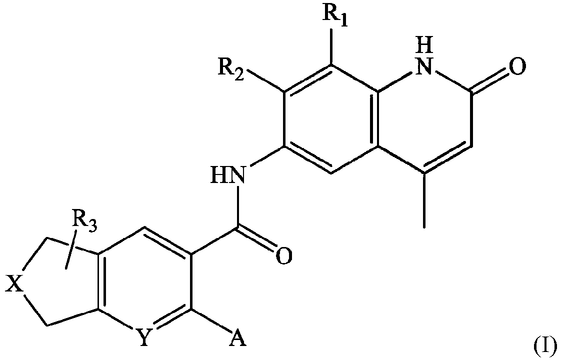
oder pharmazeutisch verträgliche Salze, Polymorphe, Stereoisomere und Tautomere davon, wobei
X aus der Gruppe ausgewählt ist, bestehend aus -C(R₄R₅)-, -S-, -O-und -N(R₆)-;
Y -N- oder -C(R₇)- ist, wobei R₇ aus der Gruppe ausgewählt ist, bestehend aus Wasserstoff und C₁₋₄-Alkyl;
A aus der Gruppe ausgewählt ist, bestehend aus unsubstituierten oder substituierten 5- oder 6-gliedrigen alicyclischen Ringsystemen; unsubstituierten oder substituierten 5- bis 10-gliedrigen heteroalicyclischen Ringsystemen; und -NR₈R₉;
R₁ aus der Gruppe ausgewählt ist, bestehend aus Wasserstoff, Hydroxy, Halogen, unsubstituiertem oder substituiertem C₁₋₄-Alkyl, unsubstituiertem oder substituiertem C₁₋₄-Alkoxy und unsubstituiertem oder substituiertem -O-R₁₀, wobei R₁₀ aus C₃₋₆-Cycloalkyl, 4-6-gliedrigem Heteroalicyclyl und C₁₋₄-Hydroxyalkyl ausgewählt ist;
R₂ aus der Gruppe ausgewählt ist, bestehend aus Wasserstoff, Hydroxy, Halogen, unsubstituiertem oder substituiertem C₁₋₄-Alkyl und unsubstituiertem oder substituiertem C₁₋₄-Alkoxy;
mit der Maßgabe, dass R₁ und R₂ nicht gleichzeitig aus Wasserstoff ausgewählt sein können; oder
R₁, R₂ und das Kohlenstoffatom, an das sie gebunden sind, zusammengenommen werden, um einen Ring auszubilden;
R₃ fehlen oder 1- oder 2-mal vorhanden sein kann und, wenn vorhanden, C₁₋₄-Alkyl ist;
R₄ und R₅ unabhängig voneinander aus der Gruppe ausgewählt sind, bestehend aus Wasserstoff, unsubstituiertem oder substituiertem C₁₋₆-Alkyl, unsubstituiertem oder substituiertem C₁₋₆-Alkenyl, unsubstituiertem oder substituiertem C₁₋₆-Alkinyl, unsubstituiertem oder substituiertem C₁₋₆-Alkoxy, -OH und -CN;
R₆ aus der Gruppe ausgewählt ist, bestehend aus Wasserstoff und unsubstituiertem oder substituiertem C₁₋₆-Alkyl, unsubstituiertem oder substituiertem C₃₋₆-Cycloalkyl, unsubstituiertem oder substituiertem C₂₋₆-Heteroalicyclyl; und
R₈ und R₉ unabhängig voneinander aus der Gruppe ausgewählt ist, bestehend aus Wasserstoff, unsubstituiertem oder substituiertem C₁₋₄-Alkyl und unsubstituiertem oder substituiertem C₃₋₄-Cycloalkyl, mit der Maßgabe, dass mindestens eines von R₈ und R₉ aus der Gruppe ausgewählt ist, bestehend aus unsubstituiertem oder substituiertem C₁₋₄-Alkyl und unsubstituiertem oder substituiertem C₃₋₄-Cycloalkyl,
und wobei, wann immer eine Gruppe als "unsubstituiert oder substituiert" beschrieben wird, falls substituiert, der/die Substituent(en), der/die einoder mehrfach vorhanden sein kann/können, unabhängig aus Alkyl, Alkenyl, Alkinyl, Cycloalkyl, Cycloalkenyl, Cycloalkinyl, Aryl, Heteroaryl, Heteroalicyclyl, Aralkyl, Heteroaralkyl, (Heteroalicyclyl)alkyl, Hydroxy, Oxo, Alkoxy, Aryloxy, Acyl, Ester, O-Carboxy, Alkylthio, Arylthio, Cyano, Halogen, Carbonyl, Thiocarbonyl, C-Amido, N-Amido, S-Sulfonamido, N-Sulfonamido, Nitro, Sulfenyl, Sulfinyl, Sulfonyl, Halogenalkyl, Halogenalkoxy, Trihalogenmethansulfonyl, Trihalogenmethansulfonamido und Amino, einschließlich mono- und disubstituierter Aminogruppen, und den geschützten Derivaten davon ausgewählt sind.

2. Verbindung, pharmazeutisch verträgliches Salz, Polymorph, Stereoisomer und Tautomer nach Anspruch 1, wobei
a) X -C(R₄R₅)- ist, wobei R₄ und R₅ unabhängig voneinander Wasserstoff oder C₁₋₄-Alkyl sind, wobei optional R₄ und R₅ beide Wasserstoff sind;
b) X -N(R₆)- ist, wobei R₆ aus der Gruppe ausgewählt ist, bestehend aus Wasserstoff, C₁₋₄-Alkyl, C₁₋₄-Halogenalkyl und einem 4- oder 5-gliedrigen Heteroalicyclyl, optional wobei R₆ aus der Gruppe ausgewählt ist, bestehend aus Wasserstoff, Methyl, Ethyl, n-Propyl, Isopropyl und Tetrahydrofuranyl, ferner optional wobei R₆ Wasserstoff, Methyl oder Ethyl ist;
oder
c) X -O- ist.

3. Verbindung, pharmazeutisch verträgliches Salz, Polymorph, Stereoisomer und Tautomer nach Anspruch 1 oder 2, wobei Y -N- ist.

4. Verbindung, pharmazeutisch verträgliches Salz, Polymorph, Stereoisomer und Tautomer nach einem der Ansprüche 1 bis 3, wobei
a) R₁ aus der Gruppe ausgewählt ist, bestehend aus Wasserstoff, Hydroxy, Fluor, Methyl, Ethyl, Propyl, Isopropyl, Butyl, Isobutyl, tert-Butyl, Methoxy, Ethoxy, Propoxy, Butoxy, -O-Azetidinyl, -O-CH₂CH₂OH -O-CH₂CH₂NHCH₃,
wobei R₁ optional Methyl, Methoxy oder Ethoxy ist;
b) R₁ Methoxy ist,
optional wobei R₂ aus der Gruppe ausgewählt ist, bestehend aus Wasserstoff, Fluor, Methyl, Ethyl, Propyl, Isopropyl, Butyl, Methoxy, Ethoxy, Propoxy und Butoxy,
ferner optional wobei R₂ Wasserstoff ist;
c) R₁, R₂ und das Kohlenstoffatom, an das sie gebunden sind, zusammengenommen werden, um ein 5- oder 6-gliedriges Ringsystem auszubilden, umfassend ein oder mehrere Sauerstoffatom(e);
oder
d) R₁, R₂ und das Kohlenstoffatom, an das sie gebunden sind, zusammengenommen werden, um ein 2,3-Dihydro-1,4-dioxin oder ein 2,3-Dihydrofuran auszubilden.

5. Verbindung, pharmazeutisch verträgliches Salz, Polymorph, Stereoisomer und Tautomer nach einem der Ansprüche 1 bis 4, wobei R₃ fehlt oder Methyl ist.

6. Verbindung, pharmazeutisch verträgliches Salz, Polymorph, Stereoisomer und Tautomer nach einem der Ansprüche 1 bis 5, wobei A aus der Gruppe ausgewählt ist, bestehend aus einem unsubstituierten oder substituierten Morpholin, einem unsubstituierten oder substituierten Morpholinderivat und einem unsubstituierten oder substituierten 5- bis 10-gliedrigen heteroalicyclischen Ringsystem außer einem unsubstituierten oder substituierten Morpholin oder einem unsubstituierten oder substituierten Morpholinderivat.

7. Verbindung, pharmazeutisch verträgliches Salz, Polymorph, Stereoisomer und Tautomer nach einem der Ansprüche 1 bis 6, wobei
a) A aus der Gruppe ausgewählt ist, bestehend aus: 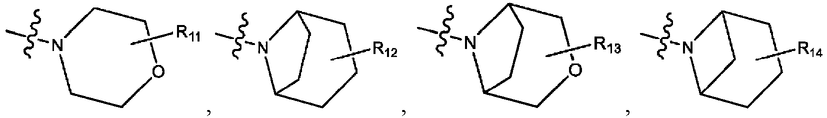 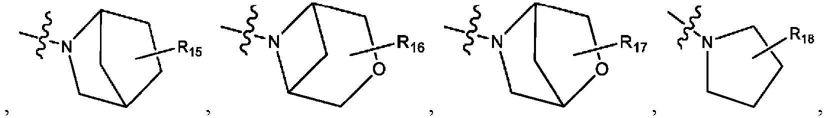 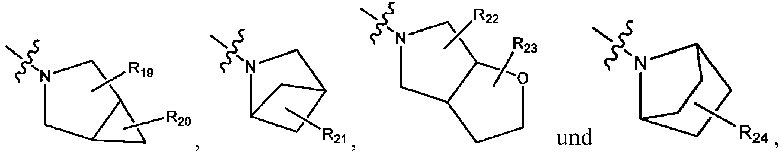 wobei R₁₁ R₁₂, R₁₃, R₁₄, R₁₅, R₁₆, R₁₇, R₁₈, R₁₉, R₂₀, R₂₁, R₂₂, R₂₃ und R₂₄ unabhängig voneinander fehlen oder 1-, 2- oder 3-mal vorhanden sind und, wenn vorhanden, aus der Gruppe von Halogen, Hydroxy, C₁₋₄-Alkyl und C₁₋₄-Alkoxy ausgewählt sind;
oder
b) wobei das 5- bis 10-gliedrige heteroalicyclische Ringsystem außer einem unsubstituierten oder substituierten Morpholin oder einem unsubstituierten oder substituierten Morpholinderivat aus der Gruppe ausgewählt ist, bestehend aus: 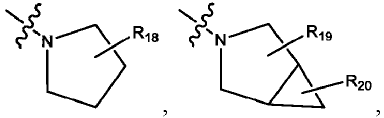 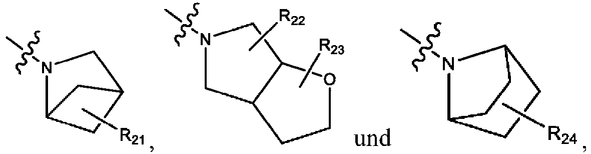
wobei R₁₈, R₁₉, R₂₀, R₂₁, R₂₂, R₂₃ und R₂₄ unabhängig voneinander fehlen oder 1- oder 2-mal vorhanden sind und, wenn vorhanden, unabhängig voneinander aus der Gruppe ausgewählt sind, bestehend aus Hydroxy, Fluor, Methyl, Ethyl und Propyl, oder
wobei R₁₈, R₁₉, R₂₀, R₂₁, R₂₂, R₂₃ und R₂₄ 1-mal vorhanden sind und unabhängig voneinander aus der Gruppe ausgewählt sind, bestehend aus Fluor und Methyl.

8. Verbindung, pharmazeutisch verträgliches Salz, Polymorph, Stereoisomer und Tautomer nach Anspruch 6, wobei das unsubstituierte oder substituierte Morpholin oder unsubstituierte oder substituierte Morpholinderivat aus der Gruppe ausgewählt ist, bestehend aus: 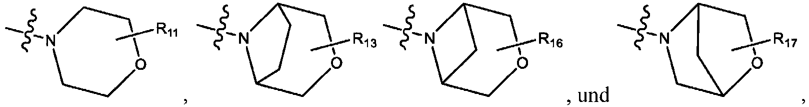
wobei R₁₁, R₁₃, R₁₆ und R₁₇ fehlen oder 1- oder 2-mal vorhanden sind und, wenn vorhanden, unabhängig voneinander aus der Gruppe ausgewählt sind, bestehend aus Hydroxy, Fluor, Methyl, Ethyl und Propyl, oder
wobei R₁₁, R₁₃, R₁₆ und R₁₇ fehlen.

9. Verbindung, pharmazeutisch verträgliches Salz, Polymorph, Stereoisomer und Tautomer nach einem der Ansprüche 1 bis 6, wobei das 5- bis 10-gliedrige heteroalicyclische Ringsystem, außer einem unsubstituierten oder substituierten Morpholin oder einem unsubstituierten oder substituierten Morpholinderivat, aus der Gruppe ausgewählt ist, bestehend aus: 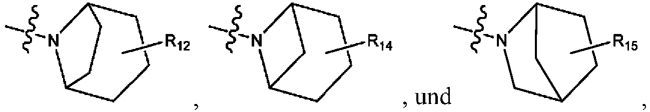
wobei R₁₂, R₁₄ und R₁₅ fehlen oder 1- oder 2-mal vorhanden sind und, wenn vorhanden, aus der Gruppe ausgewählt sind, bestehend aus Hydroxy, Fluor, Methyl, Ethyl und Propyl,
oder
wobei R₁₂, R₁₄ und R₁₅ 1-mal vorhanden sind und aus der Gruppe ausgewählt sind, bestehend aus Hydroxy, Fluor und Methyl.

10. Verbindung, pharmazeutisch verträgliches Salz, Polymorph, Stereoisomer und Tautomer nach Anspruch 1, aufweisend die folgende Formel (III) 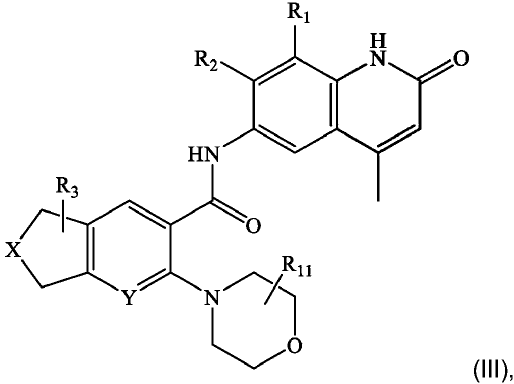 wobei R₁ C₁₋₄-Alkoxy ist; R₂ Wasserstoff ist; R₃ fehlt oder 1-mal vorhanden ist und, wenn vorhanden, aus Methyl ausgewählt ist; R₁₁ fehlt; Y -N- oder -CH- ist; X -O- oder -N(R₆)- ist, wobei R₆ aus der Gruppe ausgewählt ist, bestehend aus Wasserstoff, Methyl, Ethyl, n-Propyl, Isopropyl und Oxetanyl,
optional
wobei R₁ Methoxy ist; R₃ fehlt; Y -N- ist und X -O- ist,
oder
wobei R₁ Methoxy ist; R₃ fehlt; Y -CH- ist und X -O- ist.

11. Verbindung, pharmazeutisch verträgliches Salz, Polymorph, Stereoisomer und Tautomer nach einem der Ansprüche 1 bis 10, wobei A aus -NR₈R₉ ausgewählt ist, wobei R₈ und R₉ unabhängig voneinander aus der Gruppe ausgewählt sind, bestehend aus Wasserstoff, Methyl, Ethyl, Propyl, Cyclopropyl und Cyclopropylmethyl, wobei mindestens eines von R₈ und R₉ aus der Gruppe ausgewählt ist, bestehend aus Methyl, Ethyl, Propyl, Cyclopropyl und Cyclopropylmethyl,
optional wobei R₈ und R₉ unabhängig voneinander Methyl oder Cyclopropylmethyl sind.

12. Verbindung nach Anspruch 1, wobei die Verbindung aus der Gruppe ausgewählt ist, bestehend aus
N-(8-Methoxy-4-methyl-2-oxo-1H-chinolin-6-yl)-2-morpholino-5,7-dihydrofuro[3,4-b]pyridin-3-carboxamid,
N-(8-Methoxy-4-methyl-2-oxo-1H-chinolin-6-yl)-2-morpholino-6,7-dihydro-5H-cyclopenta[b]pyridin-3-carboxamid,
N-(8-Methoxy-4-methyl-2-oxo-1H-chinolin-6-yl)-5-methyl-2-morpholino-5,7-dihydrofuro[3,4-b]pyridin-3-carboxamid,
N-(8-Methoxy-4-methyl-2-oxo-1H-chinolin-6-yl)-2-morpholino-6,7-dihydro-5H-pyrrolo[3,4-b]pyridin-3-carboxamid,
(5R)-N-(8-Methoxy-4-methyl-2-oxo-1H-chinolin-6-yl)-5-methyl-2-morpholino-5,7-dihydrofuro[3,4-b]pyridin-3-carboxamid,
N-(8-Methoxy-4-methyl-2-oxo-1H-chinolin-6-yl)-2-(3-oxa-8-azabicyclo[3.2.1]octan-8-yl)-5,7-dihydrofuro[3,4-b]pyridin-3-carboxamid,
N-(8-Methoxy-4-methyl-2-oxo-1 H-chinolin-6-yl)-2-[(1S,4S)-2-oxa-5-azabicyclo[2.2.1]heptan-5-yl]-5,7-dihydrofuro[3,4-b]pyridin-3-carboxamid,
N-(4,8-Dimethyl-2-oxo-1H-chinolin-6-yl)-2-morpholino-5,7-dihydrofuro[3,4-b]pyridin-3-carboxamid,
N-(6-Methyl-8-oxo-3,9-dihydro-2H-furo[3,2-h]chinolin-4-yl)-2-morpholino-5,7-dihydrofuro[3,4-b]pyridin-3-carboxamid,
N-[8-(2-Hydroxyethoxy)-4-methyl-2-oxo-1H-chinolin-6-yl]-2-morpholino-5,7-dihydrofuro[3,4-b]pyridin-3-carboxamid,
N-[8-(Azetidin-3-yloxy)-4-methyl-2-oxo-1H-chinolin-6-yl]-2-morpholino-5,7-dihydrofuro[3,4-b]pyridin-3-carboxamid,
N-(7-Fluor-8-methoxy-4-methyl-2-oxo-1H-chinolin-6-yl)-2-morpholino-5,7-dihydrofuro[3,4-b]pyridin-3-carboxamid,
N-(7-Methyl-9-oxo-3,10-dihydro-2H-[1,4]dioxino[2,3-h]chinolin-5-yl)-2-morpholino-5,7-dihydrofuro[3,4-b]pyridin-3-carboxamid,
N-(4,7-Dimethyl-2-oxo-1H-chinolin-6-yl)-2-morpholino-5,7-dihydrofuro[3,4-b]pyridin-3-carboxamid,
N-(8-Methoxy-4-methyl-2-oxo-1H-chinolin-6-yl)-2-[(1R,4R)-2-oxa-5-azabicyclo[2.2.1]heptan-5-yl]-5,7-dihydrofuro[3,4-b]pyridin-3-carboxamid,
N-(8-Ethoxy-4-methyl-2-oxo-1H-chinolin-6-yl)-2-morpholino-5,7-dihydrofuro[3,4-b]pyridin-3-carboxamid,
N-[4-Methyl-8-[2-(methylamino)ethoxy]-2-oxo-1H-chinolin-6-yl]-2-morpholino-5,7-dihydrofuro[3,4-b]pyridin-3-carboxamid,
2-Morpholino-N-(4,7,8-trimethyl-2-oxo-1H-chinolin-6-yl)-5,7-dihydrofuro[3,4-b]pyridin-3-carboxamid,
N-(7-Methoxy-4-methyl-2-oxo-1H-chinolin-6-yl)-2-morpholino-5,7-dihydrofuro[3,4-b]pyridin-3-carboxamid,
N-(8-Methoxy-4-methyl-2-oxo-1H-chinolin-6-yl)-6-methyl-2-morpholino-5,7-dihydropyrrolo[3,4-b]pyridin-3-carboxamid,
N-(8-Methoxy-4-methyl-2-oxo-1H-chinolin-6-yl)-2-morpholino-6-(oxetan-3-yl)-5,7-dihydropyrrolo[3,4-b]pyridin-3-carboxamid,
6-Ethyl-N-(8-methoxy-4-methyl-2-oxo-1H-chinolin-6-yl)-2-morpholino-5,7-dihydropyrrolo[3,4-b]pyridin-3-carboxamid,
6-Isopropyl-N-(8-methoxy-4-methyl-2-oxo-1H-chinolin-6-yl)-2-morpholino-5,7-dihydropyrrolo[3,4-b]pyridin-3-carboxamid,
2-(Dimethylamino)-N-(8-methoxy-4-methyl-2-oxo-1H-chinolin-6-yl)-5,7-dihydrofuro[3,4-b]pyridin-3-carboxamid,
2-[Cyclopropylmethyl(methyl)amino]-N-(8-methoxy-4-methyl-2-oxo-1H-chinolin-6-yl)-5,7-dihydrofuro[3,4-b]pyridin-3-carboxamid,
2-(Diethylamino)-N-(8-methoxy-4-methyl-2-oxo-1H-chinolin-6-yl)-5,7-dihydrofuro[3,4-b]pyridin-3-carboxamid,
2-[Ethyl(isopropyl)amino]-N-(8-methoxy-4-methyl-2-oxo-1H-chinolin-6-yl)-5,7-dihydrofuro[3,4-b]pyridin-3-carboxamid,
2-(3,6-Dihydro-2H-pyran-4-yl)-N-(8-methoxy-4-methyl-2-oxo-1H-chinolin-6-yl)-5,7-dihydrofuro[3,4-b]pyridin-3-carboxamid,
2-(Cyclopenten-1-yl)-N-(8-methoxy-4-methyl-2-oxo-1H-chinolin-6-yl)-5,7-dihydrofuro[3,4-b]pyridin-3-carboxamid,
2-(3,3-Difluorpyrrolidin-1-yl)-N-(8-methoxy-4-methyl-2-oxo-1H-chinolin-6-yl)-5,7-dihydrofuro[3,4-b]pyridin-3-carboxamid,
N-(8-Methoxy-4-methyl-2-oxo-1H-chinolin-6-yl)-2-[(2R)-2-methylpyrrolidin-1-yl]-5,7-dihydrofuro[3,4-b]pyridin-3-carboxamid,
2-(2,3,3a,4,6,6a-Hexahydrofuro[2,3-c]pyrrol-5-yl)-N-(8-methoxy-4-methyl-2-oxo-1H-chinolin-6-yl)-5,7-dihydrofuro[3,4-b]pyridin-3-carboxamid,
N-(8-Methoxy-4-methyl-2-oxo-1H-chinolin-6-yl)-2-(1-methyl-2-azabicyclo[2.1.1]hexan-2-yl)-5,7-dihydrofuro[3,4-b]pyridin-3-carboxamid,
2-(1,3,3a,4,6,6a-Hexahydrofuro[3,4-c]pyrrol-5-yl)-N-(8-methoxy-4-methyl-2-oxo-1H-chinolin-6-yl)-5,7-dihydrofuro[3,4-b]pyridin-3-carboxamid,
2-(8-Azabicyclo[3.2.1]octan-8-yl)-N-(8-methoxy-4-methyl-2-oxo-1 H-chinolin-6-yl)-5,7-dihydrofuro[3,4-b]pyridin-3-carboxamid,
N-(8-Methoxy-4-methyl-2-oxo-1H-chinolin-6-yl)-2-[rac-(3aR,6aR)-2,3,3a,4,6,6a-hexahydrofuro[2,3-c]pyrrol-5-yl]-5,7-dihydrofuro[3,4-b]pyridin-3-carboxamid,
N-(8-Methoxy-4-methyl-2-oxo-1H-chinolin-6-yl)-2-[(2S)-2-methylpyrrolidin-1-yl]-5,7-dihydrofuro[3,4-b]pyridin-3-carboxamid,
2-(7-Azabicyclo[2.2.1]heptan-7-yl)-N-(8-methoxy-4-methyl-2-oxo-1H-chinolin-6-yl)-5,7-dihydrofuro[3,4-b]pyridin-3-carboxamid,
2-[(3R)-3-Fluorpyrrolidin-1-yl]-N-(8-methoxy-4-methyl-2-oxo-1H-chinolin-6-yl)-5,7-dihydrofuro[3,4-b]pyridin-3-carboxamid,
2-[(3S)-3-Fluorpyrrolidin-1-yl]-N-(8-methoxy-4-methyl-2-oxo-1H-chinolin-6-yl)-5,7-dihydrofuro[3,4-b]pyridin-3-carboxamid,
N-(8-Methoxy-4-methyl-2-oxo-1H-chinolin-6-yl)-2-[rac-(3R,4S)-3,4-difluorpyrrolidin-1-yl]-5,7-dihydrofuro[3,4-b]pyridin-3-carboxamid,
N-(8-Methoxy-4-methyl-2-oxo-1H-chinolin-6-yl)-2-[rac-(3S,4S)-3,4-difluorpyrrolidin-1-yl]-5,7-dihydrofuro[3,4-b]pyridin-3-carboxamid, und
2-(6,6-Difluor-3-azabicyclo[3.1.0]hexan-3-yl)-N-(8-methoxy-4-methyl-2-oxo-1H-chinolin-6-yl)-5,7-dihydrofuro[3,4-b]pyridin-3-carboxamid.

13. Pharmazeutische Zusammensetzung, umfassend eine Verbindung nach einem der vorstehenden Ansprüche und mindestens einen pharmazeutisch verträglichen Arzneistoffträger.

14. Verbindung nach einem der Ansprüche 1 bis 12 oder eine Zusammensetzung nach Anspruch 13 zur Verwendung bei einem Behandeln von Erkrankungen oder Krankheiten, die aus der Gruppe ausgewählt sind, bestehend aus systemischer oder Gewebeentzündung, Entzündungsreaktionen auf Infektionen oder Produkte von infektiösen Organismen oder Hypoxie, Autoimmun- und allergischen Prozessen, zellulärer Aktivierung und Proliferation, Krebs, Stoffwechsel, Fibrose und bei der Vorbeugung und Behandlung von Virusinfektionen.

15. Verbindung nach einem der Ansprüche 1 bis 12 oder eine Zusammensetzung nach Anspruch 13 zur Verwendung bei dem Behandeln von rheumatoider Arthritis, Osteoarthritis, Gicht, Psoriasis, Psoriasis-Arthritis, systemischem Lupus erythematodes, Multipler Sklerose, entzündlicher Darmerkrankung, entzündlichem Darmsyndrom, Morbus Crohn, Colitis ulcerosa, Colitis, Asthma, chronisch obstruktiver Atemwegserkrankung, Pneumonitis, Myokarditis, Perikarditis, Myositis, Ekzem, Dermatitis, atopischer Dermatitis, Allergie, Spondylitis ankylosans, Lupus erythematodes, Hashimoto-Krankheit, Pankreatitis, autoimmuner Augenerkrankung, Sjögren-Krankheit, Optikusneuritis, Neuromyelitis optica, Myasthenia gravis, Guillain-Barré-Syndrom, Basedow-Krankheit, Alopezie, Vitiligo, bullösen Hauterkrankungen, Nephritis, Vaskulitis, Atherosklerose, Alzheimer-Krankheit, Depression, Retinitis, Uveitis, Skleritis, Hepatitis, primärer biliärer Zirrhose, sklerosierender Cholangitis, Hypophysitis, Thyreoiditis, Addison-Krankheit, Typ-I-Diabetes und akuter Abstoßung transplantierter Organe; oder
zur Verwendung bei dem Behandeln von akuter Gicht, Riesenzellarteriitis, Nephritis einschließlich Lupus-Nephritis, Vaskulitis mit Organbeteiligung wie Glomerulonephritis, Vaskulitis einschließlich Riesenzellarteriitis, Polyarteriitis nodosa, Behçet-Krankheit, Wegener-Granulomatose, Kawasaki-Syndrom, Takayasu-Arteriitis, Vaskulitis mit Organbeteiligung und akuter Abstoßung transplantierter Organe; oder
zur Verwendung bei dem Behandeln von Sepsis, Sepsis-Syndrom, septischem Schock, Endotoxämie, systemischem inflammatorischem Response-Syndrom (SIRS), Multiorgandysfunktionssyndrom, toxischem Schocksyndrom, akuter Lungenschädigung, ARDS (Atemnotsyndrom des Erwachsenen), akutem Nierenversagen, fulminanter Hepatitis, Verbrennungen, akuter Pankreatitis, postoperativen Syndromen, Sarkoidose, Herxheimer-Reaktionen, Enzephalitis, Myelitis, Meningitis, Malaria und SIRS im Zusammenhang mit Virusinfektionen wie Influenza, Herpes Zoster, Herpes Simplex und Coronavirus; oder
zur Verwendung bei dem Behandeln von Ischämie-Reperfusionsschaden, Myokardinfarkt, zerebrovaskulärer Ischämie (Schlaganfall), akuten Koronarsyndromen, renalem Reperfusionsschaden, Organtransplantation, Koronararterien-Bypass-Transplantation, kardiopulmonalen Bypass-Prozeduren, pulmonaler, renaler, hepatischer, gastrointestinaler oder peripherer Gliedmaßenembolie; oder
zur Verwendung bei dem Behandeln von Hypercholesterinämie, Atherosklerose und Alzheimer-Krankheit; oder
zur Verwendung bei dem Behandeln von idiopathischer Lungenfibrose, Lungenfibrose, intestinaler Fibrose, Leberfibrose, nichtalkoholischer Steatohepatitis, Zirrhose, Nierenfibrose, postoperativer Striktur, Myelofibrose, Keloidbildung, Hautfibrose, Handfibrose, systemischer Sklerose, Sklerodermie und Herzfibrose; oder
zur Verwendung bei dem Behandeln oder Vorbeugen von viralen Infektionen wie Herpesvirus, humanem Papillomavirus, humanem Immundefizienzvirus (HIV), Adenovirus und Pockenvirus; oder
zur Verwendung bei dem Behandeln von Kolonkarzinomen, Mittellinienkarzinomen, Sarkomen, mesenchymalen, hepatischen, renalen und neurologischen Tumoren; akuter lymphoblastischer Leukämie, akuter myeloischer Leukämie, adulter T-Zell-Leukämie/Lymphom, Blasenkrebs, Blastom, Knochenkrebs, Brustkrebs, Gehirnkrebs, Burkitt-Lymphom, Karzinom, myeloischem Sarkom, Gebärmutterhalskrebs, chronischer lymphatischer Leukämie, chronischer myeloischer Leukämie, Kolorektalkrebs, diffus großzelligem B-Zell-Lymphom, Endometriumkrebs, Ösophaguskrebs, follikulärem Lymphom, gastrointestinalem Krebs, Glioblastoma multiforme, Gliom, Gallenblasenkrebs, Magencarcinom, Kopf-Hals-Krebs, Hodgkin-Lymphom, Non-Hodgkin-Lymphom, Darmkrebs, Nierenkrebs, Larynxkrebs, Leukämie, Lungenkrebs, Lymphom, Leberkrebs, kleinzelligem Lungenkrebs, nicht-kleinzelligem Lungenkrebs, Melanom, Mesotheliom, multiplem Myelom, Augenkrebs, Sehnervtumor, Mundhöhlenkrebs, Ovarialkrebs, Hypophysentumor, primärem zentralem Nervensystemlymphom, Prostatakrebs, Pankreaskrebs, Pharynxkrebs, Nierenzellkarzinom, Rektumkrebs, Sarkom, Hautkrebs, Spinaltumor, Dünndarmkrebs, Magenkrebs, T-Zell-Lymphom, Hodenkrebs, Schilddrüsenkrebs, Kehlkopfkrebs, Urogenitalkrebs, Urothelkarzinom, Gebärmutterkrebs, Vaginalkrebs oder Wilms-Tumor; oder
zur Verwendung bei dem Behandeln von Adipositas, wie Adipositas im Zusammenhang mit einer Krebsbehandlung oder Adipositas im Zusammenhang mit Diabetes und Herzhypertrophie.

## Revendications

1. Composé selon la formule (I) 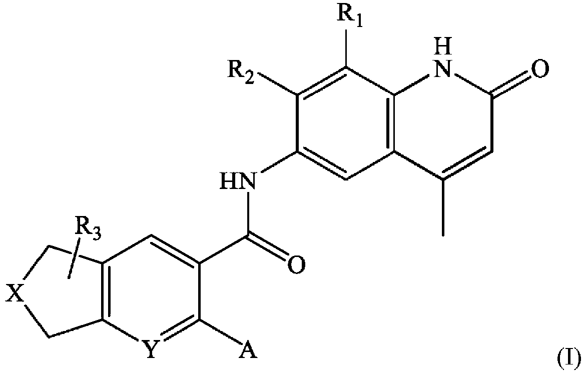
ou sels, polymorphes, stéréo-isomères, et tautomères pharmaceutiquement acceptables de celui-ci, où
X est choisi dans le groupe constitué de -C(R₄R₅)-, -S-, -O- et -N(R₆)- ;
Y est -N- ou -C(R₇)-, où R₇ est choisi dans le groupe constitué d'hydrogène et alkyle en C₁₋₄ ;
A est choisi dans le groupe constitué de systèmes de cycles alicycliques à 5 ou 6 chaînons non substitués ou substitués ; systèmes de cycles hétéroalicycliques à 5 à 10 chaînons non substitués ou substitués ; et -NR₈R₉ ;
R₁ est choisi dans le groupe constitué d'hydrogène, hydroxy, halogène, alkyle en C₁₋₄ non substitué ou substitué, alcoxy en C₁₋₄ non substitué ou substitué, et -O-R₁₀ non substitué ou substitué, où R₁₀ est choisi parmi cycloalkyle en C₃₋₆, hétéroalicyclyle à 4 6 chaînons, et hydroxyalkyle en C₁₋₄ ;
R₂ est choisi dans le groupe constitué d'hydrogène, hydroxy, halogène, alkyle en C₁₋₄ non substitué ou substitué, et alcoxy en C₁₋₄ non substitué ou substitué ;
à condition que R₁ et R₂ ne puissent pas être choisis simultanément parmi hydrogène ; ou
R₁, R₂ et l'atome de carbone auquel ils sont attachés sont pris conjointement pour former un cycle ;
R₃ peut être absent ou présent 1 ou 2 fois et, lorsqu'il est présent, est alkyle en C₁₋₄ ;
R₄ et R₅ sont indépendamment choisis dans le groupe constitué d'hydrogène, alkyle en C₁₋₆ non substitué ou substitué, alcényle en C₁₋₆ non substitué ou substitué, alcynyle en C₁₋₆ non substitué ou substitué, alcoxy en C₁₋₆ non substitué ou substitué, -OH, et -CN ;
R₆ est choisi dans le groupe constitué d'hydrogène et alkyle en C₁₋₆ non substitué ou substitué, cycloalkyle en C₃₋₆ non substitué ou substitué, hétéroalicyclyle en C₂₋₆ non substitué ou substitué ; et
R₈ et R₉ sont choisis indépendamment dans le groupe constitué d'hydrogène, alkyle en C₁₋₄ non substitué ou substitué, et cycloalkyle en C₃₋₄ non substitué ou substitué, à condition qu'au moins l'un de R₈ et R₉ soit choisi dans le groupe constitué d'alkyle en C₁₋₄ non substitué ou substitué, et cycloalkyle en C₃₋₄ non substitué ou substitué,
et où, lorsqu'un groupe est décrit comme étant « non substitué ou substitué », s'il est substitué, le ou les substituants, qui peuvent être présents une ou plusieurs fois, sont indépendamment choisis parmi alkyle, alcényle, alcynyle, cycloalkyle, cycloalcényle, cycloalcynyle, aryle, hétéroaryle, hétéroalicyclyle, aralkyle, hétéroaralkyle, (hétéroalicyclyl)alkyle, hydroxy, oxo, alcoxy, aryloxy, acyle, ester, O-carboxy, alkylthio, arylthio, cyano, halogène, carbonyle, thiocarbonyle, C-amido, N-amido, S-sulfonamido, N-sulfonamido, nitro, sulfényle, sulfinyle, sulfonyle, haloalkyle, haloalcoxy, trihalométhanesulfonyle, trihalométhanesulfonamido, et amino, y compris groupes amino mono- et di-substitués, et les dérivés protégés de ceux-ci.

2. Composé, sel, polymorphe, stéréo-isomère, et tautomère pharmaceutiquement acceptables de celui-ci, selon la revendication 1, où
a) X est -C(R₄R₅)-, où R₄ et R₅ sont indépendamment hydrogène ou alkyle en C₁₋₄, où facultativement R₄ et R₅ sont tous deux hydrogène ;
b) X est -N(R₆)-, où R₆ est choisi dans le groupe constitué d'hydrogène, alkyle en C₁₋₄, haloalkyle en C₁₋₄, et un hétéroalicyclyle à 4 ou 5 chaînons, facultativement où R₆ est choisi dans le groupe constitué d'hydrogène, méthyle, éthyle, n-propyle, isopropyle, et tétrahydrofuranyle, en outre facultativement où R₆ est hydrogène, méthyle ou éthyle ;
ou
c) X est -O-.

3. Composé, sel, polymorphe, stéréo-isomère, et tautomère pharmaceutiquement acceptables de celui-ci, selon les revendications 1 ou 2, où Y est -N-.

4. Composé, sel, polymorphe, stéréo-isomère, et tautomère pharmaceutiquement acceptables de celui-ci, selon l'une quelconque des revendications 1 à 3, où
a) R₁ est choisi dans le groupe constitué d'hydrogène, hydroxy, fluoro, méthyle, éthyle, propyle, isopropyle, butyle, isobutyle, tert-butyle, méthoxy, éthoxy, propoxy, butoxy, -O-azétidinyle, -O-CH₂CH₂OH, -O-CH₂CH₂NHCH₃,
facultativement où R₁ est méthyle, méthoxy, ou éthoxy ;
b) R₁ est méthoxy,
facultativement où R₂ est choisi dans le groupe constitué d'hydrogène, fluoro, méthyle, éthyle, propyle, isopropyle, butyle, méthoxy, éthoxy, propoxy, et butoxy,
facultativement en outre où R₂ est hydrogène ;
c) R₁, R₂ et l'atome de carbone auquel ils sont attachés sont pris conjointement pour former un système de cycle à 5 ou 6 chaînons comprenant un ou plusieurs atomes d'oxygène ;
ou
d) R₁, R₂ et l'atome de carbone auquel ils sont attachés sont pris conjointement pour former une 2,3-dihydro-1,4-dioxine ou un 2,3-dihydrofurane.

5. Composé, sel, polymorphe, stéréo-isomère, et tautomère pharmaceutiquement acceptables de celui-ci, selon l'une quelconque des revendications 1 à 4, où R₃ est absent ou méthyle.

6. Composé, sel, polymorphe, stéréo-isomère, et tautomère pharmaceutiquement acceptables de celui-ci, selon l'une quelconque des revendications 1 à 5, où A est choisi dans le groupe constitué d'une morpholine non substituée ou substituée, d'un dérivé de morpholine non substitué ou substitué, et d'un système de cycle hétéroalicyclique à 5 à 10 chaînons non substitué ou substitué autre qu'une morpholine non substituée ou substituée ou qu'un dérivé de morpholine non substitué ou substitué.

7. Composé, sel, polymorphe, stéréo-isomère, et tautomère pharmaceutiquement acceptables de celui-ci, selon l'une quelconque des revendications 1 à 6, où
a) A est choisi dans le groupe constitué de : 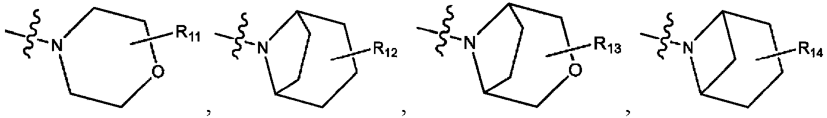 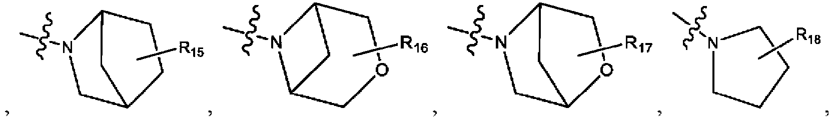 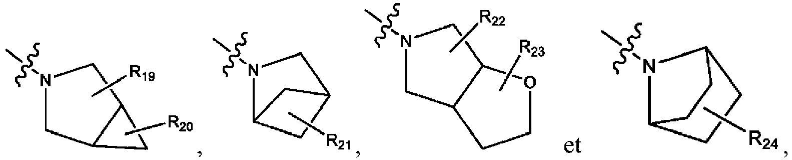 où R₁₁ R₁₂, R₁₃, R₁₄, R₁₅, R₁₆, R₁₇, R₁₈, R₁₉, R₂₀, R₂₁, R₂₂, R₂₃ et R₂₄ sont indépendamment absents ou présents 1, 2 ou 3 fois et, lorsqu'ils sont présents, sont choisis dans le groupe d'halogène, hydroxy, alkyle en C₁₋₄, et alcoxy en C₁₋₄ ; ou
b) où le système de cycle hétéroalicyclique à 5 à 10 chaînons autre qu'une morpholine non substituée ou substituée ou qu'un dérivé de morpholine non substitué ou substitué est choisi dans le groupe constitué de : 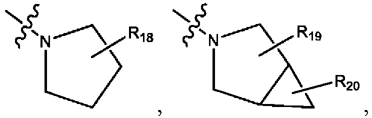 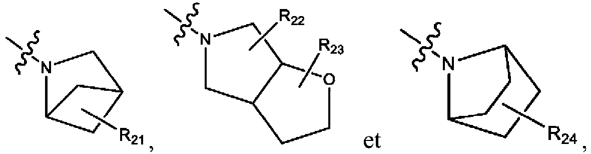
où R₁₈, R₁₉, R₂₀, R₂₁, R₂₂, R₂₃ et R₂₄ sont indépendamment absents ou présents 1 ou 2 fois et, lorsqu'ils sont présents, sont indépendamment choisis dans le groupe constitué d'hydroxy, fluoro, méthyle, éthyle, et propyle, ou
où R₁₈, R₁₉, R₂₀, R₂₁, R₂₂, R₂₃ et R₂₄ sont présents 1 fois et choisis indépendamment dans le groupe constitué de fluoro, et méthyle.

8. Composé, sel, polymorphe, stéréo-isomère, et tautomère pharmaceutiquement acceptables de celui-ci, selon la revendication 6, où la morpholine non substituée ou substituée ou le dérivé de morpholine non substitué ou substitué est choisi dans le groupe constitué de : 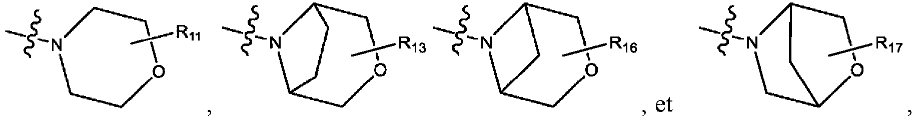
où R₁₁, R₁₃, R₁₆, et R₁₇ sont absents, ou présents 1 ou 2 fois et, lorsqu'ils sont présents, sont indépendamment choisis dans le groupe constitué d'hydroxy, fluoro, méthyle, éthyle, et propyle, ou
où R₁₁, R₁₃, R₁₆, et R₁₇ sont absents.

9. Composé, sel, polymorphe, stéréo-isomère, et tautomère pharmaceutiquement acceptables de celui-ci, selon l'une quelconque des revendications 1 à 6, où le système de cycle hétéroalicyclique à 5 à 10 chaînons autre qu'une morpholine non substituée ou substituée ou qu'un dérivé de morpholine non substitué ou substitué, est choisi dans le groupe constitué de : 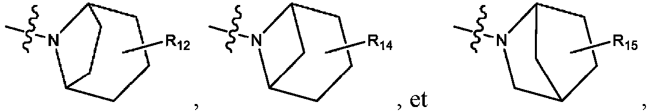
où R₁₂, R₁₄, et R₁₅ sont absents, ou présents 1 ou 2 fois et, lorsqu'ils sont présents, sont choisis dans le groupe constitué d'hydroxy, fluoro, méthyle, éthyle, et propyle,
ou
où R₁₂, R₁₄, et R₁₅ sont présents 1 fois et choisis dans le groupe constitué d'hydroxy, fluoro, et méthyle.

10. Composé, sel, polymorphe, stéréo-isomère, et tautomère pharmaceutiquement acceptables de celui-ci, selon la revendication 1, ayant la Formule (III) suivante 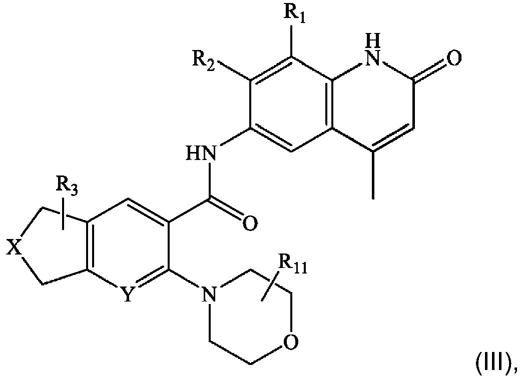 où R₁ est alcoxy en C₁₋₄ ; R₂ est hydrogène ; R₃ est absent ou présent 1 fois et, lorsqu'il est présent, est choisi parmi méthyle ; R₁₁ est absent ; Y est -N- ou -CH- ; X est -O- ou -N(R₆)-, où R₆ est choisi dans le groupe constitué d'hydrogène, méthyle, éthyle, n-propyle, isopropyle, et oxétanyle,
facultativement
où le R₁ est méthoxy ; R₃ est absent ; Y est -N- et X est -O-,
ou
où le R₁ est méthoxy ; R₃ est absent ; Y est -CH- et X est -O-.

11. Composé, sel, polymorphe, stéréo-isomère, et tautomère pharmaceutiquement acceptables de celui-ci, selon l'une quelconque des revendications 1 à 10, où A est choisi parmi -NR₈R₉, où R₈ et R₉ sont indépendamment choisis dans le groupe constitué d'hydrogène, méthyle, éthyle, propyle, cyclopropyle, et cyclopropylméthyle, où au moins l'un parmi R₈ et R₉ est choisi dans le groupe constitué de méthyle, éthyle, propyle, cyclopropyle, et cyclopropylméthyle,
facultativement où R₈ et R₉ sont indépendamment méthyle, ou cyclopropylméthyle.

12. Composé selon la revendication 1, où le composé est choisi dans le groupe constitué de
N-(8-méthoxy-4-méthyl-2-oxo-1H-quinolin-6-yl)-2-morpholino-5,7-dihydrofuro[3,4-b]pyridine-3-carboxamide,
N-(8-méthoxy-4-méthyl-2-oxo-1H-quinolin-6-yl)-2-morpholino-6,7-dihydro-5H-cyclopenta[b]pyridine-3-carboxamide,
N-(8-méthoxy-4-méthyl-2-oxo-1H-quinolin-6-yl)-5-méthyl-2-morpholino-5,7-dihydrofuro[3,4-b]pyridine-3-carboxamide,
N-(8-méthoxy-4-méthyl-2-oxo-1H-quinolin-6-yl)-2-morpholino-6,7-dihydro-5H-pyrrolo[3,4-b]pyridine-3-carboxamide,
(5R)-N-(8-méthoxy-4-méthyl-2-oxo-1H-quinolin-6-yl)-5-méthyl-2-morpholino-5,7-dihydrofuro[3,4-b]pyridine-3-carboxamide,
N-(8-méthoxy-4-méthyl-2-oxo-1H-quinolin-6-yl)-2-(3-oxa-8-azabicyclo[3.2.1]octan-8-yl)-5,7-dihydrofuro[3,4-b]pyridine-3-carboxamide,
N-(8-méthoxy-4-méthyl-2-oxo-1H-quinolin-6-yl)-2-[(1S,4S)-2-oxa-5-azabicyclo[2.2.1]heptan-5-yl]-5,7-dihydrofuro[3,4-b]pyridine-3-carboxamide,
N-(4,8-diméthyl-2-oxo-1H-quinolin-6-yl)-2-morpholino-5,7-dihydrofuro[3,4-b]pyridine-3-carboxamide,
N-(6-méthyl-8-oxo-3,9-dihydro-2H-furo[3,2-h]quinolin-4-yl)-2-morpholino-5,7-dihydrofuro[3,4-b]pyridine-3-carboxamide,
N-[8-(2-hydroxyéthoxy)-4-méthyl-2-oxo-1H-quinolin-6-yl]-2-morpholino-5,7-dihydrofuro[3,4-b]pyridine-3-carboxamide,
N-[8-(azétidin-3-yloxy)-4-méthyl-2-oxo-1H-quinolin-6-yl]-2-morpholino-5,7-dihydrofuro[3,4-b]pyridine-3-carboxamide,
N-(7-fluoro-8-méthoxy-4-méthyl-2-oxo-1H-quinolin-6-yl)-2-morpholino-5,7-dihydrofuro[3,4-b]pyridine-3-carboxamide,
N-(7-méthyl-9-oxo-3,10-dihydro-2H-[1,4]dioxino[2,3-h]quinolin-5-yl)-2-morpholino-5,7-dihydrofuro[3,4-b]pyridine-3-carboxamide,
N-(4,7-diméthyl-2-oxo-1H-quinolin-6-yl)-2-morpholino-5,7-dihydrofuro[3,4-b]pyridine-3-carboxamide,
N-(8-méthoxy-4-méthyl-2-oxo-1H-quinolin-6-yl)-2-[(1R,4R)-2-oxa-5-azabicyclo[2.2.1]heptan-5-yl]-5,7-dihydrofuro[3,4-b]pyridine-3-carboxamide,
N-(8-éthoxy-4-méthyl-2-oxo-1H-quinolin-6-yl)-2-morpholino-5,7-dihydrofuro[3,4-b]pyridine-3-carboxamide,
N-[4-méthyl-8-[2-(méthylamino)éthoxy]-2-oxo-1H-quinolin-6-yl]-2-morpholino-5,7-dihydrofuro[3,4-b]pyridine-3-carboxamide,
2-morpholino-N-(4,7,8-triméthyl-2-oxo-1H-quinolin-6-yl)-5,7-dihydrofuro[3,4-b]pyridine-3-carboxamide,
N-(7-méthoxy-4-méthyl-2-oxo-1H-quinolin-6-yl)-2-morpholino-5,7-dihydrofuro[3,4-b]pyridine-3-carboxamide,
N-(8-méthoxy-4-méthyl-2-oxo-1H-quinolin-6-yl)-6-méthyl-2-morpholino-5,7-dihydropyrrolo[3,4-b]pyridine-3-carboxamide,
N-(8-méthoxy-4-méthyl-2-oxo-1H-quinolin-6-yl)-2-morpholino-6-(oxétan-3-yl)-5,7-dihydropyrrolo[3,4-b]pyridine-3-carboxamide,
6-éthyl-N-(8-méthoxy-4-méthyl-2-oxo-1H-quinolin-6-yl)-2-morpholino-5,7-dihydropyrrolo[3,4-b]pyridine-3-carboxamide,
6-isopropyl-N-(8-méthoxy-4-méthyl-2-oxo-1H-quinolin-6-yl)-2-morpholino-5,7-dihydropyrrolo[3,4-b]pyridine-3-carboxamide,
2-(diméthylamino)-N-(8-méthoxy-4-méthyl-2-oxo-1H-quinolin-6-yl)-5,7-dihydrofuro[3,4-b]pyridine-3-carboxamide,
2-[cyclopropylméthyl(méthyl)amino]-N-(8-méthoxy-4-méthyl-2-oxo-1H-quinolin-6-yl)-5,7-dihydrofuro[3,4-b]pyridine-3-carboxamide,
2-(diéthylamino)-N-(8-méthoxy-4-méthyl-2-oxo-1H-quinolin-6-yl)-5,7-dihydrofuro[3,4-b]pyridine-3-carboxamide,
2-[éthyl(isopropyl)amino]-N-(8-méthoxy-4-méthyl-2-oxo-1H-quinolin-6-yl)-5,7-dihydrofuro[3,4-b]pyridine-3-carboxamide,
2-(3,6-dihydro-2H-pyran-4-yl)-N-(8-méthoxy-4-méthyl-2-oxo-1H-quinolin-6-yl)-5,7-dihydrofuro[3,4-b]pyridine-3-carboxamide,
2-(cyclopentén-1-yl)-N-(8-méthoxy-4-méthyl-2-oxo-1H-quinolin-6-yl)-5,7-dihydrofuro[3,4-b]pyridine-3-carboxamide,
2-(3,3-difluoropyrrolidin-1-yl)-N-(8-méthoxy-4-méthyl-2-oxo-1H-quinolin-6-yl)-5,7-dihydrofuro[3,4-b]pyridine-3-carboxamide,
N-(8-méthoxy-4-méthyl-2-oxo-1H-quinolin-6-yl)-2-[(2R)-2-méthylpyrrolidin-1-yl]-5,7-dihydrofuro[3,4-b]pyridine-3-carboxamide,
2-(2,3,3a,4,6,6a-hexahydrofuro[2,3-c]pyrrol-5-yl)-N-(8-méthoxy-4-méthyl-2-oxo-1H-quinolin-6-yl)-5,7-dihydrofuro[3,4-b]pyridine-3-carboxamide,
N-(8-méthoxy-4-méthyl-2-oxo-1H-quinolin-6-yl)-2-(I-méthyl-2-azabicyclo[2.1.I]hexan-2-yl)-5,7-dihydrofuro[3,4-b]pyridine-3-carboxamide,
2-(1,3,3a,4,6,6a-hexahydrofuro[3,4-c]pyrrol-5-yl)-N-(8-méthoxy-4-méthyl-2-oxo-1H-quinolin-6-yl)-5,7-dihydrofuro[3,4-b]pyridine-3-carboxamide,
2-(8-azabicyclo[3.2.1]octan-8-yl)-N-(8-methoxy-4-methyl-2-oxo-1 H-quinolin-6-yl)-5,7-dihydrofuro[3,4-b]pyridine-3-carboxamide,
N-(8-méthoxy-4-méthyl-2-oxo-1H-quinolin-6-yl)-2-[rac-(3aR,6aR)-2,3,3a,4,6,6a-hexahydrofuro[2,3-c]pyrrol-5-yl]-5,7-dihydrofuro[3,4-b]pyridine-3-carboxamide,
N-(8-méthoxy-4-méthyl-2-oxo-1H-quinolin-6-yl)-2-[(2S)-2-méthylpyrrolidin-1-yl]-5,7-dihydrofuro[3,4-b]pyridine-3-carboxamide,
2-(7-azabicyclo[2.2.1]heptan-7-yl)-N-(8-méthoxy-4-méthyl-2-oxo-1H-quinolin-6-yl)-5,7-dihydrofuro[3,4-b]pyridine-3-carboxamide,
2-[(3R)-3-fluoropyrrolidin-1-yl]-N-(8-méthoxy-4-méthyl-2-oxo-1H-quinolin-6-yl)-5,7-dihydrofuro[3,4-b]pyridine-3-carboxamide,
2-[(3S)-3-fluoropyrrolidin-1-yl]-N-(8-méthoxy-4-méthyl-2-oxo-1H-quinolin-6-yl)-5,7-dihydrofuro[3,4-b]pyridine-3-carboxamide,
N-(8-méthoxy-4-méthyl-2-oxo-1H-quinolin-6-yl)-2-[rac-(3R,4S)-3,4-difluoropyrrolidin-1-yl]-5,7-dihydrofuro[3,4-b]pyridine-3-carboxamide,
N-(8-méthoxy-4-méthyl-2-oxo-1H-quinolin-6-yl)-2-[rac-(3S,4S)-3,4-difluoropyrrolidin-1-yl]-5,7-dihydrofuro[3,4-b]pyridine-3-carboxamide, et
2-(6,6-difluoro-3-azabicyclo[3.1.0]hexan-3-yl)-N-(8-méthoxy-4-méthyl-2-oxo-1H-quinolin-6-yl)-5,7-dihydrofuro[3,4-b]pyridine-3-carboxamide.

13. Composition pharmaceutique comprenant un composé selon l'une quelconque des revendications précédentes et au moins un excipient pharmaceutiquement acceptable.

14. Composé selon l'une quelconque des revendications 1 à 12 ou composition selon la revendication 13 pour une utilisation dans le traitement de maladies ou d'états choisis dans le groupe constitué d'inflammation systémique ou tissulaire, réponses inflammatoires à une infection ou à des produits d'organismes infectieux ou à une hypoxie, processus auto-immuns et allergiques, activation et prolifération cellulaires, cancer, métabolisme, fibrose, et dans la prévention et le traitement d'infections virales.

15. Composé selon l'une quelconque des revendications 1 à 12 ou composition selon la revendication 13 pour une utilisation dans le traitement de polyarthrite rhumatoïde, arthrose, goutte, psoriasis, arthrite psoriasique, lupus érythémateux systémique, sclérose en plaques, maladie inflammatoire de l'intestin, syndrome inflammatoire de l'intestin, maladie de Crohn, colite ulcéreuse, colite, asthme, bronchopneumopathie chronique obstructive, pneumonite, myocardite, péricardite, myosite, eczéma, dermatite, dermatite atopique, allergie, spondylarthrite ankylosante, lupus érythémateux, maladie de Hashimoto, pancréatite, maladie oculaire auto-immune, syndrome de Sjögren, névrite optique, neuromyélite optique, myasthénie grave, syndrome de Guillain-Barré, maladie de Graves, alopécie, vitiligo, maladies bulleuses de la peau, néphrite, vascularite, athérosclérose, maladie d'Alzheimer, dépression, rétinite, uvéite, sclérite, hépatite, cirrhose biliaire primitive, cholangite sclérosante, hypophysite, thyroïdite, maladie d'Addison, diabète de type I et rejet aigu d'organes transplantés ; ou
pour une utilisation dans le traitement de goutte aiguë, artérite à cellules géantes, néphrite, y compris néphrite lupique, vascularite avec atteinte d'organes telle que glomérulonéphrite, vascularite, y compris artérite à cellules géantes, polyartérite noueuse, maladie de Behçet, granulomatose de Wegener, maladie de Kawasaki, artérite de Takayasu, vascularite avec atteinte d'organes et rejet aigu d'organes transplantés ; ou
pour une utilisation dans le traitement de septicémie, syndrome septique, choc septique, endotoxémie, syndrome de réponse inflammatoire systémique (SIRS), syndrome de défaillance multiviscérale, syndrome de choc toxique, lésion pulmonaire aiguë, SDRA (syndrome de détresse respiratoire aiguë), insuffisance rénale aiguë, hépatite fulminante, brûlures, pancréatite aiguë, syndromes post-chirurgicaux, sarcoïdose, réactions de Herxheimer, encéphalite, myélite, méningite, paludisme et SIRS associés à des infections virales telles que la grippe, le zona, l'herpès simplex et le coronavirus ; ou
pour une utilisation dans le traitement de lésion d'ischémie-reperfusion, infarctus du myocarde, ischémie cérébrovasculaire (accident vasculaire cérébral), syndromes coronariens aigus, lésion de reperfusion rénale, transplantation d'organe, pontage aorto-coronarien, interventions avec circulation extracorporelle, embolie pulmonaire, rénale, hépatique, gastro-intestinale ou des membres périphériques ; ou
pour une utilisation dans le traitement d'hypercholestérolémie, athérosclérose et maladie d'Alzheimer ; ou
pour une utilisation dans le traitement de fibrose pulmonaire idiopathique, fibrose pulmonaire, fibrose intestinale, fibrose hépatique, stéatohépatite non alcoolique, cirrhose, fibrose rénale, sténose postopératoire, myélofibrose, formation de chéloïdes, fibrose cutanée, fibrose des mains, sclérose systémique, sclérodermie et fibrose cardiaque ; ou
pour une utilisation dans le traitement ou la prévention d'infections virales telles que virus de l'herpès, virus du papillome humain, virus de l'immunodéficience humaine (VIH), adénovirus et poxvirus ; ou
pour une utilisation dans le traitement de carcinomes du côlon, carcinomes de la ligne médiane, sarcomes, tumeurs mésenchymateuses, hépatiques, rénales et neurologiques ; leucémie lymphoblastique aiguë, leucémie myéloïde aiguë, leucémie/lymphome à cellules T de l'adulte, cancer de la vessie, blastome, cancer des os, cancer du sein, cancer du cerveau, lymphome de Burkitt, carcinome, sarcome myéloïde, cancer du col de l'utérus, leucémie lymphoïde chronique, leucémie myéloïde chronique, cancer colorectal, lymphome diffus à grandes cellules B, cancer de l'endomètre, cancer de l'œsophage, lymphome folliculaire, cancer gastro-intestinal, glioblastome multiforme, gliome, cancer de la vésicule biliaire, cancer gastrique, cancer de la tête et du cou, lymphome de Hodgkin, lymphome non hodgkinien, cancer de l'intestin, cancer du rein, cancer du larynx, leucémie, cancer du poumon, lymphome, cancer du foie, cancer du poumon à petites cellules, cancer du poumon non à petites cellules, mélanome, mésothéliome, myélome multiple, cancer de l'œil, tumeur du nerf optique, cancer de la bouche, cancer de l'ovaire, tumeur hypophysaire, lymphome primaire du système nerveux central, cancer de la prostate, cancer du pancréas, cancer du pharynx, carcinome rénal, cancer du rectum, sarcome, cancer de la peau, tumeur de la moelle épinière, cancer de l'intestin grêle, cancer de l'estomac, lymphome à cellules T, cancer des testicules, cancer de la thyroïde, cancer de la gorge, cancer urogénital, carcinome urothélial, cancer de l'utérus, cancer du vagin ou tumeur de Wilms ; ou
pour une utilisation dans le traitement de l'obésité, notamment l'obésité associée au traitement du cancer ou l'obésité associée au diabète et à l'hypertrophie cardiaque.
